(19) Europäisches Patentamt
European Patent Office
Office européen des brevets

(11) Veröffentlichungsnummer: **0 400 356 B1**

# (12) EUROPÄISCHE PATENTSCHRIFT

(45) Veröffentlichungstag der Patentschrift: **10.08.94**

(51) Int. Cl.^5: **C07D 471/04**, C07D 473/00, A01N 43/90, //(C07D471/04, 235:00,221:00)

(21) Anmeldenummer: **90108547.2**

(22) Anmeldetag: **07.05.90**

Die Akte enthält technische Angaben, die nach dem Eingang der Anmeldung eingereicht wurden und die nicht in dieser Patentschrift enthalten sind.

## (54) Sulfonamide.

(30) Priorität: **20.05.89 DE 3916469**

(43) Veröffentlichungstag der Anmeldung:
**05.12.90 Patentblatt 90/49**

(45) Bekanntmachung des Hinweises auf die Patenterteilung:
**10.08.94 Patentblatt 94/32**

(84) Benannte Vertragsstaaten:
**AT BE CH DE DK ES FR GB IT LI NL**

(56) Entgegenhaltungen:
**EP-A- 0 150 974**
**EP-A- 0 244 269**
**EP-A- 0 272 628**
**EP-A- 0 329 012**

**JOURNAL OF MEDICINAL CHEMISTRY, vol. 9, 1966, pp. 373-378 ; A.G. BEAMAN et al. :"Purine sulfonamides"**

(73) Patentinhaber: **BASF Aktiengesellschaft**
**Carl-Bosch-Strasse 38**
**D-67063 Ludwigshafen (DE)**

(72) Erfinder: **Fischer, Klaus, Dr.**
**Gabelsbergerstrasse 7**
**D-6720 Speyer (DE)**
Erfinder: **Mayer, Horst, Dr.**
**Faselwiese 19**
**D-6700 Ludwigshafen (DE)**
Erfinder: **Ditrich, Klaus, Dr.**
**Paray-le-Monial-Strasse 12**
**D-6702 Bad Duerkheim (DE)**
Erfinder: **Hamprecht, Gerhard, Dr.**
**Rote-Turm-Strasse 28**
**D-6940 Weinheim (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**

EP 0 400 356 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

SYNTHESIS, Nov. 1982, Seiten 984-986 ; F.L.MERCHAN et al. :"Synthesis of 2-sulfonylaminobenzimidazoles and 4,5-dicyano-2-sulfonylaminoimidazoles from N-dichloromethylenesulfonamides"

JOURNAL OF ORGANIC CHEMISTRY, Band 42, No. 18, 1977, pp. 3065-3070 ; M.M. WEGNER et al. :"Perhydrogenation of 2,8-Diaminopurine"

## Beschreibung

Die vorliegende Erfindung betrifft Sulfonamide der allgemeinen Formel I

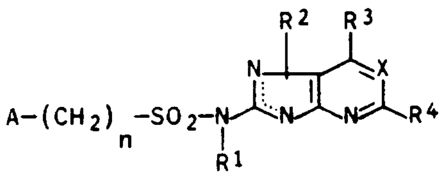

I

in der die Substituenten und Indices folgende Bedeutung haben:

$R^1$ Wasserstoff oder eine $C_1$-$C_4$-Alkylgruppe,

$R^2$ Wasserstoff,
eine $C_1$-$C_6$-Alkylgruppe, welche durch ein bis fünf Halogenatome und/oder einen der folgenden Reste substituiert sein kann; $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy oder Phenylthio;
eine $C_3$-$C_4$-Alkenylgruppe; eine $C_3$-$C_4$-Alkinylgruppe;
ein gesättigter oder einfach ungesättigter 5- bis 7-gliedriger Heterocyclus, enthaltend ein bis zwei Stickstoff-, Sauerstoff- und/oder Schwefelatome, welcher ein bis drei der folgenden Substituenten tragen kann: Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio und/oder Phenyl, Phenoxy und/oder Phenylthio;

$R^3$, $R^4$ Halogen;
$C_1$-$C_4$-Alkoxy, $C_1$-$C_4$-Alkylthio, $C_2$-$C_6$-Alkenyl, $C_2$-$C_6$-Alkenyloxy, $C_2$-$C_6$-Alkenylthio, $C_2$-$C_6$-Alkinyl, $C_2$-$C_6$-Alkinyloxy und/oder $C_2$-$C_6$-Alkinylthio, wobei die genannten Reste durch ein bis fünf Halogenatome und/oder durch eine der folgenden Gruppen substituiert sein können: $C_1$-$C_4$-Alkoxy, $C_1$-$C_4$-Halogenalkoxy, $C_2$-$C_4$-Alkylthio, $C_1$-$C_4$-Halogenalkylthio, Phenyl, Phenoxy oder Phenylthio;
$C_3$-$C_6$-Cycloalkyl, $C_3$-$C_6$-Cycloalkylthio, $C_5$-$C_6$-Cycloalkenyl, $C_3$-$C_6$-Cycloalkoxy, $C_5$-$C_6$-Cycloalkenyloxy, $C_5$-$C_6$-Cycloalkenylthio, Phenyl, Phenoxy, Phenylthio, Benzyl, Benzyloxy oder Benzylthio, wobei diese cyclischen Gruppen durch ein bis fünf Halogenatome und/oder ein bis drei der folgenden Reste substituiert sein können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy, Phenylthio, Benzyl, Benzyloxy und/oder Benzylthio; die unter $R^2$ genannten Gruppen oder $NR^7R^8$, worin
$R^7R^8$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, Phenyl und/oder Benzyl bedeuten, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy und/oder $C_1$-$C_4$-Halogenalkoxy substituiert sein können oder gemeinsam eine $C_4$-$C_6$-Alkylenbrücke, welche durch ein Sauerstoff-, Schwefel- oder Stickstoffatom unterbrochen sein kann, wobei diese Brücken ihrerseits ein bis drei $C_1$-$C_4$-Alkylgruppen tragen können;

X ein Stickstoffatom oder eine Gruppe $=CR^5$-, worin $R^5$ einen der Reste $R^3$ bedeutet,

n 0 oder 1 und

A einen ein- oder zweikernigen aromatischen Rest, der ein bis zwei Stickstoff-, Sauerstoff- und/oder Schwefelatome enthalten kann, wobei dieser Rest ein bis fünf Halogenatome und/oder ein bis drei der folgenden Gruppen tragen kann: Cyano, Nitro, Thiocyanato, $COR^6$, worin $R^6$ Hydroxy, Amino oder einen der Reste $R^3$ bedeutet, $SO_mR^6$, worin m den Wert 1 oder 2 hat und ein bis drei der unter $R^3$ genannten Reste tragen kann,

mit der Maßgabe, daß A keinen Tosylrest darstellt, wenn n für O steht und X Stickstoff, $R^1$ Wasserstoff, $R^4$ Amino ($NH_2$) und $R^3$ Wasserstoff oder Methyl bedeuten,
sowie deren landwirtschaftlich brauchbare Salze.

Weiterhin betrifft die Erfindung ein Verfahren zur Herstellung dieser Verbindungen sowie ihre Verwendung zur Bekämpfung unerwünschten Pflanzenwuchses sowie zur Beeinflussung des Pflanzenwachstums.

Von Rapoport et al. werden in Journal of Organic Chemistry, 42, 3065 (1977) Tosyl-substituierte 2,8-Diaminopurine als Zwischenprodukte zur Herstellung entsprechender 2,8-Diaminopurine offenbart.

In EP-A-150 974, EP-A-244 269 und EP-A-272 628 werden Sulfonamide beschrieben, die eine herbizide Wirkung haben. Sie genügen jedoch nicht allen Anforderungen, z.B. nach Selektivität und spezifischer Wirkung.

Der Erfindung lag daher die Aufgabe zugrunde, Substanzen mit befriedigenden Eigenschaften zu finden und zu synthetisieren.

Entsprechend dieser Aufgabe wurden die eingangs definierten Sulfonamide I gefunden.

Zur Erfindung gehören auch Verfahren zur Herstellung dieser Verbindungen I, Herbizide und Mittel zur Beeinflussung des Pflanzenwachstums, die die neuen Verbindungen als Wirkstoffe enthalten, sowie ein Verfahren zur Beeinflussung und Bekämpfung von Pflanzenwuchs mit diesen Verbindungen, einschließlich der per Disclaimer ausgenommenen Produkte. Die erfindungsgemäßen Verbindungen I sind auf vielfältige Weise analog zu bekannten Umsetzungsmethoden erhältlich. Exemplarisch seien sieben Verfahren (A bis G) im folgenden erläutert.

Verfahren A

Man erhält die Verbindungen 1 in an sich bekannter Weise (J. Med. Chem. 9, 373 (1966)) durch Umsetzung eines entsprechenden Sulfonamids II in einem inerten organischen Lösungsmittel in Gegenwart einer Base mit einem Heteroarylhalogenid III gemäß dem nachfolgenden Schema.

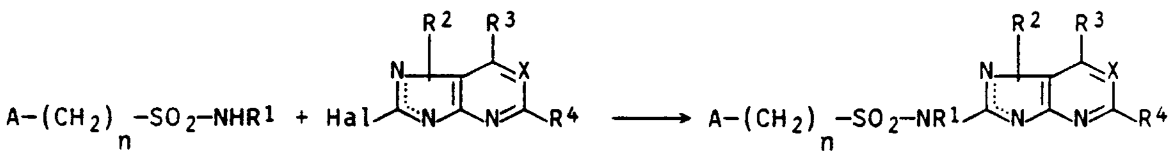

II III I

Hal in Formel III bedeutet dabei ein Halogenatom wie Fluor, Chlor, Brom und Iod, insbesondere eignen sich Verbindungen III, in denen Hal Chlor oder Brom bedeutet.

Zweckmäßigerweise verwendet man für diese Umsetzungen Lösungsmittel wie Halogenwasserstoffe, z. B. Tetrachlorethan, Methylenchlorid, Chloroform, Dichlorethan, Chlorbenzol und 1.2-Dichlorbenzol; Ether z. B. Diethylether, Methyl-tert.-Butylether, Dimethoxiethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1.3-Dimethyltetrahydro-2(1H)-pyrimidinon und 1.3-Dimethyl imidazolidin-2-on; Aromaten, z. B. Benzol, Toluol, Xylol, Pyridin und Chinolin; Ketone, z. B. Aceton, Methylethylketon; Alkohole, z. B. Methanol, Ethanol, iso-Propanol und t-Butanol oder entsprechende Gemische.

Die Umsetzung kann bei Temperaturen von 25°C bis zur Rückflußtemperatur des jeweiligen Lösungsmittels bzw. -gemisches durchgeführt werden.

Als katalytisch wirksame Basen dienen dabei aromatische Stickstoffbasen, wie Pyridin, 4-Dimethylaminopyridin und Chinolin; tertiäre aliphatische Amine, wie Triethylamin, N-Ethyl-diisopropylamin und N-Methylmorpholin; bi- und tricyclische Amine, wie B. Diazabicycloundecan (DBU) oder Diazabicyclooctan (DABCO) sowie Hydroxide, Hydride, Alkoxide, Carbonate und Hydrogencarbonate von Alkalimetall- und Erdalkalimetallkationen, insbesondere NaOH, KOH, NaH, KH, $CaH_2$, LiH, NaOMe, NaOEt, KOtBu, $Na_2CO_3$, $K_2CO_3$, $NaHCO_3$, $KHCO_3$. Mitunter ist es auch nützlich, Kombinationen der oben angeführten Basen zu verwenden.

Die molaren Verhältnisse, in denen die benötigten Ausgangsverbindungen miteinander umgesetzt werden, betragen im allgemeinen 1 : 1 bis 1 : 3 für das Verhältnis von Sulfonamid II zu Heteroarylhalogenid III und 1:1 bis 1:3 für das Verhältnis von Sulfonamid II zu katalytisch wirksamer Base.

Die Konzentration der Edukte im Lösungsmittel beträgt im allgemeinen 0,1 bis 5 mol/l, bevorzugt 0,2 bis 2 mol/l.

Besonders bevorzugt arbeitet man in aprotisch dipolaren Solventien wie Acetonitril, Dimethylformamid, Dimethylsulfoxid, N-Methylpyrrolidon, 1.3-Dimethyltetrahydro-2(1H)-pyrimidinon, 1.3-Dimethylimidazolidin-2-onoder Ethern wie 1.2-Dimethoxyethan, Diethylenglykoldimethylether, Tetrahydrofuran, oder Dioxan bei Temperaturen zwischen 80°C und 150°C unter Verwendung von Natriumhydrid, Natriummethylat, Natriumethylat oder Kalium-tertiär-butylat als Basen. Als besonders vorteilhaft hat es sich dabei erwiesen, das Sulfonamid II durch Reaktion mit der Base zunächst in sein Salz zu überführen und dieses anschließend mit dem Heteroarylhalogenid III zur Reaktion zu bringen. Eine vorherige Isolierung des Sulfonamidsalzes ist hierbei möglich, jedoch nicht notwendig, vielmehr ist sogar äußerst zweckmäßig, das Sulfonamidsalz

lediglich in situ zu erzeugen und im gleichen Reaktionsmedium mit dem Heteroarylhalogenid III weiterumzusetzen.

Die benötigten Sulfonamide der allgemeinen Formel II sind in vielen Fällen handelsüblich. Neue Sulfonamide der Formel II lassen sich nach allgemein bekannten Methoden herstellen (Pawlenko, Houben-Weyl, Methoden der organischen Chemie, Band E 11, Organische Schwefelverbindungen, J. Thieme Verlag, Stuttgart (1985), S. 1098 ff).

Die benötigten Heteroarylhalogenide der allgemeinen Formel III sind ebenfalls analog zu bekannten Methoden zugänglich (Shaw; Comprehensive Heterocyclic Chemistry (The Structure, Reaction, Synthesis and Uses of Heterocyclic Compounds), Vol. 5, 1st ed. (1984), Kap. 4.09, S. 499 ff; Montgomery, J. A. Secrist III in Comprehensive Heterocyclic Chemistry, Vol. 5, 1st ed. (1984) Kap. 4.10, S. 615 ff und S. 635 ff; Lister, The Chemistry of Heterocyclic Compounds, Fused Pyrimidines, Part II, Purines (1971) und Robins, Heterocyclic Compounds, Vol. 8, S. 162 ff (1967)).

Verfahren B

Man erhält die Verbindungen 1 mit $R^2 \neq H$ auch, indem man ein entsprechendes Sulfonylhalogenid IV in an sich bekannter Weise in einem inerten organischen Lösungsmittel in Gegenwart einer Base mit einem Heteroarylamin V, worin $R^2$ nicht Wasserstoff bedeutet, gemäß dem nachfolgenden Schema umsetzt:

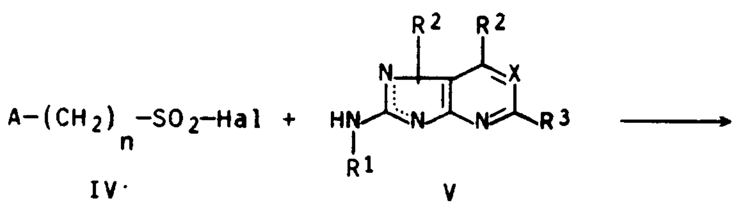

IV· V

I ($R^2 \neq H$)

Hal in Formel IV bedeutet dabei ein Halogenatom wie Fluor, Chlor und Brom, insbesondere Chlor.

Für diese Umsetzung können prinzipiell die bei Verfahren A genannten Lösungsmittel und Basen sowie deren Kombinationen bei Temperaturen von 25 °C bis zur Rückflußtemperatur des jeweiligen Lösungsmittels bzw. -gemisches verwendet werden. Bei basischen Lösungsmitteln wie Pyridin erübrigt sich die Zugabe einer Hilfsbase.

Die molaren Verhältnisse der Ausgangsverbindungen betragen im allgemeinen 1:1 bis 1:3 für das Verhältnis von Heteroarylamin V zu katalytisch wirksamer Base. Die Sulfonylhalogenide IV sowie die reaktionsbeschleunigend wirkenden Basen können aber auch jeweils in weniger als der stöchiometrischen Menge eingesetzt werden.

Die Konzentration der Edukte im Lösungsmittel beträgt im allgemeinen 0,1 bis 5 mol/l, bevorzugt 0,2 bis 2 mol/l.

Die benötigten Sulfonylhalogenide der allgemeinen Formel IV sind handelsüblich oder lassen sich nach allgemein bekannten Methoden herstellen (Clarke et al., Org. Synth. Coll. Vol. I 2. Aufl., 1941, S. 85 ff; (Hoffmann, Org. Synth., Vol. 60, Seite 121 ff). Die für die Umsetzung benötigten Heteroarylamine der allgemeinen Formel V können nach allgemein bekannten Methoden hergestellt werden. Eine Beschreibung dieser Methoden findet sich in der im Zusammenhang mit den Heteroarylhalogeniden der allgemeinen Formel II unter Verfahren A zitierten Literatur.

Verfahren C

Verbindungen I, in denen $R^1$ Wasserstoff bedeutet, erhält man beispielsweise, indem man ein Sulfonylisocyanidderivat der allgemeinen Formel VI in an sich bekannter Weise (Synthesis, 11, 984 (1982); Chem. Ber., 99, 2885 (1966)) mit einem Diaminoheteroarylderivat der allgemeinen Formel VIIa bzw. VIIb, gegebenenfalls in Gegenwart einer Base, gemäß dem folgenden Schema umsetzt.

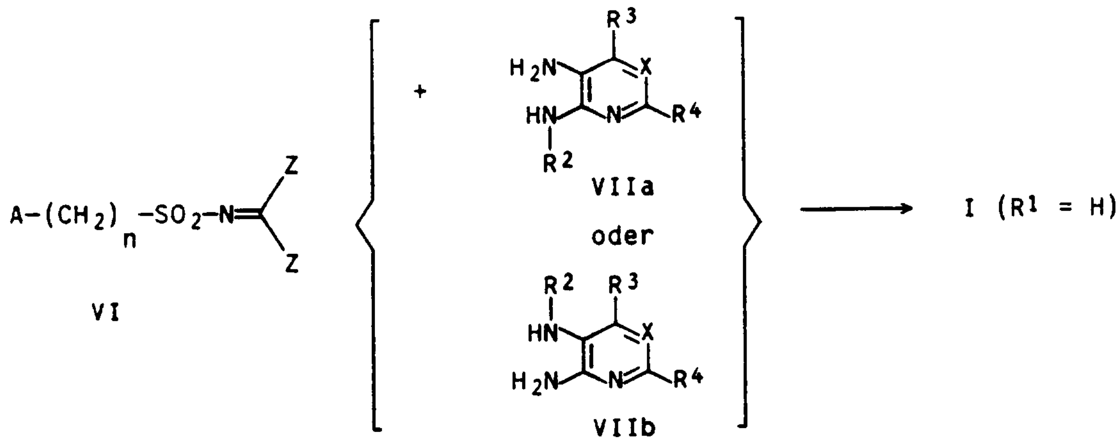

Z in Formel VI bedeutet ein Halogenatom wie Chlor und Brom oder eine $C_1$-$C_4$ Alkylthio- oder Benzylthiogruppe, wobei Chlor und Methylthio bevorzugt sind.

Diese Umsetzungen werden in einem der unter Verfahren A genannten Chlorkohlenwasserstoffe, Ether, aromatischen oder aprotisch dipolaren Lösungsmitteln bzw. entsprechenden Gemischen bei einer Temperatur von 25 °C bis zur Siedetemperatur des Lösungsmittels oder Gemischs durchgeführt.

Besonders bevorzugt sind bei diesem Verfahren Umsetzungen in Halogenkohlenwasserstoffen wie Chlorbenzol, 1.2-Dichlorbenzol; Aromaten wie Toluol, Xylol; Ethern wie 1.2-Dimethoxyethan, Diethylenglykoldimethylester, Dioxan oder aprotisch dipolaren Lösungsmitteln wie Acetonitril, Dimethylformamid, Dimethylacetamid, Dimethylsulfoxid, N-Methylpyrolidinon, 1.3-Dimethyl-tetrahydro-2(1H)-pyrinidinon und 1.3-Dimethylimidazolidin-2-on bei Temperaturen von 80 °C bis 150 °C. Die molaren Verhältnisse, in denen die benötigten Ausgangsverbindungen miteinander umgesetzt werden, betragen im allgemeinen 1:1 bis 1:5 für das Verhältnis von Derivaten der allgemeinen Formel VI zu Diaminoheteroarylverbindungen der allgemeinen Formel VIIa bzw. VIIb, wobei die Konzentration der Edukte im Lösungsmittel 0.1 bis 5 mol/l, bevorzugt 0.2 bis 2 mol/l beträgt.

Besonders bei Umsetzungen von Sulfonylisocyaniddihalogeniden der allgemeinen Formel VI, in denen Z Halogen bedeutet, ist es von Vorteil, zur Bindung des bei der Reaktion entstehenden Halogenwasserstoffes in Gegenwart von mindestens 2 mol-Äquivalenten einer Base zur arbeiten.

Bevorzugte Basen sind aromatische Stickstoffbasen wie Pyridin, 4-Dimethylaminopyridin oder Chinolin, tertiäre aliphatische Amine wie z. B. Triethylamin, N-Ethyl-diisopropylamin oder N-Methylmorpholin, bi- und tricyclische Amine wie Diazabicycloundecen (DBU) oder Diazabicyclooctan (DABCO) oder - besonders vorteilhaft - Diaminoheteroarylderivate der allgemeinen Formel VIIa bzw. VIIb.

Die benötigten Sulfonylisocyanidderivate der allgemeinen Formel VI können nach bekannten Methoden hergestellt werden (Kühler, Houben-Weyl, Methoden der Organische Chemie, Band E4, Kohlensäurederivate, G. Thieme Verlag, Stuttgart (1983) S. 540 ff, 584 ff). Die für die Umsetzung erforderlichen Diaminoheteroarylderivate der allgemeinen Formel VIIa bzw. VIIb können gemäß bekannten Methoden hergestellt werden (J. Brown, Comprehensive Heterocyclic Chemistry, Vol. 3, 1st ed. (1984), Kap. 2.13, S. 57 ff (Jones, Comprehensive Heterocyclic Chemistry Vol. 2, 1st ed. (1984), Kap. 2.08, S 395 ff).

Verfahren D

In einer Abwandlung des unter C beschriebenen Verfahren erhält man die Verbindungen I, in denen $R^1$ Wasserstoff bedeutet, auch durch Umsetzung eines geeigneten Sulfonyl-C-halogen-thioformimidsäurealkyl- oder benzylesters VIa mit einem Diaminoheteroarylderivat VIIa oder VIIb und anschließende Cyclisierung des Isothioharnstoffs VIIIa bzw. VIIIb zum gewünschten Sulfonamid I (J. Org. Chem., 42, 3065, (1977)).

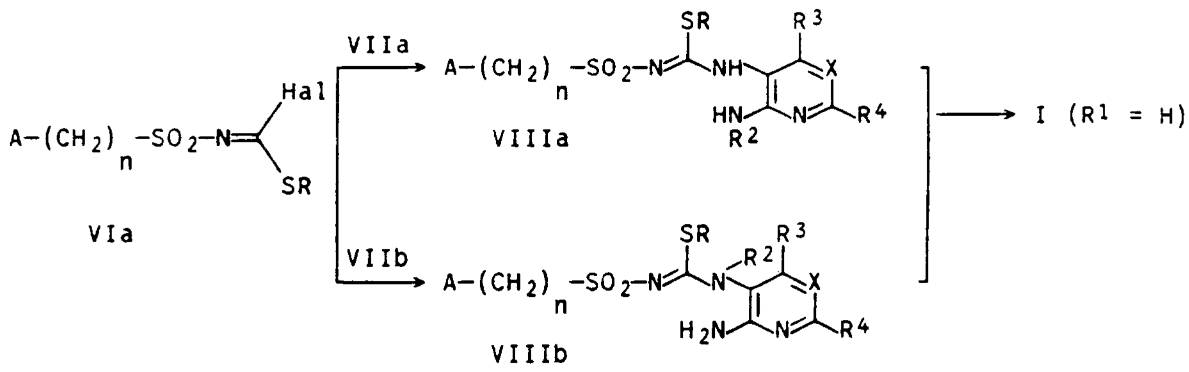

In Formel VIa hat Hal die Bedeutung Chlor oder Brom und R die Bedeutung $C_1$-$C_4$-Alkyl oder Benzyl, wobei Chlor und Methyl bevorzugt sind.

Für die dem Schema entsprechenden Umsetzungen zu den Isothioharnstoffderivaten der allgemeinen Formel VIIIa bzw. VIIIb können prinzipiell die bei Verfahren A genannten Lösungsmittel in Kombination mit den unter Verfahren C aufgeführten Basen bevorzugt bei Temperaturen von 0 °C bis 50 °C eingesetzt werden.

Die anschließende Cyclisierung der Isothioharnstoffe VIIIa bzw. VIIIb zu den Sulfonamiden I erfolgt im allgemeinen und im besonderen unter Verwendung der im Verfahren C genannten Verdünnungsmittel und Basen analog Rapoport et al. (Journal of Organic Chemistry, 42, 3065, (1977)) in Gegenwart von mindestens einem mol-Äquivalent eines Silbersalzes, wobei Silbernitrat bevorzugt wird. Die für die Umsetzung benötigten Vorprodukte der allgemeinen Formel VIa erhält man nach allgemein bekannten Methoden (z. B. Kühle, Houben-Weyl, Methoden der organischen Chemie, Band E4, Kohlensäurederivate, G. Thieme Verlag, Stuttgart (1983) auf S. 551 und dort zitierten Literatur).

Verfahren E

Verbindungen I, in denen weder $R^1$ noch $R^2$ Wasserstoff bedeuten, erhält man beispielsweise auch, indem man ein entsprechendes Sulfonamid IX in an sich bekannter Weise in einem organischen Lösungsmittel in Gegenwart einer Base mit einem Alkylierungsreagens X, worin Nu eine nucleophile Ausgangsgruppe und $R^1$ nicht Wasserstoff bedeutet, gemäß dem nachfolgenden Schema umsetzt.

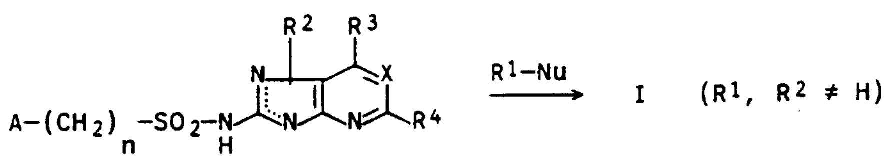


IX X

Zweckmäßigerweise verwendet man für diese Umsetzung inerte Lösungsmittel und entsprechende Gemische, wie bereits bei Verfahren A aufgeführt.

Diese Umsetzungen können bei Temperaturen von 25 °C bis zur Rückflußtemperatur des Lösungsmittels bzw. -gemisches durchgeführt werden.

Als Gasen dienen die bereits bei Verfahren A aufgeführten. Sofern die Base gleichzeitig als Lösungsmittel fungiert, kann sich die zusätzliche Verwendung eines Lösungsmittels erübrigen.

Das molare Verhältnis der Ausgangsverbindungen beträgt im allgemeinen 1:1 bis 1:3 (Verbindung IX zu Elektrophil X) bzw. 1:1 bis 1:5 (Verbindung IX zu Base). Die Konzentration der Edukte im Lösungsmittel beträgt im allgmeinen 0,1 bis 5 mol/l, bevorzugt 0,2 bis 2 mol/l.

Als Alkylierungsreagenzien X dienen insbesondere $C_1$-$C_4$-Alkylchloride, -bromide, -iodide, -benzolsulfonate, -p-Brombenzolsulfonate, -p-Methylbenzolsulfonate, $C_1$-$C_4$-Dialkylsulfate und $C_1$-$C_4$-Trialkyloxonomiumtetrafluoroborate. Die Verbindungen X sind im allgemeinen kommerziell erhältlich oder können nach allgemein bekannten Methoden hergestellt werden.

Die Sulfonamide der allgemeinen Formel IX mit $R^2 \neq H$ sind nach den bereits geschilderten Verfahren A - D zugänglich.

Verfahren F

Verbindungen der allgemeinen Formel I, in denen $R^3$ und/oder $R^4$ eine Alkoholat-, Thiolat-, Phenolat-, oder eine substituierte Aminogruppe und X Stickstoff bedeuten, erhält man besonders vorteilhaft durch Umsetzung eines Sulfonamidderivats XIa bzw. XIb, worin Hal Halogen wie Fluor, Chlor, Brom, insbesondere Chlor bedeutet und $R^3$ in der Formel XIb nicht Halogen oder Phenoxy repräsentiert, mit einem entsprechenden Alkohol, Thiol, Phenol oder Amin XIIa bzw. XIIb in an sich bekannter Weise in einem organischen Lösungsmittel in Gegenwart einer Base gemäß dem nachfolgenden Schema.

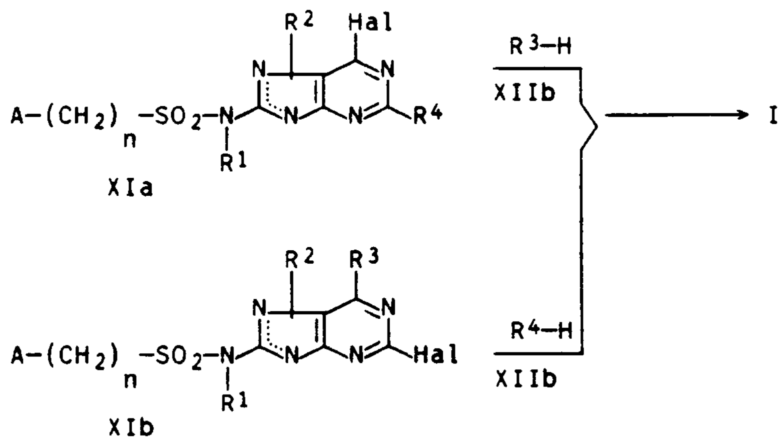


XIa

XIb

Für derartige Umsetzungen können prinzipiell die im Verfahren A genannten Lösungsmittel und Basen sowie deren Kombinationen verwendet werden. Bevorzugt wird das Reagens der Formel XIIa bzw. XIIb im Verhältnis 1:1 bis 5:1, insbesondere 3:1 bis 5:1 bezogen auf das Sulfonamid XIa bzw. XIb in einem Lösungsmittel wie Alkohol z. B. Methanol, Ethanol, Propanol, iso-Propanol, n-Butanol, iso-Butanol, tert.-Butanol, Ether z. B. Diethylether, Methyl-tert.-Butylether, 1.2-Dimethoxyethan, Tetrahydrofuran, Dioxan oder aprotisch dipolaren Solvention z. B. Acetonitril, Dimethylformamid oder Dimethylsulfoxid bei Reaktionstemperaturen zwischen Raumtemperatur und 130 °C umgesetzt.

Verfahren G

In Abwandlung des unter F beschriebenen Verfahrens erhält man Verbindungen der allgemeinen Formel I, in denen $R^3$ und $R^4$ gleich sind und Alkoholat-, Thiolat-, Phenolat-, Alkylamino-, Dialkylamino- oder Alkylenaminogruppen und X Stickstoff bedeuten durch Umsetzung eines entsprechenden Sulfonamidderivats XIc mit dem Derivat XIIa bzw. XII in einem organischen Lösungsmittel in Gegenwart einer Base gemäß dem folgenden Schema.

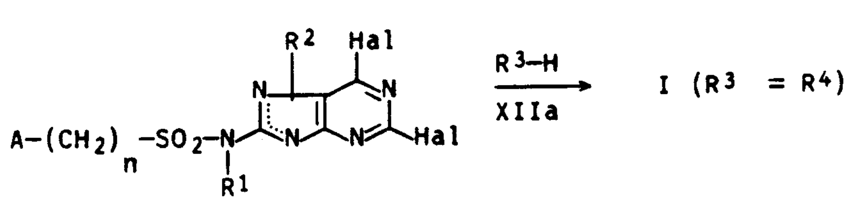


XIc

In der Formel XIc hat Hal die Bedeutung Fluor, Chlor oder Brom, insbesondere Chlor.

Die Umsetzung erfolgt im allgemeinen und im besonderen analog den in Verfahren F geschilderten Bedingungen.

Die Nucleophile der allgemeinen Formel XIIa bzw. XIIb sind im allgemeinen kommerziell erhältlich bzw. können in einfacher und allgemein bekannter Art und Weise aus bekannten Edukten hergestellt werden. Die Sulfonamide XIa, XIb und XIc sind nach den vorstehend geschilderten Verfahren A - E zugänglich.

Im Hinblick auf die bestimmungsgemäße Verwendung der Verbindungen I kommen als Substituenten bevorzugt folgende Reste in Betracht:

$R^1$ Wasserstoff oder $C_1$-$C_4$-Alkyl wie Methyl, Ethyl, n-Propyl, iso-Propyl, n-Butyl, sek.-Butyl, iso-Butyl und tert.-Butyl, insbesondere Methyl, Ethyl, Propyl und iso-Propyl

$R^2$ Wasserstoff, Allyl; Propargyl; Alkyl wie unter $R^1$ genannt, sowie Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Diemthyl, 1,2-Dimethylbutyl, 1,3-Dimethylbutyl, 2,2-Dimethylbutyl, 2,3,-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl und iso-Propyl, welches durch ein bis fünf Halogenatome, insbesondere Fluor- und/oder Chloratome oder einen der folgenden Reste substituiert sein kann: Alkoxy wie Methoxy, Ethoxy, n-Propoxy, 2-Methylethoxy, n-Butoxy, 1-Methypropoxy, 2-Methylpropoxy und 1,1-Dimethylethoxy, insbesondere Methoxy, Ethoxy, 1-Methylethoxy und 1,1-Dimethylethoxy;

Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2, 2, 2-Trifluoethoxy, 2-Chlor-1,1,2-trifluorethoxy und Pentafluorethoxy, insbesondere Trifluormethoxy und Pentafluorethoxy;

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;

Halogenalkylthio wie Difluormethylthio, Trifluormethylthio, Chlordifluormethylthlo, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Difluormethylthio und Pentafluorethylthio;

Phenyl oder Phenylthio;

ein 5- bis 7-gliedriger gesättigter oder einfach ungesättigter Heterocyclus wie Tetrahydrofuranyl, Tetrahydrothiophenyl, Dioxolanyl, Dithiolanyl, Oxathiolanyl, Pyrazolidinyl, Dihydropyrazolyl, Imidazolidinyl, Dihydroimidazolyl, Isoxazolidinyl, Dihydroisoxazolyl, Oxazolidinyl, Dihydrooxazolyl, Isothioazolidinyl, Dihydroisothiazolyl, Thiazolidinyl, Dihydrothiazolyl Tetrahydropyran-3-yl, Dihydropyran-3-yl, Tetrahydropyran-4-yl, Tetrahydropyran-2-yl, Dihydropyran-4-yl, Tetrahydrothiopyran-3-yl, Dihydrothiopyran-3yl, Tetrahydrothiopyran-4-yl, Dihydrothiopyran-4-yl und Dioxepan-5-yl, Piperidinyl, Tetrahydron-1,3-oxazinyl, Tetrahydro-1,4-oxazinyl, Tetrahydro-1,3-thiazinyl, Tetrahydro-1,4-thiazinyl, Perhydro-1,3-oxazepinyl, Perhydro-1,4-oxazepinyl, Perhydro-1,3-thiazepinyl, Perhydro-1,4-thiazepinyl, und Perhydroazepinyl, wobei dieser Ring ein bis drei der folgenden Substitutionen tragen kann:

Halogen wie Fluor, Chlor, Brom und Jod, insbesondere Fluor und Chlor;

Alkyl wie unter $R^1$ genannt, insbesondere Methyl, Ethyl, Propyl und iso-Propyl;

Alkoxy wie vorstehend genannt, insbesondere Methoxy, Ethoxy, 1-Methylethoxy und 1,1-Dimethylethoxy;

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl;

Halogenalkoxy wie vorstehend genannt, insbesondere Trifluormethoxy, Trifluorethoxy und Chlorethoxy;

Alkylthio wie vorstehend genannt, insbesondere Methylthio und Ethylthio;

Halogenalkylthio wie vorstehend genannt, insbesondere Difluormethylthio und Pentafluorethylthio und/oder Phenyl, Phenoxy oder Phenylthio;

$R^3$, $R^4$ unabhängig voneinander Halogen wie unter $R^2$ genannt, insbesondere Fluor und Chlor;

Alkoxy wie unter $R^2$ genannt, insbesondere Methoxy, Ethoxy, 1-Methylethoxy und 1,1-Dimethylethoxy;

Alkythio wie unter $R^2$ genannt, insbesondere Methylthio und Ethylthio;

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-2-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,1-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere Allyl bzw. im allgemeinen und im besonderen Alkenyloxy und/oder Alkenylthio;

Alkinyl wie Ethinyl, 1-Propinyl, Propargyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere Propargyl bzw. im allgemeinen und im besonderen entsprechendes Alkinyloxy und/oder Alkinylthio;

wobei diese Reste durch ein bis fünf Halogenatome und/oder durch eine der folgenden Gruppen substituiert sein können: Alkoxy, Halogenalkoxy, Alkylthio und Halogenalkylthio wie unter $R^2$ genannt, insbesondere Fluor, Chlor, Methoxy, Ethoxy, 1-Methylethoxy, 1,1-Dimethylethoxy, Trifluormethoxy, Pentafluorethoxy, Methylthio, Ethylthio, Difluormethylthio und Pentafluorethylthio sowie Phenyl, Phenoxy oder Phenylthio;

Cycloalkyl wie Cyclopropyl, Cyclobutyl, Cyclopentyl und Cyclohexyl, insbesondere Cyclopropyl, Cyclopentyl und Cyclohexyl bzw. entsprechendes Cycloalkoxy und/oder Cycloalkylthio, insbesondere Cyclopentyloxy, Cyclohexyloxy, Cyclopentylthio, Cyclohexylthio;

Cycloalkenyl wie Cyclopentenyl, Cyclohexenyl, Cyclohexadienyl, insbesondere Cyclopentenyl und Cyclohexenyl bzw. entsprechendes Cycloalkenyloxy und/oder Cycloalkenylthio, insbesondere Cyclopentenyloxy, Cyclohexenyloxy, Cyclopentenylthio und Cyclohexenylthio;

Phenyl, Phenoxy, Phenylthio, Benzyl, Benzyloxy, und/oder Benzylthio, wobei diese cyclischen Reste ein bis fünf Halogenatome wie unter $R^2$ genannt insbesondere Fluor und Chlor und/oder ein bis drei der folgenden Gruppen tragen können: Alkyl wie unter $R^1$ genannt, insbesondere Methyl, Ethyl, Propyl, iso-Propyl;

Halogenalkyl wie unter $R^2$ genannt, insbesondere Trifluormethyl, Alkoxy wie unter $R^2$ genannt, insbesondere Methoxy, Ethoxy, 1-Methylethoxy und 1.1-Dimethylethoxy;

Halogenalkoxy wie unter $R^2$ genannt, insbesondere Trifluormethoxy und Trifluorethoxy und Chlorethoxy; Alkylthio wie unter $R^2$ genannt, insbesondere Methylthio und Ethylthio; Halogenalkylthio wie unter $R^2$ genannt, insbesondere Difluormethylthio und Pentafluorethylthio; Phenyl, Phenoxy, Phenylthio, Benzyl, Benzyloxy- und/oder Benzylthio;

im allgemeinen und im besonderen die unter $R^2$ genannten Gruppen oder

ein Rest $NR^7R^8$, worin

$R^7$,$R^8$ Wasserstoff; Alkyl wie bei $R^2$ genannt, insbesondere wie im allgemeinen bei $R^1$ genannt; Alkenyl, Alkinyl oder Cycloalkyl mit jeweils 3 bis 6 Kohlenstoffatomen wie im allgemeinen und im besonderen bei $R^3$ genannt;

Cycloalkenyl wie im allgemeinen und im besonderen bei $R^3$ genannt;

Phenyl und oder Benzyl, wobei die aromatischen Ringe ihrerseits ein- bis fünffach durch die bei $R^3$ genannten Halogenatome und/oder ein bis dreifach durch Alkyl, Halogenalkyl, Alkoxy und/oder Halogenalkoxy mit jeweils 1 bis 4 Kohlenstoffatomen wie im allgemeinen und im besonderen bei $R^2$ genannt substituiert sein können oder

$R^7$, $R^8$ gemeinsam $C_4$-$C_6$-Alkylen, welches durch ein Heteroatom wie Sauerstoff, Schwefel oder Stickstoff unterbrochen sein kann wie $-(CH_2)_4-$, $-(CH_2)_5-$, $-(CH_2)_6-$, $-(CH_2)_2-O-(CH_2)_2-$, $-(CH_2)_2-S-(CH_2)_2-$ und $-(CH_2)_2-NH-(CH_2)_2-$, wobei diese Brücken ihrerseits ein bis drei der im allgemeinen und im besonderen bei $R^1$ genannten Alkylgruppen tragen können.

X ein Stickstoffatom oder eine Gruppe $=CR^5-$, worin $R^5$ im allgemeinen und im besonderen eine der bei $R^3$ genannten Gruppen bedeutet,

n den Wert 0 oder 1 und

A eine Aryl- oder Heteroarylgruppe wie Phenyl, Naphthyl, sechsgliedrige Heterocyclen mit einem oder mehreren Heteroatomen wie Pyridyl, Pyridazinyl, Pyrimidinyl, Pyrazinyl, 1,3,5-Triazinyl, 1,2,4-Triazinyl, 1,2,3-Triazinyl, Tetrazinyl, fünfgliedrige Heterocyclen mit einem odere mehreren Heteroatomen wie Pyrryl, Furyl, Thienyl, Pyrazolyl, Imidazolyl, Oxazolyl, Isoxazolyl, Thiazolyl, Isothiazolyl, 1,2,3-Triazolyl, 1,2,4-Triazolyl, Tetrazolyl, Thiadiazolyl, annellierte und benzokondensierte Heteroatomen, wie Indolyl, Isoindolyl, Thionaphthyl, Chinolyl, Isochinolyl, Cinnalolyl, Phthalazinyl, Chinozolyl, Chinoxalyl, Indazolyl, Naphthyrindinyl, Benzthiazolyl, Benzimidazolyl, Benzofuryl, Benzoxazolyl und Bentriazolyl, insbesondere Phenyl, Naphthyl, Thienyl, Pyridyl, Pyrazolyl, Chinolyl, wobei diese aromatischen Ringe ein bis fünf Halogenatome, insbesondere Fluor, Chlor und Brom und/oder ein bis drei der folgenden Reste tragen können: Cyano, Nitro, Thiocyanato, $-COR^6$ worin $R^6$ Hydroxy, Amino oder einen der Reste $R^3$ bedeutet, $-SO_mR^6$ worin m den Wert 1 oder 2 hat und/oder die unter $R^3$ genannten Reste.

Besonders bevorzugte Verbindungen sind Sulfonamide der allgemeinen Formel Ia

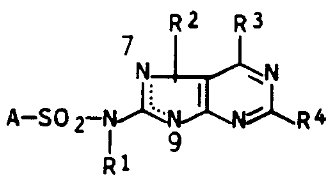

Ia

in der die Substituenten insbesondere folgende Bedeutungen haben:

- $R^1$ Wasserstoff oder Methyl;
- $R^2$ Wasserstoff oder Methyl, wobei in den Fällen, in denen $R^2$ Wasserstoff ist, infolge von Tautomerie eine Unterscheidung, an welchem der beiden Stickstoffatome (N-7 oder N-9) sich der Wasserstoff befindet, nicht möglich ist;
- $R^3$, $R^4$ Wasserstoff, Fluor, Chlor, Methyl, Ethyl, n-Propyl, iso-Propyl, Trifluormethyl, Methoxy, Ethoxy, Propoxy, 1-Methylethoxy, Methylthio, Ethylthio, Alkyl, Propargyl, Allyloxy, Allylthio, Propargyloxy, Propargylthio, Trifluormethoxy, N-Methylamino, N-Phenylamino, N,N-Dimethylamino, Pyrollidino, Piperidino, Morpholino, 3,5-Dimethylmorpholino, Phenoxy, Benzyloxy, Phenylthio, Benzylthio und/oder Phenyl;
- A Phenyl, Pyridyl, Naphthyl, Chinolinyl, Thienyl oder Pyrazolyl, wobei diese aromatischen Reste insbesondere ein bis drei der folgenden Gruppen tragen können: Nitro, Cyano, Fluor, Chlor, Ethyl, Propyl, Isopropyl, Trifluormethyl, Methoxy, Ethoxy, 1-Methylethoxy, 1,1-Dimethylethoxy, Trifluormethoxy, Trifluorethoxy, Chlorethoxy, Methoxymethyl, Ethoxymethyl, Methoxyethyl, Ethoxyethyl, Methoxyethoxy, Ethoxyethoxy, Methylthio, Ethylthio, Methyl- und Ethylsulfonyl, Methoxycarbonyl, Ethoxycarbonyl, iso-Propoxycarbonyl, Methyl- und Ethylcarbonyl, N,N-Dimethylsulfonamido und N,N-Dimethylcarboxamido sowie deren Salze.

Des weiteren sind die Verbindungen Ib bevorzugt,

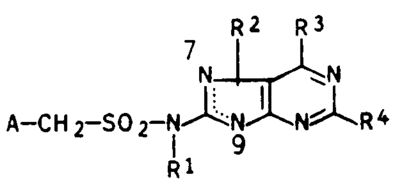

Ib

in der $R^1$, $R^2$, $R^3$ und $R^4$ die unter Formel Ia angegebene Bedeutung haben sowie deren Salze.

Bevorzugte Verbindungen sind auch Sulfonamide der allgemeinen Formel Ic

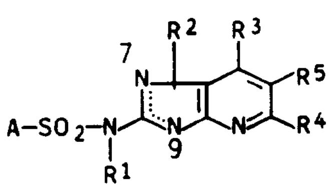

Ic

in der $R^1$, $R^2$, $R^3$, $R^4$ und A die bei Formel Ia angegebene Bedeutung haben und $R^5$ Wasserstoff, Methyl, Fluor oder Chlor bedeutet sowie deren Salze.

Des weiteren sind Sulfonamide der Formel Id bevorzugt,

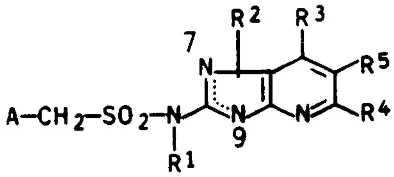

Id

in der $R^1$, $R^2$, $R^3$, $R^4$, $R^5$ und A die bei Formel Ic gegebene Bedeutung haben sowie deren Salze.

Beispiele für sehr aktive Verbindungen der Formeln Ia, Ib, Ic und Id sind in den nachstehenden Tabellen I, II, III und IV aufgeführt.

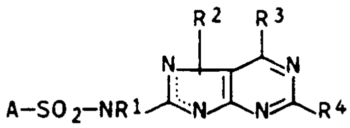

Ia

Tabelle I

| A | $R^1$ | $R^2$ | Pos. | $R^3$ | $R^4$ |
|---|---|---|---|---|---|
| Phenyl | H | H | 7/9 | $CH_3$ | $CH_3$ |
| Phenyl | H | $CH_3$ | 7 | $CH_3$ | $CH_3$ |
| Phenyl | H | H | 7/9 | Cl | $CH_3$ |
| Phenyl | H | $CH_3$ | 7 | Cl | $CH_3$ |
| Phenyl | H | H | 7/9 | $CH_3$ | Cl |
| Phenyl | H | $CH_3$ | 7 | $CH_3$ | Cl |
| Phenyl | H | H | 7/9 | Cl | Cl |
| Phenyl | H | $CH_3$ | 7 | Cl | Cl |
| Phenyl | H | H | 7/9 | $OCH_3$ | $OCH_3$ |
| Phenyl | H | $CH_3$ | 7 | $OCH_3$ | $OCH_3$ |
| Phenyl | H | H | 7/9 | $CH_3$ | $OCH_3$ |
| Phenyl | H | $CH_3$ | 7 | $CH_3$ | $OCH_3$ |
| Phenyl | H | H | 7/9 | $OCH_3$ | $CH_3$ |
| Phenyl | H | $CH_3$ | 7 | $OCH_3$ | $CH_3$ |
| Phenyl | H | H | 7/9 | Cl | $OCH_3$ |
| Phenyl | H | $CH_3$ | 7 | Cl | $OCH_3$ |
| Phenyl | H | H | 7/9 | $OCH_3$ | Cl |
| Phenyl | H | $CH_3$ | 7 | $OCH_3$ | Cl |
| 2-Cl-Phenyl | H | H | 7/9 | $CH_3$ | $CH_3$ |
| 2-Cl-Phenyl | H | $CH_3$ | 7 | $CH_3$ | $CH_3$ |
| 2-Cl-Phenyl | H | H | 7/9 | Cl | $CH_3$ |
| 2-Cl-Phenyl | H | $CH_3$ | 7 | Cl | $CH_3$ |
| 2-Cl-Phenyl | H | H | 7/9 | $CH_3$ | Cl |
| 2-Cl-Phenyl | H | $CH_3$ | 7 | $CH_3$ | Cl |
| 2-Cl-Phenyl | H | H | 7/9 | Cl | Cl |
| 2-Cl-Phenyl | H | $CH_3$ | 7 | Cl | Cl |
| 2-Cl-Phenyl | H | H | 7/9 | $OCH_3$ | $OCH_3$ |
| 2-Cl-Phenyl | H | $CH_3$ | 7 | $OCH_3$ | $OCH_3$ |
| 2-Cl-Phenyl | H | H | 7/9 | $CH_3$ | $OCH_3$ |
| 2-Cl-Phenyl | H | $CH_3$ | 7 | $CH_3$ | $OCH_3$ |
| 2-Cl-Phenyl | H | H | 7/9 | $OCH_3$ | $CH_3$ |
| 2-Cl-Phenyl | H | $CH_3$ | 7 | $OCH_3$ | $CH_3$ |
| 2-Cl-Phenyl | H | H | 7/9 | Cl | $OCH_3$ |
| 2-Cl-Phenyl | H | $CH_3$ | 7 | Cl | $OCH_3$ |
| 2-Cl-Phenyl | H | H | 7/9 | $OCH_3$ | Cl |
| 2-Cl-Phenyl | H | $CH_3$ | 7 | $OCH_3$ | Cl |
| 2-Cl-Phenyl | $CH_3$ | H | 7/9 | $CH_3$ | $CH_3$ |
| 2-Cl-Phenyl | $CH_3$ | $CH_3$ | 7 | $CH_3$ | $CH_3$ |
| 2-Cl-Phenyl | $CH_3$ | H | 7/9 | Cl | $CH_3$ |
| 2-Cl-Phenyl | $CH_3$ | $CH_3$ | 7 | Cl | $CH_3$ |

Tabelle I (Fortsetzung)

| A | $R^1$ | $R^2$ | Pos. | $R^3$ | $R^4$ |
|---|---|---|---|---|---|
| 2-Cl-Phenyl | $CH_3$ | H | 7/9 | $CH_3$ | Cl |
| 2-Cl-Phenyl | $CH_3$ | $CH_3$ | 7 | $CH_3$ | Cl |
| 2-Cl-Phenyl | $CH_3$ | H | 7/9 | Cl | Cl |
| 2-Cl-Phenyl | $CH_3$ | $CH_3$ | 7 | Cl | Cl |
| 2-Cl-Phenyl | $CH_3$ | H | 7/9 | $OCH_3$ | $OCH_3$ |
| 2-Cl-Phenyl | $CH_3$ | $CH_3$ | 7 | $OCH_3$ | $OCH_3$ |
| 2-Cl-Phenyl | $CH_3$ | H | 7/9 | $CH_3$ | $OCH_3$ |
| 2-Cl-Phenyl | $CH_3$ | $CH_3$ | 7 | $CH_3$ | $OCH_3$ |
| 2-Cl-Phenyl | $CH_3$ | H | 7/9 | $OCH_3$ | $CH_3$ |
| 2-Cl-Phenyl | $CH_3$ | $CH_3$ | 7 | $OCH_3$ | $CH_3$ |
| 2-Cl-Phenyl | $CH_3$ | H | 7/9 | Cl | $OCH_3$ |
| 2-Cl-Phenyl | $CH_3$ | $CH_3$ | 7 | Cl | $OCH_3$ |
| 2-Cl-Phenyl | $CH_3$ | H | 7/9 | $OCH_3$ | Cl |
| 2-Cl-Phenyl | $CH_3$ | $CH_3$ | 7 | $OCH_3$ | Cl |
| 2-Cl-Phenyl | H | H | 7/9 | $CF_3$ | $CF_3$ |
| 2-Cl-Phenyl | H | $CH_3$ | 7 | $CF_3$ | $CF_3$ |
| 2-Cl-Phenyl | H | H | 7/9 | Cl | $CF_3$ |
| 2-Cl-Phenyl | H | $CH_3$ | 7 | Cl | $CF_3$ |
| 2-Cl-Phenyl | H | H | 7/9 | $CF_3$ | Cl |
| 2-Cl-Phenyl | H | $CH_3$ | 7 | $CF_3$ | Cl |
| 2-Cl-Phenyl | H | H | 7/9 | F | $CF_3$ |
| 2-Cl-Phenyl | H | $CH_3$ | 7 | F | $CF_3$ |
| 2-Cl-Phenyl | H | H | 7/9 | $CF_3$ | F |
| 2-Cl-Phenyl | H | $CH_3$ | 7 | $CF_3$ | F |
| 2-Cl-Phenyl | H | H | 7/9 | $CH_3$ | $CF_3$ |
| 2-Cl-Phenyl | H | $CH_3$ | 7 | $CH_3$ | $CF_3$ |
| 2-Cl-Phenyl | H | H | 7/9 | $CF_3$ | $CH_3$ |
| 2-Cl-Phenyl | H | $CH_3$ | 7 | $CF_3$ | $CH_3$ |
| 2-Cl-Phenyl | H | H | 7/9 | $OCH_3$ | $CF_3$ |
| 2-Cl-Phenyl | H | $CH_3$ | 7 | $OCH_3$ | $CF_3$ |
| 2-Cl-Phenyl | H | H | 7/9 | $CF_3$ | $OCH_3$ |
| 2-Cl-Phenyl | H | $CH_3$ | 7 | $CF_3$ | $OCH_3$ |
| 2-Cl-Phenyl | H | H | 7/9 | F | F |
| 2-Cl-Phenyl | H | $CH_3$ | 7 | F | F |
| 2-Cl-Phenyl | H | H | 7/9 | F | Cl |
| 2-Cl-Phenyl | H | $CH_3$ | 7 | F | Cl |
| 2-Cl-Phenyl | H | H | 7/9 | Cl | F |
| 2-Cl-Phenyl | H | $CH_3$ | 7 | Cl | F |
| 2-Cl-Phenyl | H | H | 7/9 | F | $CH_3$ |
| 2-Cl-Phenyl | H | $CH_3$ | 7 | F | $CH_3$ |

Tabelle I (Fortsetzung)

| A | $R^1$ | $R^2$ | Pos. | $R^3$ | $R^4$ |
|---|---|---|---|---|---|
| 2-Cl-Phenyl | H | H | 7/9 | $CH_3$ | F |
| 2-Cl-Phenyl | H | $CH_3$ | 7 | $CH_3$ | F |
| 2-Cl-Phenyl | H | H | 7/9 | F | $OCH_3$ |
| 2-Cl-Phenyl | H | $CH_3$ | 7 | F | $OCH_3$ |
| 2-Cl-Phenyl | H | H | 7/9 | $OCH_3$ | F |
| 2-Cl-Phenyl | H | $CH_3$ | 7 | $OCH_3$ | F |
| 2-Cl-Phenyl | H | H | 7/9 | $N(CH_3)_2$ | Cl |
| 2-Cl-Phenyl | H | $CH_3$ | 7 | $N(CH_3)_2$ | Cl |
| 2-Cl-Phenyl | H | H | 7/9 | $SCH_3$ | Cl |
| 2-Cl-Phenyl | H | $CH_3$ | 7 | $SCH_3$ | Cl |
| 2-F-Phenyl | H | H | 7/9 | $CH_3$ | $CH_3$ |
| 2-F-Phenyl | H | $CH_3$ | 7 | $CH_3$ | $CH_3$ |
| 2-F-Phenyl | H | H | 7/9 | Cl | $CH_3$ |
| 2-F-Phenyl | H | $CH_3$ | 7 | Cl | $CH_3$ |
| 2-F-Phenyl | H | H | 7/9 | $CH_3$ | Cl |
| 2-F-Phenyl | H | $CH_3$ | 7 | $CH_3$ | Cl |
| 2-F-Phenyl | H | H | 7/9 | Cl | Cl |
| 2-F-Phenyl | H | $CH_3$ | 7 | Cl | Cl |
| 2-F-Phenyl | H | H | 7/9 | $OCH_3$ | $OCH_3$ |
| 2-F-Phenyl | H | $CH_3$ | 7 | $OCH_3$ | $OCH_3$ |
| 2-F-Phenyl | H | H | 7/9 | $CH_3$ | $OCH_3$ |
| 2-F-Phenyl | H | $CH_3$ | 7 | $CH_3$ | $OCH_3$ |
| 2-F-Phenyl | H | H | 7/9 | $OCH_3$ | $CH_3$ |
| 2-F-Phenyl | H | $CH_3$ | 7 | $OCH_3$ | $CH_3$ |
| 2-F-Phenyl | H | H | 7/9 | Cl | $OCH_3$ |
| 2-F-Phenyl | H | $CH_3$ | 7 | Cl | $OCH_3$ |
| 2-F-Phenyl | H | H | 7/9 | $OCH_3$ | Cl |
| 2-F-Phenyl | H | $CH_3$ | 7 | $OCH_3$ | Cl |
| 2-Br-Phenyl | H | H | 7/9 | $CH_3$ | $CH_3$ |
| 2-Br-Phenyl | H | $CH_3$ | 7 | $CH_3$ | $CH_3$ |
| 2-Br-Phenyl | H | H | 7/9 | Cl | $CH_3$ |
| 2-Br-Phenyl | H | $CH_3$ | 7 | Cl | $CH_3$ |
| 2-Br-Phenyl | H | H | 7/9 | $CH_3$ | Cl |
| 2-Br-Phenyl | H | $CH_3$ | 7 | $CH_3$ | Cl |
| 2-Br-Phenyl | H | H | 7/9 | Cl | Cl |
| 2-Br-Phenyl | H | $CH_3$ | 7 | Cl | Cl |
| 2-Br-Phenyl | H | H | 7/9 | $OCH_3$ | $OCH_3$ |
| 2-Br-Phenyl | H | $CH_3$ | 7 | $OCH_3$ | $OCH_3$ |
| 2-Br-Phenyl | H | H | 7/9 | $CH_3$ | $OCH_3$ |
| 2-Br-Phenyl | H | $CH_3$ | 7 | $CH_3$ | $OCH_3$ |

Tabelle I (Fortsetzung)

| A | $R^1$ | $R^2$ | Pos. | $R^3$ | $R^4$ |
|---|---|---|---|---|---|
| 2-Br-Phenyl | H | H | 7/9 | $OCH_3$ | $CH_3$ |
| 2-Br-Phenyl | H | $CH_3$ | 7 | $OCH_3$ | $CH_3$ |
| 2-Br-Phenyl | H | H | 7/9 | Cl | $OCH_3$ |
| 2-Br-Phenyl | H | $CH_3$ | 7 | Cl | $OCH_3$ |
| 2-Br-Phenyl | H | H | 7/9 | $OCH_3$ | Cl |
| 2-Br-Phenyl | H | $CH_3$ | 7 | $OCH_3$ | Cl |
| 2-CN-Phenyl | H | H | 7/9 | $CH_3$ | $CH_3$ |
| 2-CN-Phenyl | H | $CH_3$ | 7 | $CH_3$ | $CH_3$ |
| 2-CN-Phenyl | H | H | 7/9 | Cl | $CH_3$ |
| 2-CN-Phenyl | H | $CH_3$ | 7 | Cl | $CH_3$ |
| 2-CN-Phenyl | H | H | 7/9 | $CH_3$ | Cl |
| 2-CN-Phenyl | H | $CH_3$ | 7 | $CH_3$ | Cl |
| 2-CN-Phenyl | H | H | 7/9 | Cl | Cl |
| 2-CN-Phenyl | H | $CH_3$ | 7 | Cl | Cl |
| 2-CN-Phenyl | H | H | 7/9 | $OCH_3$ | $OCH_3$ |
| 2-CN-Phenyl | H | $CH_3$ | 7 | $OCH_3$ | $OCH_3$ |
| 2-CN-Phenyl | H | H | 7/9 | $CH_3$ | $OCH_3$ |
| 2-CN-Phenyl | H | $CH_3$ | 7 | $CH_3$ | $OCH_3$ |
| 2-CN-Phenyl | H | H | 7/9 | $OCH_3$ | $CH_3$ |
| 2-CN-Phenyl | H | $CH_3$ | 7 | $OCH_3$ | $CH_3$ |
| 2-CN-Phenyl | H | H | 7/9 | Cl | $OCH_3$ |
| 2-CN-Phenyl | H | $CH_3$ | 7 | Cl | $OCH_3$ |
| 2-CN-Phenyl | H | H | 7/9 | $OCH_3$ | Cl |
| 2-CN-Phenyl | H | $CH_3$ | 7 | $OCH_3$ | Cl |
| 2-$CF_3$-Phenyl | H | H | 7/9 | $CH_3$ | $CH_3$ |
| 2-$CF_3$-Phenyl | H | $CH_3$ | 7 | $CH_3$ | $CH_3$ |
| 2-$CF_3$-Phenyl | H | H | 7/9 | Cl | $CH_3$ |
| 2-$CF_3$-Phenyl | H | $CH_3$ | 7 | Cl | $CH_3$ |
| 2-$CF_3$-Phenyl | H | H | 7/9 | $CH_3$ | Cl |
| 2-$CF_3$-Phenyl | H | $CH_3$ | 7 | $CH_3$ | Cl |
| 2-$CF_3$-Phenyl | H | H | 7/9 | Cl | Cl |
| 2-$CF_3$-Phenyl | H | $CH_3$ | 7 | Cl | Cl |
| 2-$CF_3$-Phenyl | H | H | 7/9 | $OCH_3$ | $OCH_3$ |
| 2-$CF_3$-Phenyl | H | $CH_3$ | 7 | $OCH_3$ | $OCH_3$ |
| 2-$CF_3$-Phenyl | H | H | 7/9 | $CH_3$ | $OCH_3$ |
| 2-$CF_3$-Phenyl | H | $CH_3$ | 7 | $CH_3$ | $OCH_3$ |
| 2-$CF_3$-Phenyl | H | H | 7/9 | $OCH_3$ | $CH_3$ |
| 2-$CF_3$-Phenyl | H | $CH_3$ | 7 | $OCH_3$ | $CH_3$ |
| 2-$CF_3$-Phenyl | H | H | 7/9 | Cl | $OCH_3$ |
| 2-$CF_3$-Phenyl | H | $CH_3$ | 7 | Cl | $OCH_3$ |

Tabelle I (Fortsetzung)

| A | $R^1$ | $R^2$ | Pos. | $R^3$ | $R^4$ |
|---|---|---|---|---|---|
| 2-$CF_3$-Phenyl | H | H | 7/9 | $OCH_3$ | Cl |
| 2-$CF_3$-Phenyl | H | $CH_3$ | 7 | $OCH_3$ | Cl |
| 2-$NO_2$-Phenyl | H | H | 7/9 | $CH_3$ | $CH_3$ |
| 2-$NO_2$-Phenyl | H | $CH_3$ | 7 | $CH_3$ | $CH_3$ |
| 2-$NO_2$-Phenyl | H | H | 7/9 | Cl | $CH_3$ |
| 2-$NO_2$-Phenyl | H | $CH_3$ | 7 | Cl | $CH_3$ |
| 2-$NO_2$-Phenyl | H | H | 7/9 | $CH_3$ | Cl |
| 2-$NO_2$-Phenyl | H | $CH_3$ | 7 | $CH_3$ | Cl |
| 2-$NO_2$-Phenyl | H | H | 7/9 | Cl | Cl |
| 2-$NO_2$-Phenyl | H | $CH_3$ | 7 | Cl | Cl |
| 2-$NO_2$-Phenyl | H | H | 7/9 | $OCH_3$ | $OCH_3$ |
| 2-$NO_2$-Phenyl | H | $CH_3$ | 7 | $OCH_3$ | $OCH_3$ |
| 2-$NO_2$-Phenyl | H | H | 7/9 | $CH_3$ | $OCH_3$ |
| 2-$NO_2$-Phenyl | H | $CH_3$ | 7 | $CH_3$ | $OCH_3$ |
| 2-$NO_2$-Phenyl | H | H | 7/9 | $OCH_3$ | $CH_3$ |
| 2-$NO_2$-Phenyl | H | $CH_3$ | 7 | $OCH_3$ | $CH_3$ |
| 2-$NO_2$-Phenyl | H | H | 7/9 | Cl | $OCH_3$ |
| 2-$NO_2$-Phenyl | H | $CH_3$ | 7 | Cl | $OCH_3$ |
| 2-$NO_2$-Phenyl | H | H | 7/9 | $OCH_3$ | Cl |
| 2-$NO_2$-Phenyl | H | $CH_3$ | 7 | $OCH_3$ | Cl |
| 2-$CH_3$-Phenyl | H | H | 7/9 | $CH_3$ | $CH_3$ |
| 2-$CH_3$-Phenyl | H | $CH_3$ | 7 | $CH_3$ | $CH_3$ |
| 2-$CH_3$-Phenyl | H | H | 7/9 | Cl | $CH_3$ |
| 2-$CH_3$-Phenyl | H | $CH_3$ | 7 | Cl | $CH_3$ |
| 2-$CH_3$-Phenyl | H | H | 7/9 | $CH_3$ | Cl |
| 2-$CH_3$-Phenyl | H | $CH_3$ | 7 | $CH_3$ | Cl |
| 2-$CH_3$-Phenyl | H | H | 7/9 | Cl | Cl |
| 2-$CH_3$-Phenyl | H | $CH_3$ | 7 | Cl | Cl |
| 2-$CH_3$-Phenyl | H | H | 7/9 | $OCH_3$ | $OCH_3$ |
| 2-$CH_3$-Phenyl | H | $CH_3$ | 7 | $OCH_3$ | $OCH_3$ |
| 2-$CH_3$-Phenyl | H | H | 7/9 | $CH_3$ | $OCH_3$ |
| 2-$CH_3$-Phenyl | H | $CH_3$ | 7 | $CH_3$ | $OCH_3$ |
| 2-$CH_3$-Phenyl | H | H | 7/9 | $OCH_3$ | $CH_3$ |
| 2-$CH_3$-Phenyl | H | $CH_3$ | 7 | $OCH_3$ | $CH_3$ |
| 2-$CH_3$-Phenyl | H | H | 7/9 | Cl | $OCH_3$ |
| 2-$CH_3$-Phenyl | H | $CH_3$ | 7 | Cl | $OCH_3$ |
| 2-$CH_3$-Phenyl | H | H | 7/9 | $OCH_3$ | Cl |
| 2-$CH_3$-Phenyl | H | $CH_3$ | 7 | $OCH_3$ | Cl |
| 2-$CO_2CH_3$-Phenyl | H | H | 7/9 | $CH_3$ | $CH_3$ |
| 2-$CO_2CH_3$-Phenyl | H | $CH_3$ | 7 | $CH_3$ | $CH_3$ |

Tabelle I (Fortsetzung)

| A | $R^1$ | $R^2$ | Pos. | $R^3$ | $R^4$ |
|---|---|---|---|---|---|
| 2-$CO_2CH_3$-Phenyl | H | H | 7/9 | Cl | $CH_3$ |
| 2-$CO_2CH_3$-Phenyl | H | $CH_3$ | 7 | Cl | $CH_3$ |
| 2-$CO_2CH_3$-Phenyl | H | H | 7/9 | $CH_3$ | Cl |
| 2-$CO_2CH_3$-Phenyl | H | $CH_3$ | 7 | $CH_3$ | Cl |
| 2-$CO_2CH_3$-Phenyl | H | H | 7/9 | Cl | Cl |
| 2-$CO_2CH_3$-Phenyl | H | $CH_3$ | 7 | Cl | Cl |
| 2-$CO_2CH_3$-Phenyl | H | H | 7/9 | $OCH_3$ | $OCH_3$ |
| 2-$CO_2CH_3$-Phenyl | H | $CH_3$ | 7 | $OCH_3$ | $OCH_3$ |
| 2-$CO_2CH_3$-Phenyl | H | H | 7/9 | $CH_3$ | $OCH_3$ |
| 2-$CO_2CH_3$-Phenyl | H | $CH_3$ | 7 | $CH_3$ | $OCH_3$ |
| 2-$CO_2CH_3$-Phenyl | H | H | 7/9 | $OCH_3$ | $CH_3$ |
| 2-$CO_2CH_3$-Phenyl | H | $CH_3$ | 7 | $OCH_3$ | $CH_3$ |
| 2-$CO_2CH_3$-Phenyl | H | H | 7/9 | Cl | $OCH_3$ |
| 2-$CO_2CH_3$-Phenyl | H | $CH_3$ | 7 | Cl | $OCH_3$ |
| 2-$CO_2CH_3$-Phenyl | H | H | 7/9 | $OCH_3$ | Cl |
| 2-$CO_2CH_3$-Phenyl | H | $CH_3$ | 7 | $OCH_3$ | Cl |
| 2-$CO_2CH_3$-Phenyl | $CH_3$ | H | 7/9 | $CH_3$ | $CH_3$ |
| 2-$CO_2CH_3$-Phenyl | $CH_3$ | $CH_3$ | 7 | $CH_3$ | $CH_3$ |
| 2-$CO_2CH_3$-Phenyl | $CH_3$ | H | 7/9 | Cl | $CH_3$ |
| 2-$CO_2CH_3$-Phenyl | $CH_3$ | $CH_3$ | 7 | Cl | $CH_3$ |
| 2-$CO_2CH_3$-Phenyl | $CH_3$ | H | 7/9 | $CH_3$ | Cl |
| 2-$CO_2CH_3$-Phenyl | $CH_3$ | $CH_3$ | 7 | $CH_3$ | Cl |
| 2-$CO_2CH_3$-Phenyl | $CH_3$ | H | 7/9 | Cl | Cl |
| 2-$CO_2CH_3$-Phenyl | $CH_3$ | $CH_3$ | 7 | Cl | Cl |
| 2-$CO_2CH_3$-Phenyl | $CH_3$ | H | 7/9 | $OCH_3$ | $OCH_3$ |
| 2-$CO_2CH_3$-Phenyl | $CH_3$ | $CH_3$ | 7 | $OCH_3$ | $OCH_3$ |
| 2-$CO_2CH_3$-Phenyl | $CH_3$ | H | 7/9 | $CH_3$ | $OCH_3$ |
| 2-$CO_2CH_3$-Phenyl | $CH_3$ | $CH_3$ | 7 | $CH_3$ | $OCH_3$ |
| 2-$CO_2CH_3$-Phenyl | $CH_3$ | H | 7/9 | $OCH_3$ | $CH_3$ |
| 2-$CO_2CH_3$-Phenyl | $CH_3$ | $CH_3$ | 7 | $OCH_3$ | $CH_3$ |
| 2-$CO_2CH_3$-Phenyl | $CH_3$ | H | 7/9 | Cl | $OCH_3$ |
| 2-$CO_2CH_3$-Phenyl | $CH_3$ | $CH_3$ | 7 | Cl | $OCH_3$ |
| 2-$CO_2CH_3$-Phenyl | $CH_3$ | H | 7/9 | $OCH_3$ | Cl |
| 2-$CO_2CH_3$-Phenyl | $CH_3$ | $CH_3$ | 7 | $OCH_3$ | Cl |
| 2-$CO_2CH_3$-Phenyl | H | H | 7/9 | $CF_3$ | $CF_3$ |
| 2-$CO_2CH_3$-Phenyl | H | $CH_3$ | 7 | $CF_3$ | $CF_3$ |
| 2-$CO_2CH_3$-Phenyl | H | H | 7/9 | Cl | $CF_3$ |
| 2-$CO_2CH_3$-Phenyl | H | $CH_3$ | 7 | Cl | $CF_3$ |
| 2-$CO_2CH_3$-Phenyl | H | H | 7/9 | $CF_3$ | Cl |
| 2-$CO_2CH_3$-Phenyl | H | $CH_3$ | 7 | $CF_3$ | Cl |

Tabelle I (Fortsetzung)

| A | $R^1$ | $R^2$ | Pos. | $R^3$ | $R^4$ |
|---|---|---|---|---|---|
| 2-$CO_2CH_3$-Phenyl | H | H | 7/9 | F | $CF_3$ |
| 2-$CO_2CH_3$-Phenyl | H | $CH_3$ | 7 | F | $CF_3$ |
| 2-$CO_2CH_3$-Phenyl | H | H | 7/9 | $CF_3$ | F |
| 2-$CO_2CH_3$-Phenyl | H | $CH_3$ | 7 | $CF_3$ | F |
| 2-$CO_2CH_3$-Phenyl | H | H | 7/9 | $CH_3$ | $CF_3$ |
| 2-$CO_2CH_3$-Phenyl | H | $CH_3$ | 7 | $CH_3$ | $CF_3$ |
| 2-$CO_2CH_3$-Phenyl | H | H | 7/9 | $CF_3$ | $CH_3$ |
| 2-$CO_2CH_3$-Phenyl | H | $CH_3$ | 7 | $CF_3$ | $CH_3$ |
| 2-$CO_2CH_3$-Phenyl | H | H | 7/9 | $OCH_3$ | $CF_3$ |
| 2-$CO_2CH_3$-Phenyl | H | $CH_3$ | 7 | $OCH_3$ | $CF_3$ |
| 2-$CO_2CH_3$-Phenyl | H | H | 7/9 | $CF_3$ | $OCH_3$ |
| 2-$CO_2CH_3$-Phenyl | H | $CH_3$ | 7 | $CF_3$ | $OCH_3$ |
| 2-$CO_2CH_3$-Phenyl | H | H | 7/9 | F | F |
| 2-$CO_2CH_3$-Phenyl | H | $CH_3$ | 7 | F | F |
| 2-$CO_2CH_3$-Phenyl | H | H | 7/9 | F | Cl |
| 2-$CO_2CH_3$-Phenyl | H | $CH_3$ | 7 | F | Cl |
| 2-$CO_2CH_3$-Phenyl | H | H | 7/9 | Cl | F |
| 2-$CO_2CH_3$-Phenyl | H | $CH_3$ | 7 | Cl | F |
| 2-$CO_2CH_3$-Phenyl | H | H | 7/9 | F | $CH_3$ |
| 2-$CO_2CH_3$-Phenyl | H | $CH_3$ | 7 | F | $CH_3$ |
| 2-$CO_2CH_3$-Phenyl | H | H | 7/9 | $CH_3$ | F |
| 2-$CO_2CH_3$-Phenyl | H | $CH_3$ | 7 | $CH_3$ | F |
| 2-$CO_2CH_3$-Phenyl | H | H | 7/9 | F | $OCH_3$ |
| 2-$CO_2CH_3$-Phenyl | H | $CH_3$ | 7 | F | $OCH_3$ |
| 2-$CO_2CH_3$-Phenyl | H | H | 7/9 | $OCH_3$ | F |
| 2-$CO_2CH_3$-Phenyl | H | $CH_3$ | 7 | $OCH_3$ | F |
| 2-$CO_2CH_3$-Phenyl | H | H | 7/9 | $N(CH_3)_2$ | Cl |
| 2-$CO_2CH_3$-Phenyl | H | $CH_3$ | 7 | $N(CH_3)_2$ | Cl |
| 2-$CO_2CH_3$-Phenyl | H | H | 7/9 | $SCH_3$ | Cl |
| 2-$CO_2CH_3$-Phenyl | H | $CH_3$ | 7 | $SCH_3$ | Cl |
| 2-$CO_2C_2H_5$-Phenyl | H | H | 7/9 | $CH_3$ | $CH_3$ |
| 2-$CO_2C_2H_5$-Phenyl | H | $CH_3$ | 7 | $CH_3$ | $CH_3$ |
| 2-$CO_2C_2H_5$-Phenyl | H | H | 7/9 | Cl | $CH_3$ |
| 2-$CO_2C_2H_5$-Phenyl | H | $CH_3$ | 7 | Cl | $CH_3$ |
| 2-$CO_2C_2H_5$-Phenyl | H | H | 7/9 | $CH_3$ | Cl |
| 2-$CO_2C_2H_5$-Phenyl | H | $CH_3$ | 7 | $CH_3$ | Cl |
| 2-$CO_2C_2H_5$-Phenyl | H | H | 7/9 | Cl | Cl |
| 2-$CO_2C_2H_5$-Phenyl | H | $CH_3$ | 7 | Cl | Cl |
| 2-$CO_2C_2H_5$-Phenyl | H | H | 7/9 | $OCH_3$ | $OCH_3$ |

Tabelle I (Fortsetzung)

| A | $R^1$ | $R^2$ | Pos. | $R^3$ | $R^4$ |
|---|---|---|---|---|---|
| 2-$CO_2C_2H_5$-Phenyl | H | H | 7/9 | $CH_3$ | $OCH_3$ |
| 2-$CO_2C_2H_5$-Phenyl | H | $CH_3$ | 7 | $CH_3$ | $OCH_3$ |
| 2-$CO_2C_2H_5$-Phenyl | H | H | 7/9 | $OCH_3$ | $CH_3$ |
| 2-$CO_2C_2H_5$-Phenyl | H | $CH_3$ | 7 | $OCH_3$ | $CH_3$ |
| 2-$CO_2C_2H_5$-Phenyl | H | H | 7/9 | Cl | $OCH_3$ |
| 2-$CO_2C_2H_5$-Phenyl | H | $CH_3$ | 7 | Cl | $OCH_3$ |
| 2-$CO_2C_2H_5$-Phenyl | H | H | 7/9 | $OCH_3$ | Cl |
| 2-$CO_2C_2H_5$-Phenyl | H | $CH_3$ | 7 | $OCH_3$ | Cl |
| 2-$CON(CH_3)_2$-Phenyl | H | H | 7/9 | $CH_3$ | $CH_3$ |
| 2-$CON(CH_3)_2$-Phenyl | H | $CH_3$ | 7 | $CH_3$ | $CH_3$ |
| 2-$CON(CH_3)_2$-Phenyl | H | H | 7/9 | Cl | $CH_3$ |
| 2-$CON(CH_3)_2$-Phenyl | H | $CH_3$ | 7 | Cl | $CH_3$ |
| 2-$CON(CH_3)_2$-Phenyl | H | H | 7/9 | $CH_3$ | Cl |
| 2-$CON(CH_3)_2$-Phenyl | H | $CH_3$ | 7 | $CH_3$ | Cl |
| 2-$CON(CH_3)_2$-Phenyl | H | H | 7/9 | Cl | Cl |
| 2-$CON(CH_3)_2$-Phenyl | H | $CH_3$ | 7 | Cl | Cl |
| 2-$CON(CH_3)_2$-Phenyl | H | H | 7/9 | $OCH_3$ | $OCH_3$ |
| 2-$CON(CH_3)_2$-Phenyl | H | $CH_3$ | 7 | $OCH_3$ | $OCH_3$ |
| 2-$CON(CH_3)_2$-Phenyl | H | H | 7/9 | $CH_3$ | $OCH_3$ |
| 2-$CON(CH_3)_2$-Phenyl | H | $CH_3$ | 7 | $CH_3$ | $OCH_3$ |
| 2-$CON(CH_3)_2$-Phenyl | H | H | 7/9 | $OCH_3$ | $CH_3$ |
| 2-$CON(CH_3)_2$-Phenyl | H | $CH_3$ | 7 | $OCH_3$ | $CH_3$ |
| 2-$CON(CH_3)_2$-Phenyl | H | H | 7/9 | Cl | $OCH_3$ |
| 2-$CON(CH_3)_2$-Phenyl | H | $CH_3$ | 7 | Cl | $OCH_3$ |
| 2-$CON(CH_3)_2$-Phenyl | H | H | 7/9 | $OCH_3$ | Cl |
| 2-$CON(CH_3)_2$-Phenyl | H | $CH_3$ | 7 | $OCH_3$ | Cl |
| 2-$COCH_3$-Phenyl | H | H | 7/9 | $CH_3$ | $CH_3$ |
| 2-$COCH_3$-Phenyl | H | $CH_3$ | 7 | $CH_3$ | $CH_3$ |
| 2-$COCH_3$-Phenyl | H | H | 7/9 | Cl | $CH_3$ |
| 2-$COCH_3$-Phenyl | H | $CH_3$ | 7 | Cl | $CH_3$ |
| 2-$COCH_3$-Phenyl | H | H | 7/9 | $CH_3$ | Cl |
| 2-$COCH_3$-Phenyl | H | $CH_3$ | 7 | $CH_3$ | Cl |
| 2-$COCH_3$-Phenyl | H | H | 7/9 | Cl | Cl |
| 2-$COCH_3$-Phenyl | H | $CH_3$ | 7 | Cl | Cl |
| 2-$COCH_3$-Phenyl | H | H | 7/9 | $OCH_3$ | $OCH_3$ |
| 2-$COCH_3$-Phenyl | H | $CH_3$ | 7 | $OCH_3$ | $OCH_3$ |
| 2-$COCH_3$-Phenyl | H | H | 7/9 | $CH_3$ | $OCH_3$ |
| 2-$COCH_3$-Phenyl | H | $CH_3$ | 7 | $CH_3$ | $OCH_3$ |

Tabelle I (Fortsetzung)

| A | $R^1$ | $R^2$ | Pos. | $R^3$ | $R^4$ |
|---|---|---|---|---|---|
| 2-$COCH_3$-Phenyl | H | H | 7/9 | $OCH_3$ | $CH_3$ |
| 2-$COCH_3$-Phenyl | H | $CH_3$ | 7 | $OCH_3$ | $CH_3$ |
| 2-$COCH_3$-Phenyl | H | H | 7/9 | Cl | $OCH_3$ |
| 2-$COCH_3$-Phenyl | H | $CH_3$ | 7 | Cl | $OCH_3$ |
| 2-$COCH_3$-Phenyl | H | H | 7/9 | $OCH_3$ | Cl |
| 2-$COCH_3$-Phenyl | H | $CH_3$ | 7 | $OCH_3$ | Cl |
| 2-$OCH_3$-Phenyl | H | H | 7/9 | $CH_3$ | $CH_3$ |
| 2-$OCH_3$-Phenyl | H | $CH_3$ | 7 | $CH_3$ | $CH_3$ |
| 2-$OCH_3$-Phenyl | H | H | 7/9 | Cl | $CH_3$ |
| 2-$OCH_3$-Phenyl | H | $CH_3$ | 7 | Cl | $CH_3$ |
| 2-$OCH_3$-Phenyl | H | H | 7/9 | $CH_3$ | Cl |
| 2-$OCH_3$-Phenyl | H | $CH_3$ | 7 | $CH_3$ | Cl |
| 2-$OCH_3$-Phenyl | H | H | 7/9 | Cl | Cl |
| 2-$OCH_3$-Phenyl | H | $CH_3$ | 7 | Cl | Cl |
| 2-$OCH_3$-Phenyl | H | H | 7/9 | $OCH_3$ | $OCH_3$ |
| 2-$OCH_3$-Phenyl | H | $CH_3$ | 7 | $OCH_3$ | $OCH_3$ |
| 2-$OCH_3$-Phenyl | H | H | 7/9 | $CH_3$ | $OCH_3$ |
| 2-$OCH_3$-Phenyl | H | $CH_3$ | 7 | $CH_3$ | $OCH_3$ |
| 2-$OCH_3$-Phenyl | H | H | 7/9 | $OCH_3$ | $CH_3$ |
| 2-$OCH_3$-Phenyl | H | $CH_3$ | 7 | $OCH_3$ | $CH_3$ |
| 2-$OCH_3$-Phenyl | H | H | 7/9 | Cl | $OCH_3$ |
| 2-$OCH_3$-Phenyl | H | $CH_3$ | 7 | Cl | $OCH_3$ |
| 2-$OCH_3$-Phenyl | H | H | 7/9 | $OCH_3$ | Cl |
| 2-$OCH_3$-Phenyl | H | $CH_3$ | 7 | $OCH_3$ | Cl |
| 2-$OCH_2CH_2OCH_3$-Phenyl | H | H | 7/9 | $CH_3$ | $CH_3$ |
| 2-$OCH_2CH_2OCH_3$-Phenyl | H | $CH_3$ | 7 | $CH_3$ | $CH_3$ |
| 2-$OCH_2CH_2OCH_3$-Phenyl | H | H | 7/9 | Cl | $CH_3$ |
| 2-$OCH_2CH_2OCH_3$-Phenyl | H | $CH_3$ | 7 | Cl | $CH_3$ |
| 2-$OCH_2CH_2OCH_3$-Phenyl | H | H | 7/9 | $CH_3$ | Cl |
| 2-$OCH_2CH_2OCH_3$-Phenyl | H | $CH_3$ | 7 | $CH_3$ | Cl |
| 2-$OCH_2CH_2OCH_3$-Phenyl | H | H | 7/9 | Cl | Cl |
| 2-$OCH_2CH_2OCH_3$-Phenyl | H | $CH_3$ | 7 | Cl | Cl |
| 2-$OCH_2CH_2OCH_3$-Phenyl | H | H | 7/9 | $OCH_3$ | $OCH_3$ |
| 2-$OCH_2CH_2OCH_3$-Phenyl | H | $CH_3$ | 7 | $OCH_3$ | $OCH_3$ |
| 2-$OCH_2CH_2OCH_3$-Phenyl | H | H | 7/9 | $CH_3$ | $OCH_3$ |
| 2-$OCH_2CH_2OCH_3$-Phenyl | H | $CH_3$ | 7 | $CH_3$ | $OCH_3$ |
| 2-$OCH_2CH_2OCH_3$-Phenyl | H | H | 7/9 | $OCH_3$ | $CH_3$ |
| 2-$OCH_2CH_2OCH_3$-Phenyl | H | $CH_3$ | 7 | $OCH_3$ | $CH_3$ |

Tabelle I (Fortsetzung)

| A | $R^1$ | $R^2$ | Pos. | $R^3$ | $R^4$ |
|---|---|---|---|---|---|
| 2-$OCH_2CH_2OCH_3$-Phenyl | H | H | 7/9 | Cl | $OCH_3$ |
| 2-$OCH_2CH_2OCH_3$-Phenyl | H | $CH_3$ | 7 | Cl | $OCH_3$ |
| 2-$OCH_2CH_2OCH_3$-Phenyl | H | H | 7/9 | $OCH_3$ | Cl |
| 2-$OCH_2CH_2OCH_3$-Phenyl | H | $CH_3$ | 7 | $OCH_3$ | Cl |
| 2-$OCH_2CH_2Cl$-Phenyl | H | H | 7/9 | $CH_3$ | $CH_3$ |
| 2-$OCH_2CH_2Cl$-Phenyl | H | $CH_3$ | 7 | $CH_3$ | $CH_3$ |
| 2-$OCH_2CH_2Cl$-Phenyl | H | H | 7/9 | Cl | $CH_3$ |
| 2-$OCH_2CH_2Cl$-Phenyl | H | $CH_3$ | 7 | Cl | $CH_3$ |
| 2-$OCH_2CH_2Cl$-Phenyl | H | H | 7/9 | $CH_3$ | Cl |
| 2-$OCH_2CH_2Cl$-Phenyl | H | $CH_3$ | 7 | $CH_3$ | Cl |
| 2-$OCH_2CH_2Cl$-Phenyl | H | H | 7/9 | Cl | Cl |
| 2-$OCH_2CH_2Cl$-Phenyl | H | $CH_3$ | 7 | Cl | Cl |
| 2-$OCH_2CH_2Cl$-Phenyl | H | H | 7/9 | $OCH_3$ | $OCH_3$ |
| 2-$OCH_2CH_2Cl$-Phenyl | H | $CH_3$ | 7 | $OCH_3$ | $OCH_3$ |
| 2-$OCH_2CH_2Cl$-Phenyl | H | H | 7/9 | $CH_3$ | $OCH_3$ |
| 2-$OCH_2CH_2Cl$-Phenyl | H | $CH_3$ | 7 | $CH_3$ | $OCH_3$ |
| 2-$OCH_2CH_2Cl$-Phenyl | H | H | 7/9 | $OCH_3$ | $CH_3$ |
| 2-$OCH_2CH_2Cl$-Phenyl | H | $CH_3$ | 7 | $OCH_3$ | $CH_3$ |
| 2-$OCH_2CH_2Cl$-Phenyl | H | H | 7/9 | Cl | $OCH_3$ |
| 2-$OCH_2CH_2Cl$-Phenyl | H | $CH_3$ | 7 | Cl | $OCH_3$ |
| 2-$OCH_2CH_2Cl$-Phenyl | H | H | 7/9 | $OCH_3$ | Cl |
| 2-$OCH_2CH_2Cl$-Phenyl | H | $CH_3$ | 7 | $OCH_3$ | Cl |
| 2-$OCH_2CH_2Cl$-Phenyl | $CH_3$ | H | 7/9 | $CH_3$ | $CH_3$ |
| 2-$OCH_2CH_2Cl$-Phenyl | $CH_3$ | $CH_3$ | 7 | $CH_3$ | $CH_3$ |
| 2-$OCH_2CH_2Cl$-Phenyl | $CH_3$ | H | 7/9 | Cl | $CH_3$ |
| 2-$OCH_2CH_2Cl$-Phenyl | $CH_3$ | $CH_3$ | 7 | Cl | $CH_3$ |
| 2-$OCH_2CH_2Cl$-Phenyl | $CH_3$ | H | 7/9 | $CH_3$ | Cl |
| 2-$OCH_2CH_2Cl$-Phenyl | $CH_3$ | $CH_3$ | 7 | $CH_3$ | Cl |
| 2-$OCH_2CH_2Cl$-Phenyl | $CH_3$ | H | 7/9 | Cl | Cl |
| 2-$OCH_2CH_2Cl$-Phenyl | $CH_3$ | $CH_3$ | 7 | Cl | Cl |
| 2-$OCH_2CH_2Cl$-Phenyl | $CH_3$ | H | 7/9 | $OCH_3$ | $OCH_3$ |
| 2-$OCH_2CH_2Cl$-Phenyl | $CH_3$ | $CH_3$ | 7 | $OCH_3$ | $OCH_3$ |
| 2-$OCH_2CH_2Cl$-Phenyl | $CH_3$ | H | 7/9 | $CH_3$ | $OCH_3$ |
| 2-$OCH_2CH_2Cl$-Phenyl | $CH_3$ | $CH_3$ | 7 | $CH_3$ | $OCH_3$ |
| 2-$OCH_2CH_2Cl$-Phenyl | $CH_3$ | H | 7/9 | $OCH_3$ | $CH_3$ |
| 2-$OCH_2CH_2Cl$-Phenyl | $CH_3$ | $CH_3$ | 7 | $OCH_3$ | $CH_3$ |
| 2-$OCH_2CH_2Cl$-Phenyl | $CH_3$ | H | 7/9 | Cl | $OCH_3$ |
| 2-$OCH_2CH_2Cl$-Phenyl | $CH_3$ | $CH_3$ | 7 | Cl | $OCH_3$ |

Tabelle I (Fortsetzung)

| A | $R^1$ | $R^2$ | Pos. | $R^3$ | $R^4$ |
|---|---|---|---|---|---|
| 2-$OCH_2CH_2Cl$-Phenyl | $CH_3$ | H | 7/9 | $OCH_3$ | Cl |
| 2-$OCH_2CH_2Cl$-Phenyl | $CH_3$ | $CH_3$ | 7 | $OCH_3$ | Cl |
| 2-$OCH_2CH_2Cl$-Phenyl | H | H | 7/9 | $CF_3$ | $CF_3$ |
| 2-$OCH_2CH_2Cl$-Phenyl | H | $CH_3$ | 7 | $CF_3$ | $CF_3$ |
| 2-$OCH_2CH_2Cl$-Phenyl | H | H | 7/9 | Cl | $CF_3$ |
| 2-$OCH_2CH_2Cl$-Phenyl | H | $CH_3$ | 7 | Cl | $CF_3$ |
| 2-$OCH_2CH_2Cl$-Phenyl | H | H | 7/9 | $CF_3$ | Cl |
| 2-$OCH_2CH_2Cl$-Phenyl | H | $CH_3$ | 7 | $CF_3$ | Cl |
| 2-$OCH_2CH_2Cl$-Phenyl | H | H | 7/9 | F | $CF_3$ |
| 2-$OCH_2CH_2Cl$-Phenyl | H | $CH_3$ | 7 | F | $CF_3$ |
| 2-$OCH_2CH_2Cl$-Phenyl | H | H | 7/9 | $CF_3$ | F |
| 2-$OCH_2CH_2Cl$-Phenyl | H | $CH_3$ | 7 | $CF_3$ | F |
| 2-$OCH_2CH_2Cl$-Phenyl | H | H | 7/9 | $CH_3$ | $CF_3$ |
| 2-$OCH_2CH_2Cl$-Phenyl | H | $CH_3$ | 7 | $CH_3$ | $CF_3$ |
| 2-$OCH_2CH_2Cl$-Phenyl | H | H | 7/9 | $CF_3$ | $CH_3$ |
| 2-$OCH_2CH_2Cl$-Phenyl | H | $CH_3$ | 7 | $CF_3$ | $CH_3$ |
| 2-$OCH_2CH_2Cl$-Phenyl | H | H | 7/9 | $OCH_3$ | $CF_3$ |
| 2-$OCH_2CH_2Cl$-Phenyl | H | $CH_3$ | 7 | $OCH_3$ | $CF_3$ |
| 2-$OCH_2CH_2Cl$-Phenyl | H | H | 7/9 | $CF_3$ | $OCH_3$ |
| 2-$OCH_2CH_2Cl$-Phenyl | H | $CH_3$ | 7 | $CF_3$ | $OCH_3$ |
| 2-$OCH_2CH_2Cl$-Phenyl | H | H | 7/9 | F | F |
| 2-$OCH_2CH_2Cl$-Phenyl | H | $CH_3$ | 7 | F | F |
| 2-$OCH_2CH_2Cl$-Phenyl | H | H | 7/9 | F | Cl |
| 2-$OCH_2CH_2Cl$-Phenyl | H | $CH_3$ | 7 | F | Cl |
| 2-$OCH_2CH_2Cl$-Phenyl | H | H | 7/9 | Cl | F |
| 2-$OCH_2CH_2Cl$-Phenyl | H | $CH_3$ | 7 | Cl | F |
| 2-$OCH_2CH_2Cl$-Phenyl | H | H | 7/9 | F | $CH_3$ |
| 2-$OCH_2CH_2Cl$-Phenyl | H | $CH_3$ | 7 | F | $CH_3$ |
| 2-$OCH_2CH_2Cl$-Phenyl | H | H | 7/9 | $CH_3$ | F |
| 2-$OCH_2CH_2Cl$-Phenyl | H | $CH_3$ | 7 | $CH_3$ | F |
| 2-$OCH_2CH_2Cl$-Phenyl | H | H | 7/9 | F | $OCH_3$ |
| 2-$OCH_2CH_2Cl$-Phenyl | H | $CH_3$ | 7 | F | $OCH_3$ |
| 2-$OCH_2CH_2Cl$-Phenyl | H | H | 7/9 | $OCH_3$ | F |
| 2-$OCH_2CH_2Cl$-Phenyl | H | $CH_3$ | 7 | $OCH_3$ | F |
| 2-$OCH_2CH_2Cl$-Phenyl | H | H | 7/9 | $N(CH_3)_2$ | Cl |
| 2-$OCH_2CH_2Cl$-Phenyl | H | $CH_3$ | 7 | $N(CH_3)_2$ | Cl |
| 2-$OCH_2CH_2Cl$-Phenyl | H | H | 7/9 | $SCH_3$ | Cl |
| 2-$OCH_2CH_2Cl$-Phenyl | H | $CH_3$ | 7 | $SCH_3$ | Cl |

Tabelle I (Fortsetzung)

| A | $R^1$ | $R^2$ | Pos. | $R^3$ | $R^4$ |
|---|---|---|---|---|---|
| 2-$OCH_2CO_2CH_3$-Phenyl | H | H | 7/9 | $CH_3$ | $CH_3$ |
| 2-$OCH_2CO_2CH_3$-Phenyl | H | $CH_3$ | 7 | $CH_3$ | $CH_3$ |
| 2-$OCH_2CO_2CH_3$-Phenyl | H | H | 7/9 | Cl | $CH_3$ |
| 2-$OCH_2CO_2CH_3$-Phenyl | H | $CH_3$ | 7 | Cl | $CH_3$ |
| 2-$OCH_2CO_2CH_3$-Phenyl | H | H | 7/9 | $CH_3$ | Cl |
| 2-$OCH_2CO_2CH_3$-Phenyl | H | $CH_3$ | 7 | $CH_3$ | Cl |
| 2-$OCH_2CO_2CH_3$-Phenyl | H | H | 7/9 | Cl | Cl |
| 2-$OCH_2CO_2CH_3$-Phenyl | H | $CH_3$ | 7 | Cl | Cl |
| 2-$OCH_2CO_2CH_3$-Phenyl | H | H | 7/9 | $OCH_3$ | $OCH_3$ |
| 2-$OCH_2CO_2CH_3$-Phenyl | H | $CH_3$ | 7 | $OCH_3$ | $OCH_3$ |
| 2-$OCH_2CO_2CH_3$-Phenyl | H | H | 7/9 | $CH_3$ | $OCH_3$ |
| 2-$OCH_2CO_2CH_3$-Phenyl | H | $CH_3$ | 7 | $CH_3$ | $OCH_3$ |
| 2-$OCH_2CO_2CH_3$-Phenyl | H | H | 7/9 | $OCH_3$ | $CH_3$ |
| 2-$OCH_2CO_2CH_3$-Phenyl | H | $CH_3$ | 7 | $OCH_3$ | $CH_3$ |
| 2-$OCH_2CO_2CH_3$-Phenyl | H | H | 7/9 | Cl | $OCH_3$ |
| 2-$OCH_2CO_2CH_3$-Phenyl | H | $CH_3$ | 7 | Cl | $OCH_3$ |
| 2-$OCH_2CO_2CH_3$-Phenyl | H | H | 7/9 | $OCH_3$ | Cl |
| 2-$OCH_2CO_2CH_3$-Phenyl | H | $CH_3$ | 7 | $OCH_3$ | Cl |
| 2-$SCH_3$-Phenyl | H | H | 7/9 | $CH_3$ | $CH_3$ |
| 2-$SCH_3$-Phenyl | H | $CH_3$ | 7 | $CH_3$ | $CH_3$ |
| 2-$SCH_3$-Phenyl | H | H | 7/9 | Cl | $CH_3$ |
| 2-$SCH_3$-Phenyl | H | $CH_3$ | 7 | Cl | $CH_3$ |
| 2-$SCH_3$-Phenyl | H | H | 7/9 | $CH_3$ | Cl |
| 2-$SCH_3$-Phenyl | H | $CH_3$ | 7 | $CH_3$ | Cl |
| 2-$SCH_3$-Phenyl | H | H | 7/9 | Cl | Cl |
| 2-$SCH_3$-Phenyl | H | $CH_3$ | 7 | Cl | Cl |
| 2-$SCH_3$-Phenyl | H | H | 7/9 | $OCH_3$ | $OCH_3$ |
| 2-$SCH_3$-Phenyl | H | $CH_3$ | 7 | $OCH_3$ | $OCH_3$ |
| 2-$SCH_3$-Phenyl | H | H | 7/9 | $CH_3$ | $OCH_3$ |
| 2-$SCH_3$-Phenyl | H | $CH_3$ | 7 | $CH_3$ | $OCH_3$ |
| 2-$SCH_3$-Phenyl | H | H | 7/9 | $OCH_3$ | $CH_3$ |
| 2-$SCH_3$-Phenyl | H | $CH_3$ | 7 | $OCH_3$ | $CH_3$ |
| 2-$SCH_3$-Phenyl | H | H | 7/9 | Cl | $OCH_3$ |
| 2-$SCH_3$-Phenyl | H | $CH_3$ | 7 | Cl | $OCH_3$ |
| 2-$SCH_3$-Phenyl | H | H | 7/9 | $OCH_3$ | Cl |
| 2-$SCH_3$-Phenyl | H | $CH_3$ | 7 | $OCH_3$ | Cl |
| 2-$SO_2CH_3$-Phenyl | H | H | 7/9 | $CH_3$ | $CH_3$ |
| 2-$SO_2CH_3$-Phenyl | H | $CH_3$ | 7 | $CH_3$ | $CH_3$ |

Tabelle I (Fortsetzung)

| A | $R^1$ | $R^2$ | Pos. | $R^3$ | $R^4$ |
|---|---|---|---|---|---|
| 2-$SO_2CH_3$-Phenyl | H | H | 7/9 | Cl | $CH_3$ |
| 2-$SO_2CH_3$-Phenyl | H | $CH_3$ | 7 | Cl | $CH_3$ |
| 2-$SO_2CH_3$-Phenyl | H | H | 7/9 | $CH_3$ | Cl |
| 2-$SO_2CH_3$-Phenyl | H | $CH_3$ | 7 | $CH_3$ | Cl |
| 2-$SO_2CH_3$-Phenyl | H | H | 7/9 | Cl | Cl |
| 2-$SO_2CH_3$-Phenyl | H | $CH_3$ | 7 | Cl | Cl |
| 2-$SO_2CH_3$-Phenyl | H | H | 7/9 | $OCH_3$ | $OCH_3$ |
| 2-$SO_2CH_3$-Phenyl | H | $CH_3$ | 7 | $OCH_3$ | $OCH_3$ |
| 2-$SO_2CH_3$-Phenyl | H | H | 7/9 | $CH_3$ | $OCH_3$ |
| 2-$SO_2CH_3$-Phenyl | H | $CH_3$ | 7 | $CH_3$ | $OCH_3$ |
| 2-$SO_2CH_3$-Phenyl | H | H | 7/9 | $OCH_3$ | $CH_3$ |
| 2-$SO_2CH_3$-Phenyl | H | $CH_3$ | 7 | $OCH_3$ | $CH_3$ |
| 2-$SO_2CH_3$-Phenyl | H | H | 7/9 | Cl | $OCH_3$ |
| 2-$SO_2CH_3$-Phenyl | H | $CH_3$ | 7 | Cl | $OCH_3$ |
| 2-$SO_2CH_3$-Phenyl | H | H | 7/9 | $OCH_3$ | Cl |
| 2-$SO_2CH_3$-Phenyl | H | $CH_3$ | 7 | $OCH_3$ | Cl |
| 2-$SO_2N(CH_3)_2$-Phenyl | H | H | 7/9 | $CH_3$ | $CH_3$ |
| 2-$SO_2N(CH_3)_2$-Phenyl | H | $CH_3$ | 7 | $CH_3$ | $CH_3$ |
| 2-$SO_2N(CH_3)_2$-Phenyl | H | H | 7/9 | Cl | $CH_3$ |
| 2-$SO_2N(CH_3)_2$-Phenyl | H | $CH_3$ | 7 | Cl | $CH_3$ |
| 2-$SO_2N(CH_3)_2$-Phenyl | H | H | 7/9 | $CH_3$ | Cl |
| 2-$SO_2N(CH_3)_2$-Phenyl | H | $CH_3$ | 7 | $CH_3$ | Cl |
| 2-$SO_2N(CH_3)_2$-Phenyl | H | H | 7/9 | Cl | Cl |
| 2-$SO_2N(CH_3)_2$-Phenyl | H | $CH_3$ | 7 | Cl | Cl |
| 2-$SO_2N(CH_3)_2$-Phenyl | H | H | 7/9 | $OCH_3$ | $OCH_3$ |
| 2-$SO_2N(CH_3)_2$-Phenyl | H | $CH_3$ | 7 | $OCH_3$ | $OCH_3$ |
| 2-$SO_2N(CH_3)_2$-Phenyl | H | H | 7/9 | $CH_3$ | $OCH_3$ |
| 2-$SO_2N(CH_3)_2$-Phenyl | H | $CH_3$ | 7 | $CH_3$ | $OCH_3$ |
| 2-$SO_2N(CH_3)_2$-Phenyl | H | H | 7/9 | $OCH_3$ | $CH_3$ |
| 2-$SO_2N(CH_3)_2$-Phenyl | H | $CH_3$ | 7 | $OCH_3$ | $CH_3$ |
| 2-$SO_2N(CH_3)_2$-Phenyl | H | H | 7/9 | Cl | $OCH_3$ |
| 2-$SO_2N(CH_3)_2$-Phenyl | H | $CH_3$ | 7 | Cl | $OCH_3$ |
| 2-$SO_2N(CH_3)_2$-Phenyl | H | H | 7/9 | $OCH_3$ | Cl |
| 2-$SO_2N(CH_3)_2$-Phenyl | H | $CH_3$ | 7 | $OCH_3$ | Cl |
| 2,6-Cl,Cl-Phenyl | H | H | 7/9 | $CH_3$ | $CH_3$ |
| 2,6-Cl,Cl-Phenyl | H | $CH_3$ | 7 | $CH_3$ | $CH_3$ |
| 2,6-Cl,Cl-Phenyl | H | H | 7/9 | Cl | $CH_3$ |
| 2,6-Cl,Cl-Phenyl | H | $CH_3$ | 7 | Cl | $CH_3$ |

Tabelle I (Fortsetzung)

| A | $R^1$ | $R^2$ | Pos. | $R^3$ | $R^4$ |
|---|---|---|---|---|---|
| 2,6-Cl,Cl-Phenyl | H | H | 7/9 | $CH_3$ | Cl |
| 2,6-Cl,Cl-Phenyl | H | $CH_3$ | 7 | $CH_3$ | Cl |
| 2,6-Cl,Cl-Phenyl | H | H | 7/9 | Cl | Cl |
| 2,6-Cl,Cl-Phenyl | H | $CH_3$ | 7 | Cl | Cl |
| 2,6-Cl,Cl-Phenyl | H | H | 7/9 | $OCH_3$ | $OCH_3$ |
| 2,6-Cl,Cl-Phenyl | H | $CH_3$ | 7 | $OCH_3$ | $OCH_3$ |
| 2,6-Cl,Cl-Phenyl | H | H | 7/9 | $CH_3$ | $OCH_3$ |
| 2,6-Cl,Cl-Phenyl | H | $CH_3$ | 7 | $CH_3$ | $OCH_3$ |
| 2,6-Cl,Cl-Phenyl | H | H | 7/9 | $OCH_3$ | $CH_3$ |
| 2,6-Cl,Cl-Phenyl | H | $CH_3$ | 7 | $OCH_3$ | $CH_3$ |
| 2,6-Cl,Cl-Phenyl | H | H | 7/9 | Cl | $OCH_3$ |
| 2,6-Cl,Cl-Phenyl | H | $CH_3$ | 7 | Cl | $OCH_3$ |
| 2,6-Cl,Cl-Phenyl | H | H | 7/9 | $OCH_3$ | Cl |
| 2,6-Cl,Cl-Phenyl | H | $CH_3$ | 7 | $OCH_3$ | Cl |
| 2,6-Cl,Cl-Phenyl | $CH_3$ | H | 7/9 | $CH_3$ | $CH_3$ |
| 2,6-Cl,Cl-Phenyl | $CH_3$ | $CH_3$ | 7 | $CH_3$ | $CH_3$ |
| 2,6-Cl,Cl-Phenyl | $CH_3$ | H | 7/9 | Cl | $CH_3$ |
| 2,6-Cl,Cl-Phenyl | $CH_3$ | $CH_3$ | 7 | Cl | $CH_3$ |
| 2,6-Cl,Cl-Phenyl | $CH_3$ | H | 7/9 | $CH_3$ | Cl |
| 2,6-Cl,Cl-Phenyl | $CH_3$ | $CH_3$ | 7 | $CH_3$ | Cl |
| 2,6-Cl,Cl-Phenyl | $CH_3$ | H | 7/9 | Cl | Cl |
| 2,6-Cl,Cl-Phenyl | $CH_3$ | $CH_3$ | 7 | Cl | Cl |
| 2,6-Cl,Cl-Phenyl | $CH_3$ | H | 7/9 | $OCH_3$ | $OCH_3$ |
| 2,6-Cl,Cl-Phenyl | $CH_3$ | $CH_3$ | 7 | $OCH_3$ | $OCH_3$ |
| 2,6-Cl,Cl-Phenyl | $CH_3$ | H | 7/9 | $CH_3$ | $OCH_3$ |
| 2,6-Cl,Cl-Phenyl | $CH_3$ | $CH_3$ | 7 | $CH_3$ | $OCH_3$ |
| 2,6-Cl,Cl-Phenyl | $CH_3$ | H | 7/9 | $OCH_3$ | $CH_3$ |
| 2,6-Cl,Cl-Phenyl | $CH_3$ | $CH_3$ | 7 | $OCH_3$ | $CH_3$ |
| 2,6-Cl,Cl-Phenyl | $CH_3$ | H | 7/9 | Cl | $OCH_3$ |
| 2,6-Cl,Cl-Phenyl | $CH_3$ | $CH_3$ | 7 | Cl | $OCH_3$ |
| 2,6-Cl,Cl-Phenyl | $CH_3$ | H | 7/9 | $OCH_3$ | Cl |
| 2,6-Cl,Cl-Phenyl | $CH_3$ | $CH_3$ | 7 | $OCH_3$ | Cl |
| 2,6-Cl,Cl-Phenyl | H | H | 7/9 | $CF_3$ | $CF_3$ |
| 2,6-Cl,Cl-Phenyl | H | $CH_3$ | 7 | $CF_3$ | $CF_3$ |
| 2,6-Cl,Cl-Phenyl | H | H | 7/9 | Cl | $CF_3$ |
| 2,6-Cl,Cl-Phenyl | H | $CH_3$ | 7 | Cl | $CF_3$ |
| 2,6-Cl,Cl-Phenyl | H | H | 7/9 | $CF_3$ | Cl |
| 2,6-Cl,Cl-Phenyl | H | $CH_3$ | 7 | $CF_3$ | Cl |
| 2,6-Cl,Cl-Phenyl | H | H | 7/9 | F | $CF_3$ |

Tabelle I (Fortsetzung)

| A | $R^1$ | $R^2$ | Pos. | $R^3$ | $R^4$ |
|---|---|---|---|---|---|
| 2,6-Cl,Cl-Phenyl | H | $CH_3$ | 7 | F | $CF_3$ |
| 2,6-Cl,Cl-Phenyl | H | H | 7/9 | $CF_3$ | F |
| 2,6-Cl,Cl-Phenyl | H | $CH_3$ | 7 | $CF_3$ | F |
| 2,6-Cl,Cl-Phenyl | H | H | 7/9 | $CH_3$ | $CF_3$ |
| 2,6-Cl,Cl-Phenyl | H | $CH_3$ | 7 | $CH_3$ | $CF_3$ |
| 2,6-Cl,Cl-Phenyl | H | H | 7/9 | $CF_3$ | $CH_3$ |
| 2,6-Cl,Cl-Phenyl | H | $CH_3$ | 7 | $CF_3$ | $CH_3$ |
| 2,6-Cl,Cl-Phenyl | H | H | 7/9 | $OCH_3$ | $CF_3$ |
| 2,6-Cl,Cl-Phenyl | H | $CH_3$ | 7 | $OCH_3$ | $CF_3$ |
| 2,6-Cl,Cl-Phenyl | H | H | 7/9 | $CF_3$ | $OCH_3$ |
| 2,6-Cl,Cl-Phenyl | H | $CH_3$ | 7 | $CF_3$ | $OCH_3$ |
| 2,6-Cl,Cl-Phenyl | H | H | 7/9 | F | F |
| 2,6-Cl,Cl-Phenyl | H | $CH_3$ | 7 | F | F |
| 2,6-Cl,Cl-Phenyl | H | H | 7/9 | F | Cl |
| 2,6-Cl,Cl-Phenyl | H | $CH_3$ | 7 | F | Cl |
| 2,6-Cl,Cl-Phenyl | H | H | 7/9 | Cl | F |
| 2,6-Cl,Cl-Phenyl | H | $CH_3$ | 7 | Cl | F |
| 2,6-Cl,Cl-Phenyl | H | H | 7/9 | F | $CH_3$ |
| 2,6-Cl,Cl-Phenyl | H | $CH_3$ | 7 | F | $CH_3$ |
| 2,6-Cl,Cl-Phenyl | H | H | 7/9 | $CH_3$ | F |
| 2,6-Cl,Cl-Phenyl | H | $CH_3$ | 7 | $CH_3$ | F |
| 2,6-Cl,Cl-Phenyl | H | H | 7/9 | F | $OCH_3$ |
| 2,6-Cl,Cl-Phenyl | H | $CH_3$ | 7 | F | $OCH_3$ |
| 2,6-Cl,Cl-Phenyl | H | H | 7/9 | $OCH_3$ | F |
| 2,6-Cl,Cl-Phenyl | H | $CH_3$ | 7 | $OCH_3$ | F |
| 2,6-Cl,Cl-Phenyl | H | H | 7/9 | $N(CH_3)_2$ | Cl |
| 2,6-Cl,Cl-Phenyl | H | $CH_3$ | 7 | $N(CH_3)_2$ | Cl |
| 2,6-Cl,Cl-Phenyl | H | H | 7/9 | $SCH_3$ | Cl |
| 2,6-Cl,Cl-Phenyl | H | $CH_3$ | 7 | $SCH_3$ | Cl |
| 2-Cl,6-$CH_3$-Phenyl | H | H | 7/9 | $CH_3$ | $CH_3$ |
| 2-Cl,6-$CH_3$-Phenyl | H | $CH_3$ | 7 | $CH_3$ | $CH_3$ |
| 2-Cl,6-$CH_3$-Phenyl | H | H | 7/9 | Cl | $CH_3$ |
| 2-Cl,6-$CH_3$-Phenyl | H | $CH_3$ | 7 | Cl | $CH_3$ |
| 2-Cl,6-$CH_3$-Phenyl | H | H | 7/9 | $CH_3$ | Cl |
| 2-Cl,6-$CH_3$-Phenyl | H | $CH_3$ | 7 | $CH_3$ | Cl |
| 2-Cl,6-$CH_3$-Phenyl | H | H | 7/9 | Cl | Cl |
| 2-Cl,6-$CH_3$-Phenyl | H | $CH_3$ | 7 | Cl | Cl |
| 2-Cl,6-$CH_3$-Phenyl | H | H | 7/9 | $OCH_3$ | $OCH_3$ |
| 2-Cl,6-$CH_3$-Phenyl | H | $CH_3$ | 7 | $OCH_3$ | $OCH_3$ |
| 2-Cl,6-$CH_3$-Phenyl | H | H | 7/9 | $CH_3$ | $OCH_3$ |

Tabelle I (Fortsetzung)

| A | $R^1$ | $R^2$ | Pos. | $R^3$ | $R^4$ |
|---|---|---|---|---|---|
| 2-Cl,6-$CH_3$-Phenyl | H | $CH_3$ | 7 | $CH_3$ | $OCH_3$ |
| 2-Cl,6-$CH_3$-Phenyl | H | H | 7/9 | $OCH_3$ | $CH_3$ |
| 2-Cl,6-$CH_3$-Phenyl | H | $CH_3$ | 7 | $OCH_3$ | $CH_3$ |
| 2-Cl,6-$CH_3$-Phenyl | H | H | 7/9 | Cl | $OCH_3$ |
| 2-Cl,6-$CH_3$-Phenyl | H | $CH_3$ | 7 | Cl | $OCH_3$ |
| 2-Cl,6-$CH_3$-Phenyl | H | H | 7/9 | $OCH_3$ | Cl |
| 2-Cl,6-$CH_3$-Phenyl | $CH_3$ | H | 7/9 | $CH_3$ | $CH_3$ |
| 2-Cl,6-$CH_3$-Phenyl | $CH_3$ | $CH_3$ | 7 | $CH_3$ | $CH_3$ |
| 2-Cl,6-$CH_3$-Phenyl | $CH_3$ | H | 7/9 | Cl | $CH_3$ |
| 2-Cl,6-$CH_3$-Phenyl | $CH_3$ | $CH_3$ | 7 | Cl | $CH_3$ |
| 2-Cl,6-$CH_3$-Phenyl | $CH_3$ | H | 7/9 | $CH_3$ | Cl |
| 2-Cl,6-$CH_3$-Phenyl | $CH_3$ | $CH_3$ | 7 | $CH_3$ | Cl |
| 2-Cl,6-$CH_3$-Phenyl | $CH_3$ | H | 7/9 | Cl | Cl |
| 2-Cl,6-$CH_3$-Phenyl | $CH_3$ | $CH_3$ | 7 | Cl | Cl |
| 2-Cl,6-$CH_3$-Phenyl | $CH_3$ | H | 7/9 | $OCH_3$ | $OCH_3$ |
| 2-Cl,6-$CH_3$-Phenyl | $CH_3$ | $CH_3$ | 7 | $OCH_3$ | $OCH_3$ |
| 2-Cl,6-$CH_3$-Phenyl | $CH_3$ | H | 7/9 | $CH_3$ | $OCH_3$ |
| 2-Cl,6-$CH_3$-Phenyl | $CH_3$ | $CH_3$ | 7 | $CH_3$ | $OCH_3$ |
| 2-Cl,6-$CH_3$-Phenyl | $CH_3$ | H | 7/9 | $OCH_3$ | $CH_3$ |
| 2-Cl,6-$CH_3$-Phenyl | $CH_3$ | $CH_3$ | 7 | $OCH_3$ | $CH_3$ |
| 2-Cl,6-$CH_3$-Phenyl | $CH_3$ | H | 7/9 | Cl | $OCH_3$ |
| 2-Cl,6-$CH_3$-Phenyl | $CH_3$ | $CH_3$ | 7 | Cl | $OCH_3$ |
| 2-Cl,6-$CH_3$-Phenyl | $CH_3$ | H | 7/9 | $OCH_3$ | Cl |
| 2-Cl,6-$CH_3$-Phenyl | $CH_3$ | $CH_3$ | 7 | $OCH_3$ | Cl |
| 2-Cl,6-$CH_3$-Phenyl | H | H | 7/9 | $CF_3$ | $CF_3$ |
| 2-Cl,6-$CH_3$-Phenyl | H | $CH_3$ | 7 | $CF_3$ | $CF_3$ |
| 2-Cl,6-$CH_3$-Phenyl | H | H | 7/9 | Cl | $CF_3$ |
| 2-Cl,6-$CH_3$-Phenyl | H | $CH_3$ | 7 | Cl | $CF_3$ |
| 2-Cl,6-$CH_3$-Phenyl | H | H | 7/9 | $CF_3$ | Cl |
| 2-Cl,6-$CH_3$-Phenyl | H | $CH_3$ | 7 | $CF_3$ | Cl |
| 2-Cl,6-$CH_3$-Phenyl | H | H | 7/9 | F | $CF_3$ |
| 2-Cl,6-$CH_3$-Phenyl | H | $CH_3$ | 7 | F | $CF_3$ |
| 2-Cl,6-$CH_3$-Phenyl | H | H | 7/9 | $CF_3$ | F |
| 2-Cl,6-$CH_3$-Phenyl | H | $CH_3$ | 7 | $CF_3$ | F |
| 2-Cl,6-$CH_3$-Phenyl | H | H | 7/9 | $CH_3$ | $CF_3$ |
| 2-Cl,6-$CH_3$-Phenyl | H | $CH_3$ | 7 | $CH_3$ | $CF_3$ |
| 2-Cl,6-$CH_3$-Phenyl | H | H | 7/9 | $CF_3$ | $CH_3$ |
| 2-Cl,6-$CH_3$-Phenyl | H | $CH_3$ | 7 | $CF_3$ | $CH_3$ |
| 2-Cl,6-$CH_3$-Phenyl | H | H | 7/9 | $OCH_3$ | $CF_3$ |
| 2-Cl,6-$CH_3$-Phenyl | H | $CH_3$ | 7 | $OCH_3$ | $CF_3$ |

Tabelle I (Fortsetzung)

| A | $R^1$ | $R^2$ | Pos. | $R^3$ | $R^4$ |
|---|---|---|---|---|---|
| 2-Cl,6-$CH_3$-Phenyl | H | H | 7/9 | $CF_3$ | $OCH_3$ |
| 2-Cl,6-$CH_3$-Phenyl | H | $CH_3$ | 7 | $CF_3$ | $OCH_3$ |
| 2-Cl,6-$CH_3$-Phenyl | H | H | 7/9 | F | F |
| 2-Cl,6-$CH_3$-Phenyl | H | $CH_3$ | 7 | F | F |
| 2-Cl,6-$CH_3$-Phenyl | H | H | 7/9 | F | Cl |
| 2-Cl,6-$CH_3$-Phenyl | H | $CH_3$ | 7 | F | Cl |
| 2-Cl,6-$CH_3$-Phenyl | H | H | 7/9 | Cl | F |
| 2-Cl,6-$CH_3$-Phenyl | H | $CH_3$ | 7 | Cl | F |
| 2-Cl,6-$CH_3$-Phenyl | H | H | 7/9 | F | $CH_3$ |
| 2-Cl,6-$CH_3$-Phenyl | H | $CH_3$ | 7 | F | $CH_3$ |
| 2-Cl,6-$CH_3$-Phenyl | H | H | 7/9 | $CH_3$ | F |
| 2-Cl,6-$CH_3$-Phenyl | H | $CH_3$ | 7 | $CH_3$ | F |
| 2-Cl,6-$CH_3$-Phenyl | H | H | 7/9 | F | $OCH_3$ |
| 2-Cl,6-$CH_3$-Phenyl | H | $CH_3$ | 7 | F | $OCH_3$ |
| 2-Cl,6-$CH_3$-Phenyl | H | H | 7/9 | $OCH_3$ | F |
| 2-Cl,6-$CH_3$-Phenyl | H | $CH_3$ | 7 | $OCH_3$ | F |
| 2-Cl,6-$CH_3$-Phenyl | H | H | 7/9 | $N(CH_3)_2$ | Cl |
| 2-Cl,6-$CH_3$-Phenyl | H | $CH_3$ | 7 | $N(CH_3)_2$ | Cl |
| 2-Cl,6-$CH_3$-Phenyl | H | H | 7/9 | $SCH_3$ | Cl |
| 2-Cl,6-$CH_3$-Phenyl | H | $CH_3$ | 7 | $SCH_3$ | Cl |
| 2-Cl,6-$CO_2CH_3$-Phenyl | H | H | 7/9 | $CH_3$ | $CH_3$ |
| 2-Cl,6-$CO_2CH_3$-Phenyl | H | $CH_3$ | 7 | $CH_3$ | $CH_3$ |
| 2-Cl,6-$CO_2CH_3$-Phenyl | H | H | 7/9 | Cl | $CH_3$ |
| 2-Cl,6-$CO_2CH_3$-Phenyl | H | $CH_3$ | 7 | Cl | $CH_3$ |
| 2-Cl,6-$CO_2CH_3$-Phenyl | H | H | 7/9 | $CH_3$ | Cl |
| 2-Cl,6-$CO_2CH_3$-Phenyl | H | $CH_3$ | 7 | $CH_3$ | Cl |
| 2-Cl,6-$CO_2CH_3$-Phenyl | H | H | 7/9 | Cl | Cl |
| 2-Cl,6-$CO_2CH_3$-Phenyl | H | $CH_3$ | 7 | Cl | Cl |
| 2-Cl,6-$CO_2CH_3$-Phenyl | H | H | 7/9 | $OCH_3$ | $OCH_3$ |
| 2-Cl,6-$CO_2CH_3$-Phenyl | H | $CH_3$ | 7 | $OCH_3$ | $OCH_3$ |
| 2-Cl,6-$CO_2CH_3$-Phenyl | H | H | 7/9 | $CH_3$ | $OCH_3$ |
| 2-Cl,6-$CO_2CH_3$-Phenyl | H | $CH_3$ | 7 | $CH_3$ | $OCH_3$ |
| 2-Cl,6-$CO_2CH_3$-Phenyl | H | H | 7/9 | $OCH_3$ | $CH_3$ |
| 2-Cl,6-$CO_2CH_3$-Phenyl | H | $CH_3$ | 7 | $OCH_3$ | $CH_3$ |
| 2-Cl,6-$CO_2CH_3$-Phenyl | H | H | 7/9 | Cl | $OCH_3$ |
| 2-Cl,6-$CO_2CH_3$-Phenyl | H | $CH_3$ | 7 | Cl | $OCH_3$ |
| 2-Cl,6-$CO_2CH_3$-Phenyl | H | H | 7/9 | $OCH_3$ | Cl |
| 2-Cl,6-$CO_2CH_3$-Phenyl | H | $CH_3$ | 7 | $OCH_3$ | Cl |
| 2-Cl,6-$OCH_3$-Phenyl | H | H | 7/9 | $CH_3$ | $CH_3$ |
| 2-Cl,6-$OCH_3$-Phenyl | H | $CH_3$ | 7 | $CH_3$ | $CH_3$ |

Tabelle I (Fortsetzung)

| A | $R^1$ | $R^2$ | Pos. | $R^3$ | $R^4$ |
|---|---|---|---|---|---|
| 2-Cl,6-$OCH_3$-Phenyl | H | H | 7/9 | Cl | $CH_3$ |
| 2-Cl,6-$OCH_3$-Phenyl | H | $CH_3$ | 7 | Cl | $CH_3$ |
| 2-Cl,6-$OCH_3$-Phenyl | H | H | 7/9 | $CH_3$ | Cl |
| 2-Cl,6-$OCH_3$-Phenyl | H | $CH_3$ | 7 | $CH_3$ | Cl |
| 2-Cl,6-$OCH_3$-Phenyl | H | H | 7/9 | Cl | Cl |
| 2-Cl,6-$OCH_3$-Phenyl | H | $CH_3$ | 7 | Cl | Cl |
| 2-Cl,6-$OCH_3$-Phenyl | H | H | 7/9 | $OCH_3$ | $OCH_3$ |
| 2-Cl,6-$OCH_3$-Phenyl | H | $CH_3$ | 7 | $OCH_3$ | $OCH_3$ |
| 2-Cl,6-$OCH_3$-Phenyl | H | H | 7/9 | $CH_3$ | $OCH_3$ |
| 2-Cl,6-$OCH_3$-Phenyl | H | $CH_3$ | 7 | $CH_3$ | $OCH_3$ |
| 2-Cl,6-$OCH_3$-Phenyl | H | H | 7/9 | $OCH_3$ | $CH_3$ |
| 2-Cl,6-$OCH_3$-Phenyl | H | $CH_3$ | 7 | $OCH_3$ | $CH_3$ |
| 2-Cl,6-$OCH_3$-Phenyl | H | H | 7/9 | Cl | $OCH_3$ |
| 2-Cl,6-$OCH_3$-Phenyl | H | $CH_3$ | 7 | Cl | $OCH_3$ |
| 2-Cl,6-$OCH_3$-Phenyl | H | H | 7/9 | $OCH_3$ | Cl |
| 2-Cl,6-$OCH_3$-Phenyl | H | $CH_3$ | 7 | $OCH_3$ | Cl |
| 2-$OCH_3$,6-$CO_2CH_3$-Phenyl | H | H | 7/9 | $CH_3$ | $CH_3$ |
| 2-$OCH_3$,6-$CO_2CH_3$-Phenyl | H | $CH_3$ | 7 | $CH_3$ | $CH_3$ |
| 2-$OCH_3$,6-$CO_2CH_3$-Phenyl | H | H | 7/9 | Cl | $CH_3$ |
| 2-$OCH_3$,6-$CO_2CH_3$-Phenyl | H | $CH_3$ | 7 | Cl | $CH_3$ |
| 2-$OCH_3$,6-$CO_2CH_3$-Phenyl | H | H | 7/9 | $CH_3$ | Cl |
| 2-$OCH_3$,6-$CO_2CH_3$-Phenyl | H | $CH_3$ | 7 | $CH_3$ | Cl |
| 2-$OCH_3$,6-$CO_2CH_3$-Phenyl | H | H | 7/9 | Cl | Cl |
| 2-$OCH_3$,6-$CO_2CH_3$-Phenyl | H | $CH_3$ | 7 | Cl | Cl |
| 2-$OCH_3$,6-$CO_2CH_3$-Phenyl | H | H | 7/9 | $OCH_3$ | $OCH_3$ |
| 2-$OCH_3$,6-$CO_2CH_3$-Phenyl | H | $CH_3$ | 7 | $OCH_3$ | $OCH_3$ |
| 2-$OCH_3$,6-$CO_2CH_3$-Phenyl | H | H | 7/9 | $CH_3$ | $OCH_3$ |
| 2-$OCH_3$,6-$CO_2CH_3$-Phenyl | H | $CH_3$ | 7 | $CH_3$ | $OCH_3$ |
| 2-$OCH_3$,6-$CO_2CH_3$-Phenyl | H | H | 7/9 | $OCH_3$ | $CH_3$ |
| 2-$OCH_3$,6-$CO_2CH_3$-Phenyl | H | $CH_3$ | 7 | $OCH_3$ | $CH_3$ |
| 2-$OCH_3$,6-$CO_2CH_3$-Phenyl | H | H | 7/9 | Cl | $OCH_3$ |
| 2-$OCH_3$,6-$CO_2CH_3$-Phenyl | H | $CH_3$ | 7 | Cl | $OCH_3$ |
| 2-$OCH_3$,6-$CO_2CH_3$-Phenyl | H | H | 7/9 | $OCH_3$ | Cl |
| 2-$OCH_3$,6-$CO_2CH_3$-Phenyl | H | $CH_3$ | 7 | $OCH_3$ | Cl |
| 2-$CO_2CH_3$,6-$CH_3$-Phenyl | H | H | 7/9 | $CH_3$ | $CH_3$ |
| 2-$CO_2CH_3$,6-$CH_3$-Phenyl | H | $CH_3$ | 7 | $CH_3$ | $CH_3$ |
| 2-$CO_2CH_3$,6-$CH_3$-Phenyl | H | H | 7/9 | Cl | $CH_3$ |
| 2-$CO_2CH_3$,6-$CH_3$-Phenyl | H | $CH_3$ | 7 | Cl | $CH_3$ |
| 2-$CO_2CH_3$,6-$CH_3$-Phenyl | H | H | 7/9 | $CH_3$ | Cl |
| 2-$CO_2CH_3$,6-$CH_3$-Phenyl | H | $CH_3$ | 7 | $CH_3$ | Cl |

Tabelle I (Fortsetzung)

| A | $R^1$ | $R^2$ | Pos. | $R^3$ | $R^4$ |
|---|---|---|---|---|---|
| 2-$CO_2CH_3$,6-$CH_3$-Phenyl | H | H | 7/9 | Cl | Cl |
| 2-$CO_2CH_3$,6-$CH_3$-Phenyl | H | $CH_3$ | 7 | Cl | Cl |
| 2-$CO_2CH_3$,6-$CH_3$-Phenyl | H | H | 7/9 | $OCH_3$ | $OCH_3$ |
| 2-$CO_2CH_3$,6-$CH_3$-Phenyl | H | $CH_3$ | 7 | $OCH_3$ | $OCH_3$ |
| 2-$CO_2CH_3$,6-$CH_3$-Phenyl | H | H | 7/9 | $CH_3$ | $OCH_3$ |
| 2-$CO_2CH_3$,6-$CH_3$-Phenyl | H | $CH_3$ | 7 | $CH_3$ | $OCH_3$ |
| 2-$CO_2CH_3$,6-$CH_3$-Phenyl | H | H | 7/9 | $OCH_3$ | $CH_3$ |
| 2-$CO_2CH_3$,6-$CH_3$-Phenyl | H | $CH_3$ | 7 | $OCH_3$ | $CH_3$ |
| 2-$CO_2CH_3$,6-$CH_3$-Phenyl | H | H | 7/9 | Cl | $OCH_3$ |
| 2-$CO_2CH_3$,6-$CH_3$-Phenyl | H | $CH_3$ | 7 | Cl | $OCH_3$ |
| 2-$CO_2CH_3$,6-$CH_3$-Phenyl | H | H | 7/9 | $OCH_3$ | Cl |
| 2-$CO_2CH_3$,6-$CH_3$-Phenyl | H | $CH_3$ | 7 | $OCH_3$ | Cl |
| 2,5-Cl,Cl-Phenyl | H | H | 7/9 | $CH_3$ | $CH_3$ |
| 2,5-Cl,Cl-Phenyl | H | $CH_3$ | 7 | $CH_3$ | $CH_3$ |
| 2,5-Cl,Cl-Phenyl | H | H | 7/9 | Cl | $CH_3$ |
| 2,5-Cl,Cl-Phenyl | H | $CH_3$ | 7 | Cl | $CH_3$ |
| 2,5-Cl,Cl-Phenyl | H | H | 7/9 | $CH_3$ | Cl |
| 2,5-Cl,Cl-Phenyl | H | $CH_3$ | 7 | $CH_3$ | Cl |
| 2,5-Cl,Cl-Phenyl | H | H | 7/9 | Cl | Cl |
| 2,5-Cl,Cl-Phenyl | H | $CH_3$ | 7 | Cl | Cl |
| 2,5-Cl,Cl-Phenyl | H | H | 7/9 | $OCH_3$ | $OCH_3$ |
| 2,5-Cl,Cl-Phenyl | H | $CH_3$ | 7 | $OCH_3$ | $OCH_3$ |
| 2,5-Cl,Cl-Phenyl | H | H | 7/9 | $CH_3$ | $OCH_3$ |
| 2,5-Cl,Cl-Phenyl | H | $CH_3$ | 7 | $CH_3$ | $OCH_3$ |
| 2,5-Cl,Cl-Phenyl | H | H | 7/9 | $OCH_3$ | $CH_3$ |
| 2,5-Cl,Cl-Phenyl | H | $CH_3$ | 7 | $OCH_3$ | $CH_3$ |
| 2,5-Cl,Cl-Phenyl | H | H | 7/9 | Cl | $OCH_3$ |
| 2,5-Cl,Cl-Phenyl | H | $CH_3$ | 7 | Cl | $OCH_3$ |
| 2,5-Cl,Cl-Phenyl | H | H | 7/9 | $OCH_3$ | Cl |
| 2,5-Cl,Cl-Phenyl | H | $CH_3$ | 7 | $OCH_3$ | Cl |
| 2,5-Cl,Cl-Phenyl | $CH_3$ | H | 7/9 | $CH_3$ | $CH_3$ |
| 2,5-Cl,Cl-Phenyl | $CH_3$ | $CH_3$ | 7 | $CH_3$ | $CH_3$ |
| 2,5-Cl,Cl-Phenyl | $CH_3$ | H | 7/9 | Cl | $CH_3$ |
| 2,5-Cl,Cl-Phenyl | $CH_3$ | $CH_3$ | 7 | Cl | $CH_3$ |
| 2,5-Cl,Cl-Phenyl | $CH_3$ | H | 7/9 | $CH_3$ | Cl |
| 2,5-Cl,Cl-Phenyl | $CH_3$ | $CH_3$ | 7 | $CH_3$ | Cl |
| 2,5-Cl,Cl-Phenyl | $CH_3$ | H | 7/9 | Cl | Cl |
| 2,5-Cl,Cl-Phenyl | $CH_3$ | $CH_3$ | 7 | Cl | Cl |
| 2,5-Cl,Cl-Phenyl | $CH_3$ | H | 7/9 | $OCH_3$ | $OCH_3$ |
| 2,5-Cl,Cl-Phenyl | $CH_3$ | $CH_3$ | 7 | $OCH_3$ | $OCH_3$ |

Tabelle I (Fortsetzung)

| A | $R^1$ | $R^2$ | Pos. | $R^3$ | $R^4$ |
|---|---|---|---|---|---|
| 2,5-Cl,Cl-Phenyl | $CH_3$ | H | 7/9 | $CH_3$ | $OCH_3$ |
| 2,5-Cl,Cl-Phenyl | $CH_3$ | $CH_3$ | 7 | $CH_3$ | $OCH_3$ |
| 2,5-Cl,Cl-Phenyl | $CH_3$ | H | 7/9 | $OCH_3$ | $CH_3$ |
| 2,5-Cl,Cl-Phenyl | $CH_3$ | $CH_3$ | 7 | $OCH_3$ | $CH_3$ |
| 2,5-Cl,Cl-Phenyl | $CH_3$ | H | 7/9 | Cl | $OCH_3$ |
| 2,5-Cl,Cl-Phenyl | $CH_3$ | $CH_3$ | 7 | Cl | $OCH_3$ |
| 2,5-Cl,Cl-Phenyl | $CH_3$ | H | 7/9 | $OCH_3$ | Cl |
| 2,5-Cl,Cl-Phenyl | $CH_3$ | $CH_3$ | 7 | $OCH_3$ | Cl |
| 2,5-Cl,Cl-Phenyl | H | H | 7/9 | $CF_3$ | $CF_3$ |
| 2,5-Cl,Cl-Phenyl | H | $CH_3$ | 7 | $CF_3$ | $CF_3$ |
| 2,5-Cl,Cl-Phenyl | H | H | 7/9 | Cl | $CF_3$ |
| 2,5-Cl,Cl-Phenyl | H | $CH_3$ | 7 | Cl | $CF_3$ |
| 2,5-Cl,Cl-Phenyl | H | H | 7/9 | $CF_3$ | Cl |
| 2,5-Cl,Cl-Phenyl | H | $CH_3$ | 7 | $CF_3$ | Cl |
| 2,5-Cl,Cl-Phenyl | H | H | 7/9 | F | $CF_3$ |
| 2,5-Cl,Cl-Phenyl | H | $CH_3$ | 7 | F | $CF_3$ |
| 2,5-Cl,Cl-Phenyl | H | H | 7/9 | $CF_3$ | F |
| 2,5-Cl,Cl-Phenyl | H | $CH_3$ | 7 | $CF_3$ | F |
| 2,5-Cl,Cl-Phenyl | H | H | 7/9 | $CH_3$ | $CF_3$ |
| 2,5-Cl,Cl-Phenyl | H | $CH_3$ | 7 | $CH_3$ | $CF_3$ |
| 2,5-Cl,Cl-Phenyl | H | H | 7/9 | $CF_3$ | $CH_3$ |
| 2,5-Cl,Cl-Phenyl | H | $CH_3$ | 7 | $CF_3$ | $CH_3$ |
| 2,5-Cl,Cl-Phenyl | H | H | 7/9 | $OCH_3$ | $CF_3$ |
| 2,5-Cl,Cl-Phenyl | H | $CH_3$ | 7 | $OCH_3$ | $CF_3$ |
| 2,5-Cl,Cl-Phenyl | H | H | 7/9 | $CF_3$ | $OCH_3$ |
| 2,5-Cl,Cl-Phenyl | H | $CH_3$ | 7 | $CF_3$ | $OCH_3$ |
| 2,5-Cl,Cl-Phenyl | H | H | 7/9 | F | F |
| 2,5-Cl,Cl-Phenyl | H | $CH_3$ | 7 | F | F |
| 2,5-Cl,Cl-Phenyl | H | H | 7/9 | F | Cl |
| 2,5-Cl,Cl-Phenyl | H | $CH_3$ | 7 | F | Cl |
| 2,5-Cl,Cl-Phenyl | H | H | 7/9 | Cl | F |
| 2,5-Cl,Cl-Phenyl | H | $CH_3$ | 7 | Cl | F |
| 2,5-Cl,Cl-Phenyl | H | H | 7/9 | F | $CH_3$ |
| 2,5-Cl,Cl-Phenyl | H | $CH_3$ | 7 | F | $CH_3$ |
| 2,5-Cl,Cl-Phenyl | H | H | 7/9 | $CH_3$ | F |
| 2,5-Cl,Cl-Phenyl | H | $CH_3$ | 7 | $CH_3$ | F |
| 2,5-Cl,Cl-Phenyl | H | H | 7/9 | F | $OCH_3$ |
| 2,5-Cl,Cl-Phenyl | H | $CH_3$ | 7 | F | $OCH_3$ |
| 2,5-Cl,Cl-Phenyl | H | H | 7/9 | $OCH_3$ | F |
| 2,5-Cl,Cl-Phenyl | H | $CH_3$ | 7 | $OCH_3$ | F |

Tabelle I (Fortsetzung)

| A | $R^1$ | $R^2$ | Pos. | $R^3$ | $R^4$ |
|---|---|---|---|---|---|
| 2,5-Cl,Cl-Phenyl | H | H | 7/9 | $N(CH_3)_2$ | Cl |
| 2,5-Cl,Cl-Phenyl | H | $CH_3$ | 7 | $N(CH_3)_2$ | Cl |
| 2,5-Cl,Cl-Phenyl | H | H | 7/9 | $SCH_3$ | Cl |
| 2,5-Cl,Cl-Phenyl | H | $CH_3$ | 7 | $SCH_3$ | Cl |
| 2-$OCH_3$,5-Br-Phenyl | H | H | 7/9 | $CH_3$ | $CH_3$ |
| 2-$OCH_3$,5-Br-Phenyl | H | $CH_3$ | 7 | $CH_3$ | $CH_3$ |
| 2-$OCH_3$,5-Br-Phenyl | H | H | 7/9 | Cl | $CH_3$ |
| 2-$OCH_3$,5-Br-Phenyl | H | $CH_3$ | 7 | Cl | $CH_3$ |
| 2-$OCH_3$,5-Br-Phenyl | H | H | 7/9 | $CH_3$ | Cl |
| 2-$OCH_3$,5-Br-Phenyl | H | $CH_3$ | 7 | $CH_3$ | Cl |
| 2-$OCH_3$,5-Br-Phenyl | H | H | 7/9 | Cl | Cl |
| 2-$OCH_3$,5-Br-Phenyl | H | $CH_3$ | 7 | Cl | Cl |
| 2-$OCH_3$,5-Br-Phenyl | H | H | 7/9 | $OCH_3$ | $OCH_3$ |
| 2-$OCH_3$,5-Br-Phenyl | H | $CH_3$ | 7 | $OCH_3$ | $OCH_3$ |
| 2-$OCH_3$,5-Br-Phenyl | H | H | 7/9 | $CH_3$ | $OCH_3$ |
| 2-$OCH_3$,5-Br-Phenyl | H | $CH_3$ | 7 | $CH_3$ | $OCH_3$ |
| 2-$OCH_3$,5-Br-Phenyl | H | H | 7/9 | $OCH_3$ | $CH_3$ |
| 2-$OCH_3$,5-Br-Phenyl | H | $CH_3$ | 7 | $OCH_3$ | $CH_3$ |
| 2-$OCH_3$,5-Br-Phenyl | H | H | 7/9 | Cl | $OCH_3$ |
| 2-$OCH_3$,5-Br-Phenyl | H | $CH_3$ | 7 | Cl | $OCH_3$ |
| 2-$OCH_3$,5-Br-Phenyl | H | H | 7/9 | $OCH_3$ | Cl |
| 2-$OCH_3$,5-Br-Phenyl | H | $CH_3$ | 7 | $OCH_3$ | Cl |
| 2,5-Di-$OCH_2CF_3$-Phenyl | H | H | 7/9 | $CH^3$ | $CH^3$ |
| 2,5-Di-$OCH_2CF_3$-Phenyl | H | $CH^3$ | 7 | $CH^3$ | $CH^3$ |
| 2,5-Di-$OCH_2CF_3$-Phenyl | H | H | 7/9 | Cl | $CH^3$ |
| 2,5-Di-$OCH_2CF_3$-Phenyl | H | $CH^3$ | 7 | Cl | $CH^3$ |
| 2,5-Di-$OCH_2CF_3$-Phenyl | H | H | 7/9 | $CH^3$ | Cl |
| 2,5-Di-$OCH_2CF_3$-Phenyl | H | $CH^3$ | 7 | $CH^3$ | Cl |
| 2,5-Di-$OCH_2CF_3$-Phenyl | H | H | 7/9 | Cl | Cl |
| 2,5-Di-$OCH_2CF_3$-Phenyl | H | $CH^3$ | 7 | Cl | Cl |
| 2,5-Di-$OCH_2CF_3$-Phenyl | H | H | 7/9 | $OCH^3$ | $OCH^3$ |
| 2,5-Di-$OCH_2CF_3$-Phenyl | H | $CH^3$ | 7 | $OCH^3$ | $OCH^3$ |
| 2,5-Di-$OCH_2CF_3$-Phenyl | H | H | 7/9 | $CH^3$ | $OCH^3$ |
| 2,5-Di-$OCH_2CF_3$-Phenyl | H | $CH^3$ | 7 | $CH^3$ | $OCH^3$ |
| 2,5-Di-$OCH_2CF_3$-Phenyl | H | H | 7/9 | $OCH^3$ | $CH^3$ |
| 2,5-Di-$OCH_2CF_3$-Phenyl | H | $CH^3$ | 7 | $OCH^3$ | $CH^3$ |
| 2,5-Di-$OCH_2CF_3$-Phenyl | H | H | 7/9 | Cl | $OCH^3$ |
| 2,5-Di-$OCH_2CF_3$-Phenyl | H | $CH^3$ | 7 | Cl | $OCH^3$ |
| 2,5-Di-$OCH_2CF_3$-Phenyl | H | H | 7/9 | $OCH^3$ | Cl |
| 2,5-Di-$OCH_2CF_3$-Phenyl | H | $CH_3$ | 7 | $OCH_3$ | Cl |

Tabelle I (Fortsetzung)

| A | $R^1$ | $R^2$ | Pos. | $R^3$ | $R^4$ |
|---|---|---|---|---|---|
| 2,5-Di-$OCH_3$-Phenyl | H | H | 7/9 | $CH_3$ | $CH_3$ |
| 2,5-Di-$OCH_3$-Phenyl | H | $CH_3$ | 7 | $CH_3$ | $CH_3$ |
| 2,5-Di-$OCH_3$-Phenyl | H | H | 7/9 | Cl | $CH_3$ |
| 2,5-Di-$OCH_3$-Phenyl | H | $CH_3$ | 7 | Cl | $CH_3$ |
| 2,5-Di-$OCH_3$-Phenyl | H | H | 7/9 | $CH_3$ | Cl |
| 2,5-Di-$OCH_3$-Phenyl | H | $CH_3$ | 7 | $CH_3$ | Cl |
| 2,5-Di-$OCH_3$-Phenyl | H | H | 7/9 | Cl | Cl |
| 2,5-Di-$OCH_3$-Phenyl | H | $CH_3$ | 7 | Cl | Cl |
| 2,5-Di-$OCH_3$-Phenyl | H | H | 7/9 | $OCH_3$ | $OCH_3$ |
| 2,5-Di-$OCH_3$-Phenyl | H | $CH_3$ | 7 | $OCH_3$ | $OCH_3$ |
| 2,5-Di-$OCH_3$-Phenyl | H | H | 7/9 | $CH_3$ | $OCH_3$ |
| 2,5-Di-$OCH_3$-Phenyl | H | $CH_3$ | 7 | $CH_3$ | $OCH_3$ |
| 2,5-Di-$OCH_3$-Phenyl | H | H | 7/9 | $OCH_3$ | $CH_3$ |
| 2,5-Di-$OCH_3$-Phenyl | H | $CH_3$ | 7 | $OCH_3$ | $CH_3$ |
| 2,5-Di-$OCH_3$-Phenyl | H | H | 7/9 | Cl | $OCH_3$ |
| 2,5-Di-$OCH_3$-Phenyl | H | $CH_3$ | 7 | Cl | $OCH_3$ |
| 2,5-Di-$OCH_3$-Phenyl | H | H | 7/9 | $OCH_3$ | Cl |
| 2,5-Di-$OCH_3$-Phenyl | H | $CH_3$ | 7 | $OCH_3$ | Cl |
| 2-Cl,5-$CO_2CH_3$-Phenyl | H | H | 7/9 | $CH_3$ | $CH_3$ |
| 2-Cl,5-$CO_2CH_3$-Phenyl | H | $CH_3$ | 7 | $CH_3$ | $CH_3$ |
| 2-Cl,5-$CO_2CH_3$-Phenyl | H | H | 7/9 | Cl | $CH_3$ |
| 2-Cl,5-$CO_2CH_3$-Phenyl | H | $CH_3$ | 7 | Cl | $CH_3$ |
| 2-Cl,5-$CO_2CH_3$-Phenyl | H | H | 7/9 | $CH_3$ | Cl |
| 2-Cl,5-$CO_2CH_3$-Phenyl | H | $CH_3$ | 7 | $CH_3$ | Cl |
| 2-Cl,5-$CO_2CH_3$-Phenyl | H | H | 7/9 | Cl | Cl |
| 2-Cl,5-$CO_2CH_3$-Phenyl | H | $CH_3$ | 7 | Cl | Cl |
| 2-Cl,5-$CO_2CH_3$-Phenyl | H | H | 7/9 | $OCH_3$ | $OCH_3$ |
| 2-Cl,5-$CO_2CH_3$-Phenyl | H | $CH_3$ | 7 | $OCH_3$ | $OCH_3$ |
| 2-Cl,5-$CO_2CH_3$-Phenyl | H | H | 7/9 | $CH_3$ | $OCH_3$ |
| 2-Cl,5-$CO_2CH_3$-Phenyl | H | $CH_3$ | 7 | $CH_3$ | $OCH_3$ |
| 2-Cl,5-$CO_2CH_3$-Phenyl | H | H | 7/9 | $OCH_3$ | $CH_3$ |
| 2-Cl,5-$CO_2CH_3$-Phenyl | H | $CH_3$ | 7 | $OCH_3$ | $CH_3$ |
| 2-Cl,5-$CO_2CH_3$-Phenyl | H | H | 7/9 | Cl | $OCH_3$ |
| 2-Cl,5-$CO_2CH_3$-Phenyl | H | $CH_3$ | 7 | Cl | $OCH_3$ |
| 2-Cl,5-$CO_2CH_3$-Phenyl | H | H | 7/9 | $OCH_3$ | Cl |
| 2-Cl,5-$CO_2CH_3$-Phenyl | H | $CH_3$ | 7 | $OCH_3$ | Cl |
| 2-$CH_3$,5-$OCH_3$-Phenyl | H | H | 7/9 | $CH_3$ | $CH_3$ |
| 2-$CH_3$,5-$OCH_3$-Phenyl | H | $CH_3$ | 7 | $CH_3$ | $CH_3$ |
| 2-$CH_3$,5-$OCH_3$-Phenyl | H | H | 7/9 | Cl | $CH_3$ |
| 2-$CH_3$,5-$OCH_3$-Phenyl | H | $CH_3$ | 7 | Cl | $CH_3$ |

Tabelle I (Fortsetzung)

| A | $R^1$ | $R^2$ | Pos. | $R^3$ | $R^4$ |
|---|---|---|---|---|---|
| 2-$CH_3$, 5-$OCH_3$-Phenyl | H | H | 7/9 | $CH_3$ | Cl |
| 2-$CH_3$, 5-$OCH_3$-Phenyl | H | $CH_3$ | 7 | $CH_3$ | Cl |
| 2-$CH_3$, 5-$OCH_3$-Phenyl | H | H | 7/9 | Cl | Cl |
| 2-$CH_3$, 5-$OCH_3$-Phenyl | H | $CH_3$ | 7 | Cl | Cl |
| 2-$CH_3$, 5-$OCH_3$-Phenyl | H | H | 7/9 | $OCH_3$ | $OCH_3$ |
| 2-$CH_3$, 5-$OCH_3$-Phenyl | H | $CH_3$ | 7 | $OCH_3$ | $OCH_3$ |
| 2-$CH_3$, 5-$OCH_3$-Phenyl | H | H | 7/9 | $CH_3$ | $OCH_3$ |
| 2-$CH_3$, 5-$OCH_3$-Phenyl | H | $CH_3$ | 7 | $CH_3$ | $OCH_3$ |
| 2-$CH_3$, 5-$OCH_3$-Phenyl | H | H | 7/9 | $OCH_3$ | $CH_3$ |
| 2-$CH_3$, 5-$OCH_3$-Phenyl | H | $CH_3$ | 7 | $OCH_3$ | $CH_3$ |
| 2-$CH_3$, 5-$OCH_3$-Phenyl | H | H | 7/9 | Cl | $OCH_3$ |
| 2-$CH_3$, 5-$OCH_3$-Phenyl | H | $CH_3$ | 7 | Cl | $OCH_3$ |
| 2-$CH_3$, 5-$OCH_3$-Phenyl | H | H | 7/9 | $OCH_3$ | Cl |
| 2-$CH_3$, 5-$OCH_3$-Phenyl | H | $CH_3$ | 7 | $OCH_3$ | Cl |
| 2-Cl, 5-$NO_2$-Phenyl | H | H | 7/9 | $CH_3$ | $CH_3$ |
| 2-Cl, 5-$NO_2$-Phenyl | H | $CH_3$ | 7 | $CH_3$ | $CH_3$ |
| 2-Cl, 5-$NO_2$-Phenyl | H | H | 7/9 | Cl | $CH_3$ |
| 2-Cl, 5-$NO_2$-Phenyl | H | $CH_3$ | 7 | Cl | $CH_3$ |
| 2-Cl, 5-$NO_2$-Phenyl | H | H | 7/9 | $CH_3$ | Cl |
| 2-Cl, 5-$NO_2$-Phenyl | H | $CH_3$ | 7 | $CH_3$ | Cl |
| 2-Cl, 5-$NO_2$-Phenyl | H | H | 7/9 | Cl | Cl |
| 2-Cl, 5-$NO_2$-Phenyl | H | $CH_3$ | 7 | Cl | Cl |
| 2-Cl, 5-$NO_2$-Phenyl | H | H | 7/9 | $OCH_3$ | $OCH_3$ |
| 2-Cl, 5-$NO_2$-Phenyl | H | $CH_3$ | 7 | $OCH_3$ | $OCH_3$ |
| 2-Cl, 5-$NO_2$-Phenyl | H | H | 7/9 | $CH_3$ | $OCH_3$ |
| 2-Cl, 5-$NO_2$-Phenyl | H | $CH_3$ | 7 | $CH_3$ | $OCH_3$ |
| 2-Cl, 5-$NO_2$-Phenyl | H | H | 7/9 | $OCH_3$ | $CH_3$ |
| 2-Cl, 5-$NO_2$-Phenyl | H | $CH_3$ | 7 | $OCH_3$ | $CH_3$ |
| 2-Cl, 5-$NO_2$-Phenyl | H | H | 7/9 | Cl | $OCH_3$ |
| 2-Cl, 5-$NO_2$-Phenyl | H | $CH_3$ | 7 | Cl | $OCH_3$ |
| 2-Cl, 5-$NO_2$-Phenyl | H | H | 7/9 | $OCH_3$ | Cl |
| 2-Cl, 5-$NO_2$-Phenyl | H | $CH_3$ | 7 | $OCH_3$ | Cl |
| 2-Cl, 5-$CH_3$-Phenyl | H | H | 7/9 | $CH_3$ | $CH_3$ |
| 2-Cl, 5-$CH_3$-Phenyl | H | $CH_3$ | 7 | $CH_3$ | $CH_3$ |
| 2-Cl, 5-$CH_3$-Phenyl | H | H | 7/9 | Cl | $CH_3$ |
| 2-Cl, 5-$CH_3$-Phenyl | H | $CH_3$ | 7 | Cl | $CH_3$ |
| 2-Cl, 5-$CH_3$-Phenyl | H | H | 7/9 | $CH_3$ | Cl |
| 2-Cl, 5-$CH_3$-Phenyl | H | $CH_3$ | 7 | $CH_3$ | Cl |
| 2-Cl, 5-$CH_3$-Phenyl | H | H | 7/9 | Cl | Cl |
| 2-Cl, 5-$CH_3$-Phenyl | H | $CH_3$ | 7 | Cl | Cl |

Tabelle I (Fortsetzung)

| A | $R^1$ | $R^2$ | Pos. | $R^3$ | $R^4$ |
|---|---|---|---|---|---|
| 2-Cl,5-$CH_3$-Phenyl | H | H | 7/9 | $OCH_3$ | $OCH_3$ |
| 2-Cl,5-$CH_3$-Phenyl | H | $CH_3$ | 7 | $OCH_3$ | $OCH_3$ |
| 2-Cl,5-$CH_3$-Phenyl | H | H | 7/9 | $CH_3$ | $OCH_3$ |
| 2-Cl,5-$CH_3$-Phenyl | H | $CH_3$ | 7 | $CH_3$ | $OCH_3$ |
| 2-Cl,5-$CH_3$-Phenyl | H | H | 7/9 | $OCH_3$ | $CH_3$ |
| 2-Cl,5-$CH_3$-Phenyl | H | $CH_3$ | 7 | $OCH_3$ | $CH_3$ |
| 2-Cl,5-$CH_3$-Phenyl | H | H | 7/9 | Cl | $OCH_3$ |
| 2-Cl,5-$CH_3$-Phenyl | H | $CH_3$ | 7 | Cl | $OCH_3$ |
| 2-Cl,5-$CH_3$-Phenyl | H | H | 7/9 | $OCH_3$ | Cl |
| 2-Cl,5-$CH_3$-Phenyl | H | $CH_3$ | 7 | $OCH_3$ | Cl |
| 2,5-Di-$CH_3$-Phenyl | H | H | 7/9 | $CH_3$ | $CH_3$ |
| 2,5-Di-$CH_3$-Phenyl | H | $CH_3$ | 7 | $CH_3$ | $CH_3$ |
| 2,5-Di-$CH_3$-Phenyl | H | H | 7/9 | Cl | $CH_3$ |
| 2,5-Di-$CH_3$-Phenyl | H | $CH_3$ | 7 | Cl | $CH_3$ |
| 2,5-Di-$CH_3$-Phenyl | H | H | 7/9 | $CH_3$ | Cl |
| 2,5-Di-$CH_3$-Phenyl | H | $CH_3$ | 7 | $CH_3$ | Cl |
| 2,5-Di-$CH_3$-Phenyl | H | H | 7/9 | Cl | Cl |
| 2,5-Di-$CH_3$-Phenyl | H | $CH_3$ | 7 | Cl | Cl |
| 2,5-Di-$CH_3$-Phenyl | H | H | 7/9 | $OCH_3$ | $OCH_3$ |
| 2,5-Di-$CH_3$-Phenyl | H | $CH_3$ | 7 | $OCH_3$ | $OCH_3$ |
| 2,5-Di-$CH_3$-Phenyl | H | H | 7/9 | $CH_3$ | $OCH_3$ |
| 2,5-Di-$CH_3$-Phenyl | H | $CH_3$ | 7 | $CH_3$ | $OCH_3$ |
| 2,5-Di-$CH_3$-Phenyl | H | H | 7/9 | $OCH_3$ | $CH_3$ |
| 2,5-Di-$CH_3$-Phenyl | H | $CH_3$ | 7 | $OCH_3$ | $CH_3$ |
| 2,5-Di-$CH_3$-Phenyl | H | H | 7/9 | Cl | $OCH_3$ |
| 2,5-Di-$CH_3$-Phenyl | H | $CH_3$ | 7 | Cl | $OCH_3$ |
| 2,5-Di-$CH_3$-Phenyl | H | H | 7/9 | $OCH_3$ | Cl |
| 2,5-Di-$CH_3$-Phenyl | H | $CH_3$ | 7 | $OCH_3$ | Cl |
| 2,3-Cl,Cl-Phenyl | H | H | 7/9 | $CH_3$ | $CH_3$ |
| 2,3-Cl,Cl-Phenyl | H | $CH_3$ | 7 | $CH_3$ | $CH_3$ |
| 2,3-Cl,Cl-Phenyl | H | H | 7/9 | Cl | $CH_3$ |
| 2,3-Cl,Cl-Phenyl | H | $CH_3$ | 7 | Cl | $CH_3$ |
| 2,3-Cl,Cl-Phenyl | H | H | 7/9 | $CH_3$ | Cl |
| 2,3-Cl,Cl-Phenyl | H | $CH_3$ | 7 | $CH_3$ | Cl |
| 2,3-Cl,Cl-Phenyl | H | H | 7/9 | Cl | Cl |
| 2,3-Cl,Cl-Phenyl | H | $CH_3$ | 7 | Cl | Cl |
| 2,3-Cl,Cl-Phenyl | H | H | 7/9 | $OCH_3$ | $OCH_3$ |
| 2,3-Cl,Cl-Phenyl | H | $CH_3$ | 7 | $OCH_3$ | $OCH_3$ |
| 2,3-Cl,Cl-Phenyl | H | H | 7/9 | $CH_3$ | $OCH_3$ |
| 2,3-Cl,Cl-Phenyl | H | $CH_3$ | 7 | $CH_3$ | $OCH_3$ |

Tabelle I (Fortsetzung)

| A | $R^1$ | $R^2$ | Pos. | $R^3$ | $R^4$ |
|---|---|---|---|---|---|
| 2,3-Cl,Cl-Phenyl | H | H | 7/9 | $OCH_3$ | $CH_3$ |
| 2,3-Cl,Cl-Phenyl | H | $CH_3$ | 7 | $OCH_3$ | $CH_3$ |
| 2,3-Cl,Cl-Phenyl | H | H | 7/9 | Cl | $OCH_3$ |
| 2,3-Cl,Cl-Phenyl | H | $CH_3$ | 7 | Cl | $OCH_3$ |
| 2,3-Cl,Cl-Phenyl | H | H | 7/9 | $OCH_3$ | Cl |
| 2,3-Cl,Cl-Phenyl | H | $CH_3$ | 7 | $OCH_3$ | Cl |
| 2-$CO_2CH_3$,3-Cl-Phenyl | H | H | 7/9 | $CH_3$ | $CH_3$ |
| 2-$CO_2CH_3$,3-Cl-Phenyl | H | $CH_3$ | 7 | $CH_3$ | $CH_3$ |
| 2-$CO_2CH_3$,3-Cl-Phenyl | H | H | 7/9 | Cl | $CH_3$ |
| 2-$CO_2CH_3$,3-Cl-Phenyl | H | $CH_3$ | 7 | $CH_3$ | $CH_3$ |
| 2-$CO_2CH_3$,3-Cl-Phenyl | H | H | 7/9 | $CH_3$ | Cl |
| 2-$CO_2CH_3$,3-Cl-Phenyl | H | $CH_3$ | 7 | $CH_3$ | Cl |
| 2-$CO_2CH_3$,3-Cl-Phenyl | H | H | 7/9 | Cl | Cl |
| 2-$CO_2CH_3$,3-Cl-Phenyl | H | $CH_3$ | 7 | Cl | Cl |
| 2-$CO_2CH_3$,3-Cl-Phenyl | H | H | 7/9 | $OCH_3$ | $OCH_3$ |
| 2-$CO_2CH_3$,3-Cl-Phenyl | H | $CH_3$ | 7 | $OCH_3$ | $OCH_3$ |
| 2-$CO_2CH_3$,3-Cl-Phenyl | H | H | 7/9 | $CH_3$ | $OCH_3$ |
| 2-$CO_2CH_3$,3-Cl-Phenyl | H | $CH_3$ | 7 | $CH_3$ | $OCH_3$ |
| 2-$CO_2CH_3$,3-Cl-Phenyl | H | H | 7/9 | $OCH_3$ | $CH_3$ |
| 2-$CO_2CH_3$,3-Cl-Phenyl | H | $CH_3$ | 7 | $OCH_3$ | $CH_3$ |
| 2-$CO_2CH_3$,3-Cl-Phenyl | H | H | 7/9 | Cl | $OCH_3$ |
| 2-$CO_2CH_3$,3-Cl-Phenyl | H | $CH_3$ | 7 | Cl | $OCH_3$ |
| 2-$CO_2CH_3$,3-Cl-Phenyl | H | H | 7/9 | $OCH_3$ | Cl |
| 2-$CO_2CH_3$,3-Cl-Phenyl | H | $CH_3$ | 7 | $OCH_3$ | Cl |
| 2-$CON(CH_3)_2$,3-Cl-Phenyl | H | H | 7/9 | $CH_3$ | $CH_3$ |
| 2-$CON(CH_3)_2$,3-Cl-Phenyl | H | $CH_3$ | 7 | $CH_3$ | $CH_3$ |
| 2-$CON(CH_3)_2$,3-Cl-Phenyl | H | H | 7/9 | Cl | $CH_3$ |
| 2-$CON(CH_3)_2$,3-Cl-Phenyl | H | $CH_3$ | 7 | Cl | $CH_3$ |
| 2-$CON(CH_3)_2$,3-Cl-Phenyl | H | H | 7/9 | $CH_3$ | Cl |
| 2-$CON(CH_3)_2$,3-Cl-Phenyl | H | $CH_3$ | 7 | $CH_3$ | Cl |
| 2-$CON(CH_3)_2$,3-Cl-Phenyl | H | H | 7/9 | Cl | Cl |
| 2-$CON(CH_3)_2$,3-Cl-Phenyl | H | $CH_3$ | 7 | Cl | Cl |
| 2-$CON(CH_3)_2$,3-Cl-Phenyl | H | H | 7/9 | $OCH_3$ | $OCH_3$ |
| 2-$CON(CH_3)_2$,3-Cl-Phenyl | H | $CH_3$ | 7 | $OCH_3$ | $OCH_3$ |
| 2-$CON(CH_3)_2$,3-Cl-Phenyl | H | H | 7/9 | $CH_3$ | $OCH_3$ |
| 2-$CON(CH_3)_2$,3-Cl-Phenyl | H | $CH_3$ | 7 | $CH_3$ | $OCH_3$ |
| 2-$CON(CH_3)_2$,3-Cl-Phenyl | H | H | 7/9 | $OCH_3$ | $CH_3$ |
| 2-$CON(CH_3)_2$,3-Cl-Phenyl | H | $CH_3$ | 7 | $OCH_3$ | $CH_3$ |
| 2-$CON(CH_3)_2$,3-Cl-Phenyl | H | H | 7/9 | Cl | $OCH_3$ |
| 2-$CON(CH_3)_2$,3-Cl-Phenyl | H | $CH_3$ | 7 | Cl | $OCH_3$ |

Tabelle I (Fortsetzung)

| A | $R^1$ | $R^2$ | Pos. | $R^3$ | $R^4$ |
|---|---|---|---|---|---|
| 2-$CON(CH_3)_2$, 3-Cl-Phenyl | H | H | 7/9 | $OCH_3$ | Cl |
| 2-$CON(CH_3)_2$, 3-Cl-Phenyl | H | $CH_3$ | 7 | $OCH_3$ | Cl |
| 2-$CO_2CH_3$, 3-F-Phenyl | H | H | 7/9 | $CH_3$ | $CH_3$ |
| 2-$CO_2CH_3$, 3-F-Phenyl | H | $CH_3$ | 7 | $CH_3$ | $CH_3$ |
| 2-$CO_2CH_3$, 3-F-Phenyl | H | H | 7/9 | Cl | $CH_3$ |
| 2-$CO_2CH_3$, 3-F-Phenyl | H | $CH_3$ | 7 | Cl | $CH_3$ |
| 2-$CO_2CH_3$, 3-F-Phenyl | H | H | 7/9 | $CH_3$ | Cl |
| 2-$CO_2CH_3$, 3-F-Phenyl | H | $CH_3$ | 7 | $CH_3$ | Cl |
| 2-$CO_2CH_3$, 3-F-Phenyl | H | H | 7/9 | Cl | Cl |
| 2-$CO_2CH_3$, 3-F-Phenyl | H | $CH_3$ | 7 | Cl | Cl |
| 2-$CO_2CH_3$, 3-F-Phenyl | H | H | 7/9 | $OCH_3$ | $OCH_3$ |
| 2-$CO_2CH_3$, 3-F-Phenyl | H | $CH_3$ | 7 | $OCH_3$ | $OCH_3$ |
| 2-$CO_2CH_3$, 3-F-Phenyl | H | H | 7/9 | $CH_3$ | $OCH_3$ |
| 2-$CO_2CH_3$, 3-F-Phenyl | H | $CH_3$ | 7 | $CH_3$ | $OCH_3$ |
| 2-$CO_2CH_3$, 3-F-Phenyl | H | H | 7/9 | $OCH_3$ | $CH_3$ |
| 2-$CO_2CH_3$, 3-F-Phenyl | H | $CH_3$ | 7 | $OCH_3$ | $CH_3$ |
| 2-$CO_2CH_3$, 3-F-Phenyl | H | H | 7/9 | Cl | $OCH_3$ |
| 2-$CO_2CH_3$, 3-F-Phenyl | H | $CH_3$ | 7 | Cl | $OCH_3$ |
| 2-$CO_2CH_3$, 3-F-Phenyl | H | H | 7/9 | $OCH_3$ | Cl |
| 2-$CO_2CH_3$, 3-F-Phenyl | H | $CH_3$ | 7 | $OCH_3$ | Cl |
| 2-$CO_2CH_3$, 3-F-Phenyl | $CH_3$ | H | 7/9 | $CH_3$ | $CH_3$ |
| 2-$CO_2CH_3$, 3-F-Phenyl | $CH_3$ | $CH_3$ | 7 | $CH_3$ | $CH_3$ |
| 2-$CO_2CH_3$, 3-F-Phenyl | $CH_3$ | H | 7/9 | Cl | $CH_3$ |
| 2-$CO_2CH_3$, 3-F-Phenyl | $CH_3$ | $CH_3$ | 7 | Cl | $CH_3$ |
| 2-$CO_2CH_3$, 3-F-Phenyl | $CH_3$ | H | 7/9 | $CH_3$ | Cl |
| 2-$CO_2CH_3$, 3-F-Phenyl | $CH_3$ | $CH_3$ | 7 | $CH_3$ | Cl |
| 2-$CO_2CH_3$, 3-F-Phenyl | $CH_3$ | H | 7/9 | Cl | Cl |
| 2-$CO_2CH_3$, 3-F-Phenyl | $CH_3$ | $CH_3$ | 7 | Cl | Cl |
| 2-$CO_2CH_3$, 3-F-Phenyl | $CH_3$ | H | 7/9 | $OCH_3$ | $OCH_3$ |
| 2-$CO_2CH_3$, 3-F-Phenyl | $CH_3$ | $CH_3$ | 7 | $OCH_3$ | $OCH_3$ |
| 2-$CO_2CH_3$, 3-F-Phenyl | $CH_3$ | H | 7/9 | $CH_3$ | $OCH_3$ |
| 2-$CO_2CH_3$, 3-F-Phenyl | $CH_3$ | $CH_3$ | 7 | $CH_3$ | $OCH_3$ |
| 2-$CO_2CH_3$, 3-F-Phenyl | $CH_3$ | H | 7/9 | $OCH_3$ | $CH_3$ |
| 2-$CO_2CH_3$, 3-F-Phenyl | $CH_3$ | $CH_3$ | 7 | $OCH_3$ | $CH_3$ |
| 2-$CO_2CH_3$, 3-F-Phenyl | $CH_3$ | H | 7/9 | Cl | $OCH_3$ |
| 2-$CO_2CH_3$, 3-F-Phenyl | $CH_3$ | $CH_3$ | 7 | Cl | $OCH_3$ |
| 2-$CO_2CH_3$, 3-F-Phenyl | $CH_3$ | H | 7/9 | $OCH_3$ | Cl |
| 2-$CO_2CH_3$, 3-F-Phenyl | $CH_3$ | $CH_3$ | 7 | $OCH_3$ | Cl |
| 2-$CO_2CH_3$, 3-F-Phenyl | H | H | 7/9 | $CF_3$ | $CF_3$ |
| 2-$CO_2CH_3$, 3-F-Phenyl | H | $CH_3$ | 7 | $CF_3$ | $CF_3$ |

Tabelle I (Fortsetzung)

| A | $R^1$ | $R^2$ | Pos. | $R^3$ | $R^4$ |
|---|---|---|---|---|---|
| 2-$CO_2CH_3$, 3-F-Phenyl | H | H | 7/9 | Cl | $CF_3$ |
| 2-$CO_2CH_3$, 3-F-Phenyl | H | $CH_3$ | 7 | Cl | $CF_3$ |
| 2-$CO_2CH_3$, 3-F-Phenyl | H | H | 7/9 | $CF_3$ | Cl |
| 2-$CO_2CH_3$, 3-F-Phenyl | H | $CH_3$ | 7 | $CF_3$ | Cl |
| 2-$CO_2CH_3$, 3-F-Phenyl | H | H | 7/9 | F | $CF_3$ |
| 2-$CO_2CH_3$, 3-F-Phenyl | H | $CH_3$ | 7 | F | $CF_3$ |
| 2-$CO_2CH_3$, 3-F-Phenyl | H | H | 7/9 | $CF_3$ | F |
| 2-$CO_2CH_3$, 3-F-Phenyl | H | $CH_3$ | 7 | $CF_3$ | F |
| 2-$CO_2CH_3$, 3-F-Phenyl | H | H | 7/9 | $CH_3$ | $CF_3$ |
| 2-$CO_2CH_3$, 3-F-Phenyl | H | $CH_3$ | 7 | $CH_3$ | $CF_3$ |
| 2-$CO_2CH_3$, 3-F-Phenyl | H | H | 7/9 | $CF_3$ | $CH_3$ |
| 2-$CO_2CH_3$, 3-F-Phenyl | H | $CH_3$ | 7 | $CF_3$ | $CH_3$ |
| 2-$CO_2CH_3$, 3-F-Phenyl | H | H | 7/9 | $OCH_3$ | $CF_3$ |
| 2-$CO_2CH_3$, 3-F-Phenyl | H | $CH_3$ | 7 | $OCH_3$ | $CF_3$ |
| 2-$CO_2CH_3$, 3-F-Phenyl | H | H | 7/9 | $CF_3$ | $OCH_3$ |
| 2-$CO_2CH_3$, 3-F-Phenyl | H | $CH_3$ | 7 | $CF_3$ | $OCH_3$ |
| 2-$CO_2CH_3$, 3-F-Phenyl | H | H | 7/9 | F | F |
| 2-$CO_2CH_3$, 3-F-Phenyl | H | $CH_3$ | 7 | F | F |
| 2-$CO_2CH_3$, 3-F-Phenyl | H | H | 7/9 | F | Cl |
| 2-$CO_2CH_3$, 3-F-Phenyl | H | $CH_3$ | 7 | F | Cl |
| 2-$CO_2CH_3$, 3-F-Phenyl | H | H | 7/9 | Cl | F |
| 2-$CO_2CH_3$, 3-F-Phenyl | H | $CH_3$ | 7 | Cl | F |
| 2-$CO_2CH_3$, 3-F-Phenyl | H | H | 7/9 | F | $CH_3$ |
| 2-$CO_2CH_3$, 3-F-Phenyl | H | $CH_3$ | 7 | F | $CH_3$ |
| 2-$CO_2CH_3$, 3-F-Phenyl | H | H | 7/9 | $CH_3$ | F |
| 2-$CO_2CH_3$, 3-F-Phenyl | H | $CH_3$ | 7 | $CH_3$ | F |
| 2-$CO_2CH_3$, 3-F-Phenyl | H | H | 7/9 | F | $OCH_3$ |
| 2-$CO_2CH_3$, 3-F-Phenyl | H | $CH_3$ | 7 | F | $OCH_3$ |
| 2-$CO_2CH_3$, 3-F-Phenyl | H | H | 7/9 | $OCH_3$ | F |
| 2-$CO_2CH_3$, 3-F-Phenyl | H | $CH_3$ | 7 | $OCH_3$ | F |
| 2-$CO_2CH_3$, 3-F-Phenyl | H | H | 7/9 | $N(CH_3)_2$ | Cl |
| 2-$CO_2CH_3$, 3-F-Phenyl | H | $CH_3$ | 7 | $N(CH_3)_2$ | Cl |
| 2-$CO_2CH_3$, 3-F-Phenyl | H | H | 7/9 | $SCH_3$ | Cl |
| 2-$CO_2CH_3$, 3-F-Phenyl | H | $CH_3$ | 7 | $SCH_3$ | Cl |
| 2-$CON(CH_3)_2$, 3-F-Phenyl | H | H | 7/9 | $CH_3$ | $CH_3$ |
| 2-$CON(CH_3)_2$, 3-F-Phenyl | H | $CH_3$ | 7 | $CH_3$ | $CH_3$ |
| 2-$CON(CH_3)_2$, 3-F-Phenyl | H | H | 7/9 | Cl | $CH_3$ |
| 2-$CON(CH_3)_2$, 3-F-Phenyl | H | $CH_3$ | 7 | Cl | $CH_3$ |
| 2-$CON(CH_3)_2$, 3-F-Phenyl | H | H | 7/9 | $CH_3$ | Cl |
| 2-$CON(CH_3)_2$, 3-F-Phenyl | H | $CH_3$ | 7 | $CH_3$ | Cl |

Tabelle I (Fortsetzung)

| A | $R^1$ | $R^2$ | Pos. | $R^3$ | $R^4$ |
|---|---|---|---|---|---|
| 2-CON($CH_3$)$_2$,3-F-Phenyl | H | H | 7/9 | Cl | Cl |
| 2-CON($CH_3$)$_2$,3-F-Phenyl | H | $CH_3$ | 7 | Cl | Cl |
| 2-CON($CH_3$)$_2$,3-F-Phenyl | H | H | 7/9 | $OCH_3$ | $OCH_3$ |
| 2-CON($CH_3$)$_2$,3-F-Phenyl | H | $CH_3$ | 7 | $OCH_3$ | $OCH_3$ |
| 2-CON($CH_3$)$_2$,3-F-Phenyl | H | H | 7/9 | $CH_3$ | $OCH_3$ |
| 2-CON($CH_3$)$_2$,3-F-Phenyl | H | $CH_3$ | 7 | $CH_3$ | $OCH_3$ |
| 2-CON($CH_3$)$_2$,3-F-Phenyl | H | H | 7/9 | $OCH_3$ | $CH_3$ |
| 2-CON($CH_3$)$_2$,3-F-Phenyl | H | $CH_3$ | 7 | $OCH_3$ | $CH_3$ |
| 2-CON($CH_3$)$_2$,3-F-Phenyl | H | H | 7/9 | Cl | $OCH_3$ |
| 2-CON($CH_3$)$_2$,3-F-Phenyl | H | $CH_3$ | 7 | Cl | $OCH_3$ |
| 2-CON($CH_3$)$_2$,3-F-Phenyl | H | H | 7/9 | $OCH_3$ | Cl |
| 2-CON($CH_3$)$_2$,3-F-Phenyl | H | $CH_3$ | 7 | $OCH_3$ | Cl |
| 2,5,6-Cl,Cl,Cl-Phenyl | $CH_3$ | H | 7/9 | $CH_3$ | $CH_3$ |
| 2,5,6-Cl,Cl,Cl-Phenyl | $CH_3$ | $CH_3$ | 7 | $CH_3$ | $CH_3$ |
| 2,5,6-Cl,Cl,Cl-Phenyl | $CH_3$ | H | 7/9 | Cl | $CH_3$ |
| 2,5,6-Cl,Cl,Cl-Phenyl | $CH_3$ | $CH_3$ | 7 | Cl | $CH_3$ |
| 2,5,6-Cl,Cl,Cl-Phenyl | $CH_3$ | H | 7/9 | $CH_3$ | Cl |
| 2,5,6-Cl,Cl,Cl-Phenyl | $CH_3$ | $CH_3$ | 7 | $CH_3$ | Cl |
| 2,5,6-Cl,Cl,Cl-Phenyl | $CH_3$ | H | 7/9 | Cl | Cl |
| 2,5,6-Cl,Cl,Cl-Phenyl | $CH_3$ | $CH_3$ | 7 | Cl | Cl |
| 2,5,6-Cl,Cl,Cl-Phenyl | $CH_3$ | H | 7/9 | $OCH_3$ | $OCH_3$ |
| 2,5,6-Cl,Cl,Cl-Phenyl | $CH_3$ | $CH_3$ | 7 | $OCH_3$ | $OCH_3$ |
| 2,5,6-Cl,Cl,Cl-Phenyl | $CH_3$ | H | 7/9 | $CH_3$ | $OCH_3$ |
| 2,5,6-Cl,Cl,Cl-Phenyl | $CH_3$ | $CH_3$ | 7 | $CH_3$ | $OCH_3$ |
| 2,5,6-Cl,Cl,Cl-Phenyl | $CH_3$ | H | 7/9 | $OCH_3$ | $CH_3$ |
| 2,5,6-Cl,Cl,Cl-Phenyl | $CH_3$ | $CH_3$ | 7 | $OCH_3$ | $CH_3$ |
| 2,5,6-Cl,Cl,Cl-Phenyl | $CH_3$ | H | 7/9 | Cl | $OCH_3$ |
| 2,5,6-Cl,Cl,Cl-Phenyl | $CH_3$ | $CH_3$ | 7 | Cl | $OCH_3$ |
| 2,5,6-Cl,Cl,Cl-Phenyl | $CH_3$ | H | 7/9 | $OCH_3$ | Cl |
| 2,5,6-Cl,Cl,Cl-Phenyl | $CH_3$ | $CH_3$ | 7 | $OCH_3$ | Cl |
| 2,5,6-Cl,Cl,Cl-Phenyl | H | H | 7/9 | $CH_3$ | $CH_3$ |
| 2,5,6-Cl,Cl,Cl-Phenyl | H | $CH_3$ | 7 | $CH_3$ | $CH_3$ |
| 2,5,6-Cl,Cl,Cl-Phenyl | H | H | 7/9 | Cl | $CH_3$ |
| 2,5,6-Cl,Cl,Cl-Phenyl | H | $CH_3$ | 7 | Cl | $CH_3$ |
| 2,5,6-Cl,Cl,Cl-Phenyl | H | H | 7/9 | $CH_3$ | Cl |
| 2,5,6-Cl,Cl,Cl-Phenyl | H | $CH_3$ | 7 | $CH_3$ | Cl |
| 2,5,6-Cl,Cl,Cl-Phenyl | H | H | 7/9 | Cl | Cl |
| 2,5,6-Cl,Cl,Cl-Phenyl | H | $CH_3$ | 7 | Cl | Cl |
| 2,5,6-Cl,Cl,Cl-Phenyl | H | H | 7/9 | $OCH_3$ | $OCH_3$ |
| 2,5,6-Cl,Cl,Cl-Phenyl | H | $CH_3$ | 7 | $OCH_3$ | $OCH_3$ |

Tabelle I (Fortsetzung)

| A | $R^1$ | $R^2$ | Pos. | $R^3$ | $R^4$ |
|---|---|---|---|---|---|
| 2,5,6-Cl,Cl,Cl-Phenyl | H | H | 7/9 | $CH_3$ | $OCH_3$ |
| 2,5,6-Cl,Cl,Cl-Phenyl | H | $CH_3$ | 7 | $CH_3$ | $OCH_3$ |
| 2,5,6-Cl,Cl,Cl-Phenyl | H | H | 7/9 | $OCH_3$ | $CH_3$ |
| 2,5,6-Cl,Cl,Cl-Phenyl | H | $CH_3$ | 7 | $OCH_3$ | $CH_3$ |
| 2,5,6-Cl,Cl,Cl-Phenyl | H | H | 7/9 | Cl | $OCH_3$ |
| 2,5,6-Cl,Cl,Cl-Phenyl | H | $CH_3$ | 7 | Cl | $OCH_3$ |
| 2,5,6-Cl,Cl,Cl-Phenyl | H | H | 7/9 | $OCH_3$ | Cl |
| 2,5,6-Cl,Cl,Cl-Phenyl | H | $CH_3$ | 7 | $OCH_3$ | Cl |
| 2,3,5-Cl,Cl,Cl-Phenyl | H | H | 7/9 | $CH_3$ | $CH_3$ |
| 2,3,5-Cl,Cl,Cl-Phenyl | H | $CH_3$ | 7 | $CH_3$ | $CH_3$ |
| 2,3,5-Cl,Cl,Cl-Phenyl | H | H | 7/9 | Cl | $CH_3$ |
| 2,3,5-Cl,Cl,Cl-Phenyl | H | $CH_3$ | 7 | Cl | $CH_3$ |
| 2,3,5-Cl,Cl,Cl-Phenyl | H | H | 7/9 | $CH_3$ | Cl |
| 2,3,5-Cl,Cl,Cl-Phenyl | H | $CH_3$ | 7 | $CH_3$ | Cl |
| 2,3,5-Cl,Cl,Cl-Phenyl | H | H | 7/9 | Cl | Cl |
| 2,3,5-Cl,Cl,Cl-Phenyl | H | $CH_3$ | 7 | Cl | Cl |
| 2,3,5-Cl,Cl,Cl-Phenyl | H | H | 7/9 | $OCH_3$ | $OCH_3$ |
| 2,3,5-Cl,Cl,Cl-Phenyl | H | $CH_3$ | 7 | $OCH_3$ | $OCH_3$ |
| 2,3,5-Cl,Cl,Cl-Phenyl | H | H | 7/9 | $CH_3$ | $OCH_3$ |
| 2,3,5-Cl,Cl,Cl-Phenyl | H | $CH_3$ | 7 | $CH_3$ | $OCH_3$ |
| 2,3,5-Cl,Cl,Cl-Phenyl | H | H | 7/9 | $OCH_3$ | $CH_3$ |
| 2,3,5-Cl,Cl,Cl-Phenyl | H | $CH_3$ | 7 | $OCH_3$ | $CH_3$ |
| 2,3,5-Cl,Cl,Cl-Phenyl | H | H | 7/9 | Cl | $OCH_3$ |
| 2,3,5-Cl,Cl,Cl-Phenyl | H | $CH_3$ | 7 | Cl | $OCH_3$ |
| 2,3,5-Cl,Cl,Cl-Phenyl | H | H | 7/9 | $OCH_3$ | Cl |
| 2,3,5-Cl,Cl,Cl-Phenyl | H | $CH_3$ | 7 | $OCH_3$ | Cl |
| 1-Naphthyl | H | H | 7/9 | $CH_3$ | $CH_3$ |
| 1-Naphthyl | H | $CH_3$ | 7 | $CH_3$ | $CH_3$ |
| 1-Naphthyl | H | H | 7/9 | Cl | $CH_3$ |
| 1-Naphthyl | H | $CH_3$ | 7 | Cl | $CH_3$ |
| 1-Naphthyl | H | H | 7/9 | $CH_3$ | Cl |
| 1-Naphthyl | H | $CH_3$ | 7 | $CH_3$ | Cl |
| 1-Naphthyl | H | H | 7/9 | Cl | Cl |
| 1-Naphthyl | H | $CH_3$ | 7 | Cl | Cl |
| 1-Naphthyl | H | H | 7/9 | $OCH_3$ | $OCH_3$ |
| 1-Naphthyl | H | $CH_3$ | 7 | $OCH_3$ | $OCH_3$ |
| 1-Naphthyl | H | H | 7/9 | $CH_3$ | $OCH_3$ |
| 1-Naphthyl | H | $CH_3$ | 7 | $CH_3$ | $OCH_3$ |
| 1-Naphthyl | H | H | 7/9 | $OCH_3$ | $CH_3$ |
| 1-Naphthyl | H | $CH_3$ | 7 | $OCH_3$ | $CH_3$ |

Tabelle I (Fortsetzung)

| A | $R^1$ | $R^2$ | Pos. | $R^3$ | $R^4$ |
|---|---|---|---|---|---|
| 1-Naphthyl | H | H | 7/9 | Cl | $OCH_3$ |
| 1-Naphthyl | H | $CH_3$ | 7 | Cl | $OCH_3$ |
| 1-Naphthyl | H | H | 7/9 | $OCH_3$ | Cl |
| 1-Naphthyl | H | $CH_3$ | 7 | $OCH_3$ | Cl |
| 8-Cl, 1-Naphthyl | H | H | 7/9 | $CH_3$ | $CH_3$ |
| 8-Cl, 1-Naphthyl | H | $CH_3$ | 7 | $CH_3$ | $CH_3$ |
| 8-Cl, 1-Naphthyl | H | H | 7/9 | Cl | $CH_3$ |
| 8-Cl, 1-Naphthyl | H | $CH_3$ | 7 | Cl | $CH_3$ |
| 8-Cl, 1-Naphthyl | H | H | 7/9 | $CH_3$ | Cl |
| 8-Cl, 1-Naphthyl | H | $CH_3$ | 7 | $CH_3$ | Cl |
| 8-Cl, 1-Naphthyl | H | H | 7/9 | Cl | Cl |
| 8-Cl, 1-Naphthyl | H | $CH_3$ | 7 | Cl | Cl |
| 8-Cl, 1-Naphthyl | H | H | 7/9 | $OCH_3$ | $OCH_3$ |
| 8-Cl, 1-Naphthyl | H | $CH_3$ | 7 | $OCH_3$ | $OCH_3$ |
| 8-Cl, 1-Naphthyl | H | H | 7/9 | $CH_3$ | $OCH_3$ |
| 8-Cl, 1-Naphthyl | H | $CH_3$ | 7 | $CH_3$ | $OCH_3$ |
| 8-Cl, 1-Naphthyl | H | H | 7/9 | $OCH_3$ | $CH_3$ |
| 8-Cl, 1-Naphthyl | H | $CH_3$ | 7 | $OCH_3$ | $CH_3$ |
| 8-Cl, 1-Naphthyl | H | H | 7/9 | Cl | $OCH_3$ |
| 8-Cl, 1-Naphthyl | H | $CH_3$ | 7 | Cl | $OCH_3$ |
| 8-Cl, 1-Naphthyl | H | H | 7/9 | $OCH_3$ | Cl |
| 8-Cl, 1-Naphthyl | H | $CH_3$ | 7 | $OCH_3$ | Cl |
| 8-$CO_2CH_3$, 1-Naphthyl | H | H | 7/9 | $CH_3$ | $CH_3$ |
| 8-$CO_2CH_3$, 1-Naphthyl | H | $CH_3$ | 7 | $CH_3$ | $CH_3$ |
| 8-$CO_2CH_3$, 1-Naphthyl | H | H | 7/9 | Cl | $CH_3$ |
| 8-$CO_2CH_3$, 1-Naphthyl | H | $CH_3$ | 7 | Cl | $CH_3$ |
| 8-$CO_2CH_3$, 1-Naphthyl | H | H | 7/9 | $CH_3$ | Cl |
| 8-$CO_2CH_3$, 1-Naphthyl | H | $CH_3$ | 7 | $CH_3$ | Cl |
| 8-$CO_2CH_3$, 1-Naphthyl | H | H | 7/9 | Cl | Cl |
| 8-$CO_2CH_3$, 1-Naphthyl | H | $CH_3$ | 7 | Cl | Cl |
| 8-$CO_2CH_3$, 1-Naphthyl | H | H | 7/9 | $OCH_3$ | $OCH_3$ |
| 8-$CO_2CH_3$, 1-Naphthyl | H | $CH_3$ | 7 | $OCH_3$ | $OCH_3$ |
| 8-$CO_2CH_3$, 1-Naphthyl | H | H | 7/9 | $CH_3$ | $OCH_3$ |
| 8-$CO_2CH_3$, 1-Naphthyl | H | $CH_3$ | 7 | $CH_3$ | $OCH_3$ |
| 8-$CO_2CH_3$, 1-Naphthyl | H | H | 7/9 | $OCH_3$ | $CH_3$ |
| 8-$CO_2CH_3$, 1-Naphthyl | H | $CH_3$ | 7 | $OCH_3$ | $CH_3$ |
| 8-$CO_2CH_3$, 1-Naphthyl | H | H | 7/9 | Cl | $OCH_3$ |
| 8-$CO_2CH_3$, 1-Naphthyl | H | $CH_3$ | 7 | Cl | $OCH_3$ |
| 8-$CO_2CH_3$, 1-Naphthyl | H | H | 7/9 | $OCH_3$ | Cl |
| 8-$CO_2CH_3$, 1-Naphthyl | H | $CH_3$ | 7 | $OCH_3$ | Cl |
| 8-$OCH_3$, 1-Naphthyl | H | H | 7/9 | $CH_3$ | $CH_3$ |

Tabelle I (Fortsetzung)

| A | $R^1$ | $R^2$ | Pos. | $R^3$ | $R^4$ |
|---|---|---|---|---|---|
| 8-$OCH_3$, 1-Naphthyl | H | $CH_3$ | 7 | $CH_3$ | $CH_3$ |
| 8-$OCH_3$, 1-Naphthyl | H | H | 7/9 | Cl | $CH_3$ |
| 8-$OCH_3$, 1-Naphthyl | H | $CH_3$ | 7 | Cl | $CH_3$ |
| 8-$OCH_3$, 1-Naphthyl | H | H | 7/9 | $CH_3$ | Cl |
| 8-$OCH_3$, 1-Naphthyl | H | $CH_3$ | 7 | $CH_3$ | Cl |
| 8-$OCH_3$, 1-Naphthyl | H | H | 7/9 | Cl | Cl |
| 8-$OCH_3$, 1-Naphthyl | H | $CH_3$ | 7 | Cl | Cl |
| 8-$OCH_3$, 1-Naphthyl | H | H | 7/9 | $OCH_3$ | $OCH_3$ |
| 8-$OCH_3$, 1-Naphthyl | H | $CH_3$ | 7 | $OCH_3$ | $OCH_3$ |
| 8-$OCH_3$, 1-Naphthyl | H | H | 7/9 | $CH_3$ | $OCH_3$ |
| 8-$OCH_3$, 1-Naphthyl | H | $CH_3$ | 7 | $CH_3$ | $OCH_3$ |
| 8-$OCH_3$, 1-Naphthyl | H | H | 7/9 | $OCH_3$ | $CH_3$ |
| 8-$OCH_3$, 1-Naphthyl | H | $CH_3$ | 7 | $OCH_3$ | $CH_3$ |
| 8-$OCH_3$, 1-Naphthyl | H | H | 7/9 | Cl | $OCH_3$ |
| 8-$OCH_3$, 1-Naphthyl | H | $CH_3$ | 7 | Cl | $OCH_3$ |
| 8-$OCH_3$, 1-Naphthyl | H | H | 7/9 | $OCH_3$ | Cl |
| 8-$OCH_3$, 1-Naphthyl | H | $CH_3$ | 7 | $OCH_3$ | Cl |
| 8-$OCH_2CH_2OCH_3$, 1-Naphthyl | H | H | 7/9 | $CH_3$ | $CH_3$ |
| 8-$OCH_2CH_2OCH_3$, 1-Naphthyl | H | $CH_3$ | 7 | $CH_3$ | $CH_3$ |
| 8-$OCH_2CH_2OCH_3$, 1-Naphthyl | H | H | 7/9 | Cl | $CH_3$ |
| 8-$OCH_2CH_2OCH_3$, 1-Naphthyl | H | $CH_3$ | 7 | Cl | $CH_3$ |
| 8-$OCH_2CH_2OCH_3$, 1-Naphthyl | H | H | 7/9 | $CH_3$ | Cl |
| 8-$OCH_2CH_2OCH_3$, 1-Naphthyl | H | $CH_3$ | 7 | $CH_3$ | Cl |
| 8-$OCH_2CH_2OCH_3$, 1-Naphthyl | H | H | 7/9 | Cl | Cl |
| 8-$OCH_2CH_2OCH_3$, 1-Naphthyl | H | $CH_3$ | 7 | Cl | Cl |
| 8-$OCH_2CH_2OCH_3$, 1-Naphthyl | H | H | 7/9 | $OCH_3$ | $OCH_3$ |
| 8-$OCH_2CH_2OCH_3$, 1-Naphthyl | H | $CH_3$ | 7 | $OCH_3$ | $OCH_3$ |
| 8-$OCH_2CH_2OCH_3$, 1-Naphthyl | H | H | 7/9 | $CH_3$ | $OCH_3$ |
| 8-$OCH_2CH_2OCH_3$, 1-Naphthyl | H | $CH_3$ | 7 | $CH_3$ | $OCH_3$ |
| 8-$OCH_2CH_2OCH_3$, 1-Naphthyl | H | H | 7/9 | $OCH_3$ | $CH_3$ |
| 8-$OCH_2CH_2OCH_3$, 1-Naphthyl | H | $CH_3$ | 7 | $OCH_3$ | $CH_3$ |
| 8-$OCH_2CH_2OCH_3$, 1-Naphthyl | H | H | 7/9 | Cl | $OCH_3$ |
| 8-$OCH_2CH_2OCH_3$, 1-Naphthyl | H | $CH_3$ | 7 | Cl | $OCH_3$ |
| 8-$OCH_2CH_2OCH_3$, 1-Naphthyl | H | H | 7/9 | $OCH_3$ | Cl |
| 8-$OCH_2CH_2OCH_3$, 1-Naphthyl | H | $CH_3$ | 7 | $OCH_3$ | Cl |
| 8-$OCH_2CH_2Cl$, 1-Naphthyl | H | H | 7/9 | $CH_3$ | $CH_3$ |
| 8-$OCH_2CH_2Cl$, 1-Naphthyl | H | $CH_3$ | 7 | $CH_3$ | $CH_3$ |
| 8-$OCH_2CH_2Cl$, 1-Naphthyl | H | H | 7/9 | Cl | $CH_3$ |
| 8-$OCH_2CH_2Cl$, 1-Naphthyl | H | $CH_3$ | 7 | Cl | $CH_3$ |

Tabelle I (Fortsetzung)

| A | $R^1$ | $R^2$ | Pos. | $R^3$ | $R^4$ |
|---|---|---|---|---|---|
| 8-$OCH_2CH_2Cl$, 1-Naphthyl | H | H | 7/9 | $CH_3$ | Cl |
| 8-$OCH_2CH_2Cl$, 1-Naphthyl | H | $CH_3$ | 7 | $CH_3$ | Cl |
| 8-$OCH_2CH_2Cl$, 1-Naphthyl | H | H | 7/9 | Cl | Cl |
| 8-$OCH_2CH_2Cl$, 1-Naphthyl | H | $CH_3$ | 7 | Cl | Cl |
| 8-$OCH_2CH_2Cl$, 1-Naphthyl | H | H | 7/9 | $OCH_3$ | $OCH_3$ |
| 8-$OCH_2CH_2Cl$, 1-Naphthyl | H | $CH_3$ | 7 | $OCH_3$ | $OCH_3$ |
| 8-$OCH_2CH_2Cl$, 1-Naphthyl | H | H | 7/9 | $CH_3$ | $OCH_3$ |
| 8-$OCH_2CH_2Cl$, 1-Naphthyl | H | $CH_3$ | 7 | $CH_3$ | $OCH_3$ |
| 8-$OCH_2CH_2Cl$, 1-Naphthyl | H | H | 7/9 | $OCH_3$ | $CH_3$ |
| 8-$OCH_2CH_2Cl$, 1-Naphthyl | H | $CH_3$ | 7 | $OCH_3$ | $CH_3$ |
| 8-$OCH_2CH_2Cl$, 1-Naphthyl | H | H | 7/9 | Cl | $OCH_3$ |
| 8-$OCH_2CH_2Cl$, 1-Naphthyl | H | $CH_3$ | 7 | Cl | $OCH_3$ |
| 8-$OCH_2CH_2Cl$, 1-Naphthyl | H | H | 7/9 | $OCH_3$ | Cl |
| 8-$OCH_2CH_2Cl$, 1-Naphthyl | H | $CH_3$ | 7 | $OCH_3$ | Cl |
| 8-$OCH_2CO_2CH_3$, 1-Naphthyl | H | H | 7/9 | $CH_3$ | $CH_3$ |
| 8-$OCH_2CO_2CH_3$, 1-Naphthyl | H | $CH_3$ | 7 | $CH_3$ | $CH_3$ |
| 8-$OCH_2CO_2CH_3$, 1-Naphthyl | H | H | 7/9 | Cl | $CH_3$ |
| 8-$OCH_2CO_2CH_3$, 1-Naphthyl | H | $CH_3$ | 7 | Cl | $CH_3$ |
| 8-$OCH_2CO_2CH_3$, 1-Naphthyl | H | H | 7/9 | $CH_3$ | Cl |
| 8-$OCH_2CO_2CH_3$, 1-Naphthyl | H | $CH_3$ | 7 | $CH_3$ | Cl |
| 8-$OCH_2CO_2CH_3$, 1-Naphthyl | H | H | 7/9 | Cl | Cl |
| 8-$OCH_2CO_2CH_3$, 1-Naphthyl | H | $CH_3$ | 7 | Cl | Cl |
| 8-$OCH_2CO_2CH_3$, 1-Naphthyl | H | H | 7/9 | $OCH_3$ | $OCH_3$ |
| 8-$OCH_2CO_2CH_3$, 1-Naphthyl | H | $CH_3$ | 7 | $OCH_3$ | $OCH_3$ |
| 8-$OCH_2CO_2CH_3$, 1-Naphthyl | H | H | 7/9 | $CH_3$ | $OCH_3$ |
| 8-$OCH_2CO_2CH_3$, 1-Naphthyl | H | $CH_3$ | 7 | $CH_3$ | $OCH_3$ |
| 8-$OCH_2CO_2CH_3$, 1-Naphthyl | H | H | 7/9 | $OCH_3$ | $CH_3$ |
| 8-$OCH_2CO_2CH_3$, 1-Naphthyl | H | $CH_3$ | 7 | $OCH_3$ | $CH_3$ |
| 8-$OCH_2CO_2CH_3$, 1-Naphthyl | H | H | 7/9 | Cl | $OCH_3$ |
| 8-$OCH_2CO_2CH_3$, 1-Naphthyl | H | $CH_3$ | 7 | Cl | $OCH_3$ |
| 8-$OCH_2CO_2CH_3$, 1-Naphthyl | H | H | 7/9 | $OCH_3$ | Cl |
| 8-$OCH_2CO_2CH_3$, 1-Naphthyl | H | $CH_3$ | 7 | $OCH_3$ | Cl |
| 2-Cl, 1-Naphthyl | H | H | 7/9 | $CH_3$ | $CH_3$ |
| 2-Cl, 1-Naphthyl | H | $CH_3$ | 7 | $CH_3$ | $CH_3$ |
| 2-Cl, 1-Naphthyl | H | H | 7/9 | Cl | $CH_3$ |
| 2-Cl, 1-Naphthyl | H | $CH_3$ | 7 | Cl | $CH_3$ |
| 2-Cl, 1-Naphthyl | H | H | 7/9 | $CH_3$ | Cl |
| 2-Cl, 1-Naphthyl | H | $CH_3$ | 7 | $CH_3$ | Cl |
| 2-Cl, 1-Naphthyl | H | H | 7/9 | Cl | Cl |
| 2-Cl, 1-Naphthyl | H | $CH_3$ | 7 | Cl | Cl |

Tabelle I (Fortsetzung)

| A | $R^1$ | $R^2$ | Pos. | $R^3$ | $R^4$ |
|---|---|---|---|---|---|
| 2-Cl, 1-Naphthyl | H | H | 7/9 | $OCH_3$ | $OCH_3$ |
| 2-Cl, 1-Naphthyl | H | $CH_3$ | 7 | $OCH_3$ | $OCH_3$ |
| 2-Cl, 1-Naphthyl | H | H | 7/9 | $CH_3$ | $OCH_3$ |
| 2-Cl, 1-Naphthyl | H | $CH_3$ | 7 | $CH_3$ | $OCH_3$ |
| 2-Cl, 1-Naphthyl | H | H | 7/9 | $OCH_3$ | $CH_3$ |
| 2-Cl, 1-Naphthyl | H | $CH_3$ | 7 | $OCH_3$ | $CH_3$ |
| 2-Cl, 1-Naphthyl | H | H | 7/9 | Cl | $OCH_3$ |
| 2-Cl, 1-Naphthyl | H | $CH_3$ | 7 | Cl | $OCH_3$ |
| 2-Cl, 1-Naphthyl | H | H | 7/9 | $OCH_3$ | Cl |
| 2-Cl, 1-Naphthyl | H | $CH_3$ | 7 | $OCH_3$ | Cl |
| 2-$CO_2CH_3$, 1-Naphthyl | H | H | 7/9 | $CH_3$ | $CH_3$ |
| 2-$CO_2CH_3$, 1-Naphthyl | H | $CH_3$ | 7 | $CH_3$ | $CH_3$ |
| 2-$CO_2CH_3$, 1-Naphthyl | H | H | 7/9 | Cl | $CH_3$ |
| 2-$CO_2CH_3$, 1-Naphthyl | H | $CH_3$ | 7 | Cl | $CH_3$ |
| 2-$CO_2CH_3$, 1-Naphthyl | H | H | 7/9 | $CH_3$ | Cl |
| 2-$CO_2CH_3$, 1-Naphthyl | H | $CH_3$ | 7 | $CH_3$ | Cl |
| 2-$CO_2CH_3$, 1-Naphthyl | H | H | 7/9 | Cl | Cl |
| 2-$CO_2CH_3$, 1-Naphthyl | H | $CH_3$ | 7 | Cl | Cl |
| 2-$CO_2CH_3$, 1-Naphthyl | H | H | 7/9 | $OCH_3$ | $OCH_3$ |
| 2-$CO_2CH_3$, 1-Naphthyl | H | $CH_3$ | 7 | $OCH_3$ | $OCH_3$ |
| 2-$CO_2CH_3$, 1-Naphthyl | H | H | 7/9 | $CH_3$ | $OCH_3$ |
| 2-$CO_2CH_3$, 1-Naphthyl | H | $CH_3$ | 7 | $CH_3$ | $OCH_3$ |
| 2-$CO_2CH_3$, 1-Naphthyl | H | H | 7/9 | $OCH_3$ | $CH_3$ |
| 2-$CO_2CH_3$, 1-Naphthyl | H | $CH_3$ | 7 | $OCH_3$ | $CH_3$ |
| 2-$CO_2CH_3$, 1-Naphthyl | H | H | 7/9 | Cl | $OCH_3$ |
| 2-$CO_2CH_3$, 1-Naphthyl | H | $CH_3$ | 7 | Cl | $OCH_3$ |
| 2-$CO_2CH_3$, 1-Naphthyl | H | H | 7/9 | $OCH_3$ | Cl |
| 2-$CO_2CH_3$, 1-Naphthyl | H | $CH_3$ | 7 | $OCH_3$ | Cl |
| 2-Naphthyl | H | H | 7/9 | $CH_3$ | $CH_3$ |
| 2-Naphthyl | H | $CH_3$ | 7 | $CH_3$ | $CH_3$ |
| 2-Naphthyl | H | H | 7/9 | Cl | $CH_3$ |
| 2-Naphthyl | H | $CH_3$ | 7 | Cl | $CH_3$ |
| 2-Naphthyl | H | H | 7/9 | $CH_3$ | Cl |
| 2-Naphthyl | H | $CH_3$ | 7 | $CH_3$ | Cl |
| 2-Naphthyl | H | H | 7/9 | Cl | Cl |
| 2-Naphthyl | H | $CH_3$ | 7 | Cl | Cl |
| 2-Naphthyl | H | H | 7/9 | $OCH_3$ | $OCH_3$ |
| 2-Naphthyl | H | $CH_3$ | 7 | $OCH_3$ | $OCH_3$ |
| 2-Naphthyl | H | H | 7/9 | $CH_3$ | $OCH_3$ |
| 2-Naphthyl | H | $CH_3$ | 7 | $CH_3$ | $OCH_3$ |

Tabelle I (Fortsetzung)

| A | $R^1$ | $R^2$ | Pos. | $R^3$ | $R^4$ |
|---|---|---|---|---|---|
| 2-Naphthyl | H | H | 7/9 | $OCH_3$ | $CH_3$ |
| 2-Naphthyl | H | $CH_3$ | 7 | $OCH_3$ | $CH_3$ |
| 2-Naphthyl | H | H | 7/9 | Cl | $OCH_3$ |
| 2-Naphthyl | H | $CH_3$ | 7 | Cl | $OCH_3$ |
| 2-Naphthyl | H | H | 7/9 | $OCH_3$ | Cl |
| 2-Naphthyl | H | $CH_3$ | 7 | $OCH_3$ | Cl |
| 1-Cl, 2-Napthyl | H | H | 7/9 | $CH_3$ | $CH_3$ |
| 1-Cl, 2-Napthyl | H | $CH_3$ | 7 | $CH_3$ | $CH_3$ |
| 1-Cl, 2-Napthyl | H | H | 7/9 | Cl | $CH_3$ |
| 1-Cl, 2-Napthyl | H | $CH_3$ | 7 | Cl | $CH_3$ |
| 1-Cl, 2-Napthyl | H | H | 7/9 | $CH_3$ | Cl |
| 1-Cl, 2-Napthyl | H | $CH_3$ | 7 | $CH_3$ | Cl |
| 1-Cl, 2-Napthyl | H | H | 7/9 | Cl | Cl |
| 1-Cl, 2-Napthyl | H | $CH_3$ | 7 | Cl | Cl |
| 1-Cl, 2-Napthyl | H | H | 7/9 | $OCH_3$ | $OCH_3$ |
| 1-Cl, 2-Napthyl | H | $CH_3$ | 7 | $OCH_3$ | $OCH_3$ |
| 1-Cl, 2-Napthyl | H | H | 7/9 | $CH_3$ | $OCH_3$ |
| 1-Cl, 2-Napthyl | H | $CH_3$ | 7 | $CH_3$ | $OCH_3$ |
| 1-Cl, 2-Napthyl | H | H | 7/9 | $OCH_3$ | $CH_3$ |
| 1-Cl, 2-Napthyl | H | $CH_3$ | 7 | $OCH_3$ | $CH_3$ |
| 1-Cl, 2-Napthyl | H | H | 7/9 | Cl | $OCH_3$ |
| 1-Cl, 2-Napthyl | H | $CH_3$ | 7 | Cl | $OCH_3$ |
| 1-Cl, 2-Napthyl | H | H | 7/9 | $OCH_3$ | Cl |
| 1-Cl, 2-Napthyl | H | $CH_3$ | 7 | $OCH_3$ | Cl |
| 1-$CO_2CH_3$, 2-Napthyl | H | H | 7/9 | $CH_3$ | $CH_3$ |
| 1-$CO_2CH_3$, 2-Napthyl | H | $CH_3$ | 7 | $CH_3$ | $CH_3$ |
| 1-$CO_2CH_3$, 2-Napthyl | H | H | 7/9 | Cl | $CH_3$ |
| 1-$CO_2CH_3$, 2-Napthyl | H | $CH_3$ | 7 | Cl | $CH_3$ |
| 1-$CO_2CH_3$, 2-Napthyl | H | H | 7/9 | $CH_3$ | Cl |
| 1-$CO_2CH_3$, 2-Napthyl | H | $CH_3$ | 7 | $CH_3$ | Cl |
| 1-$CO_2CH_3$, 2-Napthyl | H | H | 7/9 | Cl | Cl |
| 1-$CO_2CH_3$, 2-Napthyl | H | $CH_3$ | 7 | Cl | Cl |
| 1-$CO_2CH_3$, 2-Napthyl | H | H | 7/9 | $OCH_3$ | $OCH_3$ |
| 1-$CO_2CH_3$, 2-Napthyl | H | $CH_3$ | 7 | $OCH_3$ | $OCH_3$ |
| 1-$CO_2CH_3$, 2-Napthyl | H | H | 7/9 | $CH_3$ | $OCH_3$ |
| 1-$CO_2CH_3$, 2-Napthyl | H | $CH_3$ | 7 | $CH_3$ | $OCH_3$ |
| 1-$CO_2CH_3$, 2-Napthyl | H | H | 7/9 | $OCH_3$ | $CH_3$ |
| 1-$CO_2CH_3$, 2-Napthyl | H | $CH_3$ | 7 | $OCH_3$ | $CH_3$ |
| 1-$CO_2CH_3$, 2-Napthyl | H | H | 7/9 | Cl | $OCH_3$ |
| 1-$CO_2CH_3$, 2-Napthyl | H | $CH_3$ | 7 | Cl | $OCH_3$ |

Tabelle I (Fortsetzung)

| A | $R^1$ | $R^2$ | Pos. | $R^3$ | $R^4$ |
|---|---|---|---|---|---|
| 1-$CO_2CH_3$, 2-Napthyl | H | H | 7/9 | $OCH_3$ | Cl |
| 1-$CO_2CH_3$, 2-Napthyl | H | $CH_3$ | 7 | $OCH_3$ | Cl |
| 1-$OCH_2CH_2OCH_3$, 2-Naphthyl | H | H | 7/9 | $CH_3$ | $CH_3$ |
| 1-$OCH_2CH_2OCH_3$, 2-Naphthyl | H | $CH_3$ | 7 | $CH_3$ | $CH_3$ |
| 1-$OCH_2CH_2OCH_3$, 2-Naphthyl | H | H | 7/9 | Cl | $CH_3$ |
| 1-$OCH_2CH_2OCH_3$, 2-Naphthyl | H | $CH_3$ | 7 | Cl | $CH_3$ |
| 1-$OCH_2CH_2OCH_3$, 2-Naphthyl | H | H | 7/9 | $CH_3$ | Cl |
| 1-$OCH_2CH_2OCH_3$, 2-Naphthyl | H | $CH_3$ | 7 | $CH_3$ | Cl |
| 1-$OCH_2CH_2OCH_3$, 2-Naphthyl | H | H | 7/9 | Cl | Cl |
| 1-$OCH_2CH_2OCH_3$, 2-Naphthyl | H | $CH_3$ | 7 | Cl | Cl |
| 1-$OCH_2CH_2OCH_3$, 2-Naphthyl | H | H | 7/9 | $OCH_3$ | $OCH_3$ |
| 1-$OCH_2CH_2OCH_3$, 2-Naphthyl | H | $CH_3$ | 7 | $OCH_3$ | $OCH_3$ |
| 1-$OCH_2CH_2OCH_3$, 2-Naphthyl | H | H | 7/9 | $CH_3$ | $OCH_3$ |
| 1-$OCH_2CH_2OCH_3$, 2-Naphthyl | H | $CH_3$ | 7 | $CH_3$ | $OCH_3$ |
| 1-$OCH_2CH_2OCH_3$, 2-Naphthyl | H | H | 7/9 | $OCH_3$ | $CH_3$ |
| 1-$OCH_2CH_2OCH_3$, 2-Naphthyl | H | $CH_3$ | 7 | $OCH_3$ | $CH_3$ |
| 1-$OCH_2CH_2OCH_3$, 2-Naphthyl | H | H | 7/9 | Cl | $OCH_3$ |
| 1-$OCH_2CH_2OCH_3$, 2-Naphthyl | H | $CH_3$ | 7 | Cl | $OCH_3$ |
| 1-$OCH_2CH_2OCH_3$, 2-Naphthyl | H | H | 7/9 | $OCH_3$ | Cl |
| 1-$OCH_2CH_2OCH_3$, 2-Naphthyl | H | $CH_3$ | 7 | $OCH_3$ | Cl |
| 1-$OCH_2CH_2Cl$, 2-Naphthyl | H | H | 7/9 | $CH_3$ | $CH_3$ |
| 1-$OCH_2CH_2Cl$, 2-Naphthyl | H | $CH_3$ | 7 | $CH_3$ | $CH_3$ |
| 1-$OCH_2CH_2Cl$, 2-Naphthyl | H | H | 7/9 | Cl | $CH_3$ |
| 1-$OCH_2CH_2Cl$, 2-Naphthyl | H | $CH_3$ | 7 | Cl | $CH_3$ |
| 1-$OCH_2CH_2Cl$, 2-Naphthyl | H | H | 7/9 | $CH_3$ | Cl |
| 1-$OCH_2CH_2Cl$, 2-Naphthyl | H | $CH_3$ | 7 | $CH_3$ | Cl |
| 1-$OCH_2CH_2Cl$, 2-Naphthyl | H | H | 7/9 | Cl | Cl |
| 1-$OCH_2CH_2Cl$, 2-Naphthyl | H | $CH_3$ | 7 | Cl | Cl |
| 1-$OCH_2CH_2Cl$, 2-Naphthyl | H | H | 7/9 | $OCH_3$ | $OCH_3$ |
| 1-$OCH_2CH_2Cl$, 2-Naphthyl | H | $CH_3$ | 7 | $OCH_3$ | $OCH_3$ |
| 1-$OCH_2CH_2Cl$, 2-Naphthyl | H | H | 7/9 | $CH_3$ | $OCH_3$ |
| 1-$OCH_2CH_2Cl$, 2-Naphthyl | H | $CH_3$ | 7 | $CH_3$ | $OCH_3$ |
| 1-$OCH_2CH_2Cl$, 2-Naphthyl | H | H | 7/9 | $OCH_3$ | $CH_3$ |
| 1-$OCH_2CH_2Cl$, 2-Naphthyl | H | $CH_3$ | 7 | $OCH_3$ | $CH_3$ |
| 1-$OCH_2CH_2Cl$, 2-Naphthyl | H | H | 7/9 | Cl | $OCH_3$ |
| 1-$OCH_2CH_2Cl$, 2-Naphthyl | H | $CH_3$ | 7 | Cl | $OCH_3$ |
| 1-$OCH_2CH_2Cl$, 2-Naphthyl | H | H | 7/9 | $OCH_3$ | Cl |
| 1-$OCH_2CH_2Cl$, 2-Naphthyl | H | $CH_3$ | 7 | $OCH_3$ | Cl |
| 1-$OCH_2CO_2CH_3$, 2-Naphthyl | H | H | 7/9 | $CH_3$ | $CH_3$ |
| 1-$OCH_2CO_2CH_3$, 2-Naphthyl | H | $CH_3$ | 7 | $CH_3$ | $CH_3$ |

Tabelle I (Fortsetzung)

| A | $R^1$ | $R^2$ | Pos. | $R^3$ | $R^4$ |
|---|---|---|---|---|---|
| 1-$OCH_2CO_2CH_3$, 2-Naphthyl | H | H | 7/9 | Cl | $CH_3$ |
| 1-$OCH_2CO_2CH_3$, 2-Naphthyl | H | $CH_3$ | 7 | Cl | $CH_3$ |
| 1-$OCH_2CO_2CH_3$, 2-Naphthyl | H | H | 7/9 | $CH_3$ | Cl |
| 1-$OCH_2CO_2CH_3$, 2-Naphthyl | H | $CH_3$ | 7 | $CH_3$ | Cl |
| 1-$OCH_2CO_2CH_3$, 2-Naphthyl | H | H | 7/9 | Cl | Cl |
| 1-$OCH_2CO_2CH_3$, 2-Naphthyl | H | $CH_3$ | 7 | Cl | Cl |
| 1-$OCH_2CO_2CH_3$, 2-Naphthyl | H | H | 7/9 | $OCH_3$ | $OCH_3$ |
| 1-$OCH_2CO_2CH_3$, 2-Naphthyl | H | $CH_3$ | 7 | $OCH_3$ | $OCH_3$ |
| 1-$OCH_2CO_2CH_3$, 2-Naphthyl | H | H | 7/9 | $CH_3$ | $OCH_3$ |
| 1-$OCH_2CO_2CH_3$, 2-Naphthyl | H | $CH_3$ | 7 | $CH_3$ | $OCH_3$ |
| 1-$OCH_2CO_2CH_3$, 2-Naphthyl | H | H | 7/9 | $OCH_3$ | $CH_3$ |
| 1-$OCH_2CO_2CH_3$, 2-Naphthyl | H | $CH_3$ | 7 | $OCH_3$ | $CH_3$ |
| 1-$OCH_2CO_2CH_3$, 2-Naphthyl | H | H | 7/9 | Cl | $OCH_3$ |
| 1-$OCH_2CO_2CH_3$, 2-Naphthyl | H | $CH_3$ | 7 | Cl | $OCH_3$ |
| 1-$OCH_2CO_2CH_3$, 2-Naphthyl | H | H | 7/9 | $OCH_3$ | Cl |
| 1-$OCH_2CO_2CH_3$, 2-Naphthyl | H | $CH_3$ | 7 | $OCH_3$ | Cl |
| 2-Thienyl | H | H | 7/9 | $CH_3$ | $CH_3$ |
| 2-Thienyl | H | $CH_3$ | 7 | $CH_3$ | $CH_3$ |
| 2-Thienyl | H | H | 7/9 | Cl | $CH_3$ |
| 2-Thienyl | H | $CH_3$ | 7 | Cl | $CH_3$ |
| 2-Thienyl | H | H | 7/9 | $CH_3$ | Cl |
| 2-Thienyl | H | $CH_3$ | 7 | $CH_3$ | Cl |
| 2-Thienyl | H | H | 7/9 | Cl | Cl |
| 2-Thienyl | H | $CH_3$ | 7 | Cl | Cl |
| 2-Thienyl | H | H | 7/9 | $OCH_3$ | $OCH_3$ |
| 2-Thienyl | H | $CH_3$ | 7 | $OCH_3$ | $OCH_3$ |
| 2-Thienyl | H | H | 7/9 | $CH_3$ | $OCH_3$ |
| 2-Thienyl | H | $CH_3$ | 7 | $CH_3$ | $OCH_3$ |
| 2-Thienyl | H | H | 7/9 | $OCH_3$ | $CH_3$ |
| 2-Thienyl | H | $CH_3$ | 7 | $OCH_3$ | $CH_3$ |
| 2-Thienyl | H | H | 7/9 | Cl | $OCH_3$ |
| 2-Thienyl | H | $CH_3$ | 7 | Cl | $OCH_3$ |
| 2-Thienyl | H | H | 7/9 | $OCH_3$ | Cl |
| 2-Thienyl | H | $CH_3$ | 7 | $OCH_3$ | Cl |
| 3-Cl, 2-Thienyl | H | H | 7/9 | $CH_3$ | $CH_3$ |
| 3-Cl, 2-Thienyl | H | $CH_3$ | 7 | $CH_3$ | $CH_3$ |
| 3-Cl, 2-Thienyl | H | H | 7/9 | Cl | $CH_3$ |
| 3-Cl, 2-Thienyl | H | $CH_3$ | 7 | Cl | $CH_3$ |
| 3-Cl, 2-Thienyl | H | H | 7/9 | $CH_3$ | Cl |
| 3-Cl, 2-Thienyl | H | $CH_3$ | 7 | $CH_3$ | Cl |

Tabelle I (Fortsetzung)

| A | $R^1$ | $R^2$ | Pos. | $R^3$ | $R^4$ |
|---|---|---|---|---|---|
| 3-Cl,2-Thienyl | H | H | 7/9 | Cl | Cl |
| 3-Cl,2-Thienyl | H | $CH_3$ | 7 | Cl | Cl |
| 3-Cl,2-Thienyl | H | H | 7/9 | $OCH_3$ | $OCH_3$ |
| 3-Cl,2-Thienyl | H | $CH_3$ | 7 | $OCH_3$ | $OCH_3$ |
| 3-Cl,2-Thienyl | H | H | 7/9 | $CH_3$ | $OCH_3$ |
| 3-Cl,2-Thienyl | H | $CH_3$ | 7 | $CH_3$ | $OCH_3$ |
| 3-Cl,2-Thienyl | H | H | 7/9 | $OCH_3$ | $CH_3$ |
| 3-Cl,2-Thienyl | H | $CH_3$ | 7 | $OCH_3$ | $CH_3$ |
| 3-Cl,2-Thienyl | H | H | 7/9 | Cl | $OCH_3$ |
| 3-Cl,2-Thienyl | H | $CH_3$ | 7 | Cl | $OCH_3$ |
| 3-Cl,2-Thienyl | H | H | 7/9 | $OCH_3$ | Cl |
| 3-Cl,2-Thienyl | H | $CH_3$ | 7 | $OCH_3$ | Cl |
| 3-Cl,2-Thienyl | $CH_3$ | H | 7/9 | $CH_3$ | $CH_3$ |
| 3-Cl,2-Thienyl | $CH_3$ | $CH_3$ | 7 | $CH_3$ | $CH_3$ |
| 3-Cl,2-Thienyl | $CH_3$ | H | 7/9 | Cl | $CH_3$ |
| 3-Cl,2-Thienyl | $CH_3$ | $CH_3$ | 7 | Cl | $CH_3$ |
| 3-Cl,2-Thienyl | $CH_3$ | H | 7/9 | $CH_3$ | Cl |
| 3-Cl,2-Thienyl | $CH_3$ | $CH_3$ | 7 | $CH_3$ | Cl |
| 3-Cl,2-Thienyl | $CH_3$ | H | 7/9 | Cl | Cl |
| 3-Cl,2-Thienyl | $CH_3$ | $CH_3$ | 7 | Cl | Cl |
| 3-Cl,2-Thienyl | $CH_3$ | H | 7/9 | $OCH_3$ | $OCH_3$ |
| 3-Cl,2-Thienyl | $CH_3$ | $CH_3$ | 7 | $OCH_3$ | $OCH_3$ |
| 3-Cl,2-Thienyl | $CH_3$ | H | 7/9 | $CH_3$ | $OCH_3$ |
| 3-Cl,2-Thienyl | $CH_3$ | $CH_3$ | 7 | $CH_3$ | $OCH_3$ |
| 3-Cl,2-Thienyl | $CH_3$ | H | 7/9 | $OCH_3$ | $CH_3$ |
| 3-Cl,2-Thienyl | $CH_3$ | $CH_3$ | 7 | $OCH_3$ | $CH_3$ |
| 3-Cl,2-Thienyl | $CH_3$ | H | 7/9 | Cl | $OCH_3$ |
| 3-Cl,2-Thienyl | $CH_3$ | $CH_3$ | 7 | Cl | $OCH_3$ |
| 3-Cl,2-Thienyl | $CH_3$ | H | 7/9 | $OCH_3$ | Cl |
| 3-Cl,2-Thienyl | $CH_3$ | $CH_3$ | 7 | $OCH_3$ | Cl |
| 3-Cl,2-Thienyl | H | H | 7/9 | $CF_3$ | $CF_3$ |
| 3-Cl,2-Thienyl | H | $CH_3$ | 7 | $CF_3$ | $CF_3$ |
| 3-Cl,2-Thienyl | H | H | 7/9 | Cl | $CF_3$ |
| 3-Cl,2-Thienyl | H | $CH_3$ | 7 | Cl | $CF_3$ |
| 3-Cl,2-Thienyl | H | H | 7/9 | $CF_3$ | Cl |
| 3-Cl,2-Thienyl | H | $CH_3$ | 7 | $CF_3$ | Cl |
| 3-Cl,2-Thienyl | H | H | 7/9 | F | $CF_3$ |
| 3-Cl,2-Thienyl | H | $CH_3$ | 7 | F | $CF_3$ |
| 3-Cl,2-Thienyl | H | H | 7/9 | $CF_3$ | F |
| 3-Cl,2-Thienyl | H | $CH_3$ | 7 | $CF_3$ | F |

Tabelle I (Fortsetzung)

| A | $R^1$ | $R^2$ | Pos. | $R^3$ | $R^4$ |
|---|---|---|---|---|---|
| 3-Cl,2-Thienyl | H | H | 7/9 | $CH_3$ | $CF_3$ |
| 3-Cl,2-Thienyl | H | $CH_3$ | 7 | $CH_3$ | $CF_3$ |
| 3-Cl,2-Thienyl | H | H | 7/9 | $CF_3$ | $CH_3$ |
| 3-Cl,2-Thienyl | H | $CH_3$ | 7 | $CF_3$ | $CH_3$ |
| 3-Cl,2-Thienyl | H | H | 7/9 | $OCH_3$ | $CF_3$ |
| 3-Cl,2-Thienyl | H | $CH_3$ | 7 | $OCH_3$ | $CF_3$ |
| 3-Cl,2-Thienyl | H | H | 7/9 | $CF_3$ | $OCH_3$ |
| 3-Cl,2-Thienyl | H | $CH_3$ | 7 | $CF_3$ | $OCH_3$ |
| 3-Cl,2-Thienyl | H | H | 7/9 | F | F |
| 3-Cl,2-Thienyl | H | $CH_3$ | 7 | F | F |
| 3-Cl,2-Thienyl | H | H | 7/9 | F | Cl |
| 3-Cl,2-Thienyl | H | $CH_3$ | 7 | F | Cl |
| 3-Cl,2-Thienyl | H | H | 7/9 | Cl | F |
| 3-Cl,2-Thienyl | H | $CH_3$ | 7 | Cl | F |
| 3-Cl,2-Thienyl | H | H | 7/9 | F | $CH_3$ |
| 3-Cl,2-Thienyl | H | $CH_3$ | 7 | F | $CH_3$ |
| 3-Cl,2-Thienyl | H | H | 7/9 | $CH_3$ | F |
| 3-Cl,2-Thienyl | H | $CH_3$ | 7 | $CH_3$ | F |
| 3-Cl,2-Thienyl | H | H | 7/9 | F | $OCH_3$ |
| 3-Cl,2-Thienyl | H | $CH_3$ | 7 | F | $OCH_3$ |
| 3-Cl,2-Thienyl | H | H | 7/9 | $OCH_3$ | F |
| 3-Cl,2-Thienyl | H | $CH_3$ | 7 | $OCH_3$ | F |
| 3-Cl,2-Thienyl | H | H | 7/9 | $N(CH_3)_2$ | Cl |
| 3-Cl,2-Thienyl | H | $CH_3$ | 7 | $N(CH_3)_2$ | Cl |
| 3-Cl,2-Thienyl | H | H | 7/9 | $SCH_3$ | Cl |
| 3-Cl,2-Thienyl | H | $CH_3$ | 7 | $SCH_3$ | Cl |
| 4-Cl,2-Thienyl | H | H | 7/9 | $CH_3$ | $CH_3$ |
| 4-Cl,2-Thienyl | H | $CH_3$ | 7 | $CH_3$ | $CH_3$ |
| 4-Cl,2-Thienyl | H | H | 7/9 | Cl | $CH_3$ |
| 4-Cl,2-Thienyl | H | $CH_3$ | 7 | Cl | $CH_3$ |
| 4-Cl,2-Thienyl | H | H | 7/9 | $CH_3$ | Cl |
| 4-Cl,2-Thienyl | H | $CH_3$ | 7 | $CH_3$ | Cl |
| 4-Cl,2-Thienyl | H | H | 7/9 | Cl | Cl |
| 4-Cl,2-Thienyl | H | $CH_3$ | 7 | Cl | Cl |
| 4-Cl,2-Thienyl | H | H | 7/9 | $OCH_3$ | $OCH_3$ |
| 4-Cl,2-Thienyl | H | $CH_3$ | 7 | $OCH_3$ | $OCH_3$ |
| 4-Cl,2-Thienyl | H | H | 7/9 | $CH_3$ | $OCH_3$ |
| 4-Cl,2-Thienyl | H | $CH_3$ | 7 | $CH_3$ | $OCH_3$ |
| 4-Cl,2-Thienyl | H | H | 7/9 | $OCH_3$ | $CH_3$ |

Tabelle I (Fortsetzung)

| A | $R^1$ | $R^2$ | Pos. | $R^3$ | $R^4$ |
|---|---|---|---|---|---|
| 4-Cl, 2-Thienyl | H | $CH_3$ | 7 | $OCH_3$ | $CH_3$ |
| 4-Cl, 2-Thienyl | H | H | 7/9 | Cl | $OCH_3$ |
| 4-Cl, 2-Thienyl | H | $CH_3$ | 7 | Cl | $OCH_3$ |
| 4-Cl, 2-Thienyl | H | H | 7/9 | $OCH_3$ | Cl |
| 4-Cl, 2-Thienyl | H | $CH_3$ | 7 | $OCH_3$ | Cl |
| 5-Cl, 2-Thienyl | H | H | 7/9 | $CH_3$ | $CH_3$ |
| 5-Cl, 2-Thienyl | H | $CH_3$ | 7 | $CH_3$ | $CH_3$ |
| 5-Cl, 2-Thienyl | H | H | 7/9 | Cl | $CH_3$ |
| 5-Cl, 2-Thienyl | H | $CH_3$ | 7 | Cl | $CH_3$ |
| 5-Cl, 2-Thienyl | H | H | 7/9 | $CH_3$ | Cl |
| 5-Cl, 2-Thienyl | H | $CH_3$ | 7 | $CH_3$ | Cl |
| 5-Cl, 2-Thienyl | H | H | 7/9 | Cl | Cl |
| 5-Cl, 2-Thienyl | H | $CH_3$ | 7 | Cl | Cl |
| 5-Cl, 2-Thienyl | H | H | 7/9 | $OCH_3$ | $OCH_3$ |
| 5-Cl, 2-Thienyl | H | $CH_3$ | 7 | $OCH_3$ | $OCH_3$ |
| 5-Cl, 2-Thienyl | H | H | 7/9 | $CH_3$ | $OCH_3$ |
| 5-Cl, 2-Thienyl | H | $CH_3$ | 7 | $CH_3$ | $OCH_3$ |
| 5-Cl, 2-Thienyl | H | H | 7/9 | $OCH_3$ | $CH_3$ |
| 5-Cl, 2-Thienyl | H | $CH_3$ | 7 | $OCH_3$ | $CH_3$ |
| 5-Cl, 2-Thienyl | H | H | 7/9 | Cl | $OCH_3$ |
| 5-Cl, 2-Thienyl | H | $CH_3$ | 7 | Cl | $OCH_3$ |
| 5-Cl, 2-Thienyl | H | H | 7/9 | $OCH_3$ | Cl |
| 5-Cl, 2-Thienyl | H | $CH_3$ | 7 | $OCH_3$ | Cl |
| $3-CO_2CH_3$, 2-Thienyl | H | H | 7/9 | $CH_3$ | $CH_3$ |
| $3-CO_2CH_3$, 2-Thienyl | H | $CH_3$ | 7 | $CH_3$ | $CH_3$ |
| $3-CO_2CH_3$, 2-Thienyl | H | H | 7/9 | Cl | $CH_3$ |
| $3-CO_2CH_3$, 2-Thienyl | H | $CH_3$ | 7 | Cl | $CH_3$ |
| $3-CO_2CH_3$, 2-Thienyl | H | H | 7/9 | $CH_3$ | Cl |
| $3-CO_2CH_3$, 2-Thienyl | H | $CH_3$ | 7 | $CH_3$ | Cl |
| $3-CO_2CH_3$, 2-Thienyl | H | H | 7/9 | Cl | Cl |
| $3-CO_2CH_3$, 2-Thienyl | H | $CH_3$ | 7 | Cl | Cl |
| $3-CO_2CH_3$, 2-Thienyl | H | H | 7/9 | $OCH_3$ | $OCH_3$ |
| $3-CO_2CH_3$, 2-Thienyl | H | $CH_3$ | 7 | $OCH_3$ | $OCH_3$ |
| $3-CO_2CH_3$, 2-Thienyl | H | H | 7/9 | $CH_3$ | $OCH_3$ |
| $3-CO_2CH_3$, 2-Thienyl | H | $CH_3$ | 7 | $CH_3$ | $OCH_3$ |
| $3-CO_2CH_3$, 2-Thienyl | H | H | 7/9 | $OCH_3$ | $CH_3$ |
| $3-CO_2CH_3$, 2-Thienyl | H | $CH_3$ | 7 | $OCH_3$ | $CH_3$ |
| $3-CO_2CH_3$, 2-Thienyl | H | H | 7/9 | Cl | $OCH_3$ |

Tabelle I (Fortsetzung)

| A | $R^1$ | $R^2$ | Pos. | $R^3$ | $R^4$ |
|---|---|---|---|---|---|
| 3-$CO_2CH_3$, 2-Thienyl | H | $CH_3$ | 7 | Cl | $OCH_3$ |
| 3-$CO_2CH_3$, 2-Thienyl | H | H | 7/9 | $OCH_3$ | Cl |
| 3-$CO_2CH_3$, 2-Thienyl | H | $CH_3$ | 7 | $OCH_3$ | Cl |
| 2-$CO_2CH_3$, 3-Thienyl | H | H | 7/9 | $CH_3$ | $CH_3$ |
| 2-$CO_2CH_3$, 3-Thienyl | H | $CH_3$ | 7 | $CH_3$ | $CH_3$ |
| 2-$CO_2CH_3$, 3-Thienyl | H | H | 7/9 | Cl | $CH_3$ |
| 2-$CO_2CH_3$, 3-Thienyl | H | $CH_3$ | 7 | Cl | $CH_3$ |
| 2-$CO_2CH_3$, 3-Thienyl | H | H | 7/9 | $CH_3$ | Cl |
| 2-$CO_2CH_3$, 3-Thienyl | H | $CH_3$ | 7 | $CH_3$ | Cl |
| 2-$CO_2CH_3$, 3-Thienyl | H | H | 7/9 | Cl | Cl |
| 2-$CO_2CH_3$, 3-Thienyl | H | $CH_3$ | 7 | Cl | Cl |
| 2-$CO_2CH_3$, 3-Thienyl | H | H | 7/9 | $OCH_3$ | $OCH_3$ |
| 2-$CO_2CH_3$, 3-Thienyl | H | $CH_3$ | 7 | $OCH_3$ | $OCH_3$ |
| 2-$CO_2CH_3$, 3-Thienyl | H | H | 7/9 | $CH_3$ | $OCH_3$ |
| 2-$CO_2CH_3$, 3-Thienyl | H | $CH_3$ | 7 | $CH_3$ | $OCH_3$ |
| 2-$CO_2CH_3$, 3-Thienyl | H | H | 7/9 | $OCH_3$ | $CH_3$ |
| 2-$CO_2CH_3$, 3-Thienyl | H | $CH_3$ | 7 | $OCH_3$ | $CH_3$ |
| 2-$CO_2CH_3$, 3-Thienyl | H | H | 7/9 | Cl | $OCH_3$ |
| 2-$CO_2CH_3$, 3-Thienyl | H | $CH_3$ | 7 | Cl | $OCH_3$ |
| 2-$CO_2CH_3$, 3-Thienyl | H | H | 7/9 | $OCH_3$ | Cl |
| 2-$CO_2CH_3$, 3-Thienyl | H | $CH_3$ | 7 | $OCH_3$ | Cl |
| 2-$CO_2CH_3$, 3-Thienyl | $CH_3$ | H | 7/9 | $CH_3$ | $CH_3$ |
| 2-$CO_2CH_3$, 3-Thienyl | $CH_3$ | $CH_3$ | 7 | $CH_3$ | $CH_3$ |
| 2-$CO_2CH_3$, 3-Thienyl | $CH_3$ | H | 7/9 | Cl | $CH_3$ |
| 2-$CO_2CH_3$, 3-Thienyl | $CH_3$ | $CH_3$ | 7 | Cl | $CH_3$ |
| 2-$CO_2CH_3$, 3-Thienyl | $CH_3$ | H | 7/9 | $CH_3$ | Cl |
| 2-$CO_2CH_3$, 3-Thienyl | $CH_3$ | $CH_3$ | 7 | $CH_3$ | Cl |
| 2-$CO_2CH_3$, 3-Thienyl | $CH_3$ | H | 7/9 | Cl | Cl |
| 2-$CO_2CH_3$, 3-Thienyl | $CH_3$ | $CH_3$ | 7 | Cl | Cl |
| 2-$CO_2CH_3$, 3-Thienyl | $CH_3$ | H | 7/9 | $OCH_3$ | $OCH_3$ |
| 2-$CO_2CH_3$, 3-Thienyl | $CH_3$ | $CH_3$ | 7 | $OCH_3$ | $OCH_3$ |
| 2-$CO_2CH_3$, 3-Thienyl | $CH_3$ | H | 7/9 | $CH_3$ | $OCH_3$ |
| 2-$CO_2CH_3$, 3-Thienyl | $CH_3$ | $CH_3$ | 7 | $CH_3$ | $OCH_3$ |
| 2-$CO_2CH_3$, 3-Thienyl | $CH_3$ | H | 7/9 | $OCH_3$ | $CH_3$ |
| 2-$CO_2CH_3$, 3-Thienyl | $CH_3$ | $CH_3$ | 7 | $OCH_3$ | $CH_3$ |
| 2-$CO_2CH_3$, 3-Thienyl | $CH_3$ | H | 7/9 | Cl | $OCH_3$ |
| 2-$CO_2CH_3$, 3-Thienyl | $CH_3$ | $CH_3$ | 7 | Cl | $OCH_3$ |
| 2-$CO_2CH_3$, 3-Thienyl | $CH_3$ | H | 7/9 | $OCH_3$ | Cl |

Tabelle I (Fortsetzung)

| A | $R^1$ | $R^2$ | Pos. | $R^3$ | $R^4$ |
|---|---|---|---|---|---|
| 2-$CO_2CH_3$,3-Thienyl | $CH_3$ | $CH_3$ | 7 | $OCH_3$ | Cl |
| 2-$CO_2CH_3$,3-Thienyl | H | H | 7/9 | $CF_3$ | $CF_3$ |
| 2-$CO_2CH_3$,3-Thienyl | H | $CH_3$ | 7 | $CF_3$ | $CF_3$ |
| 2-$CO_2CH_3$,3-Thienyl | H | H | 7/9 | Cl | $CF_3$ |
| 2-$CO_2CH_3$,3-Thienyl | H | $CH_3$ | 7 | Cl | $CF_3$ |
| 2-$CO_2CH_3$,3-Thienyl | H | H | 7/9 | $CF_3$ | Cl |
| 2-$CO_2CH_3$,3-Thienyl | H | $CH_3$ | 7 | $CF_3$ | Cl |
| 2-$CO_2CH_3$,3-Thienyl | H | H | 7/9 | F | $CF_3$ |
| 2-$CO_2CH_3$,3-Thienyl | H | $CH_3$ | 7 | F | $CF_3$ |
| 2-$CO_2CH_3$,3-Thienyl | H | H | 7/9 | $CF_3$ | F |
| 2-$CO_2CH_3$,3-Thienyl | H | $CH_3$ | 7 | $CF_3$ | F |
| 2-$CO_2CH_3$,3-Thienyl | H | H | 7/9 | $CH_3$ | $CF_3$ |
| 2-$CO_2CH_3$,3-Thienyl | H | $CH_3$ | 7 | $CH_3$ | $CF_3$ |
| 2-$CO_2CH_3$,3-Thienyl | H | H | 7/9 | $CF_3$ | $CH_3$ |
| 2-$CO_2CH_3$,3-Thienyl | H | $CH_3$ | 7 | $CF_3$ | $CH_3$ |
| 2-$CO_2CH_3$,3-Thienyl | H | H | 7/9 | $OCH_3$ | $CF_3$ |
| 2-$CO_2CH_3$,3-Thienyl | H | $CH_3$ | 7 | $OCH_3$ | $CF_3$ |
| 2-$CO_2CH_3$,3-Thienyl | H | H | 7/9 | $CF_3$ | $OCH_3$ |
| 2-$CO_2CH_3$,3-Thienyl | H | $CH_3$ | 7 | $CF_3$ | $OCH_3$ |
| 2-$CO_2CH_3$,3-Thienyl | H | H | 7/9 | F | F |
| 2-$CO_2CH_3$,3-Thienyl | H | $CH_3$ | 7 | F | F |
| 2-$CO_2CH_3$,3-Thienyl | H | H | 7/9 | F | Cl |
| 2-$CO_2CH_3$,3-Thienyl | H | $CH_3$ | 7 | F | Cl |
| 2-$CO_2CH_3$,3-Thienyl | H | H | 7/9 | Cl | F |
| 2-$CO_2CH_3$,3-Thienyl | H | $CH_3$ | 7 | Cl | F |
| 2-$CO_2CH_3$,3-Thienyl | H | H | 7/9 | F | $CH_3$ |
| 2-$CO_2CH_3$,3-Thienyl | H | $CH_3$ | 7 | F | $CH_3$ |
| 2-$CO_2CH_3$,3-Thienyl | H | H | 7/9 | $CH_3$ | F |
| 2-$CO_2CH_3$,3-Thienyl | H | $CH_3$ | 7 | $CH_3$ | F |
| 2-$CO_2CH_3$,3-Thienyl | H | H | 7/9 | F | $OCH_3$ |
| 2-$CO_2CH_3$,3-Thienyl | H | $CH_3$ | 7 | F | $OCH_3$ |
| 2-$CO_2CH_3$,3-Thienyl | H | H | 7/9 | $OCH_3$ | F |
| 2-$CO_2CH_3$,3-Thienyl | H | $CH_3$ | 7 | $OCH_3$ | F |
| 2-$CO_2CH_3$,3-Thienyl | H | H | 7/9 | $N(CH_3)_2$ | Cl |
| 2-$CO_2CH_3$,3-Thienyl | H | $CH_3$ | 7 | $N(CH_3)_2$ | Cl |
| 2-$CO_2CH_3$,3-Thienyl | H | H | 7/9 | $SCH_3$ | Cl |
| 2-$CO_2CH_3$,3-Thienyl | H | $CH_3$ | 7 | $SCH_3$ | Cl |
| 2-Cl,5-$CH_3$,3-Thienyl | H | H | 7/9 | $CH_3$ | $CH_3$ |

Tabelle I (Fortsetzung)

| A | $R^1$ | $R^2$ | Pos. | $R^3$ | $R^4$ |
|---|---|---|---|---|---|
| 2-Cl,5-$CH_3$,3-Thienyl | H | $CH_3$ | 7 | $CH_3$ | $CH_3$ |
| 2-Cl,5-$CH_3$,3-Thienyl | H | H | 7/9 | Cl | $CH_3$ |
| 2-Cl,5-$CH_3$,3-Thienyl | H | $CH_3$ | 7 | Cl | $CH_3$ |
| 2-Cl,5-$CH_3$,3-Thienyl | H | H | 7/9 | $CH_3$ | Cl |
| 2-Cl,5-$CH_3$,3-Thienyl | H | $CH_3$ | 7 | $CH_3$ | Cl |
| 2-Cl,5-$CH_3$,3-Thienyl | H | H | 7/9 | Cl | Cl |
| 2-Cl,5-$CH_3$,3-Thienyl | H | $CH_3$ | 7 | Cl | Cl |
| 2-Cl,5-$CH_3$,3-Thienyl | H | H | 7/9 | $OCH_3$ | $OCH_3$ |
| 2-Cl,5-$CH_3$,3-Thienyl | H | $CH_3$ | 7 | $OCH_3$ | $OCH_3$ |
| 2-Cl,5-$CH_3$,3-Thienyl | H | H | 7/9 | $CH_3$ | $OCH_3$ |
| 2-Cl,5-$CH_3$,3-Thienyl | H | $CH_3$ | 7 | $CH_3$ | $OCH_3$ |
| 2-Cl,5-$CH_3$,3-Thienyl | H | H | 7/9 | $OCH_3$ | $CH_3$ |
| 2-Cl,5-$CH_3$,3-Thienyl | H | $CH_3$ | 7 | $OCH_3$ | $CH_3$ |
| 2-Cl,5-$CH_3$,3-Thienyl | H | H | 7/9 | Cl | $OCH_3$ |
| 2-Cl,5-$CH_3$,3-Thienyl | H | $CH_3$ | 7 | Cl | $OCH_3$ |
| 2-Cl,5-$CH_3$,3-Thienyl | H | H | 7/9 | $OCH_3$ | Cl |
| 2-Cl,5-$CH_3$,3-Thienyl | H | $CH_3$ | 7 | $OCH_3$ | Cl |
| 4-$CO_2CH_3$,3-Thienyl | H | H | 7/9 | $CH_3$ | $CH_3$ |
| 4-$CO_2CH_3$,3-Thienyl | H | $CH_3$ | 7 | $CH_3$ | $CH_3$ |
| 4-$CO_2CH_3$,3-Thienyl | H | H | 7/9 | Cl | $CH_3$ |
| 4-$CO_2CH_3$,3-Thienyl | H | $CH_3$ | 7 | Cl | $CH_3$ |
| 4-$CO_2CH_3$,3-Thienyl | H | H | 7/9 | $CH_3$ | Cl |
| 4-$CO_2CH_3$,3-Thienyl | H | $CH_3$ | 7 | $CH_3$ | Cl |
| 4-$CO_2CH_3$,3-Thienyl | H | H | 7/9 | Cl | Cl |
| 4-$CO_2CH_3$,3-Thienyl | H | $CH_3$ | 7 | Cl | Cl |
| 4-$CO_2CH_3$,3-Thienyl | H | H | 7/9 | $OCH_3$ | $OCH_3$ |
| 4-$CO_2CH_3$,3-Thienyl | H | $CH_3$ | 7 | $OCH_3$ | $OCH_3$ |
| 4-$CO_2CH_3$,3-Thienyl | H | H | 7/9 | $CH_3$ | $OCH_3$ |
| 4-$CO_2CH_3$,3-Thienyl | H | $CH_3$ | 7 | $CH_3$ | $OCH_3$ |
| 4-$CO_2CH_3$,3-Thienyl | H | H | 7/9 | $OCH_3$ | $CH_3$ |
| 4-$CO_2CH_3$,3-Thienyl | H | $CH_3$ | 7 | $OCH_3$ | $CH_3$ |
| 4-$CO_2CH_3$,3-Thienyl | H | H | 7/9 | Cl | $OCH_3$ |
| 4-$CO_2CH_3$,3-Thienyl | H | $CH_3$ | 7 | Cl | $OCH_3$ |
| 4-$CO_2CH_3$,3-Thienyl | H | H | 7/9 | $OCH_3$ | Cl |
| 4-$CO_2CH_3$,3-Thienyl | H | $CH_3$ | 7 | $OCH_3$ | Cl |

Tabelle I (Fortsetzung)

| A | $R^1$ | $R^2$ | Pos. | $R^3$ | $R^4$ |
|---|---|---|---|---|---|
| 1-$CH_3$,4-$CO_2CH_3$,5-pyrazolyl | H | H | 7/9 | $CH_3$ | $CH_3$ |
| 1-$CH_3$,4-$CO_2CH_3$,5-pyrazolyl | H | $CH_3$ | 7 | $CH_3$ | $CH_3$ |
| 1-$CH_3$,4-$CO_2CH_3$,5-pyrazolyl | H | H | 7/9 | Cl | $CH_3$ |
| 1-$CH_3$,4-$CO_2CH_3$,5-pyrazolyl | H | $CH_3$ | 7 | Cl | $CH_3$ |
| 1-$CH_3$,4-$CO_2CH_3$,5-pyrazolyl | H | H | 7/9 | $CH_3$ | Cl |
| 1-$CH_3$,4-$CO_2CH_3$,5-pyrazolyl | H | $CH_3$ | 7 | $CH_3$ | Cl |
| 1-$CH_3$,4-$CO_2CH_3$,5-pyrazolyl | H | H | 7/9 | Cl | Cl |
| 1-$CH_3$,4-$CO_2CH_3$,5-pyrazolyl | H | $CH_3$ | 7 | Cl | Cl |
| 1-$CH_3$,4-$CO_2CH_3$,5-pyrazolyl | H | H | 7/9 | $OCH_3$ | $OCH_3$ |
| 1-$CH_3$,4-$CO_2CH_3$,5-pyrazolyl | H | $CH_3$ | 7 | $OCH_3$ | $OCH_3$ |
| 1-$CH_3$,4-$CO_2CH_3$,5-pyrazolyl | H | H | 7/9 | $CH_3$ | $OCH_3$ |
| 1-$CH_3$,4-$CO_2CH_3$,5-pyrazolyl | H | $CH_3$ | 7 | $CH_3$ | $OCH_3$ |
| 1-$CH_3$,4-$CO_2CH_3$,5-pyrazolyl | H | H | 7/9 | $OCH_3$ | $CH_3$ |
| 1-$CH_3$,4-$CO_2CH_3$,5-pyrazolyl | H | $CH_3$ | 7 | $OCH_3$ | $CH_3$ |
| 1-$CH_3$,4-$CO_2CH_3$,5-pyrazolyl | H | H | 7/9 | Cl | $OCH_3$ |
| 1-$CH_3$,4-$CO_2CH_3$,5-pyrazolyl | H | $CH_3$ | 7 | Cl | $OCH_3$ |
| 1-$CH_3$,4-$CO_2CH_3$,5-pyrazolyl | H | H | 7/9 | $OCH_3$ | Cl |
| 1-$CH_3$,4-$CO_2CH_3$,5-pyrazolyl | H | $CH_3$ | 7 | $OCH_3$ | Cl |
| 1-$CH_3$,4-$CO_2CH_3$,5-pyrazolyl | $CH_3$ | H | 7/9 | $CH_3$ | $CH_3$ |
| 1-$CH_3$,4-$CO_2CH_3$,5-pyrazolyl | $CH_3$ | $CH_3$ | 7 | $CH_3$ | $CH_3$ |
| 1-$CH_3$,4-$CO_2CH_3$,5-pyrazolyl | $CH_3$ | H | 7/9 | Cl | $CH_3$ |
| 1-$CH_3$,4-$CO_2CH_3$,5-pyrazolyl | $CH_3$ | $CH_3$ | 7 | Cl | $CH_3$ |
| 1-$CH_3$,4-$CO_2CH_3$,5-pyrazolyl | $CH_3$ | H | 7/9 | $CH_3$ | Cl |
| 1-$CH_3$,4-$CO_2CH_3$,5-pyrazolyl | $CH_3$ | $CH_3$ | 7 | $CH_3$ | Cl |
| 1-$CH_3$,4-$CO_2CH_3$,5-pyrazolyl | $CH_3$ | H | 7/9 | Cl | Cl |
| 1-$CH_3$,4-$CO_2CH_3$,5-pyrazolyl | $CH_3$ | $CH_3$ | 7 | Cl | Cl |
| 1-$CH_3$,4-$CO_2CH_3$,5-pyrazolyl | $CH_3$ | H | 7/9 | $OCH_3$ | $OCH_3$ |
| 1-$CH_3$,4-$CO_2CH_3$,5-pyrazolyl | $CH_3$ | $CH_3$ | 7 | $OCH_3$ | $OCH_3$ |
| 1-$CH_3$,4-$CO_2CH_3$,5-pyrazolyl | $CH_3$ | H | 7/9 | $CH_3$ | $OCH_3$ |
| 1-$CH_3$,4-$CO_2CH_3$,5-pyrazolyl | $CH_3$ | $CH_3$ | 7 | $CH_3$ | $OCH_3$ |
| 1-$CH_3$,4-$CO_2CH_3$,5-pyrazolyl | $CH_3$ | H | 7/9 | $OCH_3$ | $CH_3$ |
| 1-$CH_3$,4-$CO_2CH_3$,5-pyrazolyl | $CH_3$ | $CH_3$ | 7 | $OCH_3$ | $CH_3$ |
| 1-$CH_3$,4-$CO_2CH_3$,5-pyrazolyl | $CH_3$ | H | 7/9 | Cl | $OCH_3$ |
| 1-$CH_3$,4-$CO_2CH_3$,5-pyrazolyl | $CH_3$ | $CH_3$ | 7 | Cl | $OCH_3$ |
| 1-$CH_3$,4-$CO_2CH_3$,5-pyrazolyl | $CH_3$ | H | 7/9 | $OCH_3$ | Cl |
| 1-$CH_3$,4-$CO_2CH_3$,5-pyrazolyl | $CH_3$ | $CH_3$ | 7 | $OCH_3$ | Cl |
| 1-$CH_3$,4-$CO_2CH_3$,5-pyrazolyl | H | H | 7/9 | $CF_3$ | $CF_3$ |
| 1-$CH_3$,4-$CO_2CH_3$,5-pyrazolyl | H | $CH_3$ | 7 | $CF_3$ | $CF_3$ |
| 1-$CH_3$,4-$CO_2CH_3$,5-pyrazolyl | H | H | 7/9 | Cl | $CF_3$ |

Tabelle I (Fortsetzung)

| A | $R^1$ | $R^2$ | Pos. | $R^3$ | $R^4$ |
|---|---|---|---|---|---|
| 1-$CH_3$,4-$CO_2CH_3$,5-pyrazolyl | H | $CH_3$ | 7 | Cl | $CF_3$ |
| 1-$CH_3$,4-$CO_2CH_3$,5-pyrazolyl | H | H | 7/9 | $CF_3$ | Cl |
| 1-$CH_3$,4-$CO_2CH_3$,5-pyrazolyl | H | $CH_3$ | 7 | $CF_3$ | Cl |
| 1-$CH_3$,4-$CO_2CH_3$,5-pyrazolyl | H | H | 7/9 | F | $CF_3$ |
| 1-$CH_3$,4-$CO_2CH_3$,5-pyrazolyl | H | $CH_3$ | 7 | F | $CF_3$ |
| 1-$CH_3$,4-$CO_2CH_3$,5-pyrazolyl | H | H | 7/9 | $CF_3$ | F |
| 1-$CH_3$,4-$CO_2CH_3$,5-pyrazolyl | H | $CH_3$ | 7 | $CF_3$ | F |
| 1-$CH_3$,4-$CO_2CH_3$,5-pyrazolyl | H | H | 7/9 | $CH_3$ | $CF_3$ |
| 1-$CH_3$,4-$CO_2CH_3$,5-pyrazolyl | H | $CH_3$ | 7 | $CH_3$ | $CF_3$ |
| 1-$CH_3$,4-$CO_2CH_3$,5-pyrazolyl | H | H | 7/9 | $CF_3$ | $CH_3$ |
| 1-$CH_3$,4-$CO_2CH_3$,5-pyrazolyl | H | $CH_3$ | 7 | $CF_3$ | $CH_3$ |
| 1-$CH_3$,4-$CO_2CH_3$,5-pyrazolyl | H | H | 7/9 | $OCH_3$ | $CF_3$ |
| 1-$CH_3$,4-$CO_2CH_3$,5-pyrazolyl | H | $CH_3$ | 7 | $OCH_3$ | $CF_3$ |
| 1-$CH_3$,4-$CO_2CH_3$,5-pyrazolyl | H | H | 7/9 | $CF_3$ | $OCH_3$ |
| 1-$CH_3$,4-$CO_2CH_3$,5-pyrazolyl | H | $CH_3$ | 7 | $CF_3$ | $OCH_3$ |
| 1-$CH_3$,4-$CO_2CH_3$,5-pyrazolyl | H | H | 7/9 | F | F |
| 1-$CH_3$,4-$CO_2CH_3$,5-pyrazolyl | H | $CH_3$ | 7 | F | F |
| 1-$CH_3$,4-$CO_2CH_3$,5-pyrazolyl | H | H | 7/9 | F | Cl |
| 1-$CH_3$,4-$CO_2CH_3$,5-pyrazolyl | H | $CH_3$ | 7 | F | Cl |
| 1-$CH_3$,4-$CO_2CH_3$,5-pyrazolyl | H | H | 7/9 | Cl | F |
| 1-$CH_3$,4-$CO_2CH_3$,5-pyrazolyl | H | $CH_3$ | 7 | Cl | F |
| 1-$CH_3$,4-$CO_2CH_3$,5-pyrazolyl | H | H | 7/9 | F | $CH_3$ |
| 1-$CH_3$,4-$CO_2CH_3$,5-pyrazolyl | H | $CH_3$ | 7 | F | $CH_3$ |
| 1-$CH_3$,4-$CO_2CH_3$,5-pyrazolyl | H | H | 7/9 | $CH_3$ | F |
| 1-$CH_3$,4-$CO_2CH_3$,5-pyrazolyl | H | $CH_3$ | 7 | $CH_3$ | F |
| 1-$CH_3$,4-$CO_2CH_3$,5-pyrazolyl | H | H | 7/9 | F | $OCH_3$ |
| 1-$CH_3$,4-$CO_2CH_3$,5-pyrazolyl | H | $CH_3$ | 7 | F | $OCH_3$ |
| 1-$CH_3$,4-$CO_2CH_3$,5-pyrazolyl | H | H | 7/9 | $OCH_3$ | F |
| 1-$CH_3$,4-$CO_2CH_3$,5-pyrazolyl | H | $CH_3$ | 7 | $OCH_3$ | F |
| 1-$CH_3$,4-$CO_2CH_3$,5-pyrazolyl | H | H | 7/9 | $N(CH_3)_2$ | Cl |
| 1-$CH_3$,4-$CO_2CH_3$,5-pyrazolyl | H | $CH_3$ | 7 | $N(CH_3)_2$ | Cl |
| 1-$CH_3$,4-$CO_2CH_3$,5-pyrazolyl | H | H | 7/9 | $SCH_3$ | Cl |
| 1-$CH_3$,4-$CO_2CH_3$,5-pyrazolyl | H | $CH_3$ | 7 | $SCH_3$ | Cl |
| 2-Pyridyl | H | H | 7/9 | $CH_3$ | $CH_3$ |
| 2-Pyridyl | H | $CH_3$ | 7 | $CH_3$ | $CH_3$ |
| 2-Pyridyl | H | H | 7/9 | Cl | $CH_3$ |
| 2-Pyridyl | H | $CH_3$ | 7 | Cl | $CH_3$ |
| 2-Pyridyl | H | H | 7/9 | $CH_3$ | Cl |
| 2-Pyridyl | H | $CH_3$ | 7 | $CH_3$ | Cl |

Tabelle I (Fortsetzung)

| A | $R^1$ | $R^2$ | Pos. | $R^3$ | $R^4$ |
|---|---|---|---|---|---|
| 2-Pyridyl | H | H | 7/9 | Cl | Cl |
| 2-Pyridyl | H | $CH_3$ | 7 | Cl | Cl |
| 2-Pyridyl | H | H | 7/9 | $OCH_3$ | $OCH_3$ |
| 2-Pyridyl | H | $CH_3$ | 7 | $OCH_3$ | $OCH_3$ |
| 2-Pyridyl | H | H | 7/9 | $CH_3$ | $OCH_3$ |
| 2-Pyridyl | H | $CH_3$ | 7 | $CH_3$ | $OCH_3$ |
| 2-Pyridyl | H | H | 7/9 | $OCH_3$ | $CH_3$ |
| 2-Pyridyl | H | $CH_3$ | 7 | $OCH_3$ | $CH_3$ |
| 2-Pyridyl | H | H | 7/9 | Cl | $OCH_3$ |
| 2-Pyridyl | H | $CH_3$ | 7 | Cl | $OCH_3$ |
| 2-Pyridyl | H | H | 7/9 | $OCH_3$ | Cl |
| 2-Pyridyl | H | $CH_3$ | 7/9 | $OCH_3$ | Cl |
| 3-Cl,2-Pyridyl | H | H | 7/9 | $CH_3$ | $CH_3$ |
| 3-Cl,2-Pyridyl | H | $CH_3$ | 7 | $CH_3$ | $CH_3$ |
| 3-Cl,2-Pyridyl | H | H | 7/9 | Cl | $CH_3$ |
| 3-Cl,2-Pyridyl | H | $CH_3$ | 7 | Cl | $CH_3$ |
| 3-Cl,2-Pyridyl | H | H | 7/9 | $CH_3$ | Cl |
| 3-Cl,2-Pyridyl | H | $CH_3$ | 7 | $CH_3$ | Cl |
| 3-Cl,2-Pyridyl | H | H | 7/9 | Cl | Cl |
| 3-Cl,2-Pyridyl | H | $CH_3$ | 7 | Cl | Cl |
| 3-Cl,2-Pyridyl | H | H | 7/9 | $OCH_3$ | $OCH_3$ |
| 3-Cl,2-Pyridyl | H | $CH_3$ | 7 | $OCH_3$ | $OCH_3$ |
| 3-Cl,2-Pyridyl | H | H | 7/9 | $CH_3$ | $OCH_3$ |
| 3-Cl,2-Pyridyl | H | $CH_3$ | 7 | $CH_3$ | $OCH_3$ |
| 3-Cl,2-Pyridyl | H | H | 7/9 | $OCH_3$ | $CH_3$ |
| 3-Cl,2-Pyridyl | H | $CH_3$ | 7 | $OCH_3$ | $CH_3$ |
| 3-Cl,2-Pyridyl | H | H | 7/9 | Cl | $OCH_3$ |
| 3-Cl,2-Pyridyl | H | $CH_3$ | 7 | Cl | $OCH_3$ |
| 3-Cl,2-Pyridyl | H | H | 7/9 | $OCH_3$ | Cl |
| 3-Cl,2-Pyridyl | H | $CH_3$ | 7 | $OCH_3$ | Cl |
| 3-$CO_2CH_3$,2-Pyridyl | H | H | 7/9 | $CH_3$ | $CH_3$ |
| 3-$CO_2CH_3$,2-Pyridyl | H | $CH_3$ | 7 | $CH_3$ | $CH_3$ |
| 3-$CO_2CH_3$,2-Pyridyl | H | H | 7/9 | Cl | $CH_3$ |
| 3-$CO_2CH_3$,2-Pyridyl | H | $CH_3$ | 7 | Cl | $CH_3$ |
| 3-$CO_2CH_3$,2-Pyridyl | H | H | 7/9 | $CH_3$ | Cl |
| 3-$CO_2CH_3$,2-Pyridyl | H | $CH_3$ | 7 | $CH_3$ | Cl |
| 3-$CO_2CH_3$,2-Pyridyl | H | H | 7/9 | Cl | Cl |
| 3-$CO_2CH_3$,2-Pyridyl | H | $CH_3$ | 7 | Cl | Cl |
| 3-$CO_2CH_3$,2-Pyridyl | H | H | 7/9 | $OCH_3$ | $OCH_3$ |

Tabelle I (Fortsetzung)

| A | $R^1$ | $R^2$ | Pos. | $R^3$ | $R^4$ |
|---|---|---|---|---|---|
| 3-$CO_2CH_3$, 2-Pyridyl | H | $CH_3$ | 7 | $OCH_3$ | $OCH_3$ |
| 3-$CO_2CH_3$, 2-Pyridyl | H | H | 7/9 | $CH_3$ | $OCH_3$ |
| 3-$CO_2CH_3$, 2-Pyridyl | H | $CH_3$ | 7 | $CH_3$ | $OCH_3$ |
| 3-$CO_2CH_3$, 2-Pyridyl | H | H | 7/9 | $OCH_3$ | $CH_3$ |
| 3-$CO_2CH_3$, 2-Pyridyl | H | $CH_3$ | 7 | $OCH_3$ | $CH_3$ |
| 3-$CO_2CH_3$, 2-Pyridyl | H | H | 7/9 | Cl | $OCH_3$ |
| 3-$CO_2CH_3$, 2-Pyridyl | H | $CH_3$ | 7 | Cl | $OCH_3$ |
| 3-$CO_2CH_3$, 2-Pyridyl | H | H | 7/9 | $OCH_3$ | Cl |
| 3-$CO_2CH_3$, 2-Pyridyl | H | $CH_3$ | 7 | $OCH_3$ | Cl |
| 3-$CON(CH_3)_2$, 2-Pyridyl | H | H | 7/9 | $CH_3$ | $CH_3$ |
| 3-$CON(CH_3)_2$, 2-Pyridyl | H | $CH_3$ | 7 | $CH_3$ | $CH_3$ |
| 3-$CON(CH_3)_2$, 2-Pyridyl | H | H | 7/9 | Cl | $CH_3$ |
| 3-$CON(CH_3)_2$, 2-Pyridyl | H | $CH_3$ | 7 | Cl | $CH_3$ |
| 3-$CON(CH_3)_2$, 2-Pyridyl | H | H | 7/9 | $CH_3$ | Cl |
| 3-$CON(CH_3)_2$, 2-Pyridyl | H | $CH_3$ | 7 | $CH_3$ | Cl |
| 3-$CON(CH_3)_2$, 2-Pyridyl | H | H | 7/9 | Cl | Cl |
| 3-$CON(CH_3)_2$, 2-Pyridyl | H | $CH_3$ | 7 | Cl | Cl |
| 3-$CON(CH_3)_2$, 2-Pyridyl | H | H | 7/9 | $OCH_3$ | $OCH_3$ |
| 3-$CON(CH_3)_2$, 2-Pyridyl | H | $CH_3$ | 7 | $OCH_3$ | $OCH_3$ |
| 3-$CON(CH_3)_2$, 2-Pyridyl | H | H | 7/9 | $CH_3$ | $OCH_3$ |
| 3-$CON(CH_3)_2$, 2-Pyridyl | H | $CH_3$ | 7 | $CH_3$ | $OCH_3$ |
| 3-$CON(CH_3)_2$, 2-Pyridyl | H | H | 7/9 | $OCH_3$ | $CH_3$ |
| 3-$CON(CH_3)_2$, 2-Pyridyl | H | $CH_3$ | 7 | $OCH_3$ | $CH_3$ |
| 3-$CON(CH_3)_2$, 2-Pyridyl | H | H | 7/9 | Cl | $OCH_3$ |
| 3-$CON(CH_3)_2$, 2-Pyridyl | H | $CH_3$ | 7 | Cl | $OCH_3$ |
| 3-$CON(CH_3)_2$, 2-Pyridyl | H | H | 7/9 | $OCH_3$ | Cl |
| 3-$CON(CH_3)_2$, 2-Pyridyl | H | $CH_3$ | 7 | $OCH_3$ | Cl |
| 3-$CON(CH_3)_2$, 2-Pyridyl | $CH_3$ | H | 7/9 | $CH_3$ | $CH_3$ |
| 3-$CON(CH_3)_2$, 2-Pyridyl | $CH_3$ | $CH_3$ | 7 | $CH_3$ | $CH_3$ |
| 3-$CON(CH_3)_2$, 2-Pyridyl | $CH_3$ | H | 7/9 | Cl | $CH_3$ |
| 3-$CON(CH_3)_2$, 2-Pyridyl | $CH_3$ | $CH_3$ | 7 | Cl | $CH_3$ |
| 3-$CON(CH_3)_2$, 2-Pyridyl | $CH_3$ | H | 7/9 | $CH_3$ | Cl |
| 3-$CON(CH_3)_2$, 2-Pyridyl | $CH_3$ | $CH_3$ | 7 | $CH_3$ | Cl |
| 3-$CON(CH_3)_2$, 2-Pyridyl | $CH_3$ | H | 7/9 | Cl | Cl |
| 3-$CON(CH_3)_2$, 2-Pyridyl | $CH_3$ | $CH_3$ | 7 | Cl | Cl |
| 3-$CON(CH_3)_2$, 2-Pyridyl | $CH_3$ | H | 7/9 | $OCH_3$ | $OCH_3$ |
| 3-$CON(CH_3)_2$, 2-Pyridyl | $CH_3$ | $CH_3$ | 7 | $OCH_3$ | $OCH_3$ |
| 3-$CON(CH_3)_2$, 2-Pyridyl | $CH_3$ | H | 7/9 | $CH_3$ | $OCH_3$ |
| 3-$CON(CH_3)_2$, 2-Pyridyl | $CH_3$ | $CH_3$ | 7 | $CH_3$ | $OCH_3$ |

Tabelle I (Fortsetzung)

| A | $R^1$ | $R^2$ | Pos. | $R^3$ | $R^4$ |
|---|---|---|---|---|---|
| 3-CON($CH_3$)$_2$,2-Pyridyl | $CH_3$ | H | 7/9 | $OCH_3$ | $CH_3$ |
| 3-CON($CH_3$)$_2$,2-Pyridyl | $CH_3$ | $CH_3$ | 7 | $OCH_3$ | $CH_3$ |
| 3-CON($CH_3$)$_2$,2-Pyridyl | $CH_3$ | H | 7/9 | Cl | $OCH_3$ |
| 3-CON($CH_3$)$_2$,2-Pyridyl | $CH_3$ | $CH_3$ | 7 | Cl | $OCH_3$ |
| 3-CON($CH_3$)$_2$,2-Pyridyl | $CH_3$ | H | 7/9 | $OCH_3$ | Cl |
| 3-CON($CH_3$)$_2$,2-Pyridyl | $CH_3$ | $CH_3$ | 7 | $OCH_3$ | Cl |
| 3-CON($CH_3$)$_2$,2-Pyridyl | H | H | 7/9 | $CF_3$ | $CF_3$ |
| 3-CON($CH_3$)$_2$,2-Pyridyl | H | $CH_3$ | 7 | $CF_3$ | $CF_3$ |
| 3-CON($CH_3$)$_2$,2-Pyridyl | H | H | 7/9 | Cl | $CF_3$ |
| 3-CON($CH_3$)$_2$,2-Pyridyl | H | $CH_3$ | 7 | Cl | $CF_3$ |
| 3-CON($CH_3$)$_2$,2-Pyridyl | H | H | 7/9 | $CF_3$ | Cl |
| 3-CON($CH_3$)$_2$,2-Pyridyl | H | $CH_3$ | 7 | $CF_3$ | Cl |
| 3-CON($CH_3$)$_2$,2-Pyridyl | H | H | 7/9 | F | $CF_3$ |
| 3-CON($CH_3$)$_2$,2-Pyridyl | H | $CH_3$ | 7 | F | $CF_3$ |
| 3-CON($CH_3$)$_2$,2-Pyridyl | H | H | 7/9 | $CF_3$ | F |
| 3-CON($CH_3$)$_2$,2-Pyridyl | H | $CH_3$ | 7 | $CF_3$ | F |
| 3-CON($CH_3$)$_2$,2-Pyridyl | H | H | 7/9 | $CH_3$ | $CF_3$ |
| 3-CON($CH_3$)$_2$,2-Pyridyl | H | $CH_3$ | 7 | $CH_3$ | $CF_3$ |
| 3-CON($CH_3$)$_2$,2-Pyridyl | H | H | 7/9 | $CF_3$ | $CH_3$ |
| 3-CON($CH_3$)$_2$,2-Pyridyl | H | $CH_3$ | 7 | $CF_3$ | $CH_3$ |
| 3-CON($CH_3$)$_2$,2-Pyridyl | H | H | 7/9 | $OCH_3$ | $CF_3$ |
| 3-CON($CH_3$)$_2$,2-Pyridyl | H | $CH_3$ | 7 | $OCH_3$ | $CF_3$ |
| 3-CON($CH_3$)$_2$,2-Pyridyl | H | H | 7/9 | $CF_3$ | $OCH_3$ |
| 3-CON($CH_3$)$_2$,2-Pyridyl | H | $CH_3$ | 7 | $CF_3$ | $OCH_3$ |
| 3-CON($CH_3$)$_2$,2-Pyridyl | H | H | 7/9 | F | F |
| 3-CON($CH_3$)$_2$,2-Pyridyl | H | $CH_3$ | 7 | F | F |
| 3-CON($CH_3$)$_2$,2-Pyridyl | H | H | 7/9 | F | Cl |
| 3-CON($CH_3$)$_2$,2-Pyridyl | H | $CH_3$ | 7 | F | Cl |
| 3-CON($CH_3$)$_2$,2-Pyridyl | H | H | 7/9 | Cl | F |
| 3-CON($CH_3$)$_2$,2-Pyridyl | H | $CH_3$ | 7 | Cl | F |
| 3-CON($CH_3$)$_2$,2-Pyridyl | H | H | 7/9 | F | $CH_3$ |
| 3-CON($CH_3$)$_2$,2-Pyridyl | H | $CH_3$ | 7 | F | $CH_3$ |
| 3-CON($CH_3$)$_2$,2-Pyridyl | H | H | 7/9 | $CH_3$ | F |
| 3-CON($CH_3$)$_2$,2-Pyridyl | H | $CH_3$ | 7 | $CH_3$ | F |
| 3-CON($CH_3$)$_2$,2-Pyridyl | H | H | 7/9 | F | $OCH_3$ |
| 3-CON($CH_3$)$_2$,2-Pyridyl | H | $CH_3$ | 7 | F | $OCH_3$ |
| 3-CON($CH_3$)$_2$,2-Pyridyl | H | H | 7/9 | $OCH_3$ | F |
| 3-CON($CH_3$)$_2$,2-Pyridyl | H | $CH_3$ | 7 | $OCH_3$ | F |
| 3-CON($CH_3$)$_2$,2-Pyridyl | H | H | 7/9 | N($CH_3$)$_2$ | Cl |

Tabelle I (Fortsetzung)

| A | $R^1$ | $R^2$ | Pos. | $R^3$ | $R^4$ |
|---|---|---|---|---|---|
| 3-CON($CH_3$)$_2$,2-Pyridyl | H | $CH_3$ | 7 | N($CH_3$)$_2$ | Cl |
| 3-CON($CH_3$)$_2$,2-Pyridyl | H | H | 7/9 | $SCH_3$ | Cl |
| 3-CON($CH_3$)$_2$,2-Pyridyl | H | $CH_3$ | 7 | $SCH_3$ | Cl |
| 3-Pyridyl | H | H | 7/9 | $CH_3$ | $CH_3$ |
| 3-Pyridyl | H | $CH_3$ | 7 | $CH_3$ | $CH_3$ |
| 3-Pyridyl | H | H | 7/9 | Cl | $CH_3$ |
| 3-Pyridyl | H | $CH_3$ | 7 | Cl | $CH_3$ |
| 3-Pyridyl | H | H | 7/9 | $CH_3$ | Cl |
| 3-Pyridyl | H | $CH_3$ | 7 | $CH_3$ | Cl |
| 3-Pyridyl | H | H | 7/9 | Cl | Cl |
| 3-Pyridyl | H | $CH_3$ | 7 | Cl | Cl |
| 3-Pyridyl | H | H | 7/9 | $OCH_3$ | $OCH_3$ |
| 3-Pyridyl | H | $CH_3$ | 7 | $OCH_3$ | $OCH_3$ |
| 3-Pyridyl | H | H | 7/9 | $CH_3$ | $OCH_3$ |
| 3-Pyridyl | H | $CH_3$ | 7 | $CH_3$ | $OCH_3$ |
| 3-Pyridyl | H | H | 7/9 | $OCH_3$ | $CH_3$ |
| 3-Pyridyl | H | $CH_3$ | 7 | $OCH_3$ | $CH_3$ |
| 3-Pyridyl | H | H | 7/9 | Cl | $OCH_3$ |
| 3-Pyridyl | H | $CH_3$ | 7 | Cl | $OCH_3$ |
| 3-Pyridyl | H | H | 7/9 | $OCH_3$ | Cl |
| 3-Pyridyl | H | $CH_3$ | 7 | $OCH_3$ | Cl |
| 8-Chinolyl | H | H | 7/9 | $CH_3$ | $CH_3$ |
| 8-Chinolyl | H | $CH_3$ | 7 | $CH_3$ | $CH_3$ |
| 8-Chinolyl | H | H | 7/9 | Cl | $CH_3$ |
| 8-Chinolyl | H | $CH_3$ | 7 | Cl | $CH_3$ |
| 8-Chinolyl | H | H | 7/9 | $CH_3$ | Cl |
| 8-Chinolyl | H | $CH_3$ | 7 | $CH_3$ | Cl |
| 8-Chinolyl | H | H | 7/9 | Cl | Cl |
| 8-Chinolyl | H | $CH_3$ | 7 | Cl | Cl |
| 8-Chinolyl | H | H | 7/9 | $OCH_3$ | $OCH_3$ |
| 8-Chinolyl | H | $CH_3$ | 7 | $OCH_3$ | $OCH_3$ |
| 8-Chinolyl | H | H | 7/9 | $CH_3$ | $OCH_3$ |
| 8-Chinolyl | H | $CH_3$ | 7 | $CH_3$ | $OCH_3$ |
| 8-Chinolyl | H | H | 7/9 | $OCH_3$ | $CH_3$ |
| 8-Chinolyl | H | $CH_3$ | 7 | $OCH_3$ | $CH_3$ |
| 8-Chinolyl | H | H | 7/9 | Cl | $OCH_3$ |
| 8-Chinolyl | H | $CH_3$ | 7 | Cl | $OCH_3$ |
| 8-Chinolyl | H | H | 7/9 | $OCH_3$ | Cl |
| 8-Chinolyl | H | $CH_3$ | 7 | $OCH_3$ | Cl |

Tabelle I (Fortsetzung)

| A | $R^1$ | $R^2$ | Pos. | $R^3$ | $R^4$ |
|---|---|---|---|---|---|
| 7-$CO_2CH_3$,8-Chinolyl | H | H | 7/9 | $CH_3$ | $CH_3$ |
| 7-$CO_2CH_3$,8-Chinolyl | H | $CH_3$ | 7 | $CH_3$ | $CH_3$ |
| 7-$CO_2CH_3$,8-Chinolyl | H | H | 7/9 | Cl | $CH_3$ |
| 7-$CO_2CH_3$,8-Chinolyl | H | $CH_3$ | 7 | Cl | $CH_3$ |
| 7-$CO_2CH_3$,8-Chinolyl | H | H | 7/9 | $CH_3$ | Cl |
| 7-$CO_2CH_3$,8-Chinolyl | H | $CH_3$ | 7 | $CH_3$ | Cl |
| 7-$CO_2CH_3$,8-Chinolyl | H | H | 7/9 | Cl | Cl |
| 7-$CO_2CH_3$,8-Chinolyl | H | $CH_3$ | 7 | Cl | Cl |
| 7-$CO_2CH_3$,8-Chinolyl | H | H | 7/9 | $OCH_3$ | $OCH_3$ |
| 7-$CO_2CH_3$,8-Chinolyl | H | $CH_3$ | 7 | $OCH_3$ | $OCH_3$ |
| 7-$CO_2CH_3$,8-Chinolyl | H | H | 7/9 | $CH_3$ | $OCH_3$ |
| 7-$CO_2CH_3$,8-Chinolyl | H | $CH_3$ | 7 | $CH_3$ | $OCH_3$ |
| 7-$CO_2CH_3$,8-Chinolyl | H | H | 7/9 | $OCH_3$ | $CH_3$ |
| 7-$CO_2CH_3$,8-Chinolyl | H | $CH_3$ | 7 | $OCH_3$ | $CH_3$ |
| 7-$CO_2CH_3$,8-Chinolyl | H | H | 7/9 | Cl | $OCH_3$ |
| 7-$CO_2CH_3$,8-Chinolyl | H | $CH_3$ | 7 | Cl | $OCH_3$ |
| 7-$CO_2CH_3$,8-Chinolyl | H | H | 7/9 | $OCH_3$ | Cl |
| 7-$CO_2CH_3$,8-Chinolyl | H | $CH_3$ | 7 | $OCH_3$ | Cl |
| 7-Cl,8-Chinolyl | H | H | 7/9 | $CH_3$ | $CH_3$ |
| 7-Cl,8-Chinolyl | H | $CH_3$ | 7 | $CH_3$ | $CH_3$ |
| 7-Cl,8-Chinolyl | H | H | 7/9 | Cl | $CH_3$ |
| 7-Cl,8-Chinolyl | H | $CH_3$ | 7 | Cl | $CH_3$ |
| 7-Cl,8-Chinolyl | H | H | 7/9 | $CH_3$ | Cl |
| 7-Cl,8-Chinolyl | H | $CH_3$ | 7 | $CH_3$ | Cl |
| 7-Cl,8-Chinolyl | H | H | 7/9 | Cl | Cl |
| 7-Cl,8-Chinolyl | H | $CH_3$ | 7 | Cl | Cl |
| 7-Cl,8-Chinolyl | H | H | 7/9 | $OCH_3$ | $OCH_3$ |
| 7-Cl,8-Chinolyl | H | $CH_3$ | 7 | $OCH_3$ | $OCH_3$ |
| 7-Cl,8-Chinolyl | H | H | 7/9 | $CH_3$ | $OCH_3$ |
| 7-Cl,8-Chinolyl | H | $CH_3$ | 7 | $CH_3$ | $OCH_3$ |
| 7-Cl,8-Chinolyl | H | H | 7/9 | $OCH_3$ | $CH_3$ |
| 7-Cl,8-Chinolyl | H | $CH_3$ | 7 | $OCH_3$ | $CH_3$ |
| 7-Cl,8-Chinolyl | H | H | 7/9 | Cl | $OCH_3$ |
| 7-Cl,8-Chinolyl | H | $CH_3$ | 7 | Cl | $OCH_3$ |
| 7-Cl,8-Chinolyl | H | H | 7/9 | $OCH_3$ | Cl |
| 7-Cl,8-Chinolyl | H | $CH_3$ | 7 | $OCH_3$ | Cl |
| 7-Cl,8-Chinolyl | H | H | 7/9 | $CH_3$ | $CH_3$ |
| 7-Cl,8-Chinolyl | H | $CH_3$ | 7 | $CH_3$ | $CH_3$ |
| 7-Cl,8-Chinolyl | H | H | 7/9 | Cl | $CH_3$ |

Tabelle I (Fortsetzung)

| A | $R^1$ | $R^2$ | Pos. | $R^3$ | $R^4$ |
|---|---|---|---|---|---|
| 7-Cl,8-Chinolyl | H | $CH_3$ | 7 | Cl | $CH_3$ |
| 7-Cl,8-Chinolyl | H | H | 7/9 | $CH_3$ | Cl |
| 7-Cl,8-Chinolyl | H | $CH_3$ | 7 | $CH_3$ | Cl |
| 7-Cl,8-Chinolyl | H | H | 7/9 | Cl | Cl |
| 7-Cl,8-Chinolyl | H | $CH_3$ | 7 | Cl | Cl |
| 7-Cl,8-Chinolyl | H | H | 7/9 | $OCH_3$ | $OCH_3$ |
| 7-Cl,8-Chinolyl | H | $CH_3$ | 7 | $OCH_3$ | $OCH_3$ |
| 7-Cl,8-Chinolyl | H | H | 7/9 | $CH_3$ | $OCH_3$ |
| 7-Cl,8-Chinolyl | H | $CH_3$ | 7 | $CH_3$ | $OCH_3$ |
| 7-Cl,8-Chinolyl | H | H | 7/9 | $OCH_3$ | $CH_3$ |
| 7-Cl,8-Chinolyl | H | $CH_3$ | 7 | $OCH_3$ | $CH_3$ |
| 7-Cl,8-Chinolyl | H | H | 7/9 | Cl | $OCH_3$ |
| 7-Cl,8-Chinolyl | H | $CH_3$ | 7 | Cl | $OCH_3$ |
| 7-Cl,8-Chinolyl | H | H | 7/9 | $OCH_3$ | Cl |
| 7-Cl,8-Chinolyl | H | $CH_3$ | 7 | $OCH_3$ | Cl |

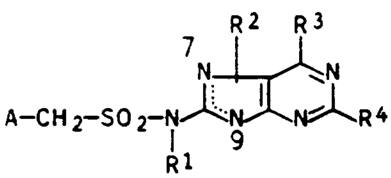

Ib

Tabelle II

| A | $R^1$ | $R^2$ | Pos. | $R^3$ | $R^4$ |
|---|---|---|---|---|---|
| Phenyl | H | H | 7/9 | $CH_3$ | $CH_3$ |
| Phenyl | H | $CH_3$ | 7 | $CH_3$ | $CH_3$ |
| Phenyl | H | H | 7/9 | Cl | $CH_3$ |
| Phenyl | H | $CH_3$ | 7 | Cl | $CH_3$ |
| Phenyl | H | H | 7/9 | $CH_3$ | Cl |
| Phenyl | H | $CH_3$ | 7 | $CH_3$ | Cl |
| Phenyl | H | H | 7/9 | Cl | Cl |
| Phenyl | H | $CH_3$ | 7 | Cl | Cl |
| Phenyl | H | H | 7/9 | $OCH_3$ | $OCH_3$ |
| Phenyl | H | $CH_3$ | 7 | $OCH_3$ | $OCH_3$ |
| Phenyl | H | H | 7/9 | $CH_3$ | $OCH_3$ |
| Phenyl | H | $CH_3$ | 7 | $CH_3$ | $OCH_3$ |
| Phenyl | H | H | 7/9 | $OCH_3$ | $CH_3$ |
| Phenyl | H | $CH_3$ | 7 | $OCH_3$ | $CH_3$ |
| Phenyl | H | H | 7/9 | Cl | $OCH_3$ |
| Phenyl | H | $CH_3$ | 7 | Cl | $OCH_3$ |
| Phenyl | H | H | 7/9 | $OCH_3$ | Cl |
| Phenyl | H | $CH_3$ | 7 | $OCH_3$ | Cl |
| 2-Cl-Phenyl | H | H | 7/9 | $CH_3$ | $CH_3$ |
| 2-Cl-Phenyl | H | $CH_3$ | 7 | $CH_3$ | $CH_3$ |
| 2-Cl-Phenyl | H | H | 7/9 | Cl | $CH_3$ |
| 2-Cl-Phenyl | H | $CH_3$ | 7 | Cl | $CH_3$ |
| 2-Cl-Phenyl | H | H | 7/9 | $CH_3$ | Cl |
| 2-Cl-Phenyl | H | $CH_3$ | 7 | $CH_3$ | Cl |
| 2-Cl-Phenyl | H | H | 7/9 | Cl | Cl |
| 2-Cl-Phenyl | H | $CH_3$ | 7 | Cl | Cl |
| 2-Cl-Phenyl | H | H | 7/9 | $OCH_3$ | $OCH_3$ |
| 2-Cl-Phenyl | H | $CH_3$ | 7 | $OCH_3$ | $OCH_3$ |
| 2-Cl-Phenyl | H | H | 7/9 | $CH_3$ | $OCH_3$ |
| 2-Cl-Phenyl | H | $CH_3$ | 7 | $CH_3$ | $OCH_3$ |
| 2-Cl-Phenyl | H | H | 7/9 | $OCH_3$ | $CH_3$ |
| 2-Cl-Phenyl | H | $CH_3$ | 7 | $OCH_3$ | $CH_3$ |
| 2-Cl-Phenyl | H | H | 7/9 | Cl | $OCH_3$ |

Tabelle II (Fortsetzung)

| A | $R^1$ | $R^2$ | Pos. | $R^3$ | $R^4$ |
|---|---|---|---|---|---|
| 2-Cl-Phenyl | H | $CH_3$ | 7 | Cl | $OCH_3$ |
| 2-Cl-Phenyl | H | H | 7/9 | $OCH_3$ | Cl |
| 2-Cl-Phenyl | H | $CH_3$ | 7 | $OCH_3$ | Cl |
| 2-F-Phenyl | H | H | 7/9 | $CH_3$ | $CH_3$ |
| 2-F-Phenyl | H | $CH_3$ | 7 | $CH_3$ | $CH_3$ |
| 2-F-Phenyl | H | H | 7/9 | Cl | $CH_3$ |
| 2-F-Phenyl | H | $CH_3$ | 7 | Cl | $CH_3$ |
| 2-F-Phenyl | H | H | 7/9 | $CH_3$ | Cl |
| 2-F-Phenyl | H | $CH_3$ | 7 | $CH_3$ | Cl |
| 2-F-Phenyl | H | H | 7/9 | Cl | Cl |
| 2-F-Phenyl | H | $CH_3$ | 7 | Cl | Cl |
| 2-F-Phenyl | H | H | 7/9 | $OCH_3$ | $OCH_3$ |
| 2-F-Phenyl | H | $CH_3$ | 7 | $OCH_3$ | $OCH_3$ |
| 2-F-Phenyl | H | H | 7/9 | $CH_3$ | $OCH_3$ |
| 2-F-Phenyl | H | $CH_3$ | 7 | $CH_3$ | $OCH_3$ |
| 2-F-Phenyl | H | H | 7/9 | $OCH_3$ | $CH_3$ |
| 2-F-Phenyl | H | $CH_3$ | 7 | $OCH_3$ | $CH_3$ |
| 2-F-Phenyl | H | H | 7/9 | Cl | $OCH_3$ |
| 2-F-Phenyl | H | $CH_3$ | 7 | Cl | $OCH_3$ |
| 2-F-Phenyl | H | H | 7/9 | $OCH_3$ | Cl |
| 2-F-Phenyl | H | $CH_3$ | 7 | $OCH_3$ | Cl |
| 2-$CH_3$-Phenyl | H | H | 7/9 | $CH_3$ | $CH_3$ |
| 2-$CH_3$-Phenyl | H | $CH_3$ | 7 | $CH_3$ | $CH_3$ |
| 2-$CH_3$-Phenyl | H | H | 7/9 | Cl | $CH_3$ |
| 2-$CH_3$-Phenyl | H | $CH_3$ | 7 | Cl | $CH_3$ |
| 2-$CH_3$-Phenyl | H | H | 7/9 | $CH_3$ | Cl |
| 2-$CH_3$-Phenyl | H | $CH_3$ | 7 | $CH_3$ | Cl |
| 2-$CH_3$-Phenyl | H | H | 7/9 | Cl | Cl |
| 2-$CH_3$-Phenyl | H | $CH_3$ | 7 | Cl | Cl |
| 2-$CH_3$-Phenyl | H | H | 7/9 | $OCH_3$ | $OCH_3$ |
| 2-$CH_3$-Phenyl | H | $CH_3$ | 7 | $OCH_3$ | $OCH_3$ |
| 2-$CH_3$-Phenyl | H | H | 7/9 | $CH_3$ | $OCH_3$ |
| 2-$CH_3$-Phenyl | H | $CH_3$ | 7 | $CH_3$ | $OCH_3$ |
| 2-$CH_3$-Phenyl | H | H | 7/9 | $OCH_3$ | $CH_3$ |
| 2-$CH_3$-Phenyl | H | $CH_3$ | 7 | $OCH_3$ | $CH_3$ |
| 2-$CH_3$-Phenyl | H | H | 7/9 | Cl | $OCH_3$ |
| 2-$CH_3$-Phenyl | H | $CH_3$ | 7 | Cl | $OCH_3$ |
| 2-$CH_3$-Phenyl | H | H | 7/9 | $OCH_3$ | Cl |
| 2-$CH_3$-Phenyl | H | $CH_3$ | 7 | $OCH_3$ | Cl |

Tabelle II (Fortsetzung)

| A | $R^1$ | $R^2$ | Pos. | $R^3$ | $R^4$ |
|---|---|---|---|---|---|
| 2-$CO_2CH_3$-Phenyl | H | H | 7/9 | $CH_3$ | $CH_3$ |
| 2-$CO_2CH_3$-Phenyl | H | $CH_3$ | 7 | $CH_3$ | $CH_3$ |
| 2-$CO_2CH_3$-Phenyl | H | H | 7/9 | Cl | $CH_3$ |
| 2-$CO_2CH_3$-Phenyl | H | $CH_3$ | 7 | Cl | $CH_3$ |
| 2-$CO_2CH_3$-Phenyl | H | H | 7/9 | $CH_3$ | Cl |
| 2-$CO_2CH_3$-Phenyl | H | $CH_3$ | 7 | $CH_3$ | Cl |
| 2-$CO_2CH_3$-Phenyl | H | H | 7/9 | Cl | Cl |
| 2-$CO_2CH_3$-Phenyl | H | $CH_3$ | 7 | Cl | Cl |
| 2-$CO_2CH_3$-Phenyl | H | H | 7/9 | $OCH_3$ | $OCH_3$ |
| 2-$CO_2CH_3$-Phenyl | H | $CH_3$ | 7 | $OCH_3$ | $OCH_3$ |
| 2-$CO_2CH_3$-Phenyl | H | H | 7/9 | $CH_3$ | $OCH_3$ |
| 2-$CO_2CH_3$-Phenyl | H | $CH_3$ | 7 | $CH_3$ | $OCH_3$ |
| 2-$CO_2CH_3$-Phenyl | H | H | 7/9 | $OCH_3$ | $CH_3$ |
| 2-$CO_2CH_3$-Phenyl | H | $CH_3$ | 7 | $OCH_3$ | $CH_3$ |
| 2-$CO_2CH_3$-Phenyl | H | H | 7/9 | Cl | $OCH_3$ |
| 2-$CO_2CH_3$-Phenyl | H | $CH_3$ | 7 | Cl | $OCH_3$ |
| 2-$CO_2CH_3$-Phenyl | H | H | 7/9 | $OCH_3$ | Cl |
| 2-$CO_2CH_3$-Phenyl | H | $CH_3$ | 7 | $OCH_3$ | Cl |
| 2-$CO_2C_2H_5$-Phenyl | H | H | 7/9 | $CH_3$ | $CH_3$ |
| 2-$CO_2C_2H_5$-Phenyl | H | $CH_3$ | 7 | $CH_3$ | $CH_3$ |
| 2-$CO_2C_2H_5$-Phenyl | H | H | 7/9 | Cl | $CH_3$ |
| 2-$CO_2C_2H_5$-Phenyl | H | $CH_3$ | 7 | Cl | $CH_3$ |
| 2-$CO_2C_2H_5$-Phenyl | H | H | 7/9 | $CH_3$ | Cl |
| 2-$CO_2C_2H_5$-Phenyl | H | $CH_3$ | 7 | $CH_3$ | Cl |
| 2-$CO_2C_2H_5$-Phenyl | H | H | 7/9 | Cl | Cl |
| 2-$CO_2C_2H_5$-Phenyl | H | $CH_3$ | 7 | Cl | Cl |
| 2-$CO_2C_2H_5$-Phenyl | H | H | 7/9 | $OCH_3$ | $OCH_3$ |
| 2-$CO_2C_2H_5$-Phenyl | H | $CH_3$ | 7 | $OCH_3$ | $OCH_3$ |
| 2-$CO_2C_2H_5$-Phenyl | H | H | 7/9 | $CH_3$ | $OCH_3$ |
| 2-$CO_2C_2H_5$-Phenyl | H | $CH_3$ | 7 | $CH_3$ | $OCH_3$ |
| 2-$CO_2C_2H_5$-Phenyl | H | H | 7/9 | $OCH_3$ | $CH_3$ |
| 2-$CO_2C_2H_5$-Phenyl | H | $CH_3$ | 7 | $OCH_3$ | $CH_3$ |
| 2-$CO_2C_2H_5$-Phenyl | H | H | 7/9 | Cl | $OCH_3$ |
| 2-$CO_2C_2H_5$-Phenyl | H | $CH_3$ | 7 | Cl | $OCH_3$ |
| 2-$CO_2C_2H_5$-Phenyl | H | H | 7/9 | $OCH_3$ | Cl |
| 2-$CO_2C_2H_5$-Phenyl | H | $CH_3$ | 7 | $OCH_3$ | Cl |
| 2-$OCH_3$-Phenyl | H | H | 7/9 | $CH_3$ | $CH_3$ |
| 2-$OCH_3$-Phenyl | H | $CH_3$ | 7 | $CH_3$ | $CH_3$ |
| 2-$OCH_3$-Phenyl | H | H | 7/9 | Cl | $CH_3$ |

Tabelle II (Fortsetzung)

| A | $R^1$ | $R^2$ | Pos. | $R^3$ | $R^4$ |
|---|---|---|---|---|---|
| 2-$OCH_3$-Phenyl | H | $CH_3$ | 7 | Cl | $CH_3$ |
| 2-$OCH_3$-Phenyl | H | H | 7/9 | $CH_3$ | Cl |
| 2-$OCH_3$-Phenyl | H | $CH_3$ | 7 | $CH_3$ | Cl |
| 2-$OCH_3$-Phenyl | H | H | 7/9 | Cl | Cl |
| 2-$OCH_3$-Phenyl | H | $CH_3$ | 7 | Cl | Cl |
| 2-$OCH_3$-Phenyl | H | H | 7/9 | $OCH_3$ | $OCH_3$ |
| 2-$OCH_3$-Phenyl | H | $CH_3$ | 7 | $OCH_3$ | $OCH_3$ |
| 2-$OCH_3$-Phenyl | H | H | 7/9 | $CH_3$ | $OCH_3$ |
| 2-$OCH_3$-Phenyl | H | $CH_3$ | 7 | $CH_3$ | $OCH_3$ |
| 2-$OCH_3$-Phenyl | H | H | 7/9 | $OCH_3$ | $CH_3$ |
| 2-$OCH_3$-Phenyl | H | $CH_3$ | 7 | $OCH_3$ | $CH_3$ |
| 2-$OCH_3$-Phenyl | H | H | 7/9 | Cl | $OCH_3$ |
| 2-$OCH_3$-Phenyl | H | $CH_3$ | 7 | Cl | $OCH_3$ |
| 2-$OCH_3$-Phenyl | H | H | 7/9 | $OCH_3$ | Cl |
| 2-$OCH_3$-Phenyl | H | $CH_3$ | 7 | $OCH_3$ | Cl |
| 2-$OCH_2CH_2OCH_3$-Phenyl | H | H | 7/9 | $CH_3$ | $CH_3$ |
| 2-$OCH_2CH_2OCH_3$-Phenyl | H | $CH_3$ | 7 | $CH_3$ | $CH_3$ |
| 2-$OCH_2CH_2OCH_3$-Phenyl | H | H | 7/9 | Cl | $CH_3$ |
| 2-$OCH_2CH_2OCH_3$-Phenyl | H | $CH_3$ | 7 | Cl | $CH_3$ |
| 2-$OCH_2CH_2OCH_3$-Phenyl | H | H | 7/9 | $CH_3$ | Cl |
| 2-$OCH_2CH_2OCH_3$-Phenyl | H | $CH_3$ | 7 | $CH_3$ | Cl |
| 2-$OCH_2CH_2OCH_3$-Phenyl | H | H | 7/9 | Cl | Cl |
| 2-$OCH_2CH_2OCH_3$-Phenyl | H | $CH_3$ | 7 | Cl | Cl |
| 2-$OCH_2CH_2OCH_3$-Phenyl | H | H | 7/9 | $OCH_3$ | $OCH_3$ |
| 2-$OCH_2CH_2OCH_3$-Phenyl | H | $CH_3$ | 7 | $OCH_3$ | $OCH_3$ |
| 2-$OCH_2CH_2OCH_3$-Phenyl | H | H | 7/9 | $CH_3$ | $OCH_3$ |
| 2-$OCH_2CH_2OCH_3$-Phenyl | H | $CH_3$ | 7 | $CH_3$ | $OCH_3$ |
| 2-$OCH_2CH_2OCH_3$-Phenyl | H | H | 7/9 | $OCH_3$ | $CH_3$ |
| 2-$OCH_2CH_2OCH_3$-Phenyl | H | $CH_3$ | 7 | $OCH_3$ | $CH_3$ |
| 2-$OCH_2CH_2OCH_3$-Phenyl | H | H | 7/9 | Cl | $OCH_3$ |
| 2-$OCH_2CH_2OCH_3$-Phenyl | H | $CH_3$ | 7 | Cl | $OCH_3$ |
| 2-$OCH_2CH_2OCH_3$-Phenyl | H | H | 7/9 | $OCH_3$ | Cl |
| 2-$OCH_2CH_2OCH_3$-Phenyl | H | $CH_3$ | 7 | $OCH_3$ | Cl |
| 2-$OCH_2CH_2Cl$-Phenyl | H | H | 7/9 | $CH_3$ | $CH_3$ |
| 2-$OCH_2CH_2Cl$-Phenyl | H | $CH_3$ | 7 | $CH_3$ | $CH_3$ |
| 2-$OCH_2CH_2Cl$-Phenyl | H | H | 7/9 | Cl | $CH_3$ |
| 2-$OCH_2CH_2Cl$-Phenyl | H | $CH_3$ | 7 | Cl | $CH_3$ |
| 2-$OCH_2CH_2Cl$-Phenyl | H | H | 7/9 | $CH_3$ | Cl |
| 2-$OCH_2CH_2Cl$-Phenyl | H | $CH_3$ | 7 | $CH_3$ | Cl |

Tabelle II (Fortsetzung)

| A | $R^1$ | $R^2$ | Pos. | $R^3$ | $R^4$ |
|---|---|---|---|---|---|
| 2-$OCH_2CH_2Cl$-Phenyl | H | H | 7/9 | Cl | Cl |
| 2-$OCH_2CH_2Cl$-Phenyl | H | $CH_3$ | 7 | Cl | Cl |
| 2-$OCH_2CH_2Cl$-Phenyl | H | H | 7/9 | $OCH_3$ | $OCH_3$ |
| 2-$OCH_2CH_2Cl$-Phenyl | H | $CH_3$ | 7 | $OCH_3$ | $OCH_3$ |
| 2-$OCH_2CH_2Cl$-Phenyl | H | H | 7/9 | $CH_3$ | $OCH_3$ |
| 2-$OCH_2CH_2Cl$-Phenyl | H | $CH_3$ | 7 | $CH_3$ | $OCH_3$ |
| 2-$OCH_2CH_2Cl$-Phenyl | H | H | 7/9 | $OCH_3$ | $CH_3$ |
| 2-$OCH_2CH_2Cl$-Phenyl | H | $CH_3$ | 7 | $OCH_3$ | $CH_3$ |
| 2-$OCH_2CH_2Cl$-Phenyl | H | H | 7/9 | Cl | $OCH_3$ |
| 2-$OCH_2CH_2Cl$-Phenyl | H | $CH_3$ | 7 | Cl | $OCH_3$ |
| 2-$OCH_2CH_2Cl$-Phenyl | H | H | 7/9 | $OCH_3$ | Cl |
| 2-$OCH_2CH_2Cl$-Phenyl | H | $CH_3$ | 7 | $OCH_3$ | Cl |
| 2-$OCH_2SO_2CH_3$-Phenyl | H | H | 7/9 | $CH_3$ | $CH_3$ |
| 2-$OCH_2SO_2CH_3$-Phenyl | H | $CH_3$ | 7 | $CH_3$ | $CH_3$ |
| 2-$OCH_2SO_2CH_3$-Phenyl | H | H | 7/9 | Cl | $CH_3$ |
| 2-$OCH_2SO_2CH_3$-Phenyl | H | $CH_3$ | 7 | Cl | $CH_3$ |
| 2-$OCH_2SO_2CH_3$-Phenyl | H | H | 7/9 | $CH_3$ | Cl |
| 2-$OCH_2SO_2CH_3$-Phenyl | H | $CH_3$ | 7 | $CH_3$ | Cl |
| 2-$OCH_2SO_2CH_3$-Phenyl | H | H | 7/9 | Cl | Cl |
| 2-$OCH_2SO_2CH_3$-Phenyl | H | $CH_3$ | 7 | Cl | Cl |
| 2-$OCH_2SO_2CH_3$-Phenyl | H | H | 7/9 | $OCH_3$ | $OCH_3$ |
| 2-$OCH_2SO_2CH_3$-Phenyl | H | $CH_3$ | 7 | $OCH_3$ | $OCH_3$ |
| 2-$OCH_2SO_2CH_3$-Phenyl | H | H | 7/9 | $CH_3$ | $OCH_3$ |
| 2-$OCH_2SO_2CH_3$-Phenyl | H | $CH_3$ | 7 | $CH_3$ | $OCH_3$ |
| 2-$OCH_2SO_2CH_3$-Phenyl | H | H | 7/9 | $OCH_3$ | $CH_3$ |
| 2-$OCH_2SO_2CH_3$-Phenyl | H | $CH_3$ | 7 | $OCH_3$ | $CH_3$ |
| 2-$OCH_2SO_2CH_3$-Phenyl | H | H | 7/9 | Cl | $OCH_3$ |
| 2-$OCH_2SO_2CH_3$-Phenyl | H | $CH_3$ | 7 | Cl | $OCH_3$ |
| 2-$OCH_2SO_2CH_3$-Phenyl | H | H | 7/9 | $OCH_3$ | Cl |
| 2-$OCH_2SO_2CH_3$-Phenyl | H | $CH_3$ | 7 | $OCH_3$ | Cl |
| 2,6-Cl,Cl-Phenyl | H | H | 7/9 | $CH_3$ | $CH_3$ |
| 2,6-Cl,Cl-Phenyl | H | $CH_3$ | 7 | $CH_3$ | $CH_3$ |
| 2,6-Cl,Cl-Phenyl | H | H | 7/9 | Cl | $CH_3$ |
| 2,6-Cl,Cl-Phenyl | H | $CH_3$ | 7 | Cl | $CH_3$ |
| 2,6-Cl,Cl-Phenyl | H | H | 7/9 | $CH_3$ | Cl |
| 2,6-Cl,Cl-Phenyl | H | $CH_3$ | 7 | $CH_3$ | Cl |
| 2,6-Cl,Cl-Phenyl | H | H | 7/9 | Cl | Cl |
| 2,6-Cl,Cl-Phenyl | H | $CH_3$ | 7 | Cl | Cl |
| 2,6-Cl,Cl-Phenyl | H | H | 7/9 | $OCH_3$ | $OCH_3$ |

Tabelle II (Fortsetzung)

| A | $R^1$ | $R^2$ | Pos. | $R^3$ | $R^4$ |
|---|---|---|---|---|---|
| 2,6-Cl,Cl-Phenyl | H | $CH_3$ | 7 | $OCH_3$ | $OCH_3$ |
| 2,6-Cl,Cl-Phenyl | H | H | 7/9 | $CH_3$ | $OCH_3$ |
| 2,6-Cl,Cl-Phenyl | H | $CH_3$ | 7 | $CH_3$ | $OCH_3$ |
| 2,6-Cl,Cl-Phenyl | H | H | 7/9 | $OCH_3$ | $CH_3$ |
| 2,6-Cl,Cl-Phenyl | H | $CH_3$ | 7 | $OCH_3$ | $CH_3$ |
| 2,6-Cl,Cl-Phenyl | H | H | 7/9 | Cl | $OCH_3$ |
| 2,6-Cl,Cl-Phenyl | H | $CH_3$ | 7 | Cl | $OCH_3$ |
| 2,6-Cl,Cl-Phenyl | H | H | 7/9 | $OCH_3$ | Cl |
| 2,6-Cl,Cl-Phenyl | H | $CH_3$ | 7 | $OCH_3$ | Cl |
| 2-Cl,6-$CH_3$-Phenyl | H | H | 7/9 | $CH_3$ | $CH_3$ |
| 2-Cl,6-$CH_3$-Phenyl | H | $CH_3$ | 7 | $CH_3$ | $CH_3$ |
| 2-Cl,6-$CH_3$-Phenyl | H | H | 7/9 | Cl | $CH_3$ |
| 2-Cl,6-$CH_3$-Phenyl | H | $CH_3$ | 7 | Cl | $CH_3$ |
| 2-Cl,6-$CH_3$-Phenyl | H | H | 7/9 | $CH_3$ | Cl |
| 2-Cl,6-$CH_3$-Phenyl | H | $CH_3$ | 7 | $CH_3$ | Cl |
| 2-Cl,6-$CH_3$-Phenyl | H | H | 7/9 | Cl | Cl |
| 2-Cl,6-$CH_3$-Phenyl | H | $CH_3$ | 7 | Cl | Cl |
| 2-Cl,6-$CH_3$-Phenyl | H | H | 7/9 | $OCH_3$ | $OCH_3$ |
| 2-Cl,6-$CH_3$-Phenyl | H | $CH_3$ | 7 | $OCH_3$ | $OCH_3$ |
| 2-Cl,6-$CH_3$-Phenyl | H | H | 7/9 | $CH_3$ | $OCH_3$ |
| 2-Cl,6-$CH_3$-Phenyl | H | $CH_3$ | 7 | $CH_3$ | $OCH_3$ |
| 2-Cl,6-$CH_3$-Phenyl | H | H | 7/9 | $OCH_3$ | $CH_3$ |
| 2-Cl,6-$CH_3$-Phenyl | H | $CH_3$ | 7 | $OCH_3$ | $CH_3$ |
| 2-Cl,6-$CH_3$-Phenyl | H | H | 7/9 | Cl | $OCH_3$ |
| 2-Cl,6-$CH_3$-Phenyl | H | $CH_3$ | 7 | Cl | $OCH_3$ |
| 2-Cl,6-$CH_3$-Phenyl | H | H | 7/9 | $OCH_3$ | Cl |
| 2-Cl,6-$CH_3$-Phenyl | H | $CH_3$ | 7 | $OCH_3$ | Cl |
| 2,5-Cl,Cl-Phenyl | H | H | 7/9 | $CH_3$ | $CH_3$ |
| 2,5-Cl,Cl-Phenyl | H | $CH_3$ | 7 | $CH_3$ | $CH_3$ |
| 2,5-Cl,Cl-Phenyl | H | H | 7/9 | Cl | $CH_3$ |
| 2,5-Cl,Cl-Phenyl | H | $CH_3$ | 7 | Cl | $CH_3$ |
| 2,5-Cl,Cl-Phenyl | H | H | 7/9 | $CH_3$ | Cl |
| 2,5-Cl,Cl-Phenyl | H | $CH_3$ | 7 | $CH_3$ | Cl |
| 2,5-Cl,Cl-Phenyl | H | H | 7/9 | Cl | Cl |
| 2,5-Cl,Cl-Phenyl | H | $CH_3$ | 7 | Cl | Cl |
| 2,5-Cl,Cl-Phenyl | H | H | 7/9 | $OCH_3$ | $OCH_3$ |
| 2,5-Cl,Cl-Phenyl | H | $CH_3$ | 7 | $OCH_3$ | $OCH_3$ |
| 2,5-Cl,Cl-Phenyl | H | H | 7/9 | $CH_3$ | $OCH_3$ |
| 2,5-Cl,Cl-Phenyl | H | $CH_3$ | 7 | $CH_3$ | $OCH_3$ |

Tabelle II (Fortsetzung)

| A | $R^1$ | $R^2$ | Pos. | $R^3$ | $R^4$ |
|---|---|---|---|---|---|
| 2,5-Cl,Cl-Phenyl | H | H | 7/9 | $OCH_3$ | $CH_3$ |
| 2,5-Cl,Cl-Phenyl | H | $CH_3$ | 7 | $OCH_3$ | $CH_3$ |
| 2,5-Cl,Cl-Phenyl | H | H | 7/9 | Cl | $OCH_3$ |
| 2,5-Cl,Cl-Phenyl | H | $CH_3$ | 7 | Cl | $OCH_3$ |
| 2,5-Cl,Cl-Phenyl | H | H | 7/9 | $OCH_3$ | Cl |
| 2,5-Cl,Cl-Phenyl | H | $CH_3$ | 7 | $OCH_3$ | Cl |

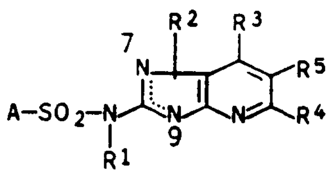

Ic

Tabelle III

| A | $R^1$ | $R^2$ | Pos. | $R^3$ | $R^4$ | $R^5$ |
|---|---|---|---|---|---|---|
| Phenyl | H | H | 7/9 | $CH_3$ | $CH_3$ | H |
| Phenyl | H | $CH_3$ | 7 | $CH_3$ | $CH_3$ | H |
| Phenyl | H | H | 7/9 | Cl | $CH_3$ | H |
| Phenyl | H | $CH_3$ | 7 | Cl | $CH_3$ | H |
| Phenyl | H | H | 7/9 | $CH_3$ | Cl | H |
| Phenyl | H | $CH_3$ | 7 | $CH_3$ | Cl | H |
| Phenyl | H | H | 7/9 | Cl | Cl | H |
| Phenyl | H | $CH_3$ | 7 | Cl | Cl | H |
| Phenyl | H | H | 7/9 | $OCH_3$ | $OCH_3$ | H |
| Phenyl | H | $CH_3$ | 7 | $OCH_3$ | $OCH_3$ | H |
| Phenyl | H | H | 7/9 | $CH_3$ | $OCH_3$ | H |
| Phenyl | H | $CH_3$ | 7 | $CH_3$ | $OCH_3$ | H |
| Phenyl | H | H | 7/9 | $OCH_3$ | $CH_3$ | H |
| Phenyl | H | $CH_3$ | 7 | $OCH_3$ | $CH_3$ | H |
| Phenyl | H | H | 7/9 | Cl | $OCH_3$ | H |
| Phenyl | H | $CH_3$ | 7 | Cl | $OCH_3$ | H |
| Phenyl | H | H | 7/9 | $OCH_3$ | Cl | H |
| Phenyl | H | $CH_3$ | 7 | $OCH_3$ | Cl | H |
| 2-Cl-Phenyl | H | H | 7/9 | $CH_3$ | $CH_3$ | H |
| 2-Cl-Phenyl | H | $CH_3$ | 7 | $CH_3$ | $CH_3$ | H |
| 2-Cl-Phenyl | H | H | 7/9 | Cl | $CH_3$ | H |
| 2-Cl-Phenyl | H | $CH_3$ | 7 | Cl | $CH_3$ | H |
| 2-Cl-Phenyl | H | H | 7/9 | $CH_3$ | Cl | H |
| 2-Cl-Phenyl | H | $CH_3$ | 7 | $CH_3$ | Cl | H |
| 2-Cl-Phenyl | H | H | 7/9 | Cl | Cl | H |
| 2-Cl-Phenyl | H | $CH_3$ | 7 | Cl | Cl | H |
| 2-Cl-Phenyl | H | H | 7/9 | $OCH_3$ | $OCH_3$ | H |
| 2-Cl-Phenyl | H | $CH_3$ | 7 | $OCH_3$ | $OCH_3$ | H |
| 2-Cl-Phenyl | H | H | 7/9 | $CH_3$ | $OCH_3$ | H |
| 2-Cl-Phenyl | H | $CH_3$ | 7 | $CH_3$ | $OCH_3$ | H |
| 2-Cl-Phenyl | H | H | 7/9 | $OCH_3$ | $CH_3$ | H |
| 2-Cl-Phenyl | H | $CH_3$ | 7 | $OCH_3$ | $CH_3$ | H |
| 2-Cl-Phenyl | H | H | 7/9 | Cl | $OCH_3$ | H |
| 2-Cl-Phenyl | H | $CH_3$ | 7 | Cl | $OCH_3$ | H |
| 2-Cl-Phenyl | H | H | 7/9 | $OCH_3$ | Cl | H |
| 2-Cl-Phenyl | H | $CH_3$ | 7 | $OCH_3$ | Cl | H |

Tabelle III (Fortsetzung)

| A | $R^1$ | $R^2$ | Pos. | $R^3$ | $R^4$ | $R^5$ |
|---|---|---|---|---|---|---|
| 2-Cl-Phenyl | H | $CH_3$ | 7 | $OCH_3$ | Cl | H |
| 2-Cl-Phenyl | H | H | 7/9 | $CF_3$ | $CF_3$ | H |
| 2-Cl-Phenyl | H | $CH_3$ | 7 | $CF_3$ | $CF_3$ | H |
| 2-Cl-Phenyl | H | H | 7/9 | Cl | $CF_3$ | H |
| 2-Cl-Phenyl | H | $CH_3$ | 7 | Cl | $CF_3$ | H |
| 2-Cl-Phenyl | H | H | 7/9 | $CF_3$ | Cl | H |
| 2-Cl-Phenyl | H | $CH_3$ | 7 | $CF_3$ | Cl | H |
| 2-Cl-Phenyl | H | H | 7/9 | F | $CF_3$ | H |
| 2-Cl-Phenyl | H | $CH_3$ | 7 | F | $CF_3$ | H |
| 2-Cl-Phenyl | H | H | 7/9 | $CF_3$ | F | H |
| 2-Cl-Phenyl | H | $CH_3$ | 7 | $CF_3$ | F | H |
| 2-Cl-Phenyl | H | H | 7/9 | $CH_3$ | $CF_3$ | H |
| 2-Cl-Phenyl | H | $CH_3$ | 7 | $CH_3$ | $CF_3$ | H |
| 2-Cl-Phenyl | H | H | 7/9 | $CF_3$ | $CH_3$ | H |
| 2-Cl-Phenyl | H | $CH_3$ | 7 | $CF_3$ | $CH_3$ | H |
| 2-Cl-Phenyl | H | H | 7/9 | $OCH_3$ | $CF_3$ | H |
| 2-Cl-Phenyl | H | $CH_3$ | 7 | $OCH_3$ | $CF_3$ | H |
| 2-Cl-Phenyl | H | H | 7/9 | $CF_3$ | $OCH_3$ | H |
| 2-Cl-Phenyl | H | $CH_3$ | 7 | $CF_3$ | $OCH_3$ | H |
| 2-Cl-Phenyl | H | H | 7/9 | F | F | H |
| 2-Cl-Phenyl | H | $CH_3$ | 7 | F | F | H |
| 2-Cl-Phenyl | H | H | 7/9 | F | Cl | H |
| 2-Cl-Phenyl | H | $CH_3$ | 7 | F | Cl | H |
| 2-Cl-Phenyl | H | H | 7/9 | Cl | F | H |
| 2-Cl-Phenyl | H | $CH_3$ | 7 | Cl | F | H |
| 2-Cl-Phenyl | H | H | 7/9 | F | $CH_3$ | H |
| 2-Cl-Phenyl | H | $CH_3$ | 7 | F | $CH_3$ | H |
| 2-Cl-Phenyl | H | H | 7/9 | $CH_3$ | F | H |
| 2-Cl-Phenyl | H | $CH_3$ | 7 | $CH_3$ | F | H |
| 2-Cl-Phenyl | H | H | 7/9 | F | $OCH_3$ | H |
| 2-Cl-Phenyl | H | $CH_3$ | 7 | F | $OCH_3$ | H |
| 2-Cl-Phenyl | H | H | 7/9 | $OCH_3$ | F | H |
| 2-Cl-Phenyl | H | $CH_3$ | 7 | $OCH_3$ | F | H |
| 2-Cl-Phenyl | H | H | 7/9 | $N(CH_3)_2$ | Cl | H |
| 2-Cl-Phenyl | H | $CH_3$ | 7 | $N(CH_3)_2$ | Cl | H |
| 2-Cl-Phenyl | H | H | 7/9 | $SCH_3$ | Cl | H |
| 2-Cl-Phenyl | H | $CH_3$ | 7 | $SCH_3$ | Cl | H |

Tabelle III (Fortsetzung)

| A | $R^1$ | $R^2$ | Pos. | $R^3$ | $R^4$ | $R^5$ |
|---|---|---|---|---|---|---|
| 2-F-Phenyl | H | H | 7/9 | $CH_3$ | $CH_3$ | H |
| 2-F-Phenyl | H | $CH_3$ | 7 | $CH_3$ | $CH_3$ | H |
| 2-F-Phenyl | H | H | 7/9 | Cl | $CH_3$ | H |
| 2-F-Phenyl | H | $CH_3$ | 7 | Cl | $CH_3$ | H |
| 2-F-Phenyl | H | H | 7/9 | $CH_3$ | Cl | H |
| 2-F-Phenyl | H | $CH_3$ | 7 | $CH_3$ | Cl | H |
| 2-F-Phenyl | H | H | 7/9 | Cl | Cl | H |
| 2-F-Phenyl | H | $CH_3$ | 7 | Cl | Cl | H |
| 2-F-Phenyl | H | H | 7/9 | $OCH_3$ | $OCH_3$ | H |
| 2-F-Phenyl | H | $CH_3$ | 7 | $OCH_3$ | $OCH_3$ | H |
| 2-F-Phenyl | H | H | 7/9 | $CH_3$ | $OCH_3$ | H |
| 2-F-Phenyl | H | $CH_3$ | 7 | $CH_3$ | $OCH_3$ | H |
| 2-F-Phenyl | H | H | 7/9 | $OCH_3$ | $CH_3$ | H |
| 2-F-Phenyl | H | $CH_3$ | 7 | $OCH_3$ | $CH_3$ | H |
| 2-F-Phenyl | H | H | 7/9 | Cl | $OCH_3$ | H |
| 2-F-Phenyl | H | $CH_3$ | 7 | Cl | $OCH_3$ | H |
| 2-F-Phenyl | H | H | 7/9 | $OCH_3$ | Cl | H |
| 2-F-Phenyl | H | $CH_3$ | 7 | $OCH_3$ | Cl | H |
| 2-CN-Phenyl | H | H | 7/9 | $CH_3$ | $CH_3$ | H |
| 2-CN-Phenyl | H | $CH_3$ | 7 | $CH_3$ | $CH_3$ | H |
| 2-CN-Phenyl | H | H | 7/9 | Cl | $CH_3$ | H |
| 2-CN-Phenyl | H | $CH_3$ | 7 | Cl | $CH_3$ | H |
| 2-CN-Phenyl | H | H | 7/9 | $CH_3$ | Cl | H |
| 2-CN-Phenyl | H | $CH_3$ | 7 | $CH_3$ | Cl | H |
| 2-CN-Phenyl | H | H | 7/9 | Cl | Cl | H |
| 2-CN-Phenyl | H | $CH_3$ | 7 | Cl | Cl | H |
| 2-CN-Phenyl | H | H | 7/9 | $OCH_3$ | $OCH_3$ | H |
| 2-CN-Phenyl | H | $CH_3$ | 7 | $OCH_3$ | $OCH_3$ | H |
| 2-CN-Phenyl | H | H | 7/9 | $CH_3$ | $OCH_3$ | H |
| 2-CN-Phenyl | H | $CH_3$ | 7 | $CH_3$ | $OCH_3$ | H |
| 2-CN-Phenyl | H | H | 7/9 | $OCH_3$ | $CH_3$ | H |
| 2-CN-Phenyl | H | $CH_3$ | 7 | $OCH_3$ | $CH_3$ | H |
| 2-CN-Phenyl | H | H | 7/9 | Cl | $OCH_3$ | H |
| 2-CN-Phenyl | H | $CH_3$ | 7 | Cl | $OCH_3$ | H |
| 2-CN-Phenyl | H | H | 7/9 | $OCH_3$ | Cl | H |
| 2-CN-Phenyl | H | $CH_3$ | 7 | $OCH_3$ | Cl | H |
| 2-$CF_3$-Phenyl | H | H | 7/9 | $CH_3$ | $CH_3$ | H |
| 2-$CF_3$-Phenyl | H | $CH_3$ | 7 | $CH_3$ | $CH_3$ | H |
| 2-$CF_3$-Phenyl | H | H | 7/9 | Cl | $CH_3$ | H |
| 2-$CF_3$-Phenyl | H | $CH_3$ | 7 | Cl | $CH_3$ | H |

Tabelle III (Fortsetzung)

| A | $R^1$ | $R^2$ | Pos. | $R^3$ | $R^4$ | $R^5$ |
|---|---|---|---|---|---|---|
| 2-$CF_3$-Phenyl | H | H | 7/9 | $CH_3$ | Cl | H |
| 2-$CF_3$-Phenyl | H | $CH_3$ | 7 | $CH_3$ | Cl | H |
| 2-$CF_3$-Phenyl | H | H | 7/9 | Cl | Cl | H |
| 2-$CF_3$-Phenyl | H | $CH_3$ | 7 | Cl | Cl | H |
| 2-$CF_3$-Phenyl | H | H | 7/9 | $OCH_3$ | $OCH_3$ | H |
| 2-$CF_3$-Phenyl | H | $CH_3$ | 7 | $OCH_3$ | $OCH_3$ | H |
| 2-$CF_3$-Phenyl | H | H | 7/9 | $CH_3$ | $OCH_3$ | H |
| 2-$CF_3$-Phenyl | H | $CH_3$ | 7 | $CH_3$ | $OCH_3$ | H |
| 2-$CF_3$-Phenyl | H | H | 7/9 | $OCH_3$ | $CH_3$ | H |
| 2-$CF_3$-Phenyl | H | $CH_3$ | 7 | $OCH_3$ | $CH_3$ | H |
| 2-$CF_3$-Phenyl | H | H | 7/9 | Cl | $OCH_3$ | H |
| 2-$CF_3$-Phenyl | H | $CH_3$ | 7 | Cl | $OCH_3$ | H |
| 2-$CF_3$-Phenyl | H | H | 7/9 | $OCH_3$ | Cl | H |
| 2-$CF_3$-Phenyl | H | $CH_3$ | 7 | $OCH_3$ | Cl | H |
| 2-$NO_2$-Phenyl | H | H | 7/9 | $CH_3$ | $CH_3$ | H |
| 2-$NO_2$-Phenyl | H | $CH_3$ | 7 | $CH_3$ | $CH_3$ | H |
| 2-$NO_2$-Phenyl | H | H | 7/9 | Cl | $CH_3$ | H |
| 2-$NO_2$-Phenyl | H | $CH_3$ | 7 | Cl | $CH_3$ | H |
| 2-$NO_2$-Phenyl | H | H | 7/9 | $CH_3$ | Cl | H |
| 2-$NO_2$-Phenyl | H | $CH_3$ | 7 | $CH_3$ | Cl | H |
| 2-$NO_2$-Phenyl | H | H | 7/9 | Cl | Cl | H |
| 2-$NO_2$-Phenyl | H | $CH_3$ | 7 | Cl | Cl | H |
| 2-$NO_2$-Phenyl | H | H | 7/9 | $OCH_3$ | $OCH_3$ | H |
| 2-$NO_2$-Phenyl | H | $CH_3$ | 7 | $OCH_3$ | $OCH_3$ | H |
| 2-$NO_2$-Phenyl | H | H | 7/9 | $CH_3$ | $OCH_3$ | H |
| 2-$NO_2$-Phenyl | H | $CH_3$ | 7 | $CH_3$ | $OCH_3$ | H |
| 2-$NO_2$-Phenyl | H | H | 7/9 | $OCH_3$ | $CH_3$ | H |
| 2-$NO_2$-Phenyl | H | $CH_3$ | 7 | $OCH_3$ | $CH_3$ | H |
| 2-$NO_2$-Phenyl | H | H | 7/9 | Cl | $OCH_3$ | H |
| 2-$NO_2$-Phenyl | H | $CH_3$ | 7 | Cl | $OCH_3$ | H |
| 2-$NO_2$-Phenyl | H | H | 7/9 | $OCH_3$ | Cl | H |
| 2-$NO_2$-Phenyl | H | $CH_3$ | 7 | $OCH_3$ | Cl | H |
| 2-$CH_3$-Phenyl | H | H | 7/9 | $CH_3$ | $CH_3$ | H |
| 2-$CH_3$-Phenyl | H | $CH_3$ | 7 | $CH_3$ | $CH_3$ | H |
| 2-$CH_3$-Phenyl | H | H | 7/9 | Cl | $CH_3$ | H |
| 2-$CH_3$-Phenyl | H | $CH_3$ | 7 | Cl | $CH_3$ | H |
| 2-$CH_3$-Phenyl | H | H | 7/9 | $CH_3$ | Cl | H |
| 2-$CH_3$-Phenyl | H | $CH_3$ | 7 | $CH_3$ | Cl | H |
| 2-$CH_3$-Phenyl | H | H | 7/9 | Cl | Cl | H |
| 2-$CH_3$-Phenyl | H | $CH_3$ | 7 | Cl | Cl | H |
| 2-$CH_3$-Phenyl | H | H | 7/9 | $OCH_3$ | $OCH_3$ | H |

Tabelle III (Fortsetzung)

| A | $R^1$ | $R^2$ | Pos. | $R^3$ | $R^4$ | $R^5$ |
|---|---|---|---|---|---|---|
| 2-$CH_3$-Phenyl | H | $CH_3$ | 7 | $OCH_3$ | $OCH_3$ | H |
| 2-$CH_3$-Phenyl | H | H | 7/9 | $CH_3$ | $OCH_3$ | H |
| 2-$CH_3$-Phenyl | H | $CH_3$ | 7 | $CH_3$ | $OCH_3$ | H |
| 2-$CH_3$-Phenyl | H | H | 7/9 | $OCH_3$ | $CH_3$ | H |
| 2-$CH_3$-Phenyl | H | $CH_3$ | 7 | $OCH_3$ | $CH_3$ | H |
| 2-$CH_3$-Phenyl | H | H | 7/9 | Cl | $OCH_3$ | H |
| 2-$CH_3$-Phenyl | H | $CH_3$ | 7 | Cl | $OCH_3$ | H |
| 2-$CH_3$-Phenyl | H | H | 7/9 | $OCH_3$ | Cl | H |
| 2-$CH_3$-Phenyl | H | $CH_3$ | 7 | $OCH_3$ | Cl | H |
| 2-$CO_2CH_3$-Phenyl | H | H | 7/9 | $CH_3$ | $CH_3$ | H |
| 2-$CO_2CH_3$-Phenyl | H | $CH_3$ | 7 | $CH_3$ | $CH_3$ | H |
| 2-$CO_2CH_3$-Phenyl | H | H | 7/9 | Cl | $CH_3$ | H |
| 2-$CO_2CH_3$-Phenyl | H | $CH_3$ | 7 | Cl | $CH_3$ | H |
| 2-$CO_2CH_3$-Phenyl | H | H | 7/9 | $CH_3$ | Cl | H |
| 2-$CO_2CH_3$-Phenyl | H | $CH_3$ | 7 | $CH_3$ | Cl | H |
| 2-$CO_2CH_3$-Phenyl | H | H | 7/9 | Cl | Cl | H |
| 2-$CO_2CH_3$-Phenyl | H | $CH_3$ | 7 | Cl | Cl | H |
| 2-$CO_2CH_3$-Phenyl | H | H | 7/9 | $OCH_3$ | $OCH_3$ | H |
| 2-$CO_2CH_3$-Phenyl | H | $CH_3$ | 7 | $OCH_3$ | $OCH_3$ | H |
| 2-$CO_2CH_3$-Phenyl | H | H | 7/9 | $CH_3$ | $OCH_3$ | H |
| 2-$CO_2CH_3$-Phenyl | H | $CH_3$ | 7 | $CH_3$ | $OCH_3$ | H |
| 2-$CO_2CH_3$-Phenyl | H | H | 7/9 | $OCH_3$ | $CH_3$ | H |
| 2-$CO_2CH_3$-Phenyl | H | $CH_3$ | 7 | $OCH_3$ | $CH_3$ | H |
| 2-$CO_2CH_3$-Phenyl | H | H | 7/9 | Cl | $OCH_3$ | H |
| 2-$CO_2CH_3$-Phenyl | H | $CH_3$ | 7 | Cl | $OCH_3$ | H |
| 2-$CO_2CH_3$-Phenyl | H | H | 7/9 | $OCH_3$ | Cl | H |
| 2-$CO_2CH_3$-Phenyl | H | $CH_3$ | 7 | $OCH_3$ | Cl | H |
| 2-$CO_2CH_3$-Phenyl | H | H | 7/9 | $CF_3$ | $CF_3$ | H |
| 2-$CO_2CH_3$-Phenyl | H | $CH_3$ | 7 | $CF_3$ | $CF_3$ | H |
| 2-$CO_2CH_3$-Phenyl | H | H | 7/9 | Cl | $CF_3$ | H |
| 2-$CO_2CH_3$-Phenyl | H | $CH_3$ | 7 | Cl | $CF_3$ | H |
| 2-$CO_2CH_3$-Phenyl | H | H | 7/9 | $CF_3$ | Cl | H |
| 2-$CO_2CH_3$-Phenyl | H | $CH_3$ | 7 | $CF_3$ | Cl | H |
| 2-$CO_2CH_3$-Phenyl | H | H | 7/9 | F | $CF_3$ | H |
| 2-$CO_2CH_3$-Phenyl | H | $CH_3$ | 7 | F | $CF_3$ | H |
| 2-$CO_2CH_3$-Phenyl | H | H | 7/9 | $CF_3$ | F | H |
| 2-$CO_2CH_3$-Phenyl | H | $CH_3$ | 7 | $CF_3$ | F | H |
| 2-$CO_2CH_3$-Phenyl | H | H | 7/9 | $CH_3$ | $CF_3$ | H |
| 2-$CO_2CH_3$-Phenyl | H | $CH_3$ | 7 | $CH_3$ | $CF_3$ | H |
| 2-$CO_2CH_3$-Phenyl | H | H | 7/9 | $CF_3$ | $CH_3$ | H |

Tabelle III (Fortsetzung)

| A | $R^1$ | $R^2$ | Pos. | $R^3$ | $R^4$ | $R^5$ |
|---|---|---|---|---|---|---|
| 2-$CO_2CH_3$-Phenyl | H | $CH_3$ | 7 | $CF_3$ | $CH_3$ | H |
| 2-$CO_2CH_3$-Phenyl | H | H | 7/9 | $OCH_3$ | $CF_3$ | H |
| 2-$CO_2CH_3$-Phenyl | H | $CH_3$ | 7 | $OCH_3$ | $CF_3$ | H |
| 2-$CO_2CH_3$-Phenyl | H | H | 7/9 | $CF_3$ | $OCH_3$ | H |
| 2-$CO_2CH_3$-Phenyl | H | $CH_3$ | 7 | $CF_3$ | $OCH_3$ | H |
| 2-$CO_2CH_3$-Phenyl | H | H | 7/9 | F | F | H |
| 2-$CO_2CH_3$-Phenyl | H | $CH_3$ | 7 | F | F | H |
| 2-$CO_2CH_3$-Phenyl | H | H | 7/9 | F | Cl | H |
| 2-$CO_2CH_3$-Phenyl | H | $CH_3$ | 7 | F | Cl | H |
| 2-$CO_2CH_3$-Phenyl | H | H | 7/9 | Cl | F | H |
| 2-$CO_2CH_3$-Phenyl | H | $CH_3$ | 7 | Cl | F | H |
| 2-$CO_2CH_3$-Phenyl | H | H | 7/9 | F | $CH_3$ | H |
| 2-$CO_2CH_3$-Phenyl | H | $CH_3$ | 7 | F | $CH_3$ | H |
| 2-$CO_2CH_3$-Phenyl | H | H | 7/9 | $CH_3$ | F | H |
| 2-$CO_2CH_3$-Phenyl | H | $CH_3$ | 7 | $CH_3$ | F | H |
| 2-$CO_2CH_3$-Phenyl | H | H | 7/9 | F | $OCH_3$ | H |
| 2-$CO_2CH_3$-Phenyl | H | $CH_3$ | 7 | F | $OCH_3$ | H |
| 2-$CO_2CH_3$-Phenyl | H | H | 7/9 | $OCH_3$ | F | H |
| 2-$CO_2CH_3$-Phenyl | H | $CH_3$ | 7 | $OCH_3$ | F | H |
| 2-$CO_2CH_3$-Phenyl | H | H | 7/9 | $N(CH_3)_2$ | Cl | H |
| 2-$CO_2CH_3$-Phenyl | H | $CH_3$ | 7 | $N(CH_3)_2$ | Cl | H |
| 2-$CO_2CH_3$-Phenyl | H | H | 7/9 | $SCH_3$ | Cl | H |
| 2-$CO_2CH_3$-Phenyl | H | $CH_3$ | 7 | $SCH_3$ | Cl | H |
| 2-$CO_2C_2H_5$-Phenyl | H | H | 7/9 | $CH_3$ | $CH_3$ | H |
| 2-$CO_2C_2H_5$-Phenyl | H | $CH_3$ | 7 | $CH_3$ | $CH_3$ | H |
| 2-$CO_2C_2H_5$-Phenyl | H | H | 7/9 | Cl | $CH_3$ | H |
| 2-$CO_2C_2H_5$-Phenyl | H | $CH_3$ | 7 | Cl | $CH_3$ | H |
| 2-$CO_2C_2H_5$-Phenyl | H | H | 7/9 | $CH_3$ | Cl | H |
| 2-$CO_2C_2H_5$-Phenyl | H | $CH_3$ | 7 | $CH_3$ | Cl | H |
| 2-$CO_2C_2H_5$-Phenyl | H | H | 7/9 | Cl | Cl | H |
| 2-$CO_2C_2H_5$-Phenyl | H | $CH_3$ | 7 | Cl | Cl | H |
| 2-$CO_2C_2H_5$-Phenyl | H | H | 7/9 | $OCH_3$ | $OCH_3$ | H |
| 2-$CO_2C_2H_5$-Phenyl | H | $CH_3$ | 7 | $OCH_3$ | $OCH_3$ | H |
| 2-$CO_2C_2H_5$-Phenyl | H | H | 7/9 | $CH_3$ | $OCH_3$ | H |
| 2-$CO_2C_2H_5$-Phenyl | H | $CH_3$ | 7 | $CH_3$ | $OCH_3$ | H |
| 2-$CO_2C_2H_5$-Phenyl | H | H | 7/9 | $OCH_3$ | $CH_3$ | H |
| 2-$CO_2C_2H_5$-Phenyl | H | $CH_3$ | 7 | $OCH_3$ | $CH_3$ | H |
| 2-$CO_2C_2H_5$-Phenyl | H | H | 7/9 | Cl | $OCH_3$ | H |
| 2-$CO_2C_2H_5$-Phenyl | H | $CH_3$ | 7 | Cl | $OCH_3$ | H |
| 2-$CO_2C_2H_5$-Phenyl | H | H | 7/9 | $OCH_3$ | Cl | H |

Tabelle III (Fortsetzung)

| A | $R^1$ | $R^2$ | Pos. | $R^3$ | $R^4$ | $R^5$ |
|---|---|---|---|---|---|---|
| 2-$CO_2C_2H_5$-Phenyl | H | $CH_3$ | 7 | $OCH_3$ | Cl | H |
| 2-$COCH_3$-Phenyl | H | H | 7/9 | $CH_3$ | $CH_3$ | H |
| 2-$COCH_3$-Phenyl | H | $CH_3$ | 7 | $CH_3$ | $CH_3$ | H |
| 2-$COCH_3$-Phenyl | H | H | 7/9 | Cl | $CH_3$ | H |
| 2-$COCH_3$-Phenyl | H | $CH_3$ | 7 | Cl | $CH_3$ | H |
| 2-$COCH_3$-Phenyl | H | H | 7/9 | $CH_3$ | Cl | H |
| 2-$COCH_3$-Phenyl | H | $CH_3$ | 7 | $CH_3$ | Cl | H |
| 2-$COCH_3$-Phenyl | H | H | 7/9 | Cl | Cl | H |
| 2-$COCH_3$-Phenyl | H | $CH_3$ | 7 | Cl | Cl | H |
| 2-$COCH_3$-Phenyl | H | H | 7/9 | $OCH_3$ | $OCH_3$ | H |
| 2-$COCH_3$-Phenyl | H | $CH_3$ | 7 | $OCH_3$ | $OCH_3$ | H |
| 2-$COCH_3$-Phenyl | H | H | 7/9 | $CH_3$ | $OCH_3$ | H |
| 2-$COCH_3$-Phenyl | H | $CH_3$ | 7 | $CH_3$ | $OCH_3$ | H |
| 2-$COCH_3$-Phenyl | H | H | 7/9 | $OCH_3$ | $CH_3$ | H |
| 2-$COCH_3$-Phenyl | H | $CH_3$ | 7 | $OCH_3$ | $CH_3$ | H |
| 2-$COCH_3$-Phenyl | H | H | 7/9 | Cl | $OCH_3$ | H |
| 2-$COCH_3$-Phenyl | H | $CH_3$ | 7 | Cl | $OCH_3$ | H |
| 2-$COCH_3$-Phenyl | H | H | 7/9 | $OCH_3$ | Cl | H |
| 2-$COCH_3$-Phenyl | H | $CH_3$ | 7 | $OCH_3$ | Cl | H |
| 2-$OCH_3$-Phenyl | H | H | 7/9 | $CH_3$ | $CH_3$ | H |
| 2-$OCH_3$-Phenyl | H | $CH_3$ | 7 | $CH_3$ | $CH_3$ | H |
| 2-$OCH_3$-Phenyl | H | H | 7/9 | Cl | $CH_3$ | H |
| 2-$OCH_3$-Phenyl | H | $CH_3$ | 7 | Cl | $CH_3$ | H |
| 2-$OCH_3$-Phenyl | H | H | 7/9 | $CH_3$ | Cl | H |
| 2-$OCH_3$-Phenyl | H | $CH_3$ | 7 | $CH_3$ | Cl | H |
| 2-$OCH_3$-Phenyl | H | H | 7/9 | Cl | Cl | H |
| 2-$OCH_3$-Phenyl | H | $CH_3$ | 7 | Cl | Cl | H |
| 2-$OCH_3$-Phenyl | H | H | 7/9 | $OCH_3$ | $OCH_3$ | H |
| 2-$OCH_3$-Phenyl | H | $CH_3$ | 7 | $OCH_3$ | $OCH_3$ | H |
| 2-$OCH_3$-Phenyl | H | H | 7/9 | $CH_3$ | $OCH_3$ | H |
| 2-$OCH_3$-Phenyl | H | $CH_3$ | 7 | $CH_3$ | $OCH_3$ | H |
| 2-$OCH_3$-Phenyl | H | H | 7/9 | $OCH_3$ | $CH_3$ | H |
| 2-$OCH_3$-Phenyl | H | $CH_3$ | 7 | $OCH_3$ | $CH_3$ | H |
| 2-$OCH_3$-Phenyl | H | H | 7/9 | Cl | $OCH_3$ | H |
| 2-$OCH_3$-Phenyl | H | $CH_3$ | 7 | Cl | $OCH_3$ | H |
| 2-$OCH_3$-Phenyl | H | H | 7/9 | $OCH_3$ | Cl | H |
| 2-$OCH_3$-Phenyl | H | $CH_3$ | 7 | $OCH_3$ | Cl | H |
| 2-$OCH_2CH_2OCH_3$-Phenyl | H | H | 7/9 | $CH_3$ | $CH_3$ | H |
| 2-$OCH_2CH_2OCH_3$-Phenyl | H | $CH_3$ | 7 | $CH_3$ | $CH_3$ | H |
| 2-$OCH_2CH_2OCH_3$-Phenyl | H | H | 7/9 | Cl | $CH_3$ | H |

Tabelle III (Fortsetzung)

| A | $R^1$ | $R^2$ | Pos. | $R^3$ | $R^4$ | $R^5$ |
|---|---|---|---|---|---|---|
| 2-$OCH_2CH_2OCH_3$-Phenyl | H | $CH_3$ | 7 | Cl | $CH_3$ | H |
| 2-$OCH_2CH_2OCH_3$-Phenyl | H | H | 7/9 | $CH_3$ | Cl | H |
| 2-$OCH_2CH_2OCH_3$-Phenyl | H | $CH_3$ | 7 | $CH_3$ | Cl | H |
| 2-$OCH_2CH_2OCH_3$-Phenyl | H | H | 7/9 | Cl | Cl | H |
| 2-$OCH_2CH_2OCH_3$-Phenyl | H | $CH_3$ | 7 | Cl | Cl | H |
| 2-$OCH_2CH_2OCH_3$-Phenyl | H | H | 7/9 | $OCH_3$ | $OCH_3$ | H |
| 2-$OCH_2CH_2OCH_3$-Phenyl | H | $CH_3$ | 7 | $OCH_3$ | $OCH_3$ | H |
| 2-$OCH_2CH_2OCH_3$-Phenyl | H | H | 7/9 | $CH_3$ | $OCH_3$ | H |
| 2-$OCH_2CH_2OCH_3$-Phenyl | H | $CH_3$ | 7 | $CH_3$ | $OCH_3$ | H |
| 2-$OCH_2CH_2OCH_3$-Phenyl | H | H | 7/9 | $OCH_3$ | $CH_3$ | H |
| 2-$OCH_2CH_2OCH_3$-Phenyl | H | $CH_3$ | 7 | $OCH_3$ | $CH_3$ | H |
| 2-$OCH_2CH_2OCH_3$-Phenyl | H | H | 7/9 | Cl | $OCH_3$ | H |
| 2-$OCH_2CH_2OCH_3$-Phenyl | H | $CH_3$ | 7 | Cl | $OCH_3$ | H |
| 2-$OCH_2CH_2OCH_3$-Phenyl | H | H | 7/9 | $OCH_3$ | Cl | H |
| 2-$OCH_2CH_2$-$OCH_3$Phenyl | H | $CH_3$ | 7 | $OCH_3$ | Cl | H |
| 2-$OCH_2CO_2CH_3$-Phenyl | H | H | 7/9 | $CH_3$ | $CH_3$ | H |
| 2-$OCH_2CO_2CH_3$-Phenyl | H | $CH_3$ | 7 | $CH_3$ | $CH_3$ | H |
| 2-$OCH_2CO_2CH_3$-Phenyl | H | H | 7/9 | Cl | $CH_3$ | H |
| 2-$OCH_2CO_2CH_3$-Phenyl | H | $CH_3$ | 7 | Cl | $CH_3$ | H |
| 2-$OCH_2CO_2CH_3$-Phenyl | H | H | 7/9 | $CH_3$ | Cl | H |
| 2-$OCH_2CO_2CH_3$-Phenyl | H | $CH_3$ | 7 | $CH_3$ | Cl | H |
| 2-$OCH_2CO_2CH_3$-Phenyl | H | H | 7/9 | Cl | Cl | H |
| 2-$OCH_2CO_2CH_3$-Phenyl | H | $CH_3$ | 7 | Cl | Cl | H |
| 2-$OCH_2CO_2CH_3$-Phenyl | H | H | 7/9 | $OCH_3$ | $OCH_3$ | H |
| 2-$OCH_2CO_2CH_3$-Phenyl | H | $CH_3$ | 7 | $OCH_3$ | $OCH_3$ | H |
| 2-$OCH_2CO_2CH_3$-Phenyl | H | H | 7/9 | $CH_3$ | $OCH_3$ | H |
| 2-$OCH_2CO_2CH_3$-Phenyl | H | $CH_3$ | 7 | $CH_3$ | $OCH_3$ | H |
| 2-$OCH_2CO_2CH_3$-Phenyl | H | H | 7/9 | $OCH_3$ | $CH_3$ | H |
| 2-$OCH_2CO_2CH_3$-Phenyl | H | $CH_3$ | 7 | $OCH_3$ | $CH_3$ | H |
| 2-$OCH_2CO_2CH_3$-Phenyl | H | H | 7/9 | Cl | $OCH_3$ | H |
| 2-$OCH_2CO_2CH_3$-Phenyl | H | $CH_3$ | 7 | Cl | $OCH_3$ | H |
| 2-$OCH_2CO_2CH_3$-Phenyl | H | H | 7/9 | $OCH_3$ | Cl | H |
| 2-$OCH_2CO_2CH_3$-Phenyl | H | $CH_3$ | 7 | $OCH_3$ | Cl | H |
| 2-$OCH_2CH_2Cl$-Phenyl | H | H | 7/9 | $CH_3$ | $CH_3$ | H |
| 2-$OCH_2CH_2Cl$-Phenyl | H | $CH_3$ | 7 | $CH_3$ | $CH_3$ | H |
| 2-$OCH_2CH_2Cl$-Phenyl | H | H | 7/9 | Cl | $CH_3$ | H |
| 2-$OCH_2CH_2Cl$-Phenyl | H | $CH_3$ | 7 | Cl | $CH_3$ | H |
| 2-$OCH_2CH_2Cl$-Phenyl | H | H | 7/9 | $CH_3$ | Cl | H |
| 2-$OCH_2CH_2Cl$-Phenyl | H | $CH_3$ | 7 | $CH_3$ | Cl | H |
| 2-$OCH_2CH_2Cl$-Phenyl | H | H | 7/9 | Cl | Cl | H |

Tabelle III (Fortsetzung)

| A | $R^1$ | $R^2$ | Pos. | $R^3$ | $R^4$ | $R^5$ |
|---|---|---|---|---|---|---|
| 2-$OCH_2CH_2Cl$-Phenyl | H | $CH_3$ | 7 | Cl | Cl | H |
| 2-$OCH_2CH_2Cl$-Phenyl | H | H | 7/9 | $OCH_3$ | $OCH_3$ | H |
| 2-$OCH_2CH_2Cl$-Phenyl | H | $CH_3$ | 7 | $OCH_3$ | $OCH_3$ | H |
| 2-$OCH_2CH_2Cl$-Phenyl | H | H | 7/9 | $CH_3$ | $OCH_3$ | H |
| 2-$OCH_2CH_2Cl$-Phenyl | H | $CH_3$ | 7 | $CH_3$ | $OCH_3$ | H |
| 2-$OCH_2CH_2Cl$-Phenyl | H | H | 7/9 | $OCH_3$ | $CH_3$ | H |
| 2-$OCH_2CH_2Cl$-Phenyl | H | $CH_3$ | 7 | $OCH_3$ | $CH_3$ | H |
| 2-$OCH_2CH_2Cl$-Phenyl | H | H | 7/9 | Cl | $OCH_3$ | H |
| 2-$OCH_2CH_2Cl$-Phenyl | H | $CH_3$ | 7 | Cl | $OCH_3$ | H |
| 2-$OCH_2CH_2Cl$-Phenyl | H | H | 7/9 | $OCH_3$ | Cl | H |
| 2-$OCH_2CH_2Cl$-Phenyl | H | $CH_3$ | 7 | $OCH_3$ | Cl | H |
| 2-$OCH_2CH_2Cl$-Phenyl | H | H | 7/9 | $CF_3$ | $CF_3$ | H |
| 2-$OCH_2CH_2Cl$-Phenyl | H | $CH_3$ | 7 | $CF_3$ | $CF_3$ | H |
| 2-$OCH_2CH_2Cl$-Phenyl | H | H | 7/9 | Cl | $CF_3$ | H |
| 2-$OCH_2CH_2Cl$-Phenyl | H | $CH_3$ | 7 | Cl | $CF_3$ | H |
| 2-$OCH_2CH_2Cl$-Phenyl | H | H | 7/9 | $CF_3$ | Cl | H |
| 2-$OCH_2CH_2Cl$-Phenyl | H | $CH_3$ | 7 | $CF_3$ | Cl | H |
| 2-$OCH_2CH_2Cl$-Phenyl | H | H | 7/9 | F | $CF_3$ | H |
| 2-$OCH_2CH_2Cl$-Phenyl | H | $CH_3$ | 7 | F | $CF_3$ | H |
| 2-$OCH_2CH_2Cl$-Phenyl | H | H | 7/9 | $CF_3$ | F | H |
| 2-$OCH_2CH_2Cl$-Phenyl | H | $CH_3$ | 7 | $CF_3$ | F | H |
| 2-$OCH_2CH_2Cl$-Phenyl | H | H | 7/9 | $CH_3$ | $CF_3$ | H |
| 2-$OCH_2CH_2Cl$-Phenyl | H | $CH_3$ | 7 | $CH_3$ | $CF_3$ | H |
| 2-$OCH_2CH_2Cl$-Phenyl | H | H | 7/9 | $CF_3$ | $CH_3$ | H |
| 2-$OCH_2CH_2Cl$-Phenyl | H | $CH_3$ | 7 | $CF_3$ | $CH_3$ | H |
| 2-$OCH_2CH_2Cl$-Phenyl | H | H | 7/9 | $OCH_3$ | $CF_3$ | H |
| 2-$OCH_2CH_2Cl$-Phenyl | H | $CH_3$ | 7 | $OCH_3$ | $CF_3$ | H |
| 2-$OCH_2CH_2Cl$-Phenyl | H | H | 7/9 | $CF_3$ | $OCH_3$ | H |
| 2-$OCH_2CH_2Cl$-Phenyl | H | $CH_3$ | 7 | $CF_3$ | $OCH_3$ | H |
| 2-$OCH_2CH_2Cl$-Phenyl | H | H | 7/9 | F | F | H |
| 2-$OCH_2CH_2Cl$-Phenyl | H | $CH_3$ | 7 | F | F | H |
| 2-$OCH_2CH_2Cl$-Phenyl | H | H | 7/9 | F | Cl | H |
| 2-$OCH_2CH_2Cl$-Phenyl | H | $CH_3$ | 7 | F | Cl | H |
| 2-$OCH_2CH_2Cl$-Phenyl | H | H | 7/9 | Cl | F | H |
| 2-$OCH_2CH_2Cl$-Phenyl | H | $CH_3$ | 7 | Cl | F | H |
| 2-$OCH_2CH_2Cl$-Phenyl | H | H | 7/9 | F | $CH_3$ | H |
| 2-$OCH_2CH_2Cl$-Phenyl | H | $CH_3$ | 7 | F | $CH_3$ | H |
| 2-$OCH_2CH_2Cl$-Phenyl | H | H | 7/9 | $CH_3$ | F | H |
| 2-$OCH_2CH_2Cl$-Phenyl | H | $CH_3$ | 7 | $CH_3$ | F | H |
| 2-$OCH_2CH_2Cl$-Phenyl | H | H | 7/9 | F | $OCH_3$ | H |

Tabelle III (Fortsetzung)

| A | $R^1$ | $R^2$ | Pos. | $R^3$ | $R^4$ | $R^5$ |
|---|---|---|---|---|---|---|
| 2-$OCH_2CH_2Cl$-Phenyl | H | $CH_3$ | 7 | F | $OCH_3$ | H |
| 2-$OCH_2CH_2Cl$-Phenyl | H | H | 7/9 | $OCH_3$ | F | H |
| 2-$OCH_2CH_2Cl$-Phenyl | H | $CH_3$ | 7 | $OCH_3$ | F | H |
| 2-$OCH_2CH_2Cl$-Phenyl | H | H | 7/9 | $N(CH_3)_2$ | Cl | H |
| 2-$OCH_2CH_2Cl$-Phenyl | H | $CH_3$ | 7 | $N(CH_3)_2$ | Cl | H |
| 2-$OCH_2CH_2Cl$-Phenyl | H | H | 7/9 | $SCH_3$ | Cl | H |
| 2-$OCH_2CH_2Cl$-Phenyl | H | $CH_3$ | 7 | $SCH_3$ | Cl | H |
| 2-$SCH_3$-Phenyl | H | H | 7/9 | $CH_3$ | $CH_3$ | H |
| 2-$SCH_3$-Phenyl | H | $CH_3$ | 7 | $CH_3$ | $CH_3$ | H |
| 2-$SCH_3$-Phenyl | H | H | 7/9 | Cl | $CH_3$ | H |
| 2-$SCH_3$-Phenyl | H | $CH_3$ | 7 | Cl | $CH_3$ | H |
| 2-$SCH_3$-Phenyl | H | H | 7/9 | $CH_3$ | Cl | H |
| 2-$SCH_3$-Phenyl | H | $CH_3$ | 7 | $CH_3$ | Cl | H |
| 2-$SCH_3$-Phenyl | H | H | 7/9 | Cl | Cl | H |
| 2-$SCH_3$-Phenyl | H | $CH_3$ | 7 | Cl | Cl | H |
| 2-$SCH_3$-Phenyl | H | H | 7/9 | $OCH_3$ | $OCH_3$ | H |
| 2-$SCH_3$-Phenyl | H | $CH_3$ | 7 | $OCH_3$ | $OCH_3$ | H |
| 2-$SCH_3$-Phenyl | H | H | 7/9 | $CH_3$ | $OCH_3$ | H |
| 2-$SCH_3$-Phenyl | H | $CH_3$ | 7 | $CH_3$ | $OCH_3$ | H |
| 2-$SCH_3$-Phenyl | H | H | 7/9 | $OCH_3$ | $CH_3$ | H |
| 2-$SCH_3$-Phenyl | H | $CH_3$ | 7 | $OCH_3$ | $CH_3$ | H |
| 2-$SCH_3$-Phenyl | H | H | 7/9 | Cl | $OCH_3$ | H |
| 2-$SCH_3$-Phenyl | H | $CH_3$ | 7 | Cl | $OCH_3$ | H |
| 2-$SCH_3$-Phenyl | H | H | 7/9 | $OCH_3$ | Cl | H |
| 2-$SCH_3$-Phenyl | H | H | 7 | $OCH_3$ | Cl | H |
| 2-$SO_2CH_3$-Phenyl | H | H | 7/9 | $CH_3$ | $CH_3$ | H |
| 2-$SO_2CH_3$-Phenyl | H | $CH_3$ | 7 | $CH_3$ | $CH_3$ | H |
| 2-$SO_2CH_3$-Phenyl | H | H | 7/9 | Cl | $CH_3$ | H |
| 2-$SO_2CH_3$-Phenyl | H | $CH_3$ | 7 | Cl | $CH_3$ | H |
| 2-$SO_2CH_3$-Phenyl | H | H | 7/9 | $CH_3$ | Cl | H |
| 2-$SO_2CH_3$-Phenyl | H | $CH_3$ | 7 | $CH_3$ | Cl | H |
| 2-$SO_2CH_3$-Phenyl | H | H | 7/9 | Cl | Cl | H |
| 2-$SO_2CH_3$-Phenyl | H | $CH_3$ | 7 | Cl | Cl | H |
| 2-$SO_2CH_3$-Phenyl | H | H | 7/9 | $OCH_3$ | $OCH_3$ | H |
| 2-$SO_2CH_3$-Phenyl | H | $CH_3$ | 7 | $OCH_3$ | $OCH_3$ | H |
| 2-$SO_2CH_3$-Phenyl | H | H | 7/9 | $CH_3$ | $OCH_3$ | H |
| 2-$SO_2CH_3$-Phenyl | H | $CH_3$ | 7 | $CH_3$ | $OCH_3$ | H |
| 2-$SO_2CH_3$-Phenyl | H | H | 7/9 | $OCH_3$ | $CH_3$ | H |
| 2-$SO_2CH_3$-Phenyl | H | $CH_3$ | 7 | $OCH_3$ | $CH_3$ | H |
| 2-$SO_2CH_3$-Phenyl | H | H | 7/9 | Cl | $OCH_3$ | H |

Tabelle III (Fortsetzung)

| A | $R^1$ | $R^2$ | Pos. | $R^3$ | $R^4$ | $R^5$ |
|---|---|---|---|---|---|---|
| 2-$SO_2CH_3$-Phenyl | H | $CH_3$ | 7 | Cl | $OCH_3$ | H |
| 2-$SO_2CH_3$-Phenyl | H | H | 7/9 | $OCH_3$ | Cl | H |
| 2-$SO_2CH_3$-Phenyl | H | $CH_3$ | 7 | $OCH_3$ | Cl | H |
| 2-$SO_2N(CH_3)_2$-Phenyl | H | H | 7/9 | $CH_3$ | $CH_3$ | H |
| 2-$SO_2N(CH_3)_2$-Phenyl | H | $CH_3$ | 7 | $CH_3$ | $CH_3$ | H |
| 2-$SO_2N(CH_3)_2$-Phenyl | H | H | 7/9 | Cl | $CH_3$ | H |
| 2-$SO_2N(CH_3)_2$-Phenyl | H | $CH_3$ | 7 | Cl | $CH_3$ | H |
| 2-$SO_2N(CH_3)_2$-Phenyl | H | H | 7/9 | $CH_3$ | Cl | H |
| 2-$SO_2N(CH_3)_2$-Phenyl | H | $CH_3$ | 7 | $CH_3$ | Cl | H |
| 2-$SO_2N(CH_3)_2$-Phenyl | H | H | 7/9 | Cl | Cl | H |
| 2-$SO_2N(CH_3)_2$-Phenyl | H | $CH_3$ | 7 | Cl | Cl | H |
| 2-$SO_2N(CH_3)_2$-Phenyl | H | H | 7/9 | $OCH_3$ | $OCH_3$ | H |
| 2-$SO_2N(CH_3)_2$-Phenyl | H | $CH_3$ | 7 | $OCH_3$ | $OCH_3$ | H |
| 2-$SO_2N(CH_3)_2$-Phenyl | H | H | 7/9 | $CH_3$ | $OCH_3$ | H |
| 2-$SO_2N(CH_3)_2$-Phenyl | H | $CH_3$ | 7 | $CH_3$ | $OCH_3$ | H |
| 2-$SO_2N(CH_3)_2$-Phenyl | H | H | 7/9 | $OCH_3$ | $CH_3$ | H |
| 2-$SO_2N(CH_3)_2$-Phenyl | H | $CH_3$ | 7 | $OCH_3$ | $CH_3$ | H |
| 2-$SO_2N(CH_3)_2$-Phenyl | H | H | 7/9 | Cl | $OCH_3$ | H |
| 2-$SO_2N(CH_3)_2$-Phenyl | H | $CH_3$ | 7 | Cl | $OCH_3$ | H |
| 2-$SO_2N(CH_3)_2$-Phenyl | H | H | 7/9 | $OCH_3$ | Cl | H |
| 2-$SO_2N(CH_3)_2$-Phenyl | H | $CH_3$ | 7 | $OCH_3$ | Cl | H |
| 2,6-Cl,Cl-Phenyl | H | H | 7/9 | $CH_3$ | $CH_3$ | H |
| 2,6-Cl,Cl-Phenyl | H | $CH_3$ | 7 | $CH_3$ | $CH_3$ | H |
| 2,6-Cl,Cl-Phenyl | H | H | 7/9 | Cl | $CH_3$ | H |
| 2,6-Cl,Cl-Phenyl | H | $CH_3$ | 7 | Cl | $CH_3$ | H |
| 2,6-Cl,Cl-Phenyl | H | H | 7/9 | $CH_3$ | Cl | H |
| 2,6-Cl,Cl-Phenyl | H | $CH_3$ | 7 | $CH_3$ | Cl | H |
| 2,6-Cl,Cl-Phenyl | H | H | 7/9 | Cl | Cl | H |
| 2,6-Cl,Cl-Phenyl | H | $CH_3$ | 7 | Cl | Cl | H |
| 2,6-Cl,Cl-Phenyl | H | H | 7/9 | $OCH_3$ | $OCH_3$ | H |
| 2,6-Cl,Cl-Phenyl | H | $CH_3$ | 7 | $OCH_3$ | $OCH_3$ | H |
| 2,6-Cl,Cl-Phenyl | H | H | 7/9 | $CH_3$ | $OCH_3$ | H |
| 2,6-Cl,Cl-Phenyl | H | $CH_3$ | 7 | $CH_3$ | $OCH_3$ | H |
| 2,6-Cl,Cl-Phenyl | H | H | 7/9 | $OCH_3$ | $CH_3$ | H |
| 2,6-Cl,Cl-Phenyl | H | $CH_3$ | 7 | $OCH_3$ | $CH_3$ | H |
| 2,6-Cl,Cl-Phenyl | H | H | 7/9 | Cl | $OCH_3$ | H |
| 2,6-Cl,Cl-Phenyl | H | $CH_3$ | 7 | Cl | $OCH_3$ | H |
| 2,6-Cl,Cl-Phenyl | H | H | 7/9 | $OCH_3$ | Cl | H |
| 2,6-Cl,Cl-Phenyl | H | $CH_3$ | 7 | $OCH_3$ | Cl | H |
| 2,6-Cl,Cl-Phenyl | H | H | 7/9 | $CF_3$ | $CF_3$ | H |

Tabelle III (Fortsetzung)

| A | $R^1$ | $R^2$ | Pos. | $R^3$ | $R^4$ | $R^5$ |
|---|---|---|---|---|---|---|
| 2,6-Cl,Cl-Phenyl | H | $CH_3$ | 7 | $CF_3$ | $CF_3$ | H |
| 2,6-Cl,Cl-Phenyl | H | H | 7/9 | Cl | $CF_3$ | H |
| 2,6-Cl,Cl-Phenyl | H | $CH_3$ | 7 | Cl | $CF_3$ | H |
| 2,6-Cl,Cl-Phenyl | H | H | 7/9 | $CF_3$ | Cl | H |
| 2,6-Cl,Cl-Phenyl | H | $CH_3$ | 7 | $CF_3$ | Cl | H |
| 2,6-Cl,Cl-Phenyl | H | H | 7/9 | F | $CF_3$ | H |
| 2,6-Cl,Cl-Phenyl | H | $CH_3$ | 7 | F | $CF_3$ | H |
| 2,6-Cl,Cl-Phenyl | H | H | 7/9 | $CF_3$ | F | H |
| 2,6-Cl,Cl-Phenyl | H | $CH_3$ | 7 | $CF_3$ | F | H |
| 2,6-Cl,Cl-Phenyl | H | H | 7/9 | $CH_3$ | $CF_3$ | H |
| 2,6-Cl,Cl-Phenyl | H | $CH_3$ | 7 | $CH_3$ | $CF_3$ | H |
| 2,6-Cl,Cl-Phenyl | H | H | 7/9 | $CF_3$ | $CH_3$ | H |
| 2,6-Cl,Cl-Phenyl | H | $CH_3$ | 7 | $CF_3$ | $CH_3$ | H |
| 2,6-Cl,Cl-Phenyl | H | H | 7/9 | $OCH_3$ | $CF_3$ | H |
| 2,6-Cl,Cl-Phenyl | H | $CH_3$ | 7 | $OCH_3$ | $CF_3$ | H |
| 2,6-Cl,Cl-Phenyl | H | H | 7/9 | $CF_3$ | $OCH_3$ | H |
| 2,6-Cl,Cl-Phenyl | H | $CH_3$ | 7 | $CF_3$ | $OCH_3$ | H |
| 2,6-Cl,Cl-Phenyl | H | H | 7/9 | F | F | H |
| 2,6-Cl,Cl-Phenyl | H | $CH_3$ | 7 | F | F | H |
| 2,6-Cl,Cl-Phenyl | H | H | 7/9 | F | Cl | H |
| 2,6-Cl,Cl-Phenyl | H | $CH_3$ | 7 | F | Cl | H |
| 2,6-Cl,Cl-Phenyl | H | H | 7/9 | Cl | F | H |
| 2,6-Cl,Cl-Phenyl | H | $CH_3$ | 7 | Cl | F | H |
| 2,6-Cl,Cl-Phenyl | H | H | 7/9 | F | $CH_3$ | H |
| 2,6-Cl,Cl-Phenyl | H | $CH_3$ | 7 | F | $CH_3$ | H |
| 2,6-Cl,Cl-Phenyl | H | H | 7/9 | $CH_3$ | F | H |
| 2,6-Cl,Cl-Phenyl | H | $CH_3$ | 7 | $CH_3$ | F | H |
| 2,6-Cl,Cl-Phenyl | H | H | 7/9 | F | $OCH_3$ | H |
| 2,6-Cl,Cl-Phenyl | H | $CH_3$ | 7 | F | $OCH_3$ | H |
| 2,6-Cl,Cl-Phenyl | H | H | 7/9 | $OCH_3$ | F | H |
| 2,6-Cl,Cl-Phenyl | H | $CH_3$ | 7 | $OCH_3$ | F | H |
| 2,6-Cl,Cl-Phenyl | H | H | 7/9 | $N(CH_3)_2$ | Cl | H |
| 2,6-Cl,Cl-Phenyl | H | $CH_3$ | 7 | $N(CH_3)_2$ | Cl | H |
| 2,6-Cl,Cl-Phenyl | H | H | 7/9 | $SCH_3$ | Cl | H |
| 2,6-Cl,Cl-Phenyl | H | $CH_3$ | 7 | $SCH_3$ | Cl | H |
| 2-Cl,6-$CH_3$-Phenyl | H | H | 7/9 | $CH_3$ | $CH_3$ | H |
| 2-Cl,6-$CH_3$-Phenyl | H | $CH_3$ | 7 | $CH_3$ | $CH_3$ | H |
| 2-Cl,6-$CH_3$-Phenyl | H | H | 7/9 | Cl | $CH_3$ | H |
| 2-Cl,6-$CH_3$-Phenyl | H | $CH_3$ | 7 | Cl | $CH_3$ | H |
| 2-Cl,6-$CH_3$-Phenyl | H | H | 7/9 | $CH_3$ | Cl | H |

Tabelle III (Fortsetzung)

| A | $R^1$ | $R^2$ | Pos. | $R^3$ | $R^4$ | $R^5$ |
|---|---|---|---|---|---|---|
| 2-Cl,6-$CH_3$-Phenyl | H | $CH_3$ | 7 | $CH_3$ | Cl | H |
| 2-Cl,6-$CH_3$-Phenyl | H | H | 7/9 | Cl | Cl | H |
| 2-Cl,6-$CH_3$-Phenyl | H | $CH_3$ | 7 | Cl | Cl | H |
| 2-Cl,6-$CH_3$-Phenyl | H | H | 7/9 | $OCH_3$ | $OCH_3$ | H |
| 2-Cl,6-$CH_3$-Phenyl | H | $CH_3$ | 7 | $OCH_3$ | $OCH_3$ | H |
| 2-Cl,6-$CH_3$-Phenyl | H | H | 7/9 | $CH_3$ | $OCH_3$ | H |
| 2-Cl,6-$CH_3$-Phenyl | H | $CH_3$ | 7 | $CH_3$ | $OCH_3$ | H |
| 2-Cl,6-$CH_3$-Phenyl | H | H | 7/9 | $OCH_3$ | $CH_3$ | H |
| 2-Cl,6-$CH_3$-Phenyl | H | $CH_3$ | 7 | $OCH_3$ | $CH_3$ | H |
| 2-Cl,6-$CH_3$-Phenyl | H | H | 7/9 | Cl | $OCH_3$ | H |
| 2-Cl,6-$CH_3$-Phenyl | H | $CH_3$ | 7 | Cl | $OCH_3$ | H |
| 2-Cl,6-$CH_3$-Phenyl | H | H | 7/9 | $OCH_3$ | Cl | H |
| 2-Cl,6-$CH_3$-Phenyl | H | $CH_3$ | 7 | $OCH_3$ | Cl | H |
| 2-Cl,6-$CH_3$-Phenyl | H | H | 7/9 | $CF_3$ | $CF_3$ | H |
| 2-Cl,6-$CH_3$-Phenyl | H | $CH_3$ | 7 | $CF_3$ | $CF_3$ | H |
| 2-Cl,6-$CH_3$-Phenyl | H | H | 7/9 | Cl | $CF_3$ | H |
| 2-Cl,6-$CH_3$-Phenyl | H | $CH_3$ | 7 | Cl | $CF_3$ | H |
| 2-Cl,6-$CH_3$-Phenyl | H | H | 7/9 | $CF_3$ | Cl | H |
| 2-Cl,6-$CH_3$-Phenyl | H | $CH_3$ | 7 | $CF_3$ | Cl | H |
| 2-Cl,6-$CH_3$-Phenyl | H | H | 7/9 | F | $CF_3$ | H |
| 2-Cl,6-$CH_3$-Phenyl | H | $CH_3$ | 7 | F | $CF_3$ | H |
| 2-Cl,6-$CH_3$-Phenyl | H | H | 7/9 | $CF_3$ | F | H |
| 2-Cl,6-$CH_3$-Phenyl | H | $CH_3$ | 7 | $CF_3$ | F | H |
| 2-Cl,6-$CH_3$-Phenyl | H | H | 7/9 | $CH_3$ | $CF_3$ | H |
| 2-Cl,6-$CH_3$-Phenyl | H | $CH_3$ | 7 | $CH_3$ | $CF_3$ | H |
| 2-Cl,6-$CH_3$-Phenyl | H | H | 7/9 | $CF_3$ | $CH_3$ | H |
| 2-Cl,6-$CH_3$-Phenyl | H | $CH_3$ | 7 | $CF_3$ | $CH_3$ | H |
| 2-Cl,6-$CH_3$-Phenyl | H | H | 7/9 | $OCH_3$ | $CF_3$ | H |
| 2-Cl,6-$CH_3$-Phenyl | H | $CH_3$ | 7 | $OCH_3$ | $CF_3$ | H |
| 2-Cl,6-$CH_3$-Phenyl | H | H | 7/9 | $CF_3$ | $OCH_3$ | H |
| 2-Cl,6-$CH_3$-Phenyl | H | $CH_3$ | 7 | $CF_3$ | $OCH_3$ | H |
| 2-Cl,6-$CH_3$-Phenyl | H | H | 7/9 | F | F | H |
| 2-Cl,6-$CH_3$-Phenyl | H | $CH_3$ | 7 | F | F | H |
| 2-Cl,6-$CH_3$-Phenyl | H | H | 7/9 | F | Cl | H |
| 2-Cl,6-$CH_3$-Phenyl | H | $CH_3$ | 7 | F | Cl | H |
| 2-Cl,6-$CH_3$-Phenyl | H | H | 7/9 | Cl | F | H |
| 2-Cl,6-$CH_3$-Phenyl | H | $CH_3$ | 7 | Cl | F | H |
| 2-Cl,6-$CH_3$-Phenyl | H | H | 7/9 | F | $CH_3$ | H |
| 2-Cl,6-$CH_3$-Phenyl | H | $CH_3$ | 7 | F | $CH_3$ | H |
| 2-Cl,6-$CH_3$-Phenyl | H | H | 7/9 | $CH_3$ | F | H |

Tabelle III (Fortsetzung)

| A | $R^1$ | $R^2$ | Pos. | $R^3$ | $R^4$ | $R^5$ |
|---|---|---|---|---|---|---|
| 2-Cl,6-$CH_3$-Phenyl | H | $CH_3$ | 7 | $CH_3$ | F | H |
| 2-Cl,6-$CH_3$-Phenyl | H | H | 7/9 | F | $OCH_3$ | H |
| 2-Cl,6-$CH_3$-Phenyl | H | $CH_3$ | 7 | F | $OCH_3$ | H |
| 2-Cl,6-$CH_3$-Phenyl | H | H | 7/9 | $OCH_3$ | F | H |
| 2-Cl,6-$CH_3$-Phenyl | H | $CH_3$ | 7 | $OCH_3$ | F | H |
| 2-Cl,6-$CH_3$-Phenyl | H | H | 7/9 | $N(CH_3)_2$ | Cl | H |
| 2-Cl,6-$CH_3$-Phenyl | H | $CH_3$ | 7 | $N(CH_3)_2$ | Cl | H |
| 2-Cl,6-$CH_3$-Phenyl | H | H | 7/9 | $SCH_3$ | Cl | H |
| 2-Cl,6-$CH_3$-Phenyl | H | $CH_3$ | 7 | $SCH_3$ | Cl | H |
| 2,5-Cl,Cl-Phenyl | H | H | 7/9 | $CH_3$ | $CH_3$ | H |
| 2,5-Cl,Cl-Phenyl | H | $CH_3$ | 7 | $CH_3$ | $CH_3$ | H |
| 2,5-Cl,Cl-Phenyl | H | H | 7/9 | Cl | $CH_3$ | H |
| 2,5-Cl,Cl-Phenyl | H | $CH_3$ | 7 | Cl | $CH_3$ | H |
| 2,5-Cl,Cl-Phenyl | H | H | 7/9 | $CH_3$ | Cl | H |
| 2,5-Cl,Cl-Phenyl | H | $CH_3$ | 7 | $CH_3$ | Cl | H |
| 2,5-Cl,Cl-Phenyl | H | H | 7/9 | Cl | Cl | H |
| 2,5-Cl,Cl-Phenyl | H | $CH_3$ | 7 | Cl | Cl | H |
| 2,5-Cl,Cl-Phenyl | H | H | 7/9 | $OCH_3$ | $OCH_3$ | H |
| 2,5-Cl,Cl-Phenyl | H | $CH_3$ | 7 | $OCH_3$ | $OCH_3$ | H |
| 2,5-Cl,Cl-Phenyl | H | H | 7/9 | $CH_3$ | $OCH_3$ | H |
| 2,5-Cl,Cl-Phenyl | H | $CH_3$ | 7 | $CH_3$ | $OCH_3$ | H |
| 2,5-Cl,Cl-Phenyl | H | H | 7/9 | $OCH_3$ | $CH_3$ | H |
| 2,5-Cl,Cl-Phenyl | H | $CH_3$ | 7 | $OCH_3$ | $CH_3$ | H |
| 2,5-Cl,Cl-Phenyl | H | H | 7/9 | Cl | $OCH_3$ | H |
| 2,5-Cl,Cl-Phenyl | H | $CH_3$ | 7 | Cl | $OCH_3$ | H |
| 2,5-Cl,Cl-Phenyl | H | H | 7/9 | $OCH_3$ | Cl | H |
| 2,5-Cl,Cl-Phenyl | H | $CH_3$ | 7 | $OCH_3$ | Cl | H |
| 2,5-Cl,Cl-Phenyl | H | H | 7/9 | $CF_3$ | $CF_3$ | H |
| 2,5-Cl,Cl-Phenyl | H | $CH_3$ | 7 | $CF_3$ | $CF_3$ | H |
| 2,5-Cl,Cl-Phenyl | H | H | 7/9 | Cl | $CF_3$ | H |
| 2,5-Cl,Cl-Phenyl | H | $CH_3$ | 7 | Cl | $CF_3$ | H |
| 2,5-Cl,Cl-Phenyl | H | H | 7/9 | $CF_3$ | Cl | H |
| 2,5-Cl,Cl-Phenyl | H | $CH_3$ | 7 | $CF_3$ | Cl | H |
| 2,5-Cl,Cl-Phenyl | H | H | 7/9 | F | $CF_3$ | H |
| 2,5-Cl,Cl-Phenyl | H | $CH_3$ | 7 | F | $CF_3$ | H |
| 2,5-Cl,Cl-Phenyl | H | H | 7/9 | $CF_3$ | F | H |
| 2,5-Cl,Cl-Phenyl | H | $CH_3$ | 7 | $CF_3$ | F | H |
| 2,5-Cl,Cl-Phenyl | H | H | 7/9 | $CH_3$ | $CF_3$ | H |
| 2,5-Cl,Cl-Phenyl | H | $CH_3$ | 7 | $CH_3$ | $CF_3$ | H |
| 2,5-Cl,Cl-Phenyl | H | H | 7/9 | $CF_3$ | $CH_3$ | H |

Tabelle III (Fortsetzung)

| A | $R^1$ | $R^2$ | Pos. | $R^3$ | $R^4$ | $R^5$ |
|---|---|---|---|---|---|---|
| 2,5-Cl,Cl-Phenyl | H | $CH_3$ | 7 | $CF_3$ | $CH_3$ | H |
| 2,5-Cl,Cl-Phenyl | H | H | 7/9 | $OCH_3$ | $CF_3$ | H |
| 2,5-Cl,Cl-Phenyl | H | $CH_3$ | 7 | $OCH_3$ | $CF_3$ | H |
| 2,5-Cl,Cl-Phenyl | H | H | 7/9 | $CF_3$ | $OCH_3$ | H |
| 2,5-Cl,Cl-Phenyl | H | $CH_3$ | 7 | $CF_3$ | $OCH_3$ | H |
| 2,5-Cl,Cl-Phenyl | H | H | 7/9 | F | F | H |
| 2,5-Cl,Cl-Phenyl | H | $CH_3$ | 7 | F | F | H |
| 2,5-Cl,Cl-Phenyl | H | H | 7/9 | F | Cl | H |
| 2,5-Cl,Cl-Phenyl | H | $CH_3$ | 7 | F | Cl | H |
| 2,5-Cl,Cl-Phenyl | H | H | 7/9 | Cl | F | H |
| 2,5-Cl,Cl-Phenyl | H | $CH_3$ | 7 | Cl | F | H |
| 2,5-Cl,Cl-Phenyl | H | H | 7/9 | F | $CH_3$ | H |
| 2,5-Cl,Cl-Phenyl | H | $CH_3$ | 7 | F | $CH_3$ | H |
| 2,5-Cl,Cl-Phenyl | H | H | 7/9 | $CH_3$ | F | H |
| 2,5-Cl,Cl-Phenyl | H | $CH_3$ | 7 | $CH_3$ | F | H |
| 2,5-Cl,Cl-Phenyl | H | H | 7/9 | F | $OCH_3$ | H |
| 2,5-Cl,Cl-Phenyl | H | $CH_3$ | 7 | F | $OCH_3$ | H |
| 2,5-Cl,Cl-Phenyl | H | H | 7/9 | $OCH_3$ | F | H |
| 2,5-Cl,Cl-Phenyl | H | $CH_3$ | 7 | $OCH_3$ | F | H |
| 2,5-Cl,Cl-Phenyl | H | H | 7/9 | $N(CH_3)_2$ | Cl | H |
| 2,5-Cl,Cl-Phenyl | H | $CH_3$ | 7 | $N(CH_3)_2$ | Cl | H |
| 2,5-Cl,Cl-Phenyl | H | H | 7/9 | $SCH_3$ | Cl | H |
| 2,5-Cl,Cl-Phenyl | H | $CH_3$ | 7 | $SCH_3$ | Cl | H |
| 2-$CO_2CH_3$,3-F-Phenyl | H | H | 7/9 | $CH_3$ | $CH_3$ | H |
| 2-$CO_2CH_3$,3-F-Phenyl | H | $CH_3$ | 7 | $CH_3$ | $CH_3$ | H |
| 2-$CO_2CH_3$,3-F-Phenyl | H | H | 7/9 | Cl | $CH_3$ | H |
| 2-$CO_2CH_3$,3-F-Phenyl | H | $CH_3$ | 7 | Cl | $CH_3$ | H |
| 2-$CO_2CH_3$,3-F-Phenyl | H | H | 7/9 | $CH_3$ | Cl | H |
| 2-$CO_2CH_3$,3-F-Phenyl | H | $CH_3$ | 7 | $CH_3$ | Cl | H |
| 2-$CO_2CH_3$,3-F-Phenyl | H | H | 7/9 | Cl | Cl | H |
| 2-$CO_2CH_3$,3-F-Phenyl | H | $CH_3$ | 7 | Cl | Cl | H |
| 2-$CO_2CH_3$,3-F-Phenyl | H | H | 7/9 | $OCH_3$ | $OCH_3$ | H |
| 2-$CO_2CH_3$,3-F-Phenyl | H | $CH_3$ | 7 | $OCH_3$ | $OCH_3$ | H |
| 2-$CO_2CH_3$,3-F-Phenyl | H | H | 7/9 | $CH_3$ | $OCH_3$ | H |
| 2-$CO_2CH_3$,3-F-Phenyl | H | $CH_3$ | 7 | $CH_3$ | $OCH_3$ | H |
| 2-$CO_2CH_3$,3-F-Phenyl | H | H | 7/9 | $OCH_3$ | $CH_3$ | H |
| 2-$CO_2CH_3$,3-F-Phenyl | H | $CH_3$ | 7 | $OCH_3$ | $CH_3$ | H |
| 2-$CO_2CH_3$,3-F-Phenyl | H | H | 7/9 | Cl | $OCH_3$ | H |
| 2-$CO_2CH_3$,3-F-Phenyl | H | $CH_3$ | 7 | Cl | $OCH_3$ | H |
| 2-$CO_2CH_3$,3-F-Phenyl | H | H | 7/9 | $OCH_3$ | Cl | H |

Tabelle III (Fortsetzung)

| A | $R^1$ | $R^2$ | Pos. | $R^3$ | $R^4$ | $R^5$ |
|---|---|---|---|---|---|---|
| 2-$CO_2CH_3$, 3-F-Phenyl | H | $CH_3$ | 7 | $OCH_3$ | Cl | H |
| 2-$CO_2CH_3$, 3-F-Phenyl | H | H | 7/9 | F | F | H |
| 2-$CO_2CH_3$, 3-F-Phenyl | H | $CH_3$ | 7 | F | F | H |
| 2-$CO_2CH_3$, 3-F-Phenyl | H | H | 7/9 | F | Cl | H |
| 2-$CO_2CH_3$, 3-F-Phenyl | H | $CH_3$ | 7 | F | Cl | H |
| 2-$CO_2CH_3$, 3-F-Phenyl | H | H | 7/9 | Cl | F | H |
| 2-$CO_2CH_3$, 3-F-Phenyl | H | $CH_3$ | 7 | Cl | F | H |
| 2-$CO_2CH_3$, 3-F-Phenyl | H | H | 7/9 | F | $CH_3$ | H |
| 2-$CO_2CH_3$, 3-F-Phenyl | H | $CH_3$ | 7 | F | $CH_3$ | H |
| 2-$CO_2CH_3$, 3-F-Phenyl | H | H | 7/9 | $CH_3$ | F | H |
| 2-$CO_2CH_3$, 3-F-Phenyl | H | $CH_3$ | 7 | $CH_3$ | F | H |
| 2-$CO_2CH_3$, 3-F-Phenyl | H | H | 7/9 | F | $OCH_3$ | H |
| 2-$CO_2CH_3$, 3-F-Phenyl | H | $CH_3$ | 7 | F | $OCH_3$ | H |
| 2-$CO_2CH_3$, 3-F-Phenyl | H | H | 7/9 | $OCH_3$ | F | H |
| 2-$CO_2CH_3$, 3-F-Phenyl | H | $CH_3$ | 7 | $OCH_3$ | F | H |
| 2-$CO_2CH_3$, 3-F-Phenyl | H | H | 7/9 | $N(CH_3)_2$ | Cl | H |
| 2-$CO_2CH_3$, 3-F-Phenyl | H | $CH_3$ | 7 | $N(CH_3)_2$ | Cl | H |
| 2-$CO_2CH_3$, 3-F-Phenyl | H | H | 7/9 | $SCH_3$ | Cl | H |
| 2-$CO_2CH_3$, 3-F-Phenyl | H | $CH_3$ | 7 | $SCH_3$ | Cl | H |
| 2-$CO_2CH_3$, 3-F-Phenyl | H | H | 7/9 | $CF_3$ | $CF_3$ | H |
| 2-$CO_2CH_3$, 3-F-Phenyl | H | $CH_3$ | 7 | $CF_3$ | $CF_3$ | H |
| 2-$CO_2CH_3$, 3-F-Phenyl | H | H | 7/9 | Cl | $CF_3$ | H |
| 2-$CO_2CH_3$, 3-F-Phenyl | H | $CH_3$ | 7 | Cl | $CF_3$ | H |
| 2-$CO_2CH_3$, 3-F-Phenyl | H | H | 7/9 | $CF_3$ | Cl | H |
| 2-$CO_2CH_3$, 3-F-Phenyl | H | $CH_3$ | 7 | $CF_3$ | Cl | H |
| 2-$CO_2CH_3$, 3-F-Phenyl | H | H | 7/9 | F | $CF_3$ | H |
| 2-$CO_2CH_3$, 3-F-Phenyl | H | $CH_3$ | 7 | F | $CF_3$ | H |
| 2-$CO_2CH_3$, 3-F-Phenyl | H | H | 7/9 | $CF_3$ | F | H |
| 2-$CO_2CH_3$, 3-F-Phenyl | H | $CH_3$ | 7 | $CF_3$ | F | H |
| 2-$CO_2CH_3$, 3-F-Phenyl | H | H | 7/9 | $CH_3$ | $CF_3$ | H |
| 2-$CO_2CH_3$, 3-F-Phenyl | H | $CH_3$ | 7 | $CH_3$ | $CF_3$ | H |
| 2-$CO_2CH_3$, 3-F-Phenyl | H | H | 7/9 | $CF_3$ | $CH_3$ | H |
| 2-$CO_2CH_3$, 3-F-Phenyl | H | $CH_3$ | 7 | $CF_3$ | $CH_3$ | H |
| 2-$CO_2CH_3$, 3-F-Phenyl | H | H | 7/9 | $OCH_3$ | $CF_3$ | H |
| 2-$CO_2CH_3$, 3-F-Phenyl | H | $CH_3$ | 7 | $OCH_3$ | $CF_3$ | H |
| 2-$CO_2CH_3$, 3-F-Phenyl | H | H | 7/9 | $CF_3$ | $OCH_3$ | H |
| 2-$CO_2CH_3$, 3-F-Phenyl | H | $CH_3$ | 7 | $CF_3$ | $OCH_3$ | H |
| 3-Cl-Thienyl | H | H | 7/9 | $CH_3$ | $CH_3$ | H |
| 3-Cl-Thienyl | H | $CH_3$ | 7 | $CH_3$ | $CH_3$ | H |
| 3-Cl-Thienyl | H | H | 7/9 | Cl | $CH_3$ | H |

Tabelle III (Fortsetzung)

| A | $R^1$ | $R^2$ | Pos. | $R^3$ | $R^4$ | $R^5$ |
|---|---|---|---|---|---|---|
| 3-Cl-Thienyl | H | $CH_3$ | 7 | Cl | $CH_3$ | H |
| 3-Cl-Thienyl | H | H | 7/9 | $CH_3$ | Cl | H |
| 3-Cl-Thienyl | H | $CH_3$ | 7 | $CH_3$ | Cl | H |
| 3-Cl-Thienyl | H | H | 7/9 | Cl | Cl | H |
| 3-Cl-Thienyl | H | $CH_3$ | 7 | Cl | Cl | H |
| 3-Cl-Thienyl | H | H | 7/9 | $OCH_3$ | $OCH_3$ | H |
| 3-Cl-Thienyl | H | $CH_3$ | 7 | $OCH_3$ | $OCH_3$ | H |
| 3-Cl-Thienyl | H | H | 7/9 | $CH_3$ | $OCH_3$ | H |
| 3-Cl-Thienyl | H | $CH_3$ | 7 | $CH_3$ | $OCH_3$ | H |
| 3-Cl-Thienyl | H | H | 7/9 | $OCH_3$ | $CH_3$ | H |
| 3-Cl-Thienyl | H | $CH_3$ | 7 | $OCH_3$ | $CH_3$ | H |
| 3-Cl-Thienyl | H | H | 7/9 | Cl | $OCH_3$ | H |
| 3-Cl-Thienyl | H | $CH_3$ | 7 | Cl | $OCH_3$ | H |
| 3-Cl-Thienyl | H | H | 7/9 | $OCH_3$ | Cl | H |
| 3-Cl-Thienyl | H | $CH_3$ | 7 | $OCH_3$ | Cl | H |
| 2-$CO_2CH_3$, 3-Thienyl | H | H | 7/9 | $CH_3$ | $CH_3$ | H |
| 2-$CO_2CH_3$, 3-Thienyl | H | $CH_3$ | 7 | $CH_3$ | $CH_3$ | H |
| 2-$CO_2CH_3$, 3-Thienyl | H | H | 7/9 | Cl | $CH_3$ | H |
| 2-$CO_2CH_3$, 3-Thienyl | H | $CH_3$ | 7 | Cl | $CH_3$ | H |
| 2-$CO_2CH_3$, 3-Thienyl | H | H | 7/9 | $CH_3$ | Cl | H |
| 2-$CO_2CH_3$, 3-Thienyl | H | $CH_3$ | 7 | $CH_3$ | Cl | H |
| 2-$CO_2CH_3$, 3-Thienyl | H | H | 7/9 | Cl | Cl | H |
| 2-$CO_2CH_3$, 3-Thienyl | H | $CH_3$ | 7 | Cl | Cl | H |
| 2-$CO_2CH_3$, 3-Thienyl | H | H | 7/9 | $OCH_3$ | $OCH_3$ | H |
| 2-$CO_2CH_3$, 3-Thienyl | H | $CH_3$ | 7 | $OCH_3$ | $OCH_3$ | H |
| 2-$CO_2CH_3$, 3-Thienyl | H | H | 7/9 | $CH_3$ | $OCH_3$ | H |
| 2-$CO_2CH_3$, 3-Thienyl | H | $CH_3$ | 7 | $CH_3$ | $OCH_3$ | H |
| 2-$CO_2CH_3$, 3-Thienyl | H | H | 7/9 | $OCH_3$ | $CH_3$ | H |
| 2-$CO_2CH_3$, 3-Thienyl | H | $CH_3$ | 7 | $OCH_3$ | $CH_3$ | H |
| 2-$CO_2CH_3$, 3-Thienyl | H | H | 7/9 | Cl | $OCH_3$ | H |
| 2-$CO_2CH_3$, 3-Thienyl | H | $CH_3$ | 7 | Cl | $OCH_3$ | H |
| 2-$CO_2CH_3$, 3-Thienyl | H | H | 7/9 | $OCH_3$ | Cl | H |
| 2-$CO_2CH_3$, 3-Thienyl | H | $CH_3$ | 7 | $OCH_3$ | Cl | H |
| 2-$CO_2CH_3$, 3-Thienyl | H | H | 7/9 | $CF_3$ | $CF_3$ | H |
| 2-$CO_2CH_3$, 3-Thienyl | H | $CH_3$ | 7 | $CF_3$ | $CF_3$ | H |
| 2-$CO_2CH_3$, 3-Thienyl | H | H | 7/9 | Cl | $CF_3$ | H |
| 2-$CO_2CH_3$, 3-Thienyl | H | $CH_3$ | 7 | Cl | $CF_3$ | H |
| 2-$CO_2CH_3$, 3-Thienyl | H | H | 7/9 | $CF_3$ | Cl | H |
| 2-$CO_2CH_3$, 3-Thienyl | H | $CH_3$ | 7 | $CF_3$ | Cl | H |
| 2-$CO_2CH_3$, 3-Thienyl | H | H | 7/9 | F | $CF_3$ | H |

Tabelle III (Fortsetzung)

| A | $R^1$ | $R^2$ | Pos. | $R^3$ | $R^4$ | $R^5$ |
|---|---|---|---|---|---|---|
| 2-$CO_2CH_3$,3-Thienyl | H | $CH_3$ | 7 | F | $CF_3$ | H |
| 2-$CO_2CH_3$,3-Thienyl | H | H | 7/9 | $CF_3$ | F | H |
| 2-$CO_2CH_3$,3-Thienyl | H | $CH_3$ | 7 | $CF_3$ | F | H |
| 2-$CO_2CH_3$,3-Thienyl | H | H | 7/9 | $CH_3$ | $CF_3$ | H |
| 2-$CO_2CH_3$,3-Thienyl | H | $CH_3$ | 7 | $CH_3$ | $CF_3$ | H |
| 2-$CO_2CH_3$,3-Thienyl | H | H | 7/9 | $CF_3$ | $CH_3$ | H |
| 2-$CO_2CH_3$,3-Thienyl | H | $CH_3$ | 7 | $CF_3$ | $CH_3$ | H |
| 2-$CO_2CH_3$,3-Thienyl | H | H | 7/9 | $OCH_3$ | $CF_3$ | H |
| 2-$CO_2CH_3$,3-Thienyl | H | $CH_3$ | 7 | $OCH_3$ | $CF_3$ | H |
| 2-$CO_2CH_3$,3-Thienyl | H | H | 7/9 | $CF_3$ | $OCH_3$ | H |
| 2-$CO_2CH_3$,3-Thienyl | H | $CH_3$ | 7 | $CF_3$ | $OCH_3$ | H |
| 2-$CO_2CH_3$,3-Thienyl | H | H | 7/9 | F | F | H |
| 2-$CO_2CH_3$,3-Thienyl | H | $CH_3$ | 7 | F | F | H |
| 2-$CO_2CH_3$,3-Thienyl | H | H | 7/9 | F | Cl | H |
| 2-$CO_2CH_3$,3-Thienyl | H | $CH_3$ | 7 | F | Cl | H |
| 2-$CO_2CH_3$,3-Thienyl | H | H | 7/9 | Cl | F | H |
| 2-$CO_2CH_3$,3-Thienyl | H | $CH_3$ | 7 | Cl | F | H |
| 2-$CO_2CH_3$,3-Thienyl | H | H | 7/9 | F | $CH_3$ | H |
| 2-$CO_2CH_3$,3-Thienyl | H | $CH_3$ | 7 | F | $CH_3$ | H |
| 2-$CO_2CH_3$,3-Thienyl | H | H | 7/9 | $CH_3$ | F | H |
| 2-$CO_2CH_3$,3-Thienyl | H | $CH_3$ | 7 | $CH_3$ | F | H |
| 2-$CO_2CH_3$,3-Thienyl | H | H | 7/9 | F | $OCH_3$ | H |
| 2-$CO_2CH_3$,3-Thienyl | H | $CH_3$ | 7 | F | $OCH_3$ | H |
| 2-$CO_2CH_3$,3-Thienyl | H | H | 7/9 | $OCH_3$ | F | H |
| 2-$CO_2CH_3$,3-Thienyl | H | $CH_3$ | 7 | $OCH_3$ | F | H |
| 2-$CO_2CH_3$,3-Thienyl | H | H | 7/9 | $N(CH_3)_2$ | Cl | H |
| 2-$CO_2CH_3$,3-Thienyl | H | $CH_3$ | 7 | $N(CH_3)_2$ | Cl | H |
| 2-$CO_2CH_3$,3-Thienyl | H | H | 7/9 | $SCH_3$ | Cl | H |
| 2-$CO_2CH_3$,3-Thienyl | H | $CH_3$ | 7 | $SCH_3$ | Cl | H |
| 1-$CH_3$,4-$CO_2CH_3$,5-Pyrazolyl | H | H | 7/9 | $CH_3$ | $CH_3$ | H |
| 1-$CH_3$,4-$CO_2CH_3$,5-Pyrazolyl | H | $CH_3$ | 7 | $CH_3$ | $CH_3$ | H |
| 1-$CH_3$,4-$CO_2CH_3$,5-Pyrazolyl | H | H | 7/9 | Cl | $CH_3$ | H |
| 1-$CH_3$,4-$CO_2CH_3$,5-Pyrazolyl | H | $CH_3$ | 7 | Cl | $CH_3$ | H |
| 1-$CH_3$,4-$CO_2CH_3$,5-Pyrazolyl | H | H | 7/9 | $CH_3$ | Cl | H |
| 1-$CH_3$,4-$CO_2CH_3$,5-Pyrazolyl | H | $CH_3$ | 7 | $CH_3$ | Cl | H |
| 1-$CH_3$,4-$CO_2CH_3$,5-Pyrazolyl | H | H | 7/9 | Cl | Cl | H |
| 1-$CH_3$,4-$CO_2CH_3$,5-Pyrazolyl | H | $CH_3$ | 7 | Cl | Cl | H |
| 1-$CH_3$,4-$CO_2CH_3$,5-Pyrazolyl | H | H | 7/9 | $OCH_3$ | $OCH_3$ | H |
| 1-$CH_3$,4-$CO_2CH_3$,5-Pyrazolyl | H | $CH_3$ | 7 | $OCH_3$ | $OCH_3$ | H |
| 1-$CH_3$,4-$CO_2CH_3$,5-Pyrazolyl | H | H | 7/9 | $CH_3$ | $OCH_3$ | H |

Tabelle III (Fortsetzung)

| A | $R^1$ | $R^2$ | Pos. | $R^3$ | $R^4$ | $R^5$ |
|---|---|---|---|---|---|---|
| 1-$CH_3$,4-$CO_2CH_3$,5-Pyrazolyl | H | $CH_3$ | 7 | $CH_3$ | $OCH_3$ | H |
| 1-$CH_3$,4-$CO_2CH_3$,5-Pyrazolyl | H | H | 7/9 | $OCH_3$ | $CH_3$ | H |
| 1-$CH_3$,4-$CO_2CH_3$,5-Pyrazolyl | H | $CH_3$ | 7 | $OCH_3$ | $CH_3$ | H |
| 1-$CH_3$,4-$CO_2CH_3$,5-Pyrazolyl | H | H | 7/9 | Cl | $OCH_3$ | H |
| 1-$CH_3$,4-$CO_2CH_3$,5-Pyrazolyl | H | $CH_3$ | 7 | Cl | $OCH_3$ | H |
| 1-$CH_3$,4-$CO_2CH_3$,5-Pyrazolyl | H | H | 7/9 | $OCH_3$ | Cl | H |
| 1-$CH_3$,4-$CO_2CH_3$,5-Pyrazolyl | H | $CH_3$ | 7 | $OCH_3$ | Cl | H |
| 1-$CH_3$,4-$CO_2CH_3$,5-Pyrazolyl | H | H | 7/9 | $CF_3$ | $CF_3$ | H |
| 1-$CH_3$,4-$CO_2CH_3$,5-Pyrazolyl | H | $CH_3$ | 7 | $CF_3$ | $CF_3$ | H |
| 1-$CH_3$,4-$CO_2CH_3$,5-Pyrazolyl | H | H | 7/9 | Cl | $CF_3$ | H |
| 1-$CH_3$,4-$CO_2CH_3$,5-Pyrazolyl | H | $CH_3$ | 7 | Cl | $CF_3$ | H |
| 1-$CH_3$,4-$CO_2CH_3$,5-Pyrazolyl | H | H | 7/9 | $CF_3$ | Cl | H |
| 1-$CH_3$,4-$CO_2CH_3$,5-Pyrazolyl | H | $CH_3$ | 7 | $CF_3$ | Cl | H |
| 1-$CH_3$,4-$CO_2CH_3$,5-Pyrazolyl | H | H | 7/9 | F | $CF_3$ | H |
| 1-$CH_3$,4-$CO_2CH_3$,5-Pyrazolyl | H | $CH_3$ | 7 | F | $CF_3$ | H |
| 1-$CH_3$,4-$CO_2CH_3$,5-Pyrazolyl | H | H | 7/9 | $CF_3$ | F | H |
| 1-$CH_3$,4-$CO_2CH_3$,5-Pyrazolyl | H | $CH_3$ | 7 | $CF_3$ | F | H |
| 1-$CH_3$,4-$CO_2CH_3$,5-Pyrazolyl | H | H | 7/9 | $CH_3$ | $CF_3$ | H |
| 1-$CH_3$,4-$CO_2CH_3$,5-Pyrazolyl | H | $CH_3$ | 7 | $CH_3$ | $CF_3$ | H |
| 1-$CH_3$,4-$CO_2CH_3$,5-Pyrazolyl | H | H | 7/9 | $CF_3$ | $CH_3$ | H |
| 1-$CH_3$,4-$CO_2CH_3$,5-Pyrazolyl | H | $CH_3$ | 7 | $CF_3$ | $CH_3$ | H |
| 1-$CH_3$,4-$CO_2CH_3$,5-Pyrazolyl | H | H | 7/9 | $OCH_3$ | $CF_3$ | H |
| 1-$CH_3$,4-$CO_2CH_3$,5-Pyrazolyl | H | $CH_3$ | 7 | $OCH_3$ | $CF_3$ | H |
| 1-$CH_3$,4-$CO_2CH_3$,5-Pyrazolyl | H | H | 7/9 | $CF_3$ | $OCH_3$ | H |
| 1-$CH_3$,4-$CO_2CH_3$,5-Pyrazolyl | H | $CH_3$ | 7 | $CF_3$ | $OCH_3$ | H |
| 1-$CH_3$,4-$CO_2CH_3$,5-Pyrazolyl | H | H | 7/9 | F | F | H |
| 1-$CH_3$,4-$CO_2CH_3$,5-Pyrazolyl | H | $CH_3$ | 7 | F | F | H |
| 1-$CH_3$,4-$CO_2CH_3$,5-Pyrazolyl | H | H | 7/9 | F | Cl | H |
| 1-$CH_3$,4-$CO_2CH_3$,5-Pyrazolyl | H | $CH_3$ | 7 | F | Cl | H |
| 1-$CH_3$,4-$CO_2CH_3$,5-Pyrazolyl | H | H | 7/9 | Cl | F | H |
| 1-$CH_3$,4-$CO_2CH_3$,5-Pyrazolyl | H | $CH_3$ | 7 | Cl | F | H |
| 1-$CH_3$,4-$CO_2CH_3$,5-Pyrazolyl | H | H | 7/9 | F | $CH_3$ | H |
| 1-$CH_3$,4-$CO_2CH_3$,5-Pyrazolyl | H | $CH_3$ | 7 | F | $CH_3$ | H |
| 1-$CH_3$,4-$CO_2CH_3$,5-Pyrazolyl | H | H | 7/9 | $CH_3$ | F | H |
| 1-$CH_3$,4-$CO_2CH_3$,5-Pyrazolyl | H | $CH_3$ | 7 | $CH_3$ | F | H |
| 1-$CH_3$,4-$CO_2CH_3$,5-Pyrazolyl | H | H | 7/9 | F | $OCH_3$ | H |
| 1-$CH_3$,4-$CO_2CH_3$,5-Pyrazolyl | H | $CH_3$ | 7 | F | $OCH_3$ | H |
| 1-$CH_3$,4-$CO_2CH_3$,5-Pyrazolyl | H | H | 7/9 | $OCH_3$ | F | H |
| 1-$CH_3$,4-$CO_2CH_3$,5-Pyrazolyl | H | $CH_3$ | 7 | $OCH_3$ | F | H |
| 1-$CH_3$,4-$CO_2CH_3$,5-Pyrazolyl | H | H | 7/9 | $N(CH_3)_2$ | Cl | H |

Tabelle III (Fortsetzung)

| A | $R^1$ | $R^2$ | Pos. | $R^3$ | $R^4$ | $R^5$ |
|---|---|---|---|---|---|---|
| 1-$CH_3$,4-$CO_2CH_3$,5-Pyrazolyl | H | $CH_3$ | 7 | $N(CH_3)_2$ | Cl | H |
| 1-$CH_3$,4-$CO_2CH_3$,5-Pyrazolyl | H | H | 7/9 | $SCH_3$ | Cl | H |
| 1-$CH_3$,4-$CO_2CH_3$,5-Pyrazolyl | H | $CH_3$ | 7 | $SCH_3$ | Cl | H |
| 3-$CON(CH_3)_2$,2-Pyridyl | H | H | 7/9 | $CH_3$ | $CH_3$ | H |
| 3-$CON(CH_3)_2$,2-Pyridyl | H | $CH_3$ | 7 | $CH_3$ | $CH_3$ | H |
| 3-$CON(CH_3)_2$,2-Pyridyl | H | H | 7/9 | Cl | $CH_3$ | H |
| 3-$CON(CH_3)_2$,2-Pyridyl | H | $CH_3$ | 7 | Cl | $CH_3$ | H |
| 3-$CON(CH_3)_2$,2-Pyridyl | H | H | 7/9 | $CH_3$ | Cl | H |
| 3-$CON(CH_3)_2$,2-Pyridyl | H | $CH_3$ | 7 | $CH_3$ | Cl | H |
| 3-$CON(CH_3)_2$,2-Pyridyl | H | H | 7/9 | Cl | Cl | H |
| 3-$CON(CH_3)_2$,2-Pyridyl | H | $CH_3$ | 7 | Cl | Cl | H |
| 3-$CON(CH_3)_2$,2-Pyridyl | H | H | 7/9 | $OCH_3$ | $OCH_3$ | H |
| 3-$CON(CH_3)_2$,2-Pyridyl | H | $CH_3$ | 7 | $OCH_3$ | $OCH_3$ | H |
| 3-$CON(CH_3)_2$,2-Pyridyl | H | H | 7/9 | $CH_3$ | $OCH_3$ | H |
| 3-$CON(CH_3)_2$,2-Pyridyl | H | $CH_3$ | 7 | $CH_3$ | $OCH_3$ | H |
| 3-$CON(CH_3)_2$,2-Pyridyl | H | H | 7/9 | $OCH_3$ | $CH_3$ | H |
| 3-$CON(CH_3)_2$,2-Pyridyl | H | $CH_3$ | 7 | $OCH_3$ | $CH_3$ | H |
| 3-$CON(CH_3)_2$,2-Pyridyl | H | H | 7/9 | Cl | $OCH_3$ | H |
| 3-$CON(CH_3)_2$,2-Pyridyl | H | $CH_3$ | 7 | Cl | $OCH_3$ | H |
| 3-$CON(CH_3)_2$,2-Pyridyl | H | H | 7/9 | $OCH_3$ | Cl | H |
| 3-$CON(CH_3)_2$,2-Pyridyl | H | $CH_3$ | 7 | $OCH_3$ | Cl | H |
| 3-$CON(CH_3)_2$,2-Pyridyl | H | H | 7/9 | $CF_3$ | $CF_3$ | H |
| 3-$CON(CH_3)_2$,2-Pyridyl | H | $CH_3$ | 7 | $CF_3$ | $CF_3$ | H |
| 3-$CON(CH_3)_2$,2-Pyridyl | H | H | 7/9 | Cl | $CF_3$ | H |
| 3-$CON(CH_3)_2$,2-Pyridyl | H | $CH_3$ | 7 | Cl | $CF_3$ | H |
| 3-$CON(CH_3)_2$,2-Pyridyl | H | H | 7/9 | $CF_3$ | Cl | H |
| 3-$CON(CH_3)_2$,2-Pyridyl | H | $CH_3$ | 7 | $CF_3$ | Cl | H |
| 3-$CON(CH_3)_2$,2-Pyridyl | H | H | 7/9 | F | $CF_3$ | H |
| 3-$CON(CH_3)_2$,2-Pyridyl | H | $CH_3$ | 7 | F | $CF_3$ | H |
| 3-$CON(CH_3)_2$,2-Pyridyl | H | H | 7/9 | $CF_3$ | F | H |
| 3-$CON(CH_3)_2$,2-Pyridyl | H | $CH_3$ | 7 | $CF_3$ | F | H |
| 3-$CON(CH_3)_2$,2-Pyridyl | H | H | 7/9 | $CH_3$ | $CF_3$ | H |
| 3-$CON(CH_3)_2$,2-Pyridyl | H | $CH_3$ | 7 | $CH_3$ | $CF_3$ | H |
| 3-$CON(CH_3)_2$,2-Pyridyl | H | H | 7/9 | $CF_3$ | $CH_3$ | H |
| 3-$CON(CH_3)_2$,2-Pyridyl | H | $CH_3$ | 7 | $CF_3$ | $CH_3$ | H |
| 3-$CON(CH_3)_2$,2-Pyridyl | H | H | 7/9 | $OCH_3$ | $CF_3$ | H |
| 3-$CON(CH_3)_2$,2-Pyridyl | H | $CH_3$ | 7 | $OCH_3$ | $CF_3$ | H |
| 3-$CON(CH_3)_2$,2-Pyridyl | H | H | 7/9 | $CF_3$ | $OCH_3$ | H |
| 3-$CON(CH_3)_2$,2-Pyridyl | H | $CH_3$ | 7 | $CF_3$ | $OCH_3$ | H |
| 3-$CON(CH_3)_2$,2-Pyridyl | H | H | 7/9 | F | F | H |

Tabelle III (Fortsetzung)

| A | $R^1$ | $R^2$ | Pos. | $R^3$ | $R^4$ | $R^5$ |
|---|---|---|---|---|---|---|
| 3-$CON(CH_3)_2$,2-Pyridyl | H | $CH_3$ | 7 | F | F | H |
| 3-$CON(CH_3)_2$,2-Pyridyl | H | H | 7/9 | F | Cl | H |
| 3-$CON(CH_3)_2$,2-Pyridyl | H | $CH_3$ | 7 | F | Cl | H |
| 3-$CON(CH_3)_2$,2-Pyridyl | H | H | 7/9 | Cl | F | H |
| 3-$CON(CH_3)_2$,2-Pyridyl | H | $CH_3$ | 7 | Cl | F | H |
| 3-$CON(CH_3)_2$,2-Pyridyl | H | H | 7/9 | F | $CH_3$ | H |
| 3-$CON(CH_3)_2$,2-Pyridyl | H | $CH_3$ | 7 | F | $CH_3$ | H |
| 3-$CON(CH_3)_2$,2-Pyridyl | H | H | 7/9 | $CH_3$ | F | H |
| 3-$CON(CH_3)_2$,2-Pyridyl | H | $CH_3$ | 7 | $CH_3$ | F | H |
| 3-$CON(CH_3)_2$,2-Pyridyl | H | H | 7/9 | F | $OCH_3$ | H |
| 3-$CON(CH_3)_2$,2-Pyridyl | H | $CH_3$ | 7 | F | $OCH_3$ | H |
| 3-$CON(CH_3)_2$,2-Pyridyl | H | H | 7/9 | $OCH_3$ | F | H |
| 3-$CON(CH_3)_2$,2-Pyridyl | H | $CH_3$ | 7 | $OCH_3$ | F | H |
| 3-$CON(CH_3)_2$,2-Pyridyl | H | H | 7/9 | $N(CH_3)_2$ | Cl | H |
| 3-$CON(CH_3)_2$,2-Pyridyl | H | $CH_3$ | 7 | $N(CH_3)_2$ | Cl | H |
| 3-$CON(CH_3)_2$,2-Pyridyl | H | H | 7/9 | $SCH_3$ | Cl | H |
| 3-$CON(CH_3)_2$,2-Pyridyl | H | $CH_3$ | 7 | $SCH_3$ | Cl | H |

Tabelle IV

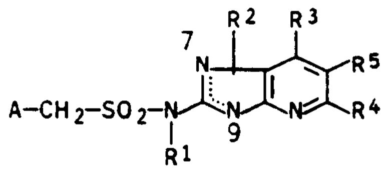

Id

| A | $R^1$ | $R^2$ | Pos. | $R^3$ | $R^4$ | $R^5$ |
|---|---|---|---|---|---|---|
| Phenyl | H | H | 7/9 | $CH_3$ | $CH_3$ | H |
| Phenyl | H | $CH_3$ | 7 | $CH_3$ | $CH_3$ | H |
| Phenyl | H | H | 7/9 | Cl | $CH_3$ | H |
| Phenyl | H | $CH_3$ | 7 | Cl | $CH_3$ | H |
| Phenyl | H | H | 7/9 | $CH_3$ | Cl | H |
| Phenyl | H | $CH_3$ | 7 | $CH_3$ | Cl | H |
| Phenyl | H | H | 7/9 | Cl | Cl | H |
| Phenyl | H | $CH_3$ | 7 | Cl | Cl | H |
| Phenyl | H | H | 7/9 | $OCH_3$ | $OCH_3$ | H |
| Phenyl | H | $CH_3$ | 7 | $OCH_3$ | $OCH_3$ | H |
| Phenyl | H | H | 7/9 | $CH_3$ | $OCH_3$ | H |
| Phenyl | H | $CH_3$ | 7 | $CH_3$ | $OCH_3$ | H |
| Phenyl | H | H | 7/9 | $OCH_3$ | $CH_3$ | H |
| Phenyl | H | $CH_3$ | 7 | $OCH_3$ | $CH_3$ | H |
| Phenyl | H | H | 7/9 | Cl | $OCH_3$ | H |
| Phenyl | H | $CH_3$ | 7 | Cl | $OCH_3$ | H |
| Phenyl | H | H | 7/9 | $OCH_3$ | Cl | H |
| Phenyl | H | $CH_3$ | 7 | $OCH_3$ | Cl | H |
| 2-Cl-Phenyl | H | H | 7/9 | $CH_3$ | $CH_3$ | H |
| 2-Cl-Phenyl | H | $CH_3$ | 7 | $CH_3$ | $CH_3$ | H |
| 2-Cl-Phenyl | H | H | 7/9 | Cl | $CH_3$ | H |
| 2-Cl-Phenyl | H | $CH_3$ | 7 | Cl | $CH_3$ | H |
| 2-Cl-Phenyl | H | H | 7/9 | $CH_3$ | Cl | H |
| 2-Cl-Phenyl | H | $CH_3$ | 7 | $CH_3$ | Cl | H |
| 2-Cl-Phenyl | H | H | 7/9 | Cl | Cl | H |
| 2-Cl-Phenyl | H | $CH_3$ | 7 | Cl | Cl | H |
| 2-Cl-Phenyl | H | H | 7/9 | $OCH_3$ | $OCH_3$ | H |
| 2-Cl-Phenyl | H | $CH_3$ | 7 | $OCH_3$ | $OCH_3$ | H |
| 2-Cl-Phenyl | H | H | 7/9 | $CH_3$ | $OCH_3$ | H |
| 2-Cl-Phenyl | H | $CH_3$ | 7 | $CH_3$ | $OCH_3$ | H |
| 2-Cl-Phenyl | H | H | 7/9 | $OCH_3$ | $CH_3$ | H |
| 2-Cl-Phenyl | H | $CH_3$ | 7 | $OCH_3$ | $CH_3$ | H |
| 2-Cl-Phenyl | H | H | 7/9 | Cl | $OCH_3$ | H |

Tabelle IV (Fortsetzung)

| A | $R^1$ | $R^2$ | Pos. | $R^3$ | $R^4$ | $R^5$ |
|---|---|---|---|---|---|---|
| 2-Cl-Phenyl | H | $CH_3$ | 7 | Cl | $OCH_3$ | H |
| 2-Cl-Phenyl | H | H | 7/9 | $OCH_3$ | Cl | H |
| 2-Cl-Phenyl | H | $CH_3$ | 7 | $OCH_3$ | Cl | H |
| 2-F-Phenyl | H | H | 7/9 | $CH_3$ | $CH_3$ | H |
| 2-F-Phenyl | H | $CH_3$ | 7 | $CH_3$ | $CH_3$ | H |
| 2-F-Phenyl | H | H | 7/9 | Cl | $CH_3$ | H |
| 2-F-Phenyl | H | $CH_3$ | 7 | Cl | $CH_3$ | H |
| 2-F-Phenyl | H | H | 7/9 | $CH_3$ | Cl | H |
| 2-F-Phenyl | H | $CH_3$ | 7 | $CH_3$ | Cl | H |
| 2-F-Phenyl | H | H | 7/9 | Cl | Cl | H |
| 2-F-Phenyl | H | $CH_3$ | 7 | Cl | Cl | H |
| 2-F-Phenyl | H | H | 7/9 | $OCH_3$ | $OCH_3$ | H |
| 2-F-Phenyl | H | $CH_3$ | 7 | $OCH_3$ | $OCH_3$ | H |
| 2-F-Phenyl | H | H | 7/9 | $CH_3$ | $OCH_3$ | H |
| 2-F-Phenyl | H | $CH_3$ | 7 | $CH_3$ | $OCH_3$ | H |
| 2-F-Phenyl | H | H | 7/9 | $OCH_3$ | $CH_3$ | H |
| 2-F-Phenyl | H | $CH_3$ | 7 | $OCH_3$ | $CH_3$ | H |
| 2-F-Phenyl | H | H | 7/9 | Cl | $OCH_3$ | H |
| 2-F-Phenyl | H | $CH_3$ | 7 | Cl | $OCH_3$ | H |
| 2-F-Phenyl | H | H | 7/9 | $OCH_3$ | Cl | H |
| 2-F-Phenyl | H | $CH_3$ | 7 | $OCH_3$ | Cl | H |
| 2-$CH_3$-Phenyl | H | H | 7/9 | $CH_3$ | $CH_3$ | H |
| 2-$CH_3$-Phenyl | H | $CH_3$ | 7 | $CH_3$ | $CH_3$ | H |
| 2-$CH_3$-Phenyl | H | H | 7/9 | Cl | $CH_3$ | H |
| 2-$CH_3$-Phenyl | H | $CH_3$ | 7 | Cl | $CH_3$ | H |
| 2-$CH_3$-Phenyl | H | H | 7/9 | $CH_3$ | Cl | H |
| 2-$CH_3$-Phenyl | H | $CH_3$ | 7 | $CH_3$ | Cl | H |
| 2-$CH_3$-Phenyl | H | H | 7/9 | Cl | Cl | H |
| 2-$CH_3$-Phenyl | H | $CH_3$ | 7 | Cl | Cl | H |
| 2-$CH_3$-Phenyl | H | H | 7/9 | $OCH_3$ | $OCH_3$ | H |
| 2-$CH_3$-Phenyl | H | $CH_3$ | 7 | $OCH_3$ | $OCH_3$ | H |
| 2-$CH_3$-Phenyl | H | H | 7/9 | $CH_3$ | $OCH_3$ | H |
| 2-$CH_3$-Phenyl | H | $CH_3$ | 7 | $CH_3$ | $OCH_3$ | H |
| 2-$CH_3$-Phenyl | H | H | 7/9 | $OCH_3$ | $CH_3$ | H |
| 2-$CH_3$-Phenyl | H | $CH_3$ | 7 | $OCH_3$ | $CH_3$ | H |
| 2-$CH_3$-Phenyl | H | H | 7/9 | Cl | $OCH_3$ | H |
| 2-$CH_3$-Phenyl | H | $CH_3$ | 7 | Cl | $OCH_3$ | H |
| 2-$CH_3$-Phenyl | H | H | 7/9 | $OCH_3$ | Cl | H |
| 2-$CH_3$-Phenyl | H | $CH_3$ | 7 | $OCH_3$ | Cl | H |
| 2-$CO_2CH_3$-Phenyl | H | H | 7/9 | $CH_3$ | $CH_3$ | H |

Tabelle IV (Fortsetzung)

| A | $R^1$ | $R^2$ | Pos. | $R^3$ | $R^4$ | $R^5$ |
|---|---|---|---|---|---|---|
| 2-$CO_2CH_3$-Phenyl | H | $CH_3$ | 7 | $CH_3$ | $CH_3$ | H |
| 2-$CO_2CH_3$-Phenyl | H | H | 7/9 | Cl | $CH_3$ | H |
| 2-$CO_2CH_3$-Phenyl | H | $CH_3$ | 7 | Cl | $CH_3$ | H |
| 2-$CO_2CH_3$-Phenyl | H | H | 7/9 | $CH_3$ | Cl | H |
| 2-$CO_2CH_3$-Phenyl | H | $CH_3$ | 7 | $CH_3$ | Cl | H |
| 2-$CO_2CH_3$-Phenyl | H | H | 7/9 | Cl | Cl | H |
| 2-$CO_2CH_3$-Phenyl | H | $CH_3$ | 7 | Cl | Cl | H |
| 2-$CO_2CH_3$-Phenyl | H | H | 7/9 | $OCH_3$ | $OCH_3$ | H |
| 2-$CO_2CH_3$-Phenyl | H | $CH_3$ | 7 | $OCH_3$ | $OCH_3$ | H |
| 2-$CO_2CH_3$-Phenyl | H | H | 7/9 | $CH_3$ | $OCH_3$ | H |
| 2-$CO_2CH_3$-Phenyl | H | $CH_3$ | 7 | $CH_3$ | $OCH_3$ | H |
| 2-$CO_2CH_3$-Phenyl | H | H | 7/9 | $OCH_3$ | $CH_3$ | H |
| 2-$CO_2CH_3$-Phenyl | H | $CH_3$ | 7 | $OCH_3$ | $CH_3$ | H |
| 2-$CO_2CH_3$-Phenyl | H | H | 7/9 | Cl | $OCH_3$ | H |
| 2-$CO_2CH_3$-Phenyl | H | $CH_3$ | 7 | Cl | $OCH_3$ | H |
| 2-$CO_2CH_3$-Phenyl | H | H | 7/9 | $OCH_3$ | Cl | H |
| 2-$CO_2CH_3$-Phenyl | H | $CH_3$ | 7 | $OCH_3$ | Cl | H |
| 2-$CO_2C_2H_5$-Phenyl | H | H | 7/9 | $CH_3$ | $CH_3$ | H |
| 2-$CO_2C_2H_5$-Phenyl | H | $CH_3$ | 7 | $CH_3$ | $CH_3$ | H |
| 2-$CO_2C_2H_5$-Phenyl | H | H | 7/9 | Cl | $CH_3$ | H |
| 2-$CO_2C_2H_5$-Phenyl | H | $CH_3$ | 7 | Cl | $CH_3$ | H |
| 2-$CO_2C_2H_5$-Phenyl | H | H | 7/9 | $CH_3$ | Cl | H |
| 2-$CO_2C_2H_5$-Phenyl | H | $CH_3$ | 7 | $CH_3$ | Cl | H |
| 2-$CO_2C_2H_5$-Phenyl | H | H | 7/9 | Cl | Cl | H |
| 2-$CO_2C_2H_5$-Phenyl | H | $CH_3$ | 7 | Cl | Cl | H |
| 2-$CO_2C_2H_5$-Phenyl | H | H | 7/9 | $OCH_3$ | $OCH_3$ | H |
| 2-$CO_2C_2H_5$-Phenyl | H | $CH_3$ | 7 | $OCH_3$ | $OCH_3$ | H |
| 2-$CO_2C_2H_5$-Phenyl | H | H | 7/9 | $CH_3$ | $OCH_3$ | H |
| 2-$CO_2C_2H_5$-Phenyl | H | $CH_3$ | 7 | $CH_3$ | $OCH_3$ | H |
| 2-$CO_2C_2H_5$-Phenyl | H | H | 7/9 | $OCH_3$ | $CH_3$ | H |
| 2-$CO_2C_2H_5$-Phenyl | H | $CH_3$ | 7 | $OCH_3$ | $CH_3$ | H |
| 2-$CO_2C_2H_5$-Phenyl | H | H | 7/9 | Cl | $OCH_3$ | H |
| 2-$CO_2C_2H_5$-Phenyl | H | $CH_3$ | 7 | Cl | $OCH_3$ | H |
| 2-$CO_2C_2H_5$-Phenyl | H | H | 7/9 | $OCH_3$ | Cl | H |
| 2-$CO_2C_2H_5$-Phenyl | H | $CH_3$ | 7 | $OCH_3$ | Cl | H |
| 2-$OCH_3$-Phenyl | H | H | 7/9 | $CH_3$ | $CH_3$ | H |
| 2-$OCH_3$-Phenyl | H | $CH_3$ | 7 | $CH_3$ | $CH_3$ | H |
| 2-$OCH_3$-Phenyl | H | H | 7/9 | Cl | $CH_3$ | H |
| 2-$OCH_3$-Phenyl | H | $CH_3$ | 7 | Cl | $CH_3$ | H |
| 2-$OCH_3$-Phenyl | H | H | 7/9 | $CH_3$ | Cl | H |

Tabelle IV (Fortsetzung)

| A | $R^1$ | $R^2$ | Pos. | $R^3$ | $R^4$ | $R^5$ |
|---|---|---|---|---|---|---|
| 2-$OCH_3$-Phenyl | H | $CH_3$ | 7 | $CH_3$ | Cl | H |
| 2-$OCH_3$-Phenyl | H | H | 7/9 | Cl | Cl | H |
| 2-$OCH_3$-Phenyl | H | $CH_3$ | 7 | Cl | Cl | H |
| 2-$OCH_3$-Phenyl | H | H | 7/9 | $OCH_3$ | $OCH_3$ | H |
| 2-$OCH_3$-Phenyl | H | $CH_3$ | 7 | $OCH_3$ | $OCH_3$ | H |
| 2-$OCH_3$-Phenyl | H | H | 7/9 | $CH_3$ | $OCH_3$ | H |
| 2-$OCH_3$-Phenyl | H | $CH_3$ | 7 | $CH_3$ | $OCH_3$ | H |
| 2-$OCH_3$-Phenyl | H | H | 7/9 | $OCH_3$ | $CH_3$ | H |
| 2-$OCH_3$-Phenyl | H | $CH_3$ | 7 | $OCH_3$ | $CH_3$ | H |
| 2-$OCH_3$-Phenyl | H | H | 7/9 | Cl | $OCH_3$ | H |
| 2-$OCH_3$-Phenyl | H | $CH_3$ | 7 | Cl | $OCH_3$ | H |
| 2-$OCH_3$-Phenyl | H | H | 7/9 | $OCH_3$ | Cl | H |
| 2-$OCH_3$-Phenyl | H | $CH_3$ | 7 | $OCH_3$ | Cl | H |
| 2-$OCH_2CH_2OCH_3$-Phenyl | H | H | 7/9 | $CH_3$ | $CH_3$ | H |
| 2-$OCH_2CH_2OCH_3$-Phenyl | H | $CH_3$ | 7 | $CH_3$ | $CH_3$ | H |
| 2-$OCH_2CH_2OCH_3$-Phenyl | H | H | 7/9 | Cl | $CH_3$ | H |
| 2-$OCH_2CH_2OCH_3$-Phenyl | H | $CH_3$ | 7 | Cl | $CH_3$ | H |
| 2-$OCH_2CH_2OCH_3$-Phenyl | H | H | 7/9 | $CH_3$ | Cl | H |
| 2-$OCH_2CH_2OCH_3$-Phenyl | H | $CH_3$ | 7 | $CH_3$ | Cl | H |
| 2-$OCH_2CH_2OCH_3$-Phenyl | H | H | 7/9 | Cl | Cl | H |
| 2-$OCH_2CH_2OCH_3$-Phenyl | H | $CH_3$ | 7 | Cl | Cl | H |
| 2-$OCH_2CH_2OCH_3$-Phenyl | H | H | 7/9 | $OCH_3$ | $OCH_3$ | H |
| 2-$OCH_2CH_2OCH_3$-Phenyl | H | $CH_3$ | 7 | $OCH_3$ | $OCH_3$ | H |
| 2-$OCH_2CH_2OCH_3$-Phenyl | H | H | 7/9 | $CH_3$ | $OCH_3$ | H |
| 2-$OCH_2CH_2OCH_3$-Phenyl | H | $CH_3$ | 7 | $CH_3$ | $OCH_3$ | H |
| 2-$OCH_2CH_2OCH_3$-Phenyl | H | H | 7/9 | $OCH_3$ | $CH_3$ | H |
| 2-$OCH_2CH_2OCH_3$-Phenyl | H | $CH_3$ | 7 | $OCH_3$ | $CH_3$ | H |
| 2-$OCH_2CH_2OCH_3$-Phenyl | H | H | 7/9 | Cl | $OCH_3$ | H |
| 2-$OCH_2CH_2OCH_3$-Phenyl | H | $CH_3$ | 7 | Cl | $OCH_3$ | H |
| 2-$OCH_2CH_2OCH_3$-Phenyl | H | H | 7/9 | $OCH_3$ | Cl | H |
| 2-$OCH_2CH_2OCH_3$-Phenyl | H | $CH_3$ | 7 | $OCH_3$ | Cl | H |
| 2-$OCH_2CH_2Cl$-Phenyl | H | H | 7/9 | $CH_3$ | $CH_3$ | H |
| 2-$OCH_2CH_2Cl$-Phenyl | H | $CH_3$ | 7 | $CH_3$ | $CH_3$ | H |
| 2-$OCH_2CH_2Cl$-Phenyl | H | H | 7/9 | Cl | $CH_3$ | H |
| 2-$OCH_2CH_2Cl$-Phenyl | H | $CH_3$ | 7 | Cl | $CH_3$ | H |
| 2-$OCH_2CH_2Cl$-Phenyl | H | H | 7/9 | $CH_3$ | Cl | H |
| 2-$OCH_2CH_2Cl$-Phenyl | H | $CH_3$ | 7 | $CH_3$ | Cl | H |
| 2-$OCH_2CH_2Cl$-Phenyl | H | H | 7/9 | Cl | Cl | H |
| 2-$OCH_2CH_2Cl$-Phenyl | H | $CH_3$ | 7 | Cl | Cl | H |
| 2-$OCH_2CH_2Cl$-Phenyl | H | H | 7/9 | $OCH_3$ | $OCH_3$ | H |

Tabelle IV (Fortsetzung)

| A | $R^1$ | $R^2$ | Pos. | $R^3$ | $R^4$ | $R^5$ |
|---|---|---|---|---|---|---|
| 2-$OCH_2CH_2Cl$-Phenyl | H | $CH_3$ | 7 | $OCH_3$ | $OCH_3$ | H |
| 2-$OCH_2CH_2Cl$-Phenyl | H | H | 7/9 | $CH_3$ | $OCH_3$ | H |
| 2-$OCH_2CH_2Cl$-Phenyl | H | $CH_3$ | 7 | $CH_3$ | $OCH_3$ | H |
| 2-$OCH_2CH_2Cl$-Phenyl | H | H | 7/9 | $OCH_3$ | $CH_3$ | H |
| 2-$OCH_2CH_2Cl$-Phenyl | H | $CH_3$ | 7 | $OCH_3$ | $CH_3$ | H |
| 2-$OCH_2CH_2Cl$-Phenyl | H | H | 7/9 | Cl | $OCH_3$ | H |
| 2-$OCH_2CH_2Cl$-Phenyl | H | $CH_3$ | 7 | Cl | $OCH_3$ | H |
| 2-$OCH_2CH_2Cl$-Phenyl | H | H | 7/9 | $OCH_3$ | Cl | H |
| 2-$OCH_2CH_2Cl$-Phenyl | H | $CH_3$ | 7 | $OCH_3$ | Cl | H |
| 2-$OCH_2CO_2CH_3$-Phenyl | H | H | 7/9 | $CH_3$ | $CH_3$ | H |
| 2-$OCH_2CO_2CH_3$-Phenyl | H | $CH_3$ | 7 | $CH_3$ | $CH_3$ | H |
| 2-$OCH_2CO_2CH_3$-Phenyl | H | H | 7/9 | Cl | $CH_3$ | H |
| 2-$OCH_2CO_2CH_3$-Phenyl | H | $CH_3$ | 7 | Cl | $CH_3$ | H |
| 2-$OCH_2CO_2CH_3$-Phenyl | H | H | 7/9 | $CH_3$ | Cl | H |
| 2-$OCH_2CO_2CH_3$-Phenyl | H | $CH_3$ | 7 | $CH_3$ | Cl | H |
| 2-$OCH_2CO_2CH_3$-Phenyl | H | H | 7/9 | Cl | Cl | H |
| 2-$OCH_2CO_2CH_3$-Phenyl | H | $CH_3$ | 7 | Cl | Cl | H |
| 2-$OCH_2CO_2CH_3$-Phenyl | H | H | 7/9 | $OCH_3$ | $OCH_3$ | H |
| 2-$OCH_2CO_2CH_3$-Phenyl | H | $CH_3$ | 7 | $OCH_3$ | $OCH_3$ | H |
| 2-$OCH_2CO_2CH_3$-Phenyl | H | H | 7/9 | $CH_3$ | $OCH_3$ | H |
| 2-$OCH_2CO_2CH_3$-Phenyl | H | $CH_3$ | 7 | $CH_3$ | $OCH_3$ | H |
| 2-$OCH_2CO_2CH_3$-Phenyl | H | H | 7/9 | $OCH_3$ | $CH_3$ | H |
| 2-$OCH_2CO_2CH_3$-Phenyl | H | $CH_3$ | 7 | $OCH_3$ | $CH_3$ | H |
| 2-$OCH_2CO_2CH_3$-Phenyl | H | H | 7/9 | Cl | $OCH_3$ | H |
| 2-$OCH_2CO_2CH_3$-Phenyl | H | $CH_3$ | 7 | Cl | $OCH_3$ | H |
| 2-$OCH_2CO_2CH_3$-Phenyl | H | H | 7/9 | $OCH_3$ | Cl | H |
| 2-$OCH_2CO_2CH_3$-Phenyl | H | $CH_3$ | 7 | $OCH_3$ | Cl | H |
| 2,6-Cl,Cl-Phenyl | H | H | 7/9 | $CH_3$ | $CH_3$ | H |
| 2,6-Cl,Cl-Phenyl | H | $CH_3$ | 7 | $CH_3$ | $CH_3$ | H |
| 2,6-Cl,Cl-Phenyl | H | H | 7/9 | Cl | $CH_3$ | H |
| 2,6-Cl,Cl-Phenyl | H | $CH_3$ | 7 | Cl | $CH_3$ | H |
| 2,6-Cl,Cl-Phenyl | H | H | 7/9 | $CH_3$ | Cl | H |
| 2,6-Cl,Cl-Phenyl | H | $CH_3$ | 7 | $CH_3$ | Cl | H |
| 2,6-Cl,Cl-Phenyl | H | H | 7/9 | Cl | Cl | H |
| 2,6-Cl,Cl-Phenyl | H | $CH_3$ | 7 | Cl | Cl | H |
| 2,6-Cl,Cl-Phenyl | H | H | 7/9 | $OCH_3$ | $OCH_3$ | H |
| 2,6-Cl,Cl-Phenyl | H | $CH_3$ | 7 | $OCH_3$ | $OCH_3$ | H |
| 2,6-Cl,Cl-Phenyl | H | H | 7/9 | $CH_3$ | $OCH_3$ | H |
| 2,6-Cl,Cl-Phenyl | H | $CH_3$ | 7 | $CH_3$ | $OCH_3$ | H |
| 2,6-Cl,Cl-Phenyl | H | H | 7/9 | $OCH_3$ | $CH_3$ | H |

Tabelle IV (Fortsetzung)

| A | $R^1$ | $R^2$ | Pos. | $R^3$ | $R^4$ | $R^5$ |
|---|---|---|---|---|---|---|
| 2,6-Cl,Cl-Phenyl | H | $CH_3$ | 7 | $OCH_3$ | $CH_3$ | H |
| 2,6-Cl,Cl-Phenyl | H | H | 7/9 | Cl | $OCH_3$ | H |
| 2,6-Cl,Cl-Phenyl | H | $CH_3$ | 7 | Cl | $OCH_3$ | H |
| 2,6-Cl,Cl-Phenyl | H | H | 7/9 | $OCH_3$ | Cl | H |
| 2,6-Cl,Cl-Phenyl | H | $CH_3$ | 7 | $OCH_3$ | Cl | H |
| 2-Cl,6-$CH_3$-Phenyl | H | H | 7/9 | $CH_3$ | $CH_3$ | H |
| 2-Cl,6-$CH_3$-Phenyl | H | $CH_3$ | 7 | $CH_3$ | $CH_3$ | H |
| 2-Cl,6-$CH_3$-Phenyl | H | H | 7/9 | Cl | $CH_3$ | H |
| 2-Cl,6-$CH_3$-Phenyl | H | $CH_3$ | 7 | Cl | $CH_3$ | H |
| 2-Cl,6-$CH_3$-Phenyl | H | H | 7/9 | $CH_3$ | Cl | H |
| 2-Cl,6-$CH_3$-Phenyl | H | $CH_3$ | 7 | $CH_3$ | Cl | H |
| 2-Cl,6-$CH_3$-Phenyl | H | H | 7/9 | Cl | Cl | H |
| 2-Cl,6-$CH_3$-Phenyl | H | $CH_3$ | 7 | Cl | Cl | H |
| 2-Cl,6-$CH_3$-Phenyl | H | H | 7/9 | $OCH_3$ | $OCH_3$ | H |
| 2-Cl,6-$CH_3$-Phenyl | H | $CH_3$ | 7 | $OCH_3$ | $OCH_3$ | H |
| 2-Cl,6-$CH_3$-Phenyl | H | H | 7/9 | $CH_3$ | $OCH_3$ | H |
| 2-Cl,6-$CH_3$-Phenyl | H | $CH_3$ | 7 | $CH_3$ | $OCH_3$ | H |
| 2-Cl,6-$CH_3$-Phenyl | H | H | 7/9 | $OCH_3$ | $CH_3$ | H |
| 2-Cl,6-$CH_3$-Phenyl | H | $CH_3$ | 7 | $OCH_3$ | $CH_3$ | H |
| 2-Cl,6-$CH_3$-Phenyl | H | H | 7/9 | Cl | $OCH_3$ | H |
| 2-Cl,6-$CH_3$-Phenyl | H | $CH_3$ | 7 | Cl | $OCH_3$ | H |
| 2-Cl,6-$CH_3$-Phenyl | H | H | 7/9 | $OCH_3$ | Cl | H |
| 2-Cl,6-$CH_3$-Phenyl | H | $CH_3$ | 7 | $OCH_3$ | Cl | H |
| 2,5-Cl,Cl-Phenyl | H | H | 7/9 | $CH_3$ | $CH_3$ | H |
| 2,5-Cl,Cl-Phenyl | H | $CH_3$ | 7 | $CH_3$ | $CH_3$ | H |
| 2,5-Cl,Cl-Phenyl | H | H | 7/9 | Cl | $CH_3$ | H |
| 2,5-Cl,Cl-Phenyl | H | $CH_3$ | 7 | Cl | $CH_3$ | H |
| 2,5-Cl,Cl-Phenyl | H | H | 7/9 | $CH_3$ | Cl | H |
| 2,5-Cl,Cl-Phenyl | H | $CH_3$ | 7 | $CH_3$ | Cl | H |
| 2,5-Cl,Cl-Phenyl | H | H | 7/9 | Cl | Cl | H |
| 2,5-Cl,Cl-Phenyl | H | $CH_3$ | 7 | Cl | Cl | H |
| 2,5-Cl,Cl-Phenyl | H | H | 7/9 | $OCH_3$ | $OCH_3$ | H |
| 2,5-Cl,Cl-Phenyl | H | $CH_3$ | 7 | $OCH_3$ | $OCH_3$ | H |
| 2,5-Cl,Cl-Phenyl | H | H | 7/9 | $CH_3$ | $OCH_3$ | H |
| 2,5-Cl,Cl-Phenyl | H | $CH_3$ | 7 | $CH_3$ | $OCH_3$ | H |
| 2,5-Cl,Cl-Phenyl | H | H | 7/9 | $OCH_3$ | $CH_3$ | H |
| 2,5-Cl,Cl-Phenyl | H | $CH_3$ | 7 | $OCH_3$ | $CH_3$ | H |
| 2,5-Cl,Cl-Phenyl | H | H | 7/9 | Cl | $OCH_3$ | H |
| 2,5-Cl,Cl-Phenyl | H | $CH_3$ | 7 | Cl | $OCH_3$ | H |
| 2,5-Cl,Cl-Phenyl | H | H | 7/9 | $OCH_3$ | Cl | H |
| 2,5-Cl,Cl-Phenyl | H | $CH_3$ | 7 | $OCH_3$ | Cl | H |

Die substituierten Sulfonamide I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall

möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromotischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Die Wirkstoffe werden beispielsweise wie folgt formuliert:

I. Man vermischt 90 Gewichtsteile der Verbindung Nr. 1.005 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.006 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III 20 Gewichtsteile der Verbindung Nr. 1.007 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.008 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.009 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.010 werden mit 97 Gewichtsteilen feinteiligem Kaolin vermischt. Man erhält auf diese Weise ein Stäubemittel, das 3 Gew.% des Wirkstoffs enthält.

VII 30 Gewichtsteile des Wirkstoffs Nr. 1.011 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.012 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0 kg/ha, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden.

Einige der Sulfonamide der allgemeinen Formel I können daneben praktisch alle Entwicklungsstadien einer Pflanze verschiedenartig beeinflussen und werden deshalb auch als Wachstumsregulatoren eingesetzt. Die Wirkungsvielfalt der Verbindungen hängt von verschiedenen Faktoren ab. Maßgeblich sind dabei vor allem

a) Pflanzenart und -sorte,

b) Zeitpunkt der Applikation, bezüglich Entwicklungsstadium der Pflanze und Jahreszeit,

c) Applikationsort und -verfahren (Samenbeize, Bodenbehandlung oder Blattapplikation)

d) klimatische Faktoren, z.B. Temperatur, Niederschlagsmenge, außerdem auch Tageslänge und Lichtintensität

e) Bodenbeschaffenheit (einschließlich Düngung),

f) Formulierung bzw. Anwendungsform des Wirkstoffs und

g) Aufwandmenge und Wirkstoffgehalt der Mittel.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, sind nachstehend einige Beispiele aufgeführt.

A. Mit den wachstumsregulierenden Sulfonamiden I läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Bei Obstgehölzen und anderen Bäumen oder Sträuchern können dadurch kostenintensive Schnittmaßnahmen reduziert werden.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmver-Stärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der Verbindungen I kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Verbindungen I läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen

Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den wachstumsregulierenden Mitteln auf der Basis von Sulfonamiden lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Sulfonamide der Formel I können Ertragssteigerungen durch Eingriffe in den pflanzlichen Stoffwechsel bzw. durch Förderung oder Hemmung des vegetativen und/oder des generativen Wachstums verursachen.

C. Mit den Sulfonamiden lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen z.B. Baumwolle wesentlich.

D. Mit den Sulfonamiden I kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die wachstumsregulierenden Verbindungen I kann die Aufwandmenge stark variiert werden. Bei der Saatgutbehandlung werden im allgemeinen Wirkstoffmengen von 0,001 bis 50 g je Kilogramm Saatgut, vorzugsweise 0,01 bis 1 g, benötigt. Für die Blatt- und Bodenbehandlung sind im allgemeinen Gaben von 0,01 bis 10 kg/ha, bevorzugt 0,1 bis 5 kg/ha ausreichend.

Zur Verbreiterung des Wirkungsspektrums und zur Erzielung synergistischer Effekte können die Sulfonamide der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Imidazolinone, Sulfonylharnstoffe, Cyclohexan-1,3-dionderivate, Chinolincarbonsäurederivate, Phenyloxy- bzw. Heteroaryloxyphenylpropionsäuren sowie deren Salze, Ester und Amide und andere in Betracht.

Außerdem kann es von Nutzen sein, die neuen Verbindungen der Formel I allein oder in Kombination mit anderen Herbiziden und Wachstumsregulatoren auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die in den nachstehenden Synthesebeispielen wiedergegebenen Arbeitsvorschriften wurden unter entsprechender Abwandlung der Ausgangsverbindungen zur Herstellung weiterer Verbindungen I benutzt. Die so erhaltenen Verbindungen sind in den anschließenden Tabellen 1 - 3 mit physikalischen Angaben aufgeführt.

Beispiel 1

Synthese von $N^1$-(2,6-Dichlor-7-methyl-8-purinyl)-benzolsulfonamid (Verfahren A)

10,8 g (50,0 mMol) Benzolsulfonamid-Natriumsalz wurden in 100 ml DMF suspendiert und bei 60 °C 11,9 g (50,0 mMol) 7-Methyl-2,6,8-trichlorpurin zugegeben. Nach 8 h bei 80 °C und Abkühlung auf Raumtemp. wurde mit $H_2O$/Eis versetzt, mit 10 %iger HCl angesäuert, der entstandene Niederschlag abgesaugt und getrocknet.
11,7 g farblose Kristalle (65 %) (Wirkstoffbeispiel 1.001)
$^1$H-NMR (250 MHz, $d^6$DMSO, $\delta$ in ppm); 3,60 (s,3H, N-$CH_3$); 7,60 (mc, 3H, Aryl-H) ; 7,95 (mc, 2H, Aryl-H);

Beispiel 2

Synthese von $N^1$-(2-Chlor-6-methoxy-7-methyl-8-purinyl)-benzolsulfonamid (Verfahren F)

3,00 g (8,00 mMol) des in Beispiel 1 dargestellten Sulfonamids und 2,90 g (16,0 mMol) 30 %ige Natriummethylatlösung wurden in 50 ml Methanol 12 h bei Raumtemperatur gerührt und anschließend am Rotavapor eingeengt. Durch Einrühren des Rückstandes in $H_2O$ und Ansäuern mit verdünnter HCl konnten 2,30 g (81 %) des gewünschten Produkts in Form farbloser Kristalle isoliert werden (Wirkstoffbeispiel 1.002).
$^1$H-NMR (250 MHz, $d^6$-DMSO, $\delta$ in ppm): 3,50 (s,3H, N-$CH_3$); 4,05 (s, 3H, $OCH_3$); 7,55 (mc, 3H, Aryl-H); 7,95 (mc, 2H, Aryl-H);

Beispiel 3

Synthese von $N^1$-(2,6-Dimethoxy-7-methyl-8-purinyl)-benzolsulfonamid (Verfahren G)

3,60 g (10,0 mMol) des in Beispiel 1 synthetisierten Sulfonamids und 5,40 g (30,0 mMol) 30 %ige Natriummethylatlösung wurden in 200 ml Methanol in einem Autoklaven 12 h bei 120 °C erhitzt und die Reaktionsmischung nach Abkühlung auf Raumtemperatur am Rotavapor eingeengt. Nach Verrühren des Rückstandes mit $H_2O$ und Ansäuern mit 10 %iger HCl erhielt man 3,20 g farblose Kristalle in einer Ausbeute von 92 % (Wirkstoffbeispiel 1.003).
$^1$H-NMR (250 MHz, $d^6$-DMSO, $\delta$ in ppm): 3,45 (s, 3H, N-$CH_3$); 3,90 (s,3H,$OCH_3$); 4,05 (s,3H,$OCH_3$) ; 7,50 (mc,3H,Aryl-H); 7,95 (mc, 2H, Aryl-H);

Beispiel 4

Synthese von $N^1$-(2,6-Dichlor-7-methyl-8-purinyl)-2-carbmethoxybenzolsulfonamid (Verfahren B)

Die Suspension von 3,00 g (14,0 mMol) 8-Amino-2,6-dichlor-7-methylpurin in 150 ml Pyridin wurde bei 70 °C mit 6,60 g (28,0 mMol) 2-Carbmethoxy-benzolsulfonamid versetzt. Nach 3 h bei 70-80 °C wurde die Reaktionsmischung am Rotavapor eingeengt, der Rückstand in Eis/$H_2O$ eingerührt und mit verdünnter HCl angesäuert. Man erhielt des gewünschte Produkt in Form eines braunen Pulvers mit den folgenden physikalischen Daten (Wirkstoffbeispiel 1.020)
$^1$H-NMR (250 MHz, $d^6$-DMSO, $\delta$ in ppm) 3,60 (s,3H,N-$CH_3$); 3,80 (s,3H,$CO_2CH_3$); 7,60 (mc,1H,Aryl-H); 7,70 (mc,2H,Aryl-H); 8,10 (mc, 1H,Aryl-H).

Beispiel 5

Synthese von N1-(2,6-Dimethoxy-8-purinyl)-benzolsulfonamid (Verfahren C)

Zu 2,20 (13,0 mMol) 4,5-Diamino-2,6-dimethoxy-pyrimidin und 2,60 g (26,0 mMol) Triethylamin in 50 ml Dioxan tropfte man unter Rühren bei Raumtemperatur langsam 3,00 g (13,0 mMol) Benzolsulfonylisocyaniddichlorid zu. Nach 5 h bei 100 °C wurde auf Raumtemperatur abgekühlt vom entstandenen Feststoff abfiltriert und das Filtrat am Rotavapor eingeengt. Aus dem Rückstand konnte durch Kristallisation mit Methylenchlorid/Methyl-t-Butylether das gewünschte Produkt isoliert werden. (Wirkstoffbeispiel 1.004)
$^1$H-NMR (250 MHz, $d^6$-DMSO, $\delta$ in ppm): 3,90 (s,3H, $OCH_3$); 4,00 (s,3H,$OCH_3$); 7,55 (mc,3H,Aryl-H); 7,95 (mc,2H,Aryl-H);

Tabelle 1

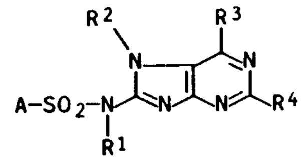

Ia

| Nr. | A | $R^1$ | $R^2$ | $R^3$ | $R^4$ | $^1$HNMR [$d^6$ DMSO, δ (ppm)]; Fp [°C] |
|---|---|---|---|---|---|---|
| 1.001 | Phenyl | H | $CH_3$ | Cl | Cl | 3,60(s,3H);7,60(mc,3H);7.95(mc,2H); |
| 1.002 | Phenyl | H | $CH_3$ | $OCH_3$ | Cl | 3,50(s,3H);4,05(s,3H);7,55(mc,3H);7,95(mc,2H) |
| 1.003 | Phenyl | H | $CH_3$ | $OCH_3$ | $OCH_3$ | 3,45(s,3H);3,90(s,3H);4.05(s,3H);7,50(mc,3H); 7,95(mc,2H); |
| 1.004 | Phenyl | H | H | $OCH_3$ | $OCH_3$ | 3,90(s,3H);4,00(s,3H);7,55(mc,3H):7,95(mc,2H); |
| 1.005 | 2-Cl-Phenyl | H | $CH_3$ | Cl | Cl | 3,60(s,3H);7,50-7,60(m,3H);8,15(mc,1H); |
| 1.006 | 2-Cl-Phenyl | H | $CH_3$ | $OCH_3$ | Cl | 3,55(s,3H);4,05(s,3H);7,45-7,60(m,3H); 8,15(mc,1H); |
| 1.007 | 2-Cl-Phenyl | H | $CH_3$ | $OCH_3$ | $OCH_3$ | 3,50(s,3H);3,90(s,3H);4,05(s,3H); 7,45-7,60(m,3H);8,15(mc,1H); |
| 1.008 | 2,6-Cl,Cl-Phenyl | H | $CH_3$ | Cl | Cl | 3,60(s,3H);7.45-7,60(m,3H); |
| 1.009 | 2,6-Cl,Cl-Phenyl | H | $CH_3$ | $OCH_3$ | Cl | 3,55(s,3H);4,10(s,3H);7,50-7,65(m,3H); |
| 1.010 | 2,6-Cl,Cl-Phenyl | H | $CH_3$ | $OCH_3$ | $OCH_3$ | 3,50(s,3H);3,80(s,3H);3,95(s,3H);7,40- 7,50(m,3H); |
| 1.011 | 2,6-Cl,Cl-Phenyl | H | $CH_3$ | $N(CH_3)_2$ | Cl | 3,05(s,6H);3,55(s,3H);7,40-7,55(m,3H); |
| 1.012 | 2,5-Cl,Cl-Phenyl | H | $CH_3$ | Cl | Cl | 3,60(s,3H);7,60(mc,2H);8,20(mc,1H); |
| 1.013 | 2,5-Cl,Cl-Phenyl | H | $CH_3$ | $OCH_3$ | Cl | 3,55(s,3H);4,05(s,3H);7,65(mc,2H);8,20(mc,1H); |
| 1.014 | 2,5-Cl,Cl-Phenyl | H | $CH_3$ | $OCH_3$ | $OCH_3$ | 3,55(s,3H);3,90(s,3H);4,05(s,3H);7,65(mc,2H); 8,20(mc,1H); |
| 1.015 | 3-F-Phenyl | H | $CH_3$ | Cl | Cl | 3,65(s,3H);7,40(mc,1H);7,60(mc,1H);7,80(mc,2H); |
| 1.016 | 3-F-Phenyl | H | $CH_3$ | $OCH_3$ | Cl | 3,50(s,3H);4,10(s,3H);7,40(mc,1H);7,60(mc,1H); 7,80(mc,2H); |

Tabelle 1 (Fortsetzung)

| Nr. | A | $R^1$ | $R^2$ | $R^3$ | $R^4$ | $^1HNMR$ [$d^6$ DMSO, $\delta$ (ppm)]; Fp [°C] |
|---|---|---|---|---|---|---|
| 1.017 | 3-F-Phenyl | H | $CH_3$ | $OCH_3$ | $OCH_3$ | 3,50(s,3H);3,90(s,3H);4,00(s,3H);7,40(mc,1H); 7,60(mc,1H);7,80(mc,2H); |
| 1.018 | 4-F-Phenyl | H | $CH_3$ | Cl | Cl | 3,60(s,3H);7,40(mc,2H);8,05(mc,2H); |
| 1.019 | 4-F-Phenyl | H | $CH_3$ | $OCH_3$ | Cl | 3,50(s,3H);4,05(s,3H);7,30(mc,2H);8,00(mc,2H); |
| 1.020 | 2-$CO_2CH_3$-Phenyl | H | $CH_3$ | Cl | Cl | 3,60(s,3H); 3,80(s,3H);7,60(mc,1H);7,70(mc,2H); 8,10(mc,1H); |
| 1.021 | 2-Cl,6-$CH_3$-Phenyl | H | $CH_3$ | Cl | Cl | 2,70(s,3H);3,55(s,3H);7,30-7,50(m,3H); |
| 1.022 | 2-Cl,6-$CH_3$-Phenyl | H | $CH_3$ | $OCH_3$ | Cl | 2,70(s,3H);3,50(s,3H);4,00(s,3H);7,30-7,50(m,3H); |
| 1.023 | 2-Cl,6-$CH_3$-Phenyl | H | $CH_3$ | $OCH_3$ | $OCH_3$ | 2,70(s,3H);3,50(s,3H);3,90(s,3H);4,00(s,3H); 7,20-7,40(m,3H); |
| 1.024 | 2-$OCH_3$-Phenyl | H | $CH_3$ | Cl | Cl | 3,55(s,3H);3,70(s,3H);7,05(t,1H);7,15(d,1H);7,55 (t,1H);7,85(d,1H); |
| 1.025 | 2-$OCH_3$-Phenyl | H | $CH_3$ | $OCH_3$ | Cl | 3,45(s,3H);3,65(s,3H);4,05(s,3H);7,05(t,1H); 7,15(d,1H);7,55(t,1H);7,85(d,1H); |
| 1.026 | 2-$OCH_3$-Phenyl | H | $CH_3$ | $OCH_3$ | $OCH_3$ | 3,45(s,3H);3,60(s,3H);3,90(s,3H);4,05(s,3H); 7,00(t,1H);7,10(d,1H);7,50(t,1H);7,85(d,1H); |
| 1.027 | 2-$OCH_3$-Phenyl | H | $CH_3$ | $N(CH_3)_2$ | Cl | 3,00(s,6H);3,45(s,3H);3,60(s,3H);7,05(t,1H); 7,15(d,1H);7,55(t,1H);7,85(d,1H); |
| 1.028 | 4-$OCH_3$-Phenyl | H | $CH_3$ | $OCH_3$ | $OCH_3$ | 3,50(s,3H);3,80(s,3H); 3,90(s,3H); 4,00(s,3H); 6,95(mc,2H); 7,90(mc,2H); |
| 1.029 | 2-Naphthyl | H | $CH_3$ | Cl | Cl | 3,60(s,3H);7,70(mc,2H);7,90-8,15(m,4H);8,65(s,1H); |
| 1.030 | 2-Naphthyl | H | $CH_3$ | $OCH_3$ | Cl | 3,50(s,3H);4,00(s,3H);7,55(mc,2H);7,90-8,15 (m,4H);8,65(s,1H); |

Tabelle 1 (Fortsetzung)

| Nr. | A | $R^1$ | $R^2$ | $R^3$ | $R^4$ | $^1$HNMR [$d^6$ DMSO, δ (ppm)]; Fp [°C] |
|---|---|---|---|---|---|---|
| 1.031 | 2-Naphthyl | H | $CH_3$ | $OCH_3$ | $OCH_3$ | 3,50(s,3H);3,85(s,3H);3,95(s,3H);7,60(mc,2H); 7,90-8,10(m,4H);8,60(s,1H); |
| 1.032 | 1-Naphthyl | H | $CH_3$ | Cl | Cl | 3,55(s,3H);7,60-7,75(m,3H);8,00(mc,1H);8,15(mc,1H); 8,40(mc,1H);8,80(mc,1H); |
| 1.033 | 1-Naphthyl | H | $CH_3$ | $OCH_3$ | Cl | 3,45(s,3H);3,95(s,3H);7,55-7,70(m,3H);7,95(mc, 1H);7,95(mc,1H);8,05(mc,1H);8,20(mc,1H);8,35(mc,1H); 8,85(mc,1H); |
| 1.034 | 1-Naphthyl | H | $CH_3$ | $OCH_3$ | $OCH_3$ | 3,50(s,3H); 3,80 (s,3H);3,90(s,3H);7,45-7,60 (m,3H);8,15(mc,1H); |
| 1.035 | 8-Chinolyl | H | $CH_3$ | Cl | Cl | 3,50(s,3H);7,65(mc,1H);7,80(mc,1H);8,30(mc,1H); 8,50(mc,1H);8,55(mc,1H);8,80(mc,1H); |
| 1.036 | 8-Chinolyl | H | $CH_3$ | $OCH_3$ | Cl | 3,40(s,3H);4,10(s,3H);7,55(mc,1H);7,70(mc,1H); 8,20(mc,1H);8,50(mc,2H);8,70(mc,1H); |
| 1.037 | 2-Thienyl | H | $CH_3$ | Cl | Cl | 3,65(s,3H);7,10(mc,1H);7,75(mc,1H);7,85(mc,1H); |
| 1.038 | 2-Thienyl | H | $CH_3$ | $OCH_3$ | Cl | 3,55(s,3H);4,05(s,3H);7,10(mc,1H);7,75(mc,2H); |
| 1.039 | 2-Thienyl | H | $CH_3$ | $OCH_3$ | $OCH_3$ | 3,50(s,3H);3,95(s,3H);4,05(S,3H);7,10(mc,1H); 7,80(mc,2H); |
| 1.040 | 3-Cl,2-Thienyl | H | $CH_3$ | Cl | Cl | 3,70(s,3H);7,10(d,1H);7,90(d,1H); |
| 1.041 | 3-Cl,2-Thienyl | H | $CH_3$ | $OCH_3$ | Cl | 3,50(s,3H),4,10(s,3H);7,10(d,1H);7,90(d,1H); |
| 1.042 | 3-Cl,2-Thienyl | H | $CH_3$ | $OCH_3$ | $OCH_3$ | 3,55(s,3H);3,90(s,3H);4,05(s,3H);7,10(d,1H), 7,85(d,1H); |
| 1.043 | 4-$CO_2CH_3$,3-Thienyl | H | $CH_3$ | Cl | Cl | 3,55(s,3H);3,70(s,3H);8,35(mc,2H); |
| 1.044 | 4-$CO_2CH_3$,3-Thienyl | H | $CH_3$ | $OCH_3$ | Cl | 3,50(s,3H);3,70(s,3H);4,10(s,3H);8,30(mc,2H); |
| 1.045 | 4-$CO_2CH_3$,3-Thienyl | H | $CH_3$ | $OCH_3$ | $OCH_3$ | 3.45(s,3H);3,70(s,3H);3,90(s,3H);4,05(s,3H) 8,30(mc,2H); |

Tabelle 1 (Fortsetzung)

| Nr. | A | $R^1$ | $R^2$ | $R^3$ | $R^4$ | $^1HNMR$ [$d^6$ DMSO, δ (ppm)]; Fp [°C] |
|---|---|---|---|---|---|---|
| 1.046 | 2-Chlorphenyl | H | H | $OCH_3$ | $CH_3$ | 2,48(s,3H);34,00s,3H);7,51(mc,3H);8,19(d,1H); 12,50(s;1H); 12,70(s;1H); |
| 1.047 | 2-Chlorphenyl | H | H | $OCH_3$ | $OCH_3$ | 3,90(s,3H);4,05(s,3H);7,51(mc,3H);8,15(d,1H); 12,30(s,1H);12,60(s,1H); |
| 1.048 | 2-Chlorphenyl | H | $CH_3$ | F | F | 193 |
| 1.049 | 2-Chlorphenyl | H | $CH_3$ | $OCH_3$ | F | 3,53(s,3H);7,37(mc,2H);7,63(mc,1H);7,97(mc,1H); 13,0(m,1H); |
| 1.050 | 2,6-Cl,Cl-phenyl | H | $CH_3$ | F | F | 3,53(s,3H);7,22-7,63(m,3H); |
| 1.051 | 2,6-Cl,Cl-phenyl | H | $CH_3$ | $OCH_3$ | F | 3,53(s,3H);7,51(mc,1H);7,60(mc,2H); |
| 1.052 | 2,6-Cl,Cl-phenyl | H | $CH_3$ | $OCH_2CF_3$ | Cl | 3,53(s,3H);5,19(q,2H);7,54(mc,3H); |
| 1.053 | 2,3-Cl,Cl-phenyl | H | $CH_3$ | Cl | Cl | 244 |
| 1.054 | 2,3-Cl,Cl-phenyl | H | $CH_3$ | $OCH_3$ | Cl | 215 |
| 1.055 | 2,4,5-Cl,Cl,Cl-phenyl | H | $CH_3$ | Cl | Cl | 255-227 |
| 1.056 | 2-F-phenyl | H | $CH_3$ | Cl | Cl | 115-119 |
| 1.057 | 2-F-phenyl | H | $CH_3$ | $OCH_3$ | Cl | 210-212 |
| 1.058 | 2-F-phenyl | H | $CH_3$ | F | F | 3,53(s,33H);7,53(mc,1H);7,64(mc,2H);8,19(d,1H); |
| 1.059 | 2,6-F,F-phenyl | H | $CH_3$ | Cl | Cl | 3,65(s,3H);7,63(mc,1H);8,05(mc,1H);8,91(mc,1H); |
| 1.060 | 2,6-F,F-phenyl | H | $CH_3$ | $OCH_3$ | Cl | *3,61(s,3H);4,15(s,3H);6,91(mc,2H);7,41(mc,2H); |
| 1.061 | 2,6-F,F-phenyl | H | $CH_3$ | F | F | 167-169 |
| 1.062 | 2,6-F,F-phenyl | H | $CH_3$ | $OCH_3$ | F | 3,56(s,3H);4,08(s,3H);7,23(mc,2H);7,64(mc,2H); |
| 1.063 | 2-$CO_2CH_3$-phenyl | H | $CH_3$ | $OCH_3$ | Cl | 203-205 |
| 1.064 | 2-$CH_3$-phenyl | H | $CH_3$ | Cl | Cl | 2,63(s,3H);3,61(s,3H);7,40(mc,3H);8,07(d,1H); |

* $^1H$-NMR-Spektrum in $CDCl_3$ als Lösungsmittel aufgenommen

Tabelle 1 (Fortsetzung)

| Nr. | A | $R^1$ | $R^2$ | $R^3$ | $R^4$ | $^1HNMR$ [$d^6$ DMSO, δ (ppm)]; Fp [°C] |
|---|---|---|---|---|---|---|
| 1.065 | 2-$CH_3$-phenyl | H | $CH_3$ | $OCH_3$ | Cl | 2,61(s,3H);3,49(s,3H);4,03(s,3H);7,39(mc,3H); 8,05(d,1H);0,87(s,1H); |
| 1.066 | 2-$CH_3$-phenyl | H | $CH_3$ | $OCH_3$ | $OCH_3$ | 2,63(s,3H);3,50(s,3H);3,89(s,3H);4,01(s,3H); 7,38(mc,3H);8,09(d,1H);12,45(s,1H); |
| 1.067 | 2,5-$CH_3$,$CH_3$-phenyl | H | $CH_3$ | Cl | Cl | 189-190 |
| 1.068 | 2,5-$CH_3$,$CH_3$-phenyl | H | $CH_3$ | $OCH_3$ | Cl | 206-208 |
| 1.069 | 2,5-$CH_3$,$CH_3$-phenyl | H | $CH_3$ | $OCH_3$ | $OCH_3$ | 182-184 |
| 1.070 | 2-$CF_3$-Phenyl | H | $CH_3$ | Cl | Cl | 208-210 |
| 1.071 | 2-$CF_3$-Phenyl | H | $CH_3$ | $OCH_3$ | Cl | 205-206 |
| 1.072 | 2-$CF_3$-Phenyl | H | $CH_3$ | $OCH_3$ | $OCH_3$ | 199-203 |
| 1.073 | 2-$O(CH_2)_2Cl$-phenyl | H | $CH_3$ | Cl | Cl | 115-119 |
| 1.074 | 2-$O(CH_2)_2OCH_3$-phenyl | H | $CH_3$ | Cl | Cl | 191-193 |
| 1.075 | 2-$O(CH_2)_2OCH_3$-phenyl | H | $CH_3$ | $OCH_3$ | Cl | 188 |
| 1.076 | 2,5-$OCH_3$,$OCH_3$-phenyl | H | $CH_3$ | Cl | Cl | 3,54(s,3H);3,61(s,3H);3,76(s,3H);7,11(s,2H); 7,44(s,1H);12,85(s,1H); |
| 1.077 | 2,5-$OCH_3$,$OCH_3$-phenyl | H | $CH_3$ | $OCH_3$ | Cl | 3,46(s,3H);3,61(s,3H);3,76(s,3H);4,04(s,3H); 7,07(s,2H);7,44(s,1,H);12,62(s,1H); |
| 1.078 | 2-$OCH_3$,5-Br-phenyl | H | $CH_3$ | Cl | Cl | 3,57(s,3H);3,71(s-3H);7,13(mc,1H);7,73(mc-1H); 7,98(mc,1H); |

Tabelle 2

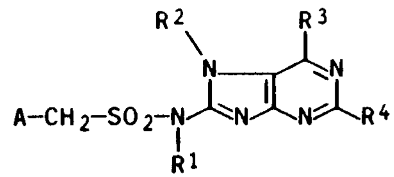

Ib

| Nr. | A | $R^1$ | $R^2$ | $R^3$ | $R^4$ | $^1HNMR$ [$d^6$ DMSO, δ (ppm)]; Fp [°C] |
|---|---|---|---|---|---|---|
| 2.001 | 2,6-Cl,Cl-Phenyl | H | $CH_3$ | Cl | Cl | 3,60(s,3H);4,70(s,2H);7,40(mc,1H); 7,55(mc,2H); |
| 2.002 | 2,6-Cl,Cl-Phenyl | H | $CH_3$ | $OCH_3$ | Cl | 3.50 (s, 3H), 4.05 (s, 2H), 4.70 (s, 2H), 7.40 (mc, 1H), 7.55 (mc, 2H) |
| 2.003 | 2-F-phenyl | H | $CH_3$ | Cl | Cl | 3,57(s,3H);4,49(s,2H);7,08-7,60(m-4H); |
| 2.004 | 2-F-phenyl | H | $CH_3$ | $OCH_3$ | Cl | 3,48(s,3H);4,07(s,3H);4,44(s,2H); 7,18(mc,2H);7,35(mc,1H);7,52(mc,1H); |

Tabelle 3

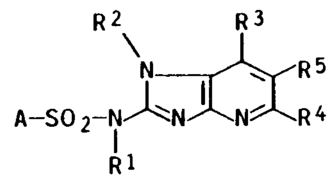

Ic

| Nr. | A | $R^1$ | $R^2$ | $R^3$ | $R^4$ | $R^5$ | $^1HNMR$ [$d^6$ DMSO, δ (ppm)]; Fp [°C] |
|---|---|---|---|---|---|---|---|
| 3.001 | Phenyl | H | H | H | H | H | 7,15(dd,1H);7,55(mc,3H);7,65(dd,1H); 7,90(mc,2H);8,05(dd,1H); |

## Anwendungsbeispiele

Die herbizide Wirkung der Sulfonamide der Formel I auf das Wachstum der Testpflanzen wird durch folgende Gewächshausversuche gezeigt.

Zur Anzucht der Testpflanzen dienten Plastikblumentöpfe mit 300 $cm^3$ Inhalt und lehmigem Sand mit etwa 3,0 % Humus als Substrat. Die Samen der Testpflanzen wurden nach Arten getrennt eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung wurden entweder direkt gesäte oder in den gleichen Gefäßen aufgewachsene Pflanzen ausgewählt oder sie wurden erst als Keimpflanzen getrennt angezogen und einige Tage vor der Behandlung in die Versuchsgefäße verpflanzt. Je nach Wuchsform wurden die Testpflanzen bei einer Wuchshöhe von 3 - 15 cm dann mit den in Wasser als Verteilungsmittel suspendierten oder emulgierten Wirkstoffen, die durch fein verteilende Düsen gespritzt wurden, behandelt. Die Aufwandmenge für die Nachauflaufbehandlung betrug 0,5 kg/ha aktive Substanz.

Die Versuchsgefäße wurden im Gewächshaus aufgestellt, wobei für wärmeliebende Arten wärmere Bereiche (20 bis 35 ° C) und für solche gemäßigter Klimate 10 bis 20 ° C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

In den Gewächshausversuchen wurden Pflanzen der folgenden Arten verwendet:

| Lateinischer Name | Deutscher Name |
|---|---|
| Chenopodium album | Weißer Gänsefuß |
| Chrysanthemum corinarium | Kronenwucherblume |
| Matricaria IN ODORA | Duftlose Kamille |
| Sinapis alba | Weißer Senf |
| Zea mays | Mais |

Mit 0,5 kg/ha a.S. im Nachauflaufverfahren eingesetzt, lassen sich mit Beispiel 1.009 und mit Beispiel 1.057 breitblättrige unerwünschte Pflanzen sehr gut bekämpfen bei gleichzeitiger Verträglichkeit für eine Beispielkultur.

**Patentansprüche**
**Patentansprüche für folgende Vertragsstaaten : AT, BE, CH, DE, DK, GB, IT, LI, NL**

**1.** Sulfonamide der allgemeinen Formel I,

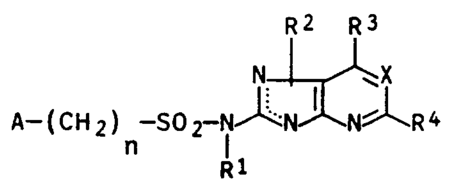

I

in der die Substituenten und Indices folgende Bedeutung haben:

$R^1$ Wasserstoff oder eine $C_1$-$C_4$-Alkylgruppe,

$R^2$ Wasserstoff,
eine $C_1$-$C_6$-Alkylgruppe, welche durch ein bis fünf Halogenatome und/oder einen der folgenden Reste substituiert sein kann; $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy oder Phenylthio;
eine $C_3$-$C_4$-Alkenylgruppe; eine $C_3$-$C_4$-Alkinylgruppe;
ein gesättigter oder einfach ungesättigter 5- bis 7-gliedriger Heterocyclus, enthaltend ein bis zwei Stickstoff-, Sauerstoff- und/oder Schwefelatome, welcher ein bis drei der folgenden Substituenten tragen kann: Halogen, $C_1$-$C_4$-Alkyl, $C_1$-$C_4$-Alkoxy, $C_1$-$C_4$-Halogenalkyl,

$C_1$-$C_4$-Halogenalkoxy, $C_1$-$C_4$-Alkylthio, $C_1$-$C_4$-Halogenalkylthio und/oder Phenyl, Phenoxy und/oder Phenylthio;

$R^3$, $R^4$ Halogen;

$C_1$-$C_4$-Alkoxy, $C_1$-$C_4$-Alkylthio, $C_2$-$C_6$-Alkenyl, $C_2$-$C_6$-Alkenyloxy, $C_2$-$C_6$-Alkenylthio, $C_2$-$C_6$-Alkinyl, $C_2$-$C_6$-Alkinyloxy und/oder $C_2$-$C_6$-Alkinylthio, wobei die genannten Reste durch ein bis fünf Halogenatome und/oder durch eine der folgenden Gruppen substituiert sein können: $C_1$-$C_4$-Alkoxy, $C_1$-$C_4$-Halogenalkoxy, $C_2$-$C_4$-Alkylthio, $C_1$-$C_4$-Halogenalkylthio, Phenyl, Phenoxy oder Phenylthio;

$C_3$-$C_6$-Cycloalkyl, $C_3$-$C_6$-Cycloalkylthio, $C_5$-$C_6$-Cycloalkenyl, $C_3$-$C_6$-Cycloalkoxy, $C_5$-$C_6$-Cycloalkenyloxy, $C_5$-$C_6$-Cycloalkenylthio, Phenyl, Phenoxy, Phenylthio, Benzyl, Benzyloxy oder Benzylthio, wobei diese cyclischen Gruppen durch ein bis fünf Halogenatome und/oder ein bis drei der folgenden Reste substituiert sein können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy, Phenylthio, Benzyl, Benzyloxy und/oder Benzylthio;

die unter $R^2$ genannten Gruppen oder

$NR^7R^8$, worin

$R^7R^8$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, Phenyl und/oder Benzyl bedeuten, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy und/oder $C_1$-$C_4$-Halogenalkoxy substituiert sein können oder gemeinsam eine $C_4$-$C_6$-Alkylenbrücke, welche durch ein Sauerstoff-, Schwefel- oder Stickstoffatom unterbrochen sein kann, wobei diese Brücke ihrerseits ein bis drei $C_1$-$C_4$-Alkylgruppen tragen kann;

X ein Stickstoffatom oder eine Gruppe $=CR^5$-, worin $R^5$ einen der Reste $R^3$ bedeutet,

n 0 oder 1 und

A einen ein- oder zweikernigen aromatischen Rest, der ein bis zwei Stickstoff-, Sauerstoff- und/oder Schwefelatome enthalten kann, wobei dieser Rest ein bis fünf Halogenatome und/oder ein bis drei der folgenden Gruppen tragen kann: Cyano, Nitro, Thiocyanato,

$COR^6$, worin $R^6$ Hydroxy, Amino oder einen der Reste $R^3$ bedeutet,

$SO_mR^6$, worin m den Wert 1 oder 2 hat und ein bis drei der unter $R^3$ genannten Reste,

mit der Maßgabe, daß A keinen Tosylrest darstellt, wenn n für 0 steht und X Stickstoff, $R^1$ Wasserstoff, $R^4$ Amino ($NH_2$) und $R^3$ Wasserstoff oder Methyl bedeuten,
sowie deren landwirtschaftlich brauchbare Salze.

**2.** Verfahren zur Herstellung der Verbindungen I gemäß Anspruch 1, dadurch gekennzeichnet, daß man ein entsprechendes Sulfonamid II

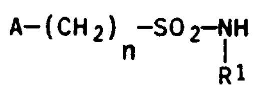

II

in an sich bekannter Weise in einem inerten organischen Lösungsmittel in Anwesenheit einer Base mit einem Heteroarylhalogenid III

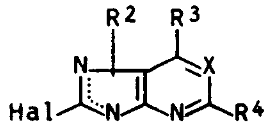

III

umsetzt, worin Hal ein Halogenatom bedeutet.

**3.** Verfahren zur Herstellung der Verbindungen I gemäß Anspruch 1, in denen $R^2$ nicht Wasserstoff bedeutet, dadurch gekennzeichnet, daß man ein entsprechendes Sulfonylhalogenid IV,

$A-(CH_2)_n-SO_2-Hal$ IV

worin Hal die in Anspruch 2 gegebene Bedeutung hat, in an sich bekannter Weise in einem inerten organischen Lösungsmittel in Gegenwart einer Base mit einem Heteroarylamin V

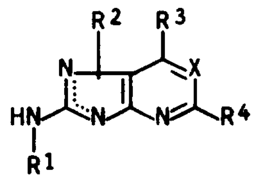

V

umsetzt.

**4.** Verfahren zur Herstellung der Verbindungen I gemäß Anspruch 1, in denen $R^1$ Wasserstoff bedeutet, dadurch gekennzeichnet, daß man eine entsprechende Verbindung VI,

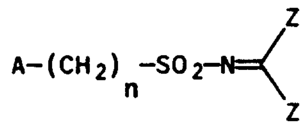

VI

worin Z ein Halogenatom oder eine Alkylthio- oder eine Benzylthiogruppe bedeutet, in an sich bekannter Weise in einem inerten organischen Lösungsmittel mit einem Diaminoheteroarylderivat VIIa bzw. VIIb,

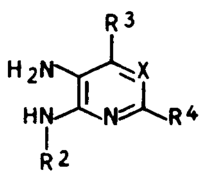

VIIa

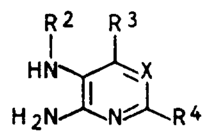

VIIb

umsetzt.

**5.** Verfahren zur Herstellung der Verbindungen I gemäß Anspruch 4, dadurch gekennzeichnet, daß man ein entsprechendes Derivat VIa,

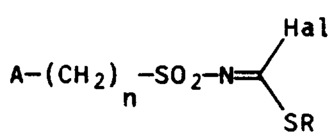

VIa

worin Hal die in Anspruch 2 gegebene Bedeutung hat und R eine Alkyl- oder Benzylgruppe bedeutet, in an sich bekannter Weise in einem inerten organischen Lösungsmittel mit einem Diaminoheteroarylderivat VIIa bzw. VIIb gemäß Anspruch 4, zum Isothioharnstoff VIIIa bzw. VIIIb

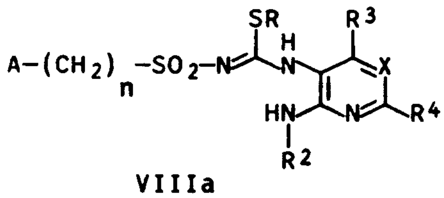

VIIIa

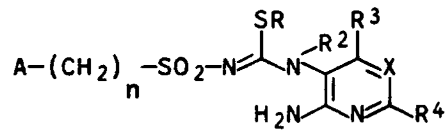

VIIIb

umsetzt und dieses anschließend in Gegenwart einer Base und eines Silbersalzes in einem inerten organischen Lösungsmittel cyclisiert.

6. Verfahren zur Herstellung der Verbindungen I gemäß Anspruch 1, worin $R^1$ und $R^2$ nicht Wasserstoff bedeuten, dadurch gekennzeichnet, daß man ein entsprechendes Derivat IX,

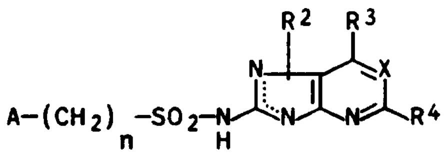

IX

in dem $R^2$ nicht Wasserstoff bedeutet, in an sich bekannter Weise in einem inerten organischen Lösungsmittel in Gegenwart einer Base mit einem Alkylierungsreagens X,

$R^1$-Nu    X

worin Nu eine nucleophile Abgangsgruppe und $R^1$ nicht Wasserstoff bedeutet, umsetzt.

7. Verfahren zur Herstellung der Verbindungen I gemäß Anspruch 1, in denen entweder $R^3$ oder $R^4$ eine nucleophile Gruppe und X ein Stickstoffatom bedeutet, dadurch gekennzeichnet, daß man ein entsprechendes Derivat der Formel XIa bzw. XIb

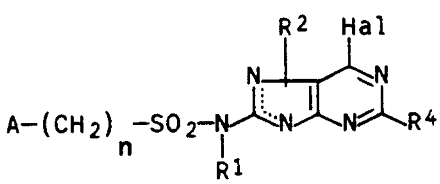

XIa

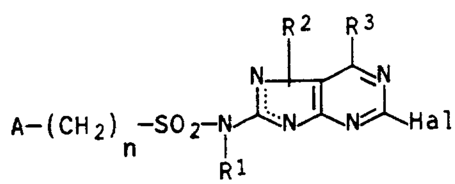

XIb

worin Hal die in Anspruch 2 gegebene Bedeutung hat, in an sich bekannter Weise in Gegenwart einer Base in einem inerten organischen Lösungsmittel mit der entsprechenden Verbindung XIIa bzw. XIIb

$R^3$-H    XIIa $R^4$-H    XIIb

umsetzt.

8. Verfahren zur Herstellung der Verbindungen I gemäß Anspruch 1, in denen $R^3$ und $R^4$ die gleiche nucleophile Gruppe bedeuten und X Stickstoff bedeutet, dadurch gekennzeichnet, daß man ein entsprechendes Derivat XIc

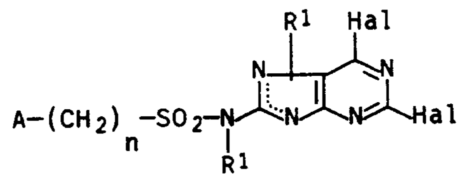

XIc

worin Hal die in Anspruch 2 gegebene Bedeutung hat, in an sich bekannter Weise in Gegenwart einer Base in einem inerten organischen Lösungsmittel mit der entsprechenden Verbindung XIIa oder XIIb gemäß Anspruch 7 umsetzt.

**9.** Herbizides Mittel, enthaltend ein Sulfonamid der allgemeinen Formel I, gemäß Anspruch 1, oder dessen landwirtschaftlich brauchbares Salz sowie übliche Formulierungshilfsmittel.

**10.** Verfahren zur Bekämpfung unerwünschten Pflanzenwachstums, dadurch gekennzeichnet, daß man ein Sulfonamid der Formel I

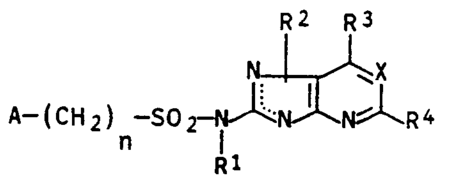

I

in der die Substituenten und Indices folgende Bedeutung haben:

$R^1$ Wasserstoff oder eine $C_1$-$C_4$-Alkylgruppe,

$R^2$ Wasserstoff,
eine $C_1$-$C_6$-Alkylgruppe, welche durch ein bis fünf Halogenatome und/oder einen der folgenden Reste substituiert sein kann; $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy oder Phenylthio;
eine $C_3$-$C_4$-Alkenylgruppe; eine $C_3$-$C_4$-Alkinylgruppe;
ein gesättigter oder einfach ungesättigter 5- bis 7-gliedriger Heterocyclus, enthaltend ein bis zwei Stickstoff-, Sauerstoff- und/oder Schwefelatome, welcher ein bis drei der folgenden Substituenten tragen kann: Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio und/oder Phenyl, Phenoxy und/oder Phenylthio;

$R^3$, $R^4$ Halogen;
$C_1$-$C_4$-Alkoxy, $C_1$-$C_4$-Alkylthio, $C_2$-$C_6$-Alkenyl, $C_2$-$C_6$-Alkenyloxy, $C_2$-$C_6$-Alkenylthio, $C_2$-$C_6$-Alkinyl, $C_2$-$C_6$-Alkinyloxy und/oder $C_2$-$C_6$-Alkinylthio, wobei die genannten Reste durch ein bis fünf Halogenatome und/oder durch eine der folgenden Gruppen substituiert sein können: $C_1$-$C_4$-Alkoxy, $C_1$-$C_4$-Halogenalkoxy, $C_2$-$C_4$-Alkylthio, $C_1$-$C_4$-Halogenalkylthio, Phenyl, Phenoxy oder Phenylthio;
$C_3$-$C_6$-Cycloalkyl, $C_3$-$C_6$-Cycloalkylthio, $C_5$-$C_6$-Cycloalkenyl, $C_3$-$C_6$-Cycloalkoxy, $C_5$-$C_6$-Cycloalkenyloxy, $C_5$-$C_6$-Cycloalkenylthio, Phenyl, Phenoxy, Phenylthio, Benzyl, Benzyloxy oder Benzylthio, wobei diese cyclischen Gruppen durch ein bis fünf Halogenatome und/oder ein bis drei der folgenden Reste substituiert sein können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy, Phenylthio, Benzyl, Benzyloxy und/oder Benzylthio;
die unter $R^2$ genannten Gruppen oder
$NR^7R^8$, worin
$R^7R^8$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, Phenyl und/oder Benzyl bedeuten, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy und/oder $C_1$-$C_4$-Halogenalkoxy substituiert sein können oder gemeinsam eine $C_4$-$C_6$-Alkylenbrücke, welche durch ein Sauerstoff-, Schwefel- oder Stickstoffatom unterbrochen sein kann, wobei diese Brücke ihrerseits ein bis drei $C_1$-$C_4$-Alkylgruppen tragen kann;

X ein Stickstoffatom oder eine Gruppe $=CR^5$-, worin $R^5$ einen der Reste $R^3$ bedeutet,

n 0 oder 1 und

A einen ein- oder zweikernigen aromatischen Rest, der ein bis zwei Stickstoff-, Sauerstoff- und/oder Schwefelatome enthalten kann, wobei dieser Rest ein bis fünf Halogenatome und/oder ein bis drei der folgenden Gruppen tragen kann: Cyano, Nitro, Thiocyanato, $COR^6$, worin $R^6$ Hydroxy, Amino oder einen der Reste $R^3$ bedeutet, $SO_mR^6$, worin m den Wert 1 oder 2 hat und ein bis drei der unter $R^3$ genannten Reste,

sowie deren landwirtschaftlich brauchbare Salze auf die Pflanzen und/oder ihren Lebensraum einwirken läßt.

**11.** Mittel zur Beeinflussung des Pflanzenwuchses, enthaltend ein Sulfonamid der Formel I gemäß Anspruch 1, oder dessen Satz sowie übliche Formulierungshilfsmittel.

**12.** Verfahren zur Beeinflussung des Pflanzenwuchses, dadurch gekennzeichnet, daß man ein Sulfonamid der Formel I gemäß Anspruch 10 oder dessen Salz auf die Pflanzen und/oder ihren Lebensraum einwirken läßt.

**Patentansprüche für folgenden Vertragsstaat : ES**

**1.** Herbizides Mittel, enthaltend übliche Formulierungshilfsmittel und Sulfonamide der allgemeinen Formel I,

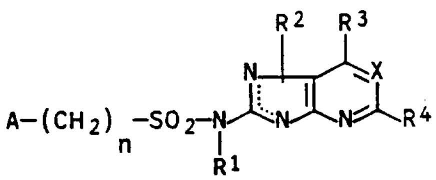

I

in der die Substituenten und Indices folgende Bedeutung haben:

$R^1$ Wasserstoff oder eine $C_1$-$C_4$-Alkylgruppe,

$R^2$ Wasserstoff,
eine $C_1$-$C_6$-Alkylgruppe, welche durch ein bis fünf Halogenatome und/oder einen der folgenden Reste substituiert sein kann; $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy oder Phenylthio;
eine $C_3$-$C_4$-Alkenylgruppe; eine $C_3$-$C_4$-Alkinylgruppe;
ein gesättigter oder einfach ungesättigter 5- bis 7-gliedriger Heterocyclus, enthaltend ein bis zwei Stickstoff-, Sauerstoff- und/oder Schwefelatome, welcher ein bis drei der folgenden Substituenten tragen kann: Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio und/oder Phenyl, Phenoxy und/oder Phenylthio;

$R^3$, $R^4$ Halogen;
$C_1$-$C_4$-Alkoxy, $C_1$-$C_4$-Alkylthio, $C_2$-$C_6$-Alkenyl, $C_2$-$C_6$-Alkenyloxy, $C_2$-$C_6$-Alkenylthio, $C_2$-$C_6$-Alkinyl, $C_2$-$C_6$-Alkinyloxy und/oder $C_2$-$C_6$-Alkinylthio, wobei die genannten Reste durch ein bis fünf Halogenatome und/oder durch eine der folgenden Gruppen substituiert sein können: $C_1$-$C_4$-Alkoxy, $C_1$-$C_4$-Halogenalkoxy, $C_2$-$C_4$-Alkylthio, $C_1$-$C_4$-Halogenalkylthio, Phenyl, Phenoxy oder Phenylthio;
$C_3$-$C_6$-Cycloalkyl, $C_3$-$C_6$-Cycloalkylthio, $C_5$-$C_6$-Cycloalkenyl, $C_3$-$C_6$-Cycloalkoxy, $C_5$-$C_6$-Cycloalkenyloxy, $C_5$-$C_6$-Cycloalkenylthio, Phenyl, Phenoxy, Phenylthio, Benzyl, Benzyloxy oder Benzylthio, wobei diese cyclischen Gruppen durch ein bis fünf Halogenatome und/oder ein bis drei der folgenden Reste substituiert sein können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy, Phenylthio, Benzyl, Benzyloxy und/oder Benzylthio;
die unter $R^2$ genannten Gruppen oder
$NR^7R^8$, worin
$R^7R^8$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, Phenyl und/oder Benzyl bedeuten, wobei die aromatischen Ringe ihrerseits ein- bis fünffach durch Halogen und/oder ein- bis dreifach durch $C_1$-$C_4$-Alkyl, $C_1$-$C_4$-

Halogenalkyl, $C_1$-$C_4$-Alkoxy und/oder $C_1$-$C_4$-Halogenalkoxy substituiert sein können oder gemeinsam eine $C_4$-$C_6$-Alkylenbrücke, welche durch ein Sauerstoff-, Schwefel- oder Stickstoffatom unterbrochen sein kann, wobei diese Brücke ihrerseits ein bis drei $C_1$-$C_4$-Alkylgruppen tragen kann;

X ein Stickstoffatom oder eine Gruppe $=CR^5$-, worin $R^5$ einen der Reste $R^3$ bedeutet,

n 0 oder 1 und

A einen ein- oder zweikernigen aromatischen Rest, der ein bis zwei Stickstoff-, Sauerstoff- und/oder Schwefelatome enthalten kann, wobei dieser Rest ein bis fünf Halogenatome und/oder ein bis drei der folgenden Gruppen tragen kann: Cyano, Nitro, Thiocyanato, $COR^6$, worin $R^6$ Hydroxy, Amino oder einen der Reste $R^3$ bedeutet, $SO_mR^6$, worin m den Wert 1 oder 2 hat und ein bis drei der unter $R^3$ genannten Reste,

mit der Maßgabe, daß A keinen Tosylrest darstellt, wenn n für 0 steht und X Stickstoff, $R^1$ Wasserstoff, $R^4$ Amino ($NH_2$) und $R^3$ Wasserstoff oder Methyl bedeuten,
sowie deren landwirtschaftlich brauchbare Salze.

2. Verfahren zur Herstellung der Verbindungen I gemäß Anspruch 1, dadurch gekennzeichnet, daß man ein entsprechendes Sulfonamid II

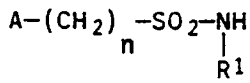

II

in an sich bekannter Weise in einem inerten organischen Lösungsmittel in Anwesenheit einer Base mit einem Heteroarylhalogenid III

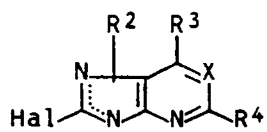

III

umsetzt, worin Hal ein Halogenatom bedeutet.

3. Verfahren zur Herstellung der Verbindungen I gemäß Anspruch 1, in denen $R^2$ nicht Wasserstoff bedeutet, dadurch gekennzeichnet, daß man ein entsprechendes Sulfonylhalogenid IV,

$A-(CH_2)_n-SO_2-Hal$ IV

worin Hal die in Anspruch 2 gegebene Bedeutung hat, in an sich bekannter Weise in einem inerten organischen Lösungsmittel in Gegenwart einer Base mit einem Heteroarylamin V

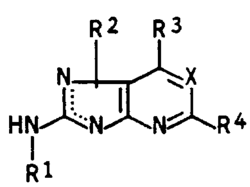

V

umsetzt.

4. Verfahren zur Herstellung der Verbindungen I gemäß Anspruch 1, in denen $R^1$ Wasserstoff bedeutet, dadurch gekennzeichnet, daß man eine entsprechende Verbindung VI,

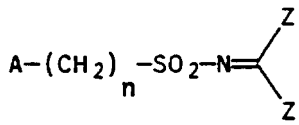

VI

worin Z ein Halogenatom oder eine Alkylthio- oder eine Benzylthiogruppe bedeutet, in an sich bekannter Weise in einem inerten organischen Lösungsmittel mit einem Diaminoheteroarylderivat VIIa bzw. VIIb,

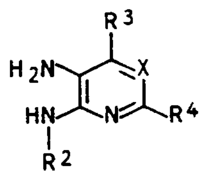

VIIa

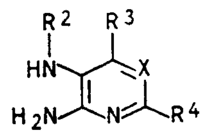

VIIb

umsetzt.

**5.** Verfahren zur Herstellung der Verbindungen I gemäß Anspruch 4, dadurch gekennzeichnet, daß man ein entsprechendes Derivat VIa,

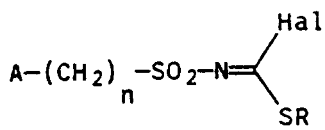

VIa

worin Hal die in Anspruch 2 gegebene Bedeutung hat und R eine Alkyl- oder Benzylgruppe bedeutet, in an sich bekannter Weise in einem inerten organischen Lösungsmittel mit einem Diaminoheteroarylderivat VIIa bzw. VIIb gemäß Anspruch 4, zum Isothioharnstoff VIIIa bzw. VIIIb

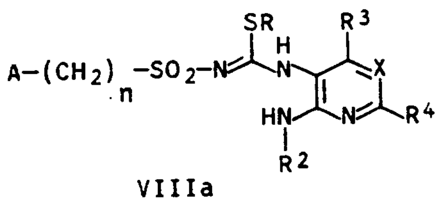

VIIIa

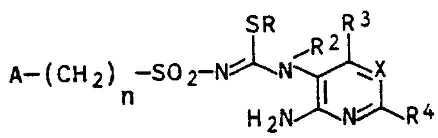

VIIIb

umsetzt und dieses anschließend in Gegenwart einer Base und eines Silbersalzes in einem inerten organischen Lösungsmittel cyclisiert.

**6.** Verfahren zur Herstellung der Verbindungen I gemäß Anspruch 1, worin $R^1$ und $R^2$ nicht Wasserstoff bedeuten, dadurch gekennzeichnet, daß man ein entsprechendes Derivat IX,

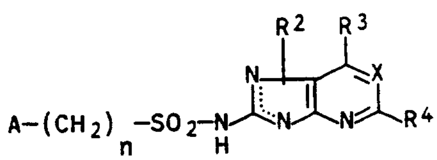

IX

in dem $R^2$ nicht Wasserstoff bedeutet, in an sich bekannter Weise in einem inerten organischen Lösungsmittel in Gegenwart einer Base mit einem Alkylierungsreagens X,

$R^1$-Nu X

worin Nu eine nucleophile Abgangsgruppe und $R^1$ nicht Wasserstoff bedeutet, umsetzt.

**7.** Verfahren zur Herstellung der Verbindungen I gemäß Anspruch 1, in denen entweder $R^3$ oder $R^4$ eine nucleophile Gruppe und X ein Stickstoffatom bedeutet, dadurch gekennzeichnet, daß man ein entsprechendes Derivat der Formel XIa bzw. XIb

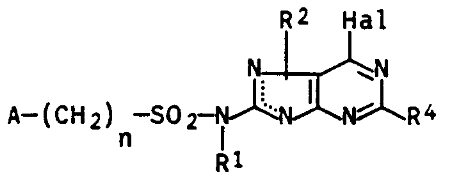

XIa

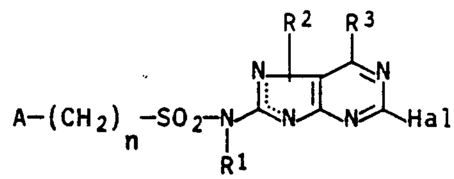

XIb

worin Hal die in Anspruch 2 gegebene Bedeutung hat, in an sich bekannter Weise in Gegenwart einer Base in einem inerten organischen Lösungsmittel mit der entsprechenden Verbindung XIIa bzw. XIIb

$R^3$-H XIIa $R^4$-H XIIb

umsetzt.

**8.** Verfahren zur Herstellung der Verbindungen I gemäß Anspruch 1, in denen $R^3$ und $R^4$ die gleiche nucleophile Gruppe bedeuten und X Stickstoff bedeutet, dadurch gekennzeichnet, daß man ein entsprechendes Derivat XIc

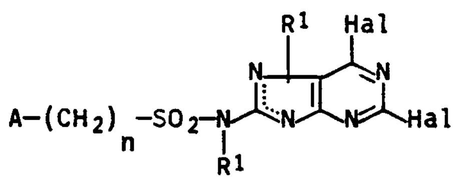

XIc

worin Hal die in Anspruch 2 gegebene Bedeutung hat, in an sich bekannter Weise in Gegenwart einer Base in einem inerten organischen Lösungsmittel mit der entsprechenden Verbindung XIIa oder XIIb gemäß Anspruch 7 umsetzt.

**9.** Verfahren zur Bekämpfung unerwünschten Pflanzenwachstums, dadurch gekennzeichnet, daß man ein Sulfonamid der Formel I

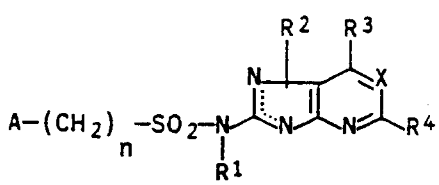

I

in der die Substituenten und Indices folgende Bedeutung haben:

$R^1$ Wasserstoff oder eine $C_1$-$C_4$-Alkylgruppe,

$R^2$ Wasserstoff,

eine $C_1$-$C_6$-Alkylgruppe, welche durch ein bis fünf Halogenatome und/oder einen der folgenden Reste substituiert sein kann; $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy oder Phenylthio;

eine $C_3$-$C_4$-Alkenylgruppe; eine $C_3$-$C_4$-Alkinylgruppe;

ein gesättigter oder einfach ungesättigter 5- bis 7-gliedriger Heterocyclus, enthaltend ein bis zwei Stickstoff-, Sauerstoff- und/oder Schwefelatome, welcher ein bis drei der folgenden Substituenten tragen kann: Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_1$-$C_4$-Halogenalkylthio und/oder Phenyl, Phenoxy und/oder Phenylthio;

$R^3$, $R^4$ Halogen;

$C_1$-$C_4$-Alkoxy, $C_1$-$C_4$-Alkylthio, $C_2$-$C_6$-Alkenyl, $C_2$-$C_6$-Alkenyloxy, $C_2$-$C_6$-Alkenylthio, $C_2$-$C_6$-Alkinyl, $C_2$-$C_6$-Alkinyloxy und/oder $C_2$-$C_6$-Alkinylthio, wobei die genannten Reste durch ein bis fünf Halogenatome und/oder durch eine der folgenden Gruppen substituiert sein können: $C_1$-$C_4$-Alkoxy, $C_1$-$C_4$-Halogenalkoxy, $C_2$-$C_4$-Alkylthio, $C_1$-$C_4$-Halogenalkylthio, Phenyl, Phenoxy oder Phenylthio;

$C_3$-$C_6$-Cycloalkyl, $C_3$-$6_6$-Cycloalkylthio, $C_5$-$C_6$-Cycloalkenyl, $C_3$-$C_6$-Cycloalkoxy, $C_5$-$C_6$-Cycloalkenyloxy, $C_5$-$C_6$-Cycloalkenylthio, Phenyl, Phenoxy, Phenylthio, Benzyl, Benzyloxy oder Benzylthio, wobei diese cyclischen Gruppen durch ein bis fünf Halogenatome und/oder ein bis drei der folgenden Reste substituiert sein können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Phenyl, Phenoxy, Phenylthio, Benzyl, Benzyloxy und/oder Benzylthio;

die unter $R^2$ genannten Gruppen oder

$NR^7R^8$, worin

$R^7R^8$ Wasserstoff, $C_1$-$C_6$-Alkyl, $C_3$-$C_6$-Alkenyl, $C_3$-$C_6$-Alkinyl, $C_3$-$C_6$-Cycloalkyl, $C_5$-$C_6$-Cycloalkenyl, Phenyl und/oder Benzyl bedeuten, wobei die aromatischen Ringe ihrerseits ein- bis fünffach durch Halogen und/oder ein- bis dreifach durch $C_1$-$C_4$-Alkyl, $C_1$-$C_4$-Halogenalkyl, $C_1$-$C_4$-Alkoxy und/oder $C_1$-$C_4$-Halogenalkoxy substituiert sein können oder gemeinsam eine $C_4$-$C_6$-Alkylenbrücke, welche durch ein Sauerstoff-, Schwefel- oder Stickstoffatom unterbrochen sein kann, wobei diese Brücke ihrerseits ein bis drei $C_1$-$C_4$-Alkylgruppen tragen kann;

X ein Stickstoffatom oder eine Gruppe $=CR^5$-, worin $R^5$ einen der Reste $R^3$ bedeutet,

n 0 oder 1 und

A einen ein- oder zweikernigen aromatischen Rest, der ein bis zwei Stickstoff-, Sauerstoff- und/oder Schwefelatome enthalten kann, wobei dieser Rest ein bis fünf Halogenatome und/oder ein bis drei der folgenden Gruppen tragen kann: Cyano, Nitro, Thiocyanato, $COR^6$, worin $R^6$ Hydroxy, Amino oder einen der Reste $R^3$ bedeutet, $SO_mR^6$, worin m den Wert 1 oder 2 hat und ein bis drei der unter $R^3$ genannten Reste,

sowie deren landwirtschaftlich brauchbare Salze auf die Pflanzen und/oder ihren Lebensraum einwirken läßt.

**10.** Verfahren zur Beeinflussung des Pflanzenwuchses, dadurch gekennzeichnet, daß man ein Sulfonamid der Formel I gemäß Anspruch 9, oder dessen Salz auf die Pflanzen und/oder ihren Lebensraum einwirken läßt.

## Claims

## Claims for the following Contracting States : AT, BE, CH, DE, DK, GB, IT, LI, NL

**1.** A sulfonamide of the general formula I

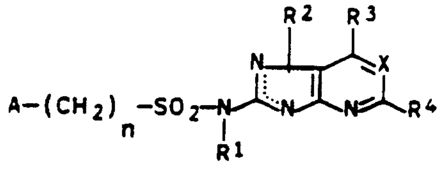

I

where

$R^1$ is hydrogen or $C_1$-$C_4$-alkyl,

$R^2$ is hydrogen,

$C_1$-$C_6$-alkyl which can be substituted by one to five halogens and/or one of the following: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio;

$C_3$-$C_4$-alkenyl, $C_3$-$C_4$-alkynyl;

a saturated or singly unsaturated 5- to 7-membered heterocycle which contains one or two nitrogen, oxygen and/or sulfur atoms and which can have one to three of the following substituents: halogen, $C_1$-$C_4$-alkyl, $C_1$-$C_4$-alkoxy, $C_1$-$C_4$-haloalkyl, $C_1$-$C_4$-haloalkoxy, $C_1$-$C_4$-alkylthio, $C_1$-$C_4$-haloalkylthio and/or phenyl, phenoxy and/or phenylthio;

$R^3$ and $R^4$ are halogen;

$C_1$-$C_4$-alkoxy, $C_1$-$C_4$-alkylthio, $C_2$-$C_6$-alkenyl, $C_2$-$C_6$-alkenyloxy, $C_2$-$C_6$-alkenylthio, $C_2$-$C_6$-alkynyl, $C_2$-$C_6$-alkynyloxy and/or $C_2$-$C_6$-alkynylthio, it being possible for these radicals to be substituted by one to five halogens and/or by one of the following groups: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio; $C_3$-$C_6$-cycloalkyl, $C_3$-$C_6$-cycloalkylthio, $C_5$-$C_6$-cycloalkenyl, $C_3$-$C_6$-cycloalkoxy, $C_5$-$C_6$-cycloalkenyloxy, $C_5$-$C_6$-cycloalkenylthio, phenyl, phenoxy, phenylthio, benzyl, benzyloxy or benzylthio, it being possible for these cyclic groups to be substituted by one to five halogens and/or one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, phenyl, phenoxy, phenylthio, benzyl, benzyloxy and/or benzylthio;

the groups mentioned under $R^2$ or $NR^7R^8$ where

$R^7$ and $R^8$ are hydrogen, $C_1$-$C_6$-alkyl, $C_3$-$C_6$-alkenyl, $C_3$-$C_6$-alkynyl, $C_3$-$C_6$-cycloalkyl, $C_5$-$C_6$-cycloalkenyl, phenyl, and/or benzyl, it being possible for the aromatic rings in turn to be substituted once to five times by halogen and/or once to three times by $C_1$-$C_4$-alkyl, $C_1$-$C_4$-haloalkyl, $C_1$-$C_4$-alkoxy and/or $C_1$-$C_4$-haloalkoxy, or together are a $C_4$-$C_6$-alkylene bridge which can be interrupted by an oxygen, sulfur or a nitrogen atom, it being possible for this bridge in turn to carry one to three $C_1$-$C_4$-alkyl groups;

X is nitrogen or $=CR^5$- where $R^5$ is one of the radicals $R^3$,

n is 0 or 1 and

A is a mono- or dinuclear aromatic radical which can contain one or two nitrogen, oxygen and/or sulfur atoms and can carry one to five halogens and/or one to three of the following: cyano, nitro, thiocyanato, $COR^6$ where $R^6$ is hydroxyl, amino and one of the radicals $R^3$,

$SO_mR^6$ where m is 1 or 2, and can carry one to three of the radicals mentioned under $R^3$, with the proviso that A is not tosyl when n is 0, X is nitrogen, $R^1$ is hydrogen, $^4$ is amino ($NH_2$) and $R^3$ is hydrogen or methyl,

or an agriculturally useful salt thereof.

2. A process for the preparation of a compound I as claimed in claim 1, which comprises reacting an appropriate sulfonamide II

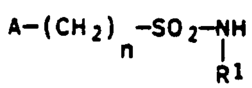

II

in a conventional manner in an inert organic solvent in the presence of a base with a heteroaryl halide III

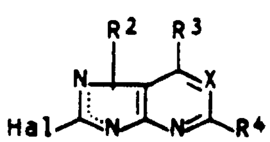

III

where Hal is halogen.

3. A process for the preparation of a compound I as claimed in claim 1, where $R^2$ is not hydrogen, which comprises reacting an appropriate sulfonyl halide IV

$A$-$(CH_2)_n$-$SO_2$-Hal IV

where Hal has the meaning given in claim 2, in a conventional manner in an inert organic solvent in the presence of a base with a heteroarylamine V

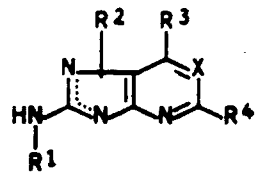

V

4. A process for the preparation of a compound I as claimed in claim 1, where $R^1$ is hydrogen, which comprises reacting an appropriate compound VI

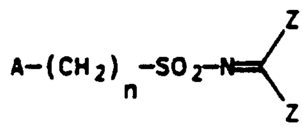

VI

where Z is halogen or alkylthio or benzylthio in a conventional manner in an inert organic solvent with a diaminoheteroaryl derivative VIIa or VIIb

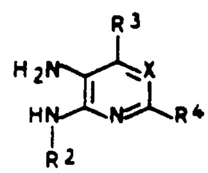

VIIa

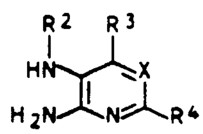

VIIb

5. A process for the preparation of a compound I as claimed in claim 4, which comprises reacting an appropriate derivative VIa

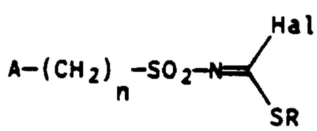

VIa

where Hal has the meaning given in claim 2 and R is alkyl or benzyl, in a conventional manner in an inert organic solvent with a diaminoheteroaryl derivative VIIa or VIIb as claimed in claim 4, to give an isothiourea VIIIa or VIIIb

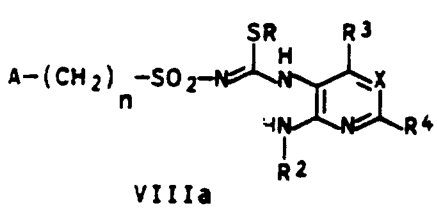

VIIIa

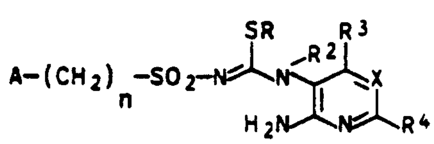

VIIIb

which is then cyclized in the presence of a base and of a silver salt in an inert organic solvent.

6. A process for the preparation of a compound I as claimed in claim 1, where $R^1$ and $R^2$ are not hydrogen, which comprises reacting an appropriate derivative IX

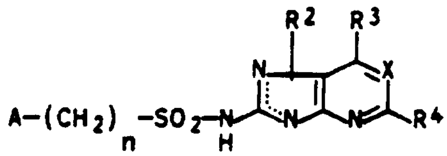

IX

where $R^2$ is not hydrogen, in a conventional manner in an inert organic solvent in the presence of a base with an alkylating reagent X

$R^1$-Nu X

where Nu is a nucleophilic leaving group and $R^1$ is not hydrogen.

**7.** A process for the preparation of a compound I as claimed in claim 1, where either $R^3$ or $R^4$ is a nucleophilic group and X is nitrogen, which comprises reacting an appropriate derivative of the formula XIa or XIb

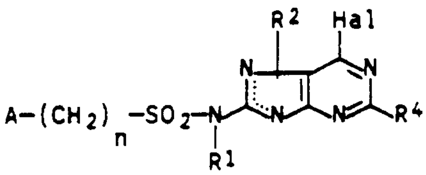

XIa

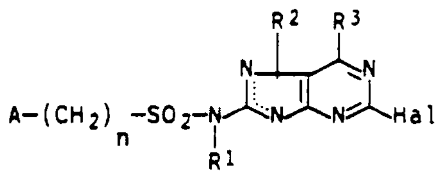

XIb

where Hal has the meaning given in claim 2, in a conventional manner in the presence of a base in an inert organic solvent with the appropriate compound XIIa or XIIb

$R^3$-H XIIa $R^4$-H XIIb

**8.** A process for the preparation of a compound I as claimed in claim 1, where $R^3$ and $R^4$ are each the same nucleophilic group and X is nitrogen, which comprises reacting an appropriate derivative IXc

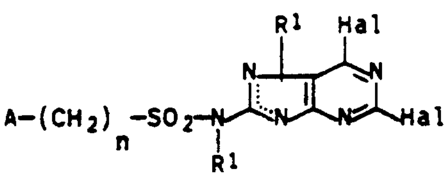

XIc

where Hal has the meaning given in claim 2, in a conventional manner in the presence of a base in an inert organic solvent with the appropriate compound XIIa or XIIb as claimed in claim 7.

**9.** A herbicidal composition containing a sulfonamide of the general formula I as claimed in claim 1, or an agriculturally useful salt thereof, and conventional formulation auxiliaries.

**10.** A method for controlling undesired plant growth, which comprises treating the plants and/or their habitat with a sulfonamide of the formula I

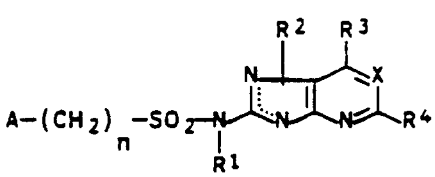

I

where
$R^1$ is hydrogen or $C_1$-$C_4$-alkyl,
$R^2$ is hydrogen,
$C_1$-$C_6$-alkyl which can be substituted by one to five halogens and/or one of the following: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio;
$C_3$-$C_4$-alkenyl, $C_3$-$C_4$-alkynyl;
a saturated or singly unsaturated 5- to 7-membered heterocycle which contains one or two nitrogen, oxygen and/or sulfur atoms and which can have one to three of the following substituents: halogen, $C_1$-$C_4$-alkyl, $C_1$-$C_4$-alkoxy, $C_1$-$C_4$-haloalkyl, $C_1$-$C_4$-haloalkoxy, $C_1$-$C_4$-alkylthio, $C_1$-$C_4$-haloalkylthio and/or phenyl, phenoxy and/or phenylthio;
$R^3$ and $R^4$ are halogen;
$C_1$-$C_4$-alkoxy, $C_1$-$C_4$-alkylthio, $C_2$-$C_6$-alkenyl, $C_2$-$C_6$-alkenyloxy, $C_2$-$C_6$-alkenylthio, $C_2$-$C_6$-alkynyl, $C_2$-$C_6$-alkynyloxy and/or $C_2$-$C_6$-alkynylthio, it being possible for these radicals to be substituted by one to five halogens and/or by one of the folowing groups: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio; $C_3$-$C_6$-cycloalkyl, $C_3$-$C_6$-cycloalkylthio, $C_5$-$C_6$-cycloalkenyl, $C_3$-$C_6$-cycloalkoxy, $C_5$-$C_6$-cycloalkenyloxy, $C_5$-$C_6$-cycloalkenylthio, phenyl, phenoxy, phenylthio, benzyl, benzyloxy or benzylthio, it being possible for these cyclic groups to be substituted by one to five halogens and/or one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, phenyl, phenoxy, phenylthio, benzyl, benzyloxy and/or benzylthio;
the groups mentioned under $R^2$ or $NR^7R^8$ where
$R^7$ and $R^8$ are hydrogen, $C_1$-$C_6$-alkyl, $C_3$-$C_6$-alkenyl, $C_3$-$C_6$-alkynyl, $C_3$-$C_6$-cycloalkyl, $C_5$-$C_6$-cycloalkenyl, phenyl, and/or benzyl, it being possible for the aromatic rings in turn to be substituted once to five times by halogen and/or once to three times by $C_1$-$C_4$-alkyl, $C_1$-$C_4$-haloalkyl, $C_1$-$C_4$-alkoxy and/or $C_1$-$C_4$-haloalkoxy, or together are a $C_4$-$C_6$-alkylene bridge which can be interrupted by an oxygen, sulfur or a nitrogen atom, it being possible for this bridge in turn to carry one to three $C_1$-$C_4$-alkyl groups;
X is nitrogen or $=CR^5$- where $R^5$ is one of the radicals $R^3$,
n is 0 or 1 and
A is a mono- or dinuclear aromatic radical which can contain one or two nitrogen, oxygen and/or sulfur atoms and can carry one to five halogens and/or one to three of the following: cyano, nitro, thiocyanato, $COR^6$ where $R^6$ is hydroxyl, amino and one of the radicals $R^3$,
$SO_mR^6$ where m is 1 or 2, and can carry one to three of the radicals mentioned under $R^3$, with the proviso that A is not tosyl when n is 0, X is nitrogen, $R^1$ is hydrogen, $^4$ is amino ($NH_2$) and $R^3$ is hydrogen or methyl,
or an agriculturally useful salt thereof.

**11.** An agent for influencing plant growth, containing a sulfonamide of the formula I as claimed in claim 1, or a salt thereof, and conventional formulation auxiliaries.

**12.** A method for influencing plant growth, which comprises treating the plants and/or their habitat with a sulfonamide of the formula I as claimed in claim 10, or a salt thereof.

**Claims for the following Contracting State : ES**

**1.** A herbicidal composition containing conventional formulation auxiliaries and a sulfonamide of the general formula I

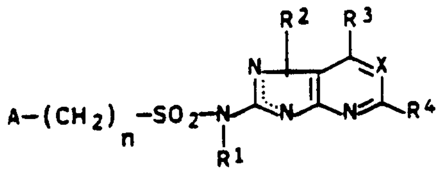

I

where
$R^1$ is hydrogen or $C_1$-$C_4$-alkyl,
$R^2$ is hydrogen,

$C_1$-$C_6$-alkyl which can be substituted by one to five halogens and/or one of the following: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio;
$C_3$-$C_4$-alkenyl, $C_3$-$C_4$-alkynyl;
a saturated or singly unsaturated 5- to 7-membered heterocycle which contains one or two nitrogen, oxygen and/or sulfur atoms and which can have one to three of the following substituents: halogen, $C_1$-$C_4$-alkyl, $C_1$-$C_4$-alkoxy, $C_1$-$C_4$-haloalkyl, $C_1$-$C_4$-haloalkoxy, $C_1$-$C_4$-alkylthio, $C_1$-$C_4$-haloalkylthio and/or phenyl, phenoxy and/or phenylthio;
$R^3$ and $R^4$ are halogen;
$C_1$-$C_4$-alkoxy, $C_1$-$C_4$-alkylthio, $C_2$-$C_6$-alkenyl, $C_2$-$C_6$-alkenyloxy, $C_2$-$C_6$-alkenylthio, $C_2$-$C_6$-alkynyl, $C_2$-$C_6$-alkynyloxy and/or $C_2$-$C_6$-alkynylthio, it being possible for these radicals to be substituted by one to five halogens and/or by one of the following groups: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio; $C_3$-$C_6$-cycloalkyl, $C_5$-$C_6$-cycloalkylthio, $C_5$-$C_6$-cycloalkenyl, $C_3$-$C_6$-cycloalkoxy, $C_5$-$C_6$-cycloalkenyloxy,$C_5$-$C_6$-cycloalkenylthio, phenyl, phenoxy, phenylthio, benzyl, benzyloxy or benzylthio, it being possible for these cyclic groups to be substituted by one to five halogens and/or one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, phenyl, phenoxy, phenylthio, benzyl, benzyloxy and/or benzylthio;
the groups mentioned under $R^2$ or $NR^7R^8$ where
$R^7$ and $R^8$ are hydrogen, $C_1$-$C_6$-alkyl, $C_3$-$C_6$-alkenyl, $C_3$-$C_6$-alkynyl, $C_3$-$C_6$-cycloalkyl, $C_5$-$C_6$-cycloalkenyl, phenyl, and/or benzyl, it being possible for the aromatic rings in turn to be substituted once to five times by halogen and/or once to three times by $C_1$-$C_4$-alkyl, $C_1$-$C_4$-haloalkyl, $C_1$-$C_4$-alkoxy and/or $C_1$-$C_4$-haloalkoxy, or together are a $C_4$-$C_6$-alkylene bridge which can be interrupted by an oxygen, sulfur or a nitrogen atom, it being possible for this bridge in turn to carry one to three $C_1$-$C_4$-alkyl groups;
X is nitrogen or $=CR^5$- where $R^5$ is one of the radicals $R^3$,
n is 0 or 1 and
A is a mono- or dinuclear aromatic radical which can contain one or two nitrogen, oxygen and/or sulfur atoms and can carry one to five halogens and/or one to three of the following: cyano, nitro, thiocyanato, $COR^6$ where $R^6$ is hydroxyl, amino or one of the radicals $R^3$,
$SO_mR^6$ where m is 1 or 2, and can carry one to three of the radicals mentioned under $R^3$, with the proviso that A is not tosyl when n is 0, X is nitrogen, $R^1$ is hydrogen, $^4$ is amino ($NH_2$) and $R^3$ is hydrogen or methyl,
or an agriculturally useful salt throughout.

2. A process for the preparation of a compound I as claimed in claim 1, which comprises reacting an appropriate sulfonamide II

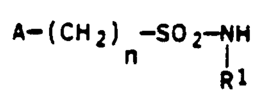

II

in a conventional manner in an inert organic solvent in the presence of a base with a heteroaryl halide III

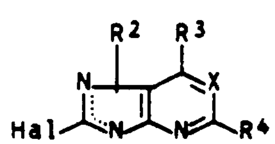

III

where Hal is halogen.

3. A process for the preparation of a compound I as claimed in claim 1, where $R^2$ is not hydrogen, which comprises reacting an appropriate sulfonyl halide IV

$A$-$(CH_2)_n$-$SO_2$-Hal IV

where Hal has the meaning given in claim 2, in a conventional manner in an inert organic solvent in the presence of a base with a heteroarylamine V

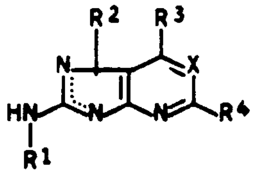

V

4. A process for the preparation of a compound I as claimed in claim 1, where $R^1$ is hydrogen, which comprises reacting an appropriate compound VI

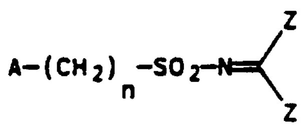

VI

where Z is halogen or alkylthio or benzylthio in a conventional manner in an inert organic solvent with a diaminoheteroaryl derivative VIIa or VIIb

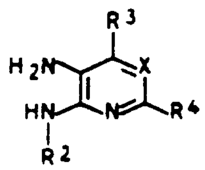

VIIa

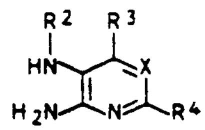

VIIb

5. A process for the preparation of a compound I as claimed in claim 4, which comprises reacting an appropriate derivative VIa

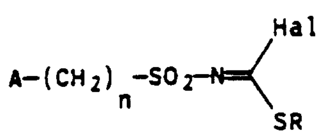

VIa

where Hal has the meaning given in claim 2 and R is alkyl or benzyl, in a conventional manner in an inert organic solvent with a diaminoheteroaryl derivative VIIa or VIIb as claimed in claim 4, to give an isothiourea VIIIa or VIIIb

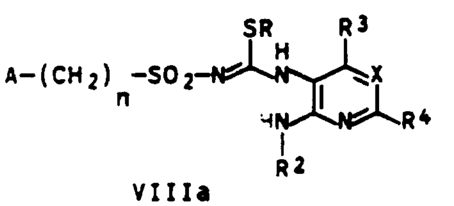

VIIIa

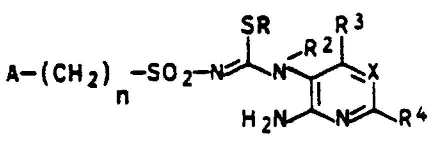

VIIIb

which is then cyclized in the presence of a base and of a silver salt in an inert organic solvent.

**6.** A process for the preparation of a compound I as claimed in claim 1, where $R^1$ and $R^2$ are not hydrogen, which comprises reacting an appropriate derivative IX

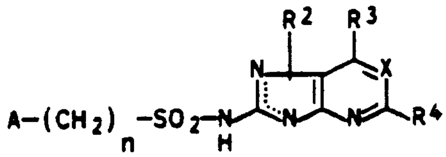

IX

where $R^2$ is not hydrogen, in a conventional manner in an inert organic solvent in the presence of a base with an alkylating reagent X

$R^1$-Nu X

where Nu is a nucleophilic leaving group and $R^1$ is not hydrogen.

**7.** A process for the preparation of a compound I as claimed in claim 1, where either $R^3$ or $R^4$ is a nucleophilic group and X is nitrogen, which comprises reacting an appropriate derivative of the formula XIa or XIb

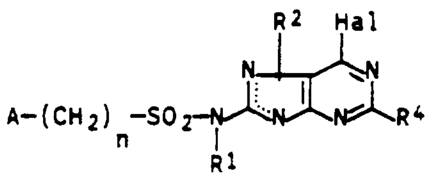

XIa

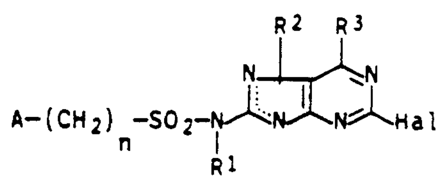

XIb

where Hal has the meaning given in claim 2, in a conventional manner in the presence of a base in an inert organic solvent with the appropriate compound XIIa or XIIb

$R^3$-H XIIa $R^4$-H XIIb

**8.** A process for the preparation of a compound I as claimed in claim 1, where $R^3$ and $R^4$ are each the same nucleophilic group and X is nitrogen, which comprises reacting an appropriate derivative IXc

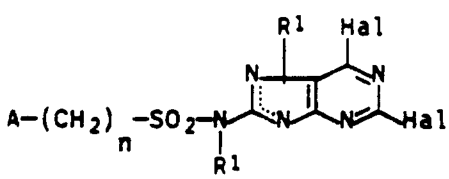

XIc

where Hal has the meaning given in claim 2, in a conventional manner in the presence of a base in an inert organic solvent with the appropriate compound XIIa or XIIb as claimed in claim 7.

**9.** A method for controlling undesired plant growth, which comprises treating the plants and/or their habitat with a sulfonamide of the formula I

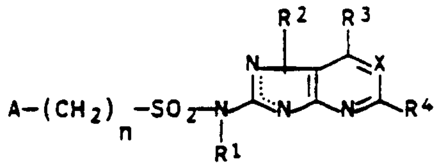

I

where
$R^1$ is hydrogen or $C_1$-$C_4$-alkyl,
$R^2$ is hydrogen,
$C_1$-$C_6$-alkyl which can be substituted by one to five halogens and/or one of the following: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio;
$C_3$-$C_4$-alkenyl, $C_3$-$C_4$-alkynyl;
a saturated or singly unsaturated 5- to 7-membered heterocycle which contains one or two nitrogen, oxygen and/or sulfur atoms and which can have one to three of the following substituents: halogen, $C_1$-$C_4$-alkyl, $C_1$-$C_4$-alkoxy, $C_1$-$C_4$-haloalkyl, $C_1$-$C_4$-haloalkoxy, $C_1$-$C_4$-alkylthio, $C_1$-$C_4$-haloalkylthio and/or phenyl, phenoxy and/or phenylthio;
$R^3$ and $R^4$ are halogen;
$C_1$-$C_4$-alkoxy, $C_1$-$C_4$-alkylthio, $C_2$-$C_6$-alkenyl, $C_2$-$C_6$-alkenyloxy, $C_2$-$C_6$-alkenylthio, $C_2$-$C_6$-alkynyl, $C_2$-$C_6$-alkynyloxy and/or $C_2$-$C_6$-alkynylthio, it being possible for these radicals to be substituted by one to five halogens and/or by one of the following groups: $C_1$-$C_4$-alkoxy, $C_1$-$C_4$-haloalkoxy, $C_1$-$C_4$-alkylthio, $C_1$-$C_4$-haloalkylthio, phenyl, phenoxy or phenylthio; $C_3$-$C_6$-cycloalkyl, $C_3$-$C_6$-cycloalkylthio, $C_5$-$C_6$-cycloalkenyl, $C_3$-$C_6$-cycloalkoxy, $C_5$-$C_6$-cycloalkenyloxy,$C_5$-$C_6$-cycloalkenylthio, phenyl, phenoxy, phenylthio, benzyl, benzyloxy or benzylthio, it being possible for these cyclic groups to be substituted by one to five halogens and/or one to three of the following: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, phenyl, phenoxy, phenylthio, benzyl, benzyloxy and/or benzylthio;
the groups mentioned under $R^2$ or $NR^7R^8$ where
$R^7$ and $R^8$ are hydrogen, $C_1$-$C_6$-alkyl, $C_3$-$C_6$-alkenyl, $C_3$-$C_6$-alkynyl, $C_3$-$C_6$-cycloalkyl, $C_5$-$C_6$-cycloalkenyl, phenyl, and/or benzyl, it being possible for the aromatic rings in turn to be substituted once to five times by halogen and/or once to three times by $C_1$-$C_4$-alkyl, $C_1$-$C_4$-haloalkyl, $C_1$-$C_4$-alkoxy and/or $C_1$-$C_4$-haloalkoxy, or together are a $C_4$-$C_6$-alkylene bridge which can be interrupted by an oxygen, sulfur or a nitrogen atom, it being possible for this bridge in turn to carry one to three $C_1$-$C_4$-alkyl groups;
X is nitrogen or $=CR^5$- where $R^5$ is one of the radicals $R^3$, n is 0 or 1 and
A is a mono- or dinuclear aromatic radical which can contain one or two nitrogen, oxygen and/or sulfur atoms and can carry one to five halogens and/or one to three of the following: cyano, nitro, thiocyanato, $COR^6$ where $R^6$ is hydroxyl, amino or one of the radicals $R^3$,
$SO_mR^6$ where m is 1 or 2, and can carry one to three of the radicals mentioned under $R^3$, with the proviso that A is not tosyl when n is 0, X is nitrogen, $R^1$ is hydrogen, $^4$ is amino ($NH_2$) and $R^3$ is hydrogen or methyl,
or an agriculturally useful salt thereof.

**10.** A method for influencing plant growth, which comprises treating the plants and/or their habitat with a sulfonamide of the formula I as claimed in claim 9, or a salt thereof.

## Revendications

## Revendications pour les Etats contractants suivants : AT, BE, CH, DE, DK, GB, IT, LI, NL

**1.** Sulfonamides de formule générale 1

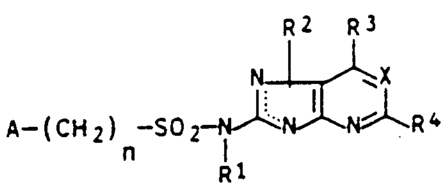

dans les symboles et indices ont les significations suivantes :
$R^1$ représente l'hydrogène ou un groupe alkyle en C1-C4,
$R^2$ représente l'hydrogène,
un groupe alkyle en C1-C6 qui peut être substitué par 1 à 5 atomes d'halogènes et/ou un des groupes suivants : alcoxy en C1-C4, halogénoalcoxy en C1-C4, alkylthio en C1-C4, halogénoalkylthio en C1-C4, phényle, phénoxy ou phénylthio,
un groupe alcényle en C3-C4; un groupe alcynyle en C3-C4;
un hétérocycle saturé ou mono-insaturé de 5 à 7 chaînons contenant 1 à 2 atomes d'azote, d'oxygène et/ou de soufre et qui peut porter 1 à 3 des substituants suivants : les halogènes, les groupes alkyle en C1-C4, alcoxy en C1-C4, halogénoalkyle en C1-C4, halogènoalcoxy en C1-C4, alkylthio en C1-C4, halogénoalkylthio en C1-C4, et/ou phényle, phénoxy et/ou phénylthio;
$R^3$, $R^4$ représentent des halogénes;
des groupes alcoxy en C1-C4, alkylthio en C1-C4, alcényle en C2-C6, alcényloxy en C2-C6, alcénylthio en C2-C6, alcynyle en C2-C6, alcynyloxy en C2-C6 et/ou alcynylthio en C2-C6, tous ces groupes pouvant être substitués par 1 à 5 atomes d'halogènes et/ou par un des groupes suivants : alcoxy en C1-C4, halogénoalcoxy en C1-C4, alkylthio en C2-C4, halogénoalkylthio en C2-C4, phényle, phénoxy ou phénylthio;
des groupes cycloalkyle en C3-C6, cycloalkylthio en C3-C6, cycloalcényle en C5-C6, cycloalcoxy en C3-C6, cycloalcényloxy en C5-C6, cycloalcénylthio en C5-C6, phényle, phénoxy, phénylthio, benzyle, benzyloxy ou benzylthio, ces groupes cycliques pouvant être substitués par 1 à 5 atomes d'halogènes et/ou par 1 à 3 des groupes suivants : alkyle en C1-C4, halogénoalkyle en C1-C4, alcoxy en C1-C4, halogénoalcoxy en C1-C4, alkylthio en C1-C4, halogénoalkylthio en C1-C4, phényle, phénoxy, phénylthio, benzyle, benzyloxy et/ou benzylthio;
les groupes mentionnés en référence à $R^2$ ou bien
des groupes $NR^7R^8$ dans lesquels
$R^7R^8$ représentent l'hydrogène, des groupes alkyle en C1-C6, alcényle en C3-C6, alcynyle en C3-C6, cycloalkyle en C3-C6, cycloalcényle en C5-C6, phényle et/ou benzyle, les noyaux aromatiques pouvant eux-mêmes être substitués une à cinq fois par halogène et/ou 1 à 3 fois par alkyle en C1-C4, halogénoalkyle en C1-C4, alcoxy en C1-C4 et/ou halogénoalcoxy en C1-C4, ou bien $R^7$ et $R^8$ représentent ensemble un pont alkylène en C4-C6 qui peut être interrompu par un atome d'oxygène, de soufre ou d'azote, ce pont pouvant lui-même porter 1 à 3 groupes alkyle en C1-C4;
X représente un atome d'azote ou un groupe $=CR^5-$ dans lequel $R^5$ a l'une des significations indiquées pour R3,
n est égal à 0 ou 1, et
A représente un radical aromatique mono- ou bi-cyclique qui peut contenir 1 à 2 atomes d'azote, d'oxygène et/ou de soufre, ce radical pouvant porter 1 à 5 atomes d'halogènes et/ou 1 à 3 des groupes suivants :
cyano, nitro, thiocyanato,
$COR^6$ dans lequel $R^6$ représente un groupe hydroxy, amino ou a l'une des significations indiquées pour $R^3$,
$SO_mR^6$ dans lequel m est égal à 1 ou 2 et 1 à 3 des groupes mentionnés en référence à $R^3$,
sous réserve que A ne peut représenter un groupe toluène-sulfonyle lorsque n est égal à O et que X représente l'azote, $R^1$ l'hydrogène, $R^4$ un groupe amino ($NH_2$) et $R^3$ l'hydrogène ou un groupe méthyle,
et leurs sels acceptables pour des usages agricoles.

**2.** Procédé de préparation des composés de la revendication 1, caractérisé en ce que l'on fait réagir un sulfonamide correspondant II

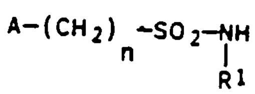

II

de manière connue en soi, dans un solvant organique inerte, en présence d'une base, avec un halogénure d'hétéroaryle III

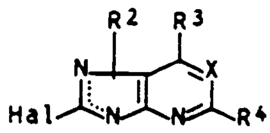

III

Hal représentant un atome d'halogène.

**3.** Procédé de préparation des composés I de la revendication 1 pour lesquels $R^2$ a une signification autre que l'hydrogéne, caractérisé en ce que l'on fait réagir un halogènure de sulfonyle correspondant de formule IV

$A-(CH_2)_n-SO_2-Hal$ IV

dans laquelle Hal a les significations indiquées dans la revendication 2 de manière connue en soi, dans un solvant orgnique inerte, en présence d'une base, avec une hétéroarylamine V

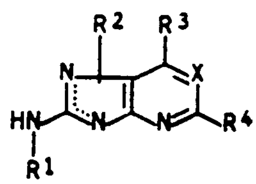

V

**4.** Procédé de préparation des composés I de la revendication 1 pour lesquels $R^1$ représente l'hydrogène, caractérisé en ce que l'on fait réagir un composé correspondant VI

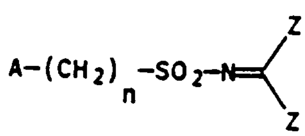

VI

pour lequel Z représente un atome d'halogène ou un groupe alkylthio ou benzylthio, de manière connue en soi, dans un solvant organique inerte, avec une dérivé diaminohétéroarylique VIIa ou VIIb respectivement

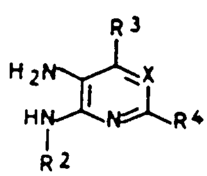

VIIa

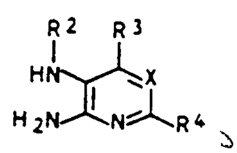

VIIb

**5.** Procédé de préparation des composés I de la revendication 4, caractérisé en ce que l'on fait réagir un dérivé correspondant VIa

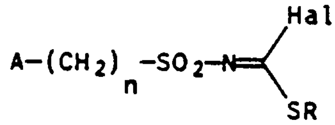

VIa

pour lequel Hal a les significations indiquées dans la revendication 2 et R représente un groupe alkyle ou benzyle, de manière connue en soi, dans un solvant organique inerte, avec un dérivé diaminohétéroarylique VIIa ou VIIb respectivement selon la revendication 4, ce qui donne une isothiourée de formule respective VIIIa ou VIIIb

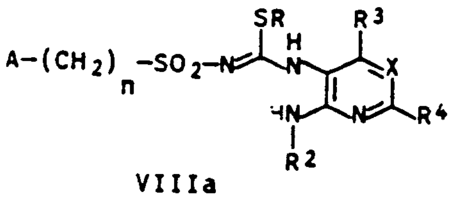

VIIIa

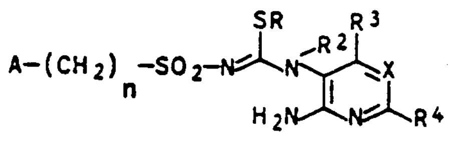

VIIIb

qu'on cyclise ensuite en présence d'une base et d'un sel d'argent dans un solvant organique inerte.

6. Procédé de préparation des composés I de la revendication 1 pour lesquels $R^1$ et $R^2$ ont des significations autres que l'hydrogène, caractérisé en ce que l'on fait réagir un dérivé correspondant de formule IX

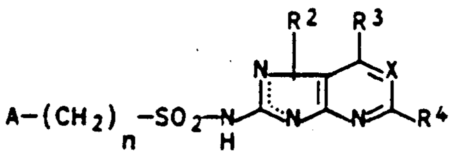

IX

dans laquelle $R^2$ a une signification autre que l'hydrogène, de manière connue en soi, dans un solvant organique inerte et en présence d'une base, avec un réactif alkylant de formule X

$R^1$-Nu X

pour lequel Nu représente un groupe éliminable nucléophile et $R^1$ a une signification autre que l'hydrogène.

7. Procédé de préparation des composés I de la revendication 1 pour lesquels $R^3$ ou $R^4$ représente un groupe nucléophile et X un atome d'azote, caractérisé en ce que l'on fait réagir un dérivé correspondant de formule respective XIa ou XIb

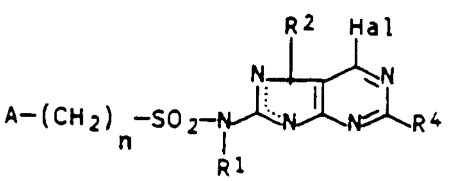

XIa

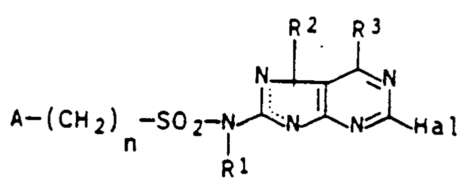

XIb

dans lesquelles Hal a les significations indiquées dans la revendication 2, de manière connue en soi, en présence d'une base, dans un solvant organique inerte, avec le dérivé correspondant de formule respective XIIa ou XIIb

$R^3$-H XIIa $R^4$-H XIIb

**8.** Procédé de préparation des composés I de la revendication 1 pour lesquels $R^3$ et $R^4$ représentent le même groupe nucléophile et X représente l'azote, caractérisé en ce que l'on fait réagir un dérivé correspondant de formule XIc

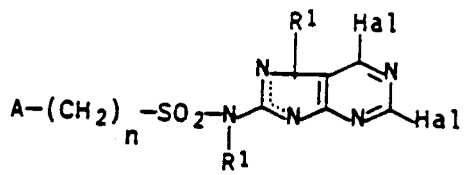

XIc

dans laquelle Hal a les significations indiquées dans la revendication 2, de manière connue en soi, en présence d'une base, dans un solvant organique inerte, avec le dérivé correspondant XIIa ou XIIb selon la revendication 7.

**9.** Produit herbicide contenant un sulfonamide de formule générale I de la revendication 1 ou l'un de ses sels acceptables pour des usages agricoles avec des produits auxiliaires usuels de formulation.

**10.** Procédé pour combattre les croissances de végétaux indésirables, caractérisé en ce que l'on fait agir sur les végétaux et/ou leur habitat un sulfonamide de formule I

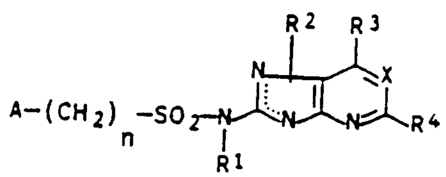

I

dans laquelle les symboles et les indices ont les significations suivantes :
$R^1$ représente l'hydrogène ou un groupe alkyle en C1-C4,
$R^2$ représente l'hydrogène,
un groupe alkyle en C1-C6 qui peut être substitué par 1 à 5 atomes d'halogènes et/ou un des groupes suivants : alcoxy en C1-C4, halogénoalcoxy en C1-C4, alkylthio en C1-C4, halogénoalkylthio en C1-C4, phényle, phénoxy ou phénylthio,
un groupe alcényle en C3-C4; un groupe alcynyle en C3-C4;
un hétérocycle saturé ou mono-insaturé de 5 à 7 chaînons contenant 1 à 2 atomes d'azote, d'oxygène et/ou de soufre et qui peut porter 1 à 3 des substituants suivants : les halogènes, les groupes alkyle en C1-C4, alcoxy en C1-C4, halogénoalkyle en C1-C4, halogènoalcoxy en C1-C4, alkylthio en C1-C4, halogénoalkylthio en C1-C4, et/ou phényle, phénoxy et/ou phénylthio;
$R^3$, $R^4$ représentent des halogènes;
des groupes alcoxy en C1-C4, alkylthio en C1-C4, alcényle en C2-C6, alcényloxy en C2-C6, alcénylthio en C2-C6, alcynyle en C2-C6, alcynyloxy en C2-C6 et/ou alcynylthio en C2-C6, tous ces groupes pouvant être substitués par 1 à 5 atomes d'halogènes et/ou par un des groupes suivants : alcoxy en C1-C4, halogénoalcoxy en C1-C4, alkylthio en C2-C4, halogénoalkylthio en C2-C4, phényle, phénoxy ou phénylthio;
des groupes cycloalkyle en C3-C6, cycloalkylthio en C3-C6, cycloalcényle en C5-C6, cycloalcoxy en C3-C6, cycloalcényloxy en C5-C6, cycloalcénylthio en C5-C6, phényle, phénoxy, phénylthio, benzyle, benzyloxy ou benzylthio, ces groupes cycliques pouvant être substitués par 1 à 5 atomes d'halogènes et/ou par 1 à 3 des groupes suivants : alkyle en C1-C4, halogénoalkyle en C1-C4, alcoxy en C1-C4, halogénoalcoxy en C1-C4, alkylthio en C1-C4, halogénoalkylthio en C1-C4, phényle, phénoxy, phénylthio, benzyle, benzyloxy et/ou benzylthio;
les groupes mentionnés en référence à $R^2$ ou bien

des groupes $NR^7R^8$ dans lesquels
$R^7R^8$ représentent l'hydrogène, des groupes alkyle en C1-C6, alcényle en C3-C6, alcynyle en C3-C6, cycloalkyle en C3-C6, cycloalcényle en C5-C6, phényle et/ou benzyle, les noyaux aromatiques pouvant eux-mêmes être substitués une à cinq fois par halogène et/ou 1 à 3 fois par alkyle en C1-C4, halogénoalkyle en C1-C4, alcoxy en C1-C4 et/ou halogénoalcoxy en C1-C4, ou bien $R^7$ et $R^8$ représentent ensemble un pont alkylène en C4-C6 qui peut être interrompu par un atome d'oxygène, de soufre ou d'azote, ce pont pouvant lui-même porter 1 à 3 groupes alkyle en C1-C4;
X représente un atome d'azote ou un groupe $=CR^5$- dans lequel $R^5$ a l'une des significations indiquées pour $R^3$,
n est égal à 0 ou 1, et
A représente un radical aromatique mono- ou bi-cyclique qui peut contenir 1 à 2 atomes d'azote, d'oxygène et/ou de soufre, ce radical pouvant porter 1 à 5 atomes d'halogènes et/ou 1 à 3 des groupes suivants :
cyano, nitro, thiocyanato,
$COR^6$ dans lequel $R^6$ représente un groupe hydroxy, amino ou a l'une des significations indiquées pour $R^3$,
$SO_mR^6$ dans lequel m est égal à 1 ou 2 et 1 à 3 des groupes mentionnés en référence à $R^3$,
sous réserve que A ne peut représenter un groupe toluène-sulfonyle lorsque n est égal à O et que X représente l'azote, $R^1$ l'hydrogène, $R^4$ un groupe amino ($NH_2$) et $R^3$ l'hydrogène ou un groupe méthyle,
et leurs sels acceptables pour des usages agricoles,
ou ses sels acceptables pour des usages agricoles.

**11.** Produit pour agir sur la croissance des végétaux, contenant un sulfonamide de formule I de la revendication 1 ou l'un de ses sels avec des produits auxiliaires usuels de formulation.

**12.** Procédé pour agir sur la croissance des végétaux, caractérisé en ce que l'on fait agir sur les végétaux et/ou leur habitat un sulfonamide de formule I selon la revendication 10 ou l'un de ses sels.

**Revendications pour l'Etat contractant suivant : ES**

**1.** Produit herbicide contenant des produits auxiliaires usuels de formulation et des sulfonamides de formule générale I

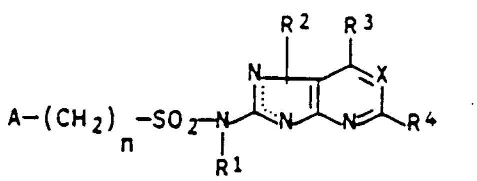

I

dans laquelle les symboles et indices ont les significations suivantes :
$R^1$ représente l'hydrogène ou un groupe alkyle en C1-C4,
$R^2$ représente l'hydrogène,
un groupe alkyle en C1-C6 qui peut être substitué par 1 à 5 atomes d'halogènes et/ou un des groupes suivants : alcoxy en C1-C4, halogénoalcoxy en C1-C4, alkylthio en C1-C4, halogénoalkylthio en C1-C4, phényle, phénoxy ou phénylthio,
un groupe alcényle en C3-C4; un groupe alcynyle en C3-C4;
un hétérocycle saturé ou mono-insaturé de 5 à 7 chaînons contenant 1 à 2 atomes d'azote, d'oxygène et/ou de soufre et qui peut porter 1 à 3 des substituants suivants : les halogènes, les groupes alkyle en C1-C4, alcoxy en C1-C4, halogénoalkyle en C1-C4, halogènoalcoxy en C1-C4, alkylthio en C1-C4, halogénoalkylthio en C1-C4, et/ou phényle, phénoxy et/ou phénylthio;
$R^3$, $R^4$ représentent des halogènes;
des groupes alcoxy en C1-C4, alkylthio en C1-C4, alcényle en C2-C6, alcényloxy en C2-C6, alcénylthio en C2-C6, alcynyle en C2-C6, alcynyloxy en C2-C6 et/ou alcynylthio en C2-C6, tous ces groupes pouvant être substitués par 1 à 5 atomes d'halogènes et/ou par un des groupes suivants : alcoxy en C1-C4, halogénoalcoxy en C1-C4, alkylthio en C2-C4, halogénoalkylthio en C2-C4, phényle, phénoxy ou phénylthio;
des groupes cycloalkyle en C3-C6, cycloalkylthio en C3-C6, cycloalcényle en C5-C6, cycloalcoxy en

C3-C6, cycloalcényloxy en C5-C6, cycloalcénylthio en C5-C6, phényle, phénoxy, phénylthio, benzyle, benzyloxy ou benzylthio, ces groupes cycliques pouvant être substitués par 1 à 5 atomes d'halogènes et/ou par 1 à 3 des groupes suivants : alkyle en C1-C4, halogénoalkyle en C1-C4, alcoxy en C1-C4, halogénoalcoxy en C1-C4, alkylthio en C1-C4, halogénoalkylthio en C1-C4, phényle, phénoxy, phénylthio, benzyle, benzyloxy et/ou benzylthio;

les groupes mentionnés en référence à $R^2$ ou bien

des groupes $NR^7R^8$ dans lesquels

$R^7R^8$ représentent l'hydrogène, des groupes alkyle en C1-C6, alcényle en C3-C6, alcynyle en C3-C6, cycloalkyle en C3-C6, cycloalcényle en C5-C6, phényle et/ou benzyle, les noyaux aromatiques pouvant eux-mêmes être substitués une à cinq fois par halogène et/ou 1 à 3 fois par alkyle en C1-C4, halogénoalkyle en C1-C4, alcoxy en C1-C4 et/ou halogénoalcoxy en C1-C4, ou bien $R^7$ et $R^8$ représentent ensemble un pont alkylène en C4-C6 qui peut être interrompu par un atome d'oxygène, de soufre ou d'azote, ce pont pouvant lui-même porter 1 à 3 groupes alkyle en C1-C4;

X représente un atome d'azote ou un groupe $=CR^5$- dans lequel $R^5$ a l'une des significations indiquées pour $R^3$,

n est égal à 0 ou 1, et

A représente un radical aromatique mono- ou bi-cyclique qui peut contenir 1 à 2 atomes d'azote, d'oxygène et/ou de soufre, ce radical pouvant porter 1 à 5 atomes d'halogènes et/ou 1 à 3 des groupes suivants :

cyano, nitro, thiocyanato,

$COR^6$ dans lequel $R^6$ représente un groupe hydroxy, amino ou a l'une des significations indiquées pour $R^3$,

$SO_mR^6$ dans lequel m est égal à 1 ou 2 et 1 à 3 des groupes mentionnés en référence à $R^3$,

sous réserve que A ne peut représenter un groupe toluène-sulfonyle lorsque n est égal à O et que X représente l'azote, $R^1$ l'hydrogène, $R^4$ un groupe amino ($NH_2$) et $R^3$ l'hydrogène ou un groupe méthyle, et leurs sels acceptables pour des usages agricoles,

ou leurs sels acceptables pour des usages agricoles.

2. Procédé de préparation des composés de la revendication 1, caractérisé en ce que l'on fait réagir un sulfonamide correspondant II

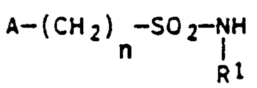

II

de manière connue en soi, dans un solvant organique inerte, en présence d'une base, avec un halogénure d'hétéroaryle III

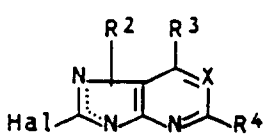

III

Hal représentant un atome d'halogène.

3. Procédé de préparation des composés I de la revendication 1 pour lesquels $R^2$ a une signification autre que l'hydrogène, caractérisé en ce que l'on fait réagir un halogènure de sulfonyle correspondant de formule IV

$A-(CH_2)_n-SO_2-Hal$ IV

dans laquelle Hal a les significations indiquées dans la revendication 2 de manière connue en soi, dans un solvant orgnique inerte, en présence d'une base, avec une hétéroarylamine V

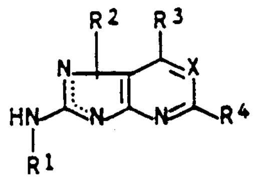

V

4. Procédé de préparation des composés I de la revendication 1 pour lesquels $R^1$ représente l'hydrogène, caractérisé en ce que l'on fait réagir un composé correspondant VI

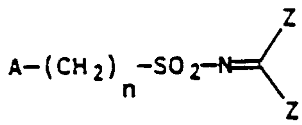

VI

pour lequel Z représente un atome d'halogène ou un groupe alkylthio ou benzylthio, de manière connue en soi, dans un solvant organique inerte, avec une dérivé diaminohétéroarylique VIIa ou VIIb respectivement

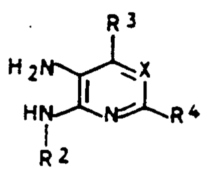

VIIa

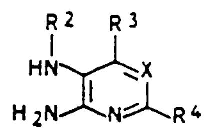

VIIb

5. Procédé de préparation des composés I de la revendication 4, caractérisé en ce que l'on fait réagir un dérivé correspondant VIa

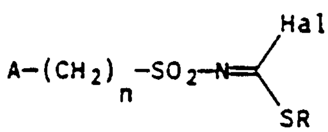

VIa

pour lequel Hal a les significations indiquées dans la revendication 2 et R représente un groupe alkyle ou benzyle, de manière connue en soi, dans un solvant organique inerte, avec un dérivé diaminohétéroarylique VIIa ou VIIb respectivement selon la revendication 4, ce qui donne une isothiourée de formule respective VIIIa ou VIIIb

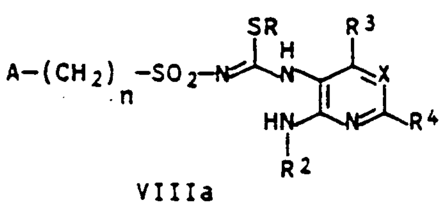

VIIIa

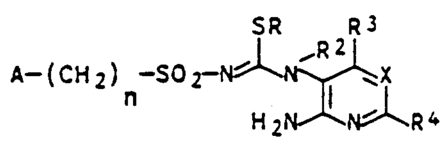

VIIIb

qu'on cyclise ensuite en présence d'une base et d'un sel d'argent dans un solvant organique inerte.

6. Procédé de préparation des composés I de la revendication 1 pour lesquels $R^1$ et $R^2$ ont des significations autres que l'hydrogène, caractérisé en ce que l'on fait réagir un dérivé correspondant de formule IX

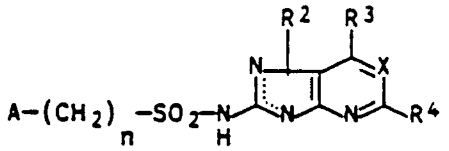

IX

dans laquelle $R^2$ a une signification autre que l'hydrogène, de manière connue en soi, dans un solvant organique inerte et en présence d'une base, avec un réactif alkylant de formule X

$R^1$-Nu X

pour lequel Nu représente un groupe éliminable nucléophile et $R^1$ a une signification autre que l'hydrogène.

**7.** Procédé de préparation des composés I de la revendication 1 pour lesquels $R^3$ ou $R^4$ représente un groupe nucléophile et X un atome d'azote, caractérisé en ce que l'on fait réagir un dérivé correspondant de formule respective XIa ou XIb

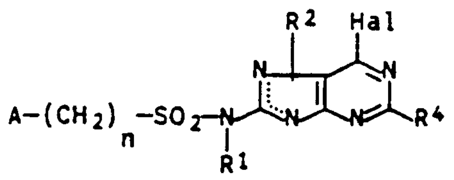

XIa

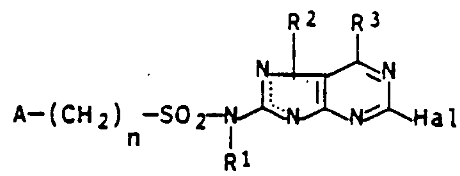

XIb

dans lesquelles Hal a les significations indiquées dans la revendication 2, de manière connue en soi, en présence d'une base, dans un solvant organique inerte, avec le dérivé correspondant de formule respective XIIa ou XIIb

$R^3$-H XIIa $R^4$-H XIIb

**8.** Procédé de préparation des composés I de la revendication 1 pour lesquels $R^3$ et $R^4$ représentent le même groupe nucléophile et X représente l'azote, caractérisé en ce que l'on fait réagir un dérivé correspondant de formule XIc

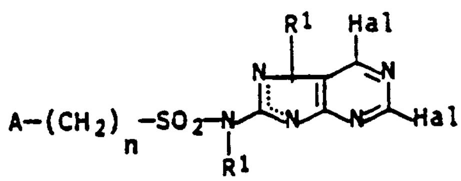

XIc

dans laquelle Hal a les significations indiquées dans la revendication 2, de manière connue en soi, en présence d'une base, dans un solvant organique inerte, avec le dérivé correspondant XIIa ou XIIb selon la revendication 7.

**9.** Procédé pour combattre les croissances de végétaux indésirables, caractérisé en ce que l'on fait agir sur les végétaux et/ou leur habitat un sulfonamide de formule I

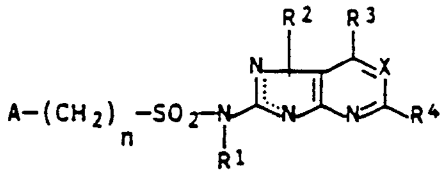

I

dans laquelle les symboles et les indices ont les significations suivantes :

$R^1$ représente l'hydrogène ou un groupe alkyle en C1-C4,

$R^2$ représente l'hydrogène,

un groupe alkyle en C1-C6 qui peut être substitué par 1 à 5 atomes d'halogènes et/ou un des groupes suivants : alcoxy en C1-C4, halogénoalcoxy en C1-C4, alkylthio en C1-C4, halogénoalkylthio en C1-C4, phényle, phénoxy ou phénylthio,

un groupe alcényle en C3-C4; un groupe alcynyle en C3-C4;

un hétérocycle saturé ou mono-insaturé de 5 à 7 chaînons contenant 1 à 2 atomes d'azote, d'oxygène et/ou de soufre et qui peut porter 1 à 3 des substituants suivants : les halogènes, les groupes alkyle en C1-C4, alcoxy en C1-C4, halogénoalkyle en C1-C4, halogènoalcoxy en C1-C4, alkylthio en C1-C4, halogénoalkylthio en C1-C4, et/ou phényle, phénoxy et/ou phénylthio;

$R^3$, $R^4$ représentent des halogènes;

des groupes alcoxy en C1-C4, alkylthio en C1-C4, alcényle en C2-C6, alcényloxy en C2-C6, alcénylthio en C2-C6, alcynyle en C2-C6, alcynyloxy en C2-C6 et/ou alcynylthio en C2-C6, tous ces groupes pouvant être substitués par 1 à 5 atomes d'halogènes et/ou par un des groupes suivants : alcoxy en C1-C4, halogénoalcoxy en C1-C4, alkylthio en C2-C4, halogénoalkylthio en C2-C4, phényle, phénoxy ou phénylthio;

des groupes cycloalkyle en C3-C6, cycloalkylthio en C3-C6, cycloalcényle en C5-C6, cycloalcoxy en C3-C6, cycloalcényloxy en C5-C6, cycloalcénylthio en C5-C6, phényle, phénoxy, phénylthio, benzyle, benzyloxy ou benzylthio, ces groupes cycliques pouvant être substitués par 1 à 5 atomes d'halogènes et/ou par 1 à 3 des groupes suivants : alkyle en C1-C4, halogénoalkyle en C1-C4, alcoxy en C1-C4, halogénoalcoxy en C1-C4, alkylthio en C1-C4, halogénoalkylthio en C1-C4, phényle, phénoxy, phénylthio, benzyle, benzyloxy et/ou benzylthio;

les groupes mentionnés en référence à $R^2$ ou bien

des groupes $NR^7R^8$ dans lesquels

$R^7R^8$ représentent l'hydrogène, des groupes alkyle en C1-C6, alcényle en C3-C6, alcynyle en C3-C6, cycloalkyle en C3-C6, cycloalcényle en C5-C6, phényle et/ou benzyle, les noyaux aromatiques pouvant eux-mêmes être substitués une à cinq fois par halogène et/ou 1 à 3 fois par alkyle en C1-C4, halogénoalkyle en C1-C4, alcoxy en C1-C4 et/ou halogénoalcoxy en C1-C4, ou bien $R^7$ et $R^8$ représentent ensemble un pont alkylène en C4-C6 qui peut être interrompu par un atome d'oxygène, de soufre ou d'azote, ce pont pouvant lui-même porter 1 à 3 groupes alkyle en C1-C4;

X représente un atome d'azote ou un groupe $=CR^5$- dans lequel $R^5$ a l'une des significations indiquées pour $R^3$,

n est égal à 0 ou 1, et

A représente un radical aromatique mono- ou bi-cyclique qui peut contenir 1 à 2 atomes d'azote, d'oxygène et/ou de soufre, ce radical pouvant porter 1 à 5 atomes d'halogènes et/ou 1 à 3 des groupes suivants :

cyano, nitro, thiocyanato,

$COR^6$ dans lequel $R^6$ représente un groupe hydroxy, amino ou a l'une des significations indiquées pour $R^3$,

$SO_mR^6$ dans lequel m est égal à 1 ou 2 et 1 à 3 des groupes mentionnés en référence à $R^3$,

sous réserve que A ne peut représenter un groupe toluène-sulfonyle lorsque n est égal à O et que X représente l'azote, $R^1$ l'hydrogène, $R^4$ un groupe amino ($NH_2$) et $R^3$ l'hydrogène ou un groupe méthyle,

et leurs sels acceptables pour des usages agricoles,

ou ses sels acceptables pour des usages agricoles.

**10.** Procédé pour agir sur le croissance des végétaux, caractérisé en ce que l'on fait agir un sulfonamide de formule I de la revendication 9 ou l'un de ses sels sur les végétaux et/ou leur habitat.